# EUROPEAN PATENT APPLICATION

(11) **EP 4 166 675 A1**
(43) Date of publication of application: **19.04.2023**
(21) Application number: 21828111.1
(22) Date of filing: 25.06.2021
(51) Int. Cl.: C12P 21/02, C12N 15/09, C40B 30/04, C40B 40/10

(54) **TRANSLATION SYSTEM PROVIDED WITH MODIFIED GENETIC CODE TABLE**

(30) Priority: 25.06.2020 JP 2020109576
(71) Applicant: CHUGAI SEIYAKU KABUSHIKI KAISHA, Tokyo 115-8543 (JP)
(72) Inventor: NISHIMURA, Kaori, Kamakura-shi, Kanagawa 247-8530 (JP); TANIGUCHI, Takaaki, Synapse, 138623 (SG); SHINOHARA, Shojiro, Kamakura-shi, Kanagawa 247-8530 (JP); KAGOTANI, Mana, Kamakura-shi, Kanagawa 247-8530 (JP); MISAIZU, Miki, Kamakura-shi, Kanagawa 247-8530 (JP)
(74) Representative: Vossius & Partner Patentanwälte Rechtsanwälte mbB
(86) International application number: PCT/JP2021/024075
(87) International publication number: WO 2021/261577

(57) **Abstract**

The present inventors used aminoacylated tRNAs prepared by ligating a pCpA-amino acid to a tRNA lacking the 3'-terminal CA sequence using a ligase, and thereby succeeded in discrimination between the NNA and NNG codons in specific codon boxes, which had been difficult to achieve due to the presence of wobble base pairing. Furthermore, the inventors assigned another amino acid to the NNU or NNC codon in the same codon box, and performed translation of a sequence containing the NNU, NNA, and NNG codons, or the NNC, NNA, and NNG codons to evaluate the codon discrimination ability. As a result, the codons of interest were specifically translated into only the corresponding amino acids, achieving accurate discrimination successfully. Furthermore, the inventors confirmed that similar effects can also be obtained even when the nucleotide sequence of the tRNA body is altered.

## Description

### [Technical Field]

The present disclosure relates to translation systems that have an altered genetic code table, and methods for their use.

### [Background Art]

Display library is a very useful technology by which molecules binding to a target protein can be obtained efficiently in an evolutionary engineering manner. In order to use a display library to obtain a molecule that exhibits high binding ability to an arbitrary target molecule, or to obtain many molecules each of which respectively bind to different epitopes, panning of a highly diverse library is required. To construct a highly diverse library, the number or variety of building blocks of the library may be increased; however, when there is a limit on the molecular weight from the viewpoint of membrane permeability, the number of building blocks will also be limited. Therefore, the strategy of increasing the variety of building blocks is important for increasing library diversity.

In reconstituted cell-free translation systems such as PURESYSTEM (Non-Patent Literature (NPL) 1), natural codon-amino acid correspondences can be altered because the concentrations of components such as amino acids, tRNAs, and aminoacyl-tRNA synthetases (ARSs) can be adjusted. The use of such translation systems has enabled construction of display libraries into which 20 or more different arbitrary building blocks are introduced. However, in the *Escherichia coli* translation system using three-base codons, only up to 32 different building blocks may be introduced in principle, because of the wobble rule. To give a more specific explanation, there is some "play" in the pairing of the third letter of codons and the first letter of anticodons, and this allows pairing between G and U, called a wobble base pair, in addition to Watson-Crick base pairs. Therefore, the anticodon GNN decodes the NNU and NNC codons, and the anticodon UNN decodes the NNA and NNG codons. Thus, the discrimination between these codons is not possible, limiting the maximum number of different amino acids that can be introduced into one codon box to two (NPL 2).

So far, there have been reports of assigning different amino acids to the NNA and NNG codons in specific codon boxes, (NPL 3, NPL 4, and Patent Literature (PTL) 1), but there have been no reports of successfully expanding the number of building blocks by further assigning another amino acid in the same codon box and simultaneously and accurately discriminating a total of three amino acids. Furthermore, versatility of the selectable amino acids may be low for the already reported methods. Moreover, when an aminoacyl tRNA prepared outside the translation system is used for translation, a higher concentration of aminoacyl tRNA is required than aminoacylated tRNA prepared by ARS in the translation system, in which case, it has been shown mathematically that discrimination between the NNA and NNG codons will become difficult (NPL 5).

In this technical field, assigning various amino acids to a codon table, and translating by accurately discriminating the individual codons determines the quality of the display library. It is very important that the amino acids assigned to the individual codons are specifically translated from the respective codons.

### [Citation List]

### [Patent Literature]

[PTL 1] WO2016/154675

### [Non-Patent Literature]

[NPL 1] Shimizu et al., Nat Biotechnol. 2001; 19(8): 751-755.
[NPL 2] Iwane et al., Nat Chem. 2016; 8(4): 317-325.
[NPL 3] Mukai et al., Nucleic Acids Res. 2015; 43(16): 8111-8122.
[NPL 4] Cui et al., J Am Chem Soc. 2015; 137(13): 4404-4413.
[NPL 5] Frenkel-Morgenstern et al., Mol Syst Biol. 2012; 8: 572.

### [Summary of Invention]

### [Technical Problem]

Various attempts have so far been made for codon expansion; however, there is no report of assigning different amino acids to the NNA and NNG codons and clearly showing accurate discrimination of these codons. Furthermore, there is no report of assigning 3 amino acids within the same codon box and clearly showing their accurate discrimination. The present invention was achieved in view of such circumstances. An objective of the present invention is to provide novel means for enabling discrimination of codons.

### [Solution to Problem]

This time, the present inventors succeeded in discrimination of the NNA and NNG codons in specific codon boxes, which had been difficult to achieve due to the presence of wobble base pairing. Furthermore, the inventors assigned another amino acid to the NNU or NNC codon in the same codon box. When a sequence containing these 3 codons was actually translated to evaluate the discrimination ability, the codons of interest were specifically translated into only the corresponding amino acids, and the successful accurate discrimination was also confirmed numerically.

The present disclosure is based on such findings, and specifically encompasses the embodiments exemplified in [1] to [32] below:
[1] A translation system, comprising a tRNA having an anticodon complementary to a codon represented by M₁M₂A and a tRNA having an anticodon complementary to a codon represented by M₁M₂G;
   wherein M₁ and M₂ represent nucleosides for the first and second letters of the codon respectively, and each of M₁ and M₂ is independently selected from any of adenosine (A), guanosine (G), cytidine (C), and uridine (U); and
   wherein each of the two tRNAs is attached to an amino acid or an amino acid analog that is different from each other.
[2] The translation system of [1], wherein at least two amino acids or amino acid analogs can be translated from a codon box constituted by M₁M₂U, M₁M₂C, M₁M₂A, and M₁M₂G.
[3] A translation system, comprising
   (i) a tRNA having an anticodon complementary to a codon represented by M₁M₂U, a tRNA having an anticodon complementary to a codon represented by M₁M₂A, and a tRNA having an anticodon complementary to a codon represented by M₁M₂G, or
   (ii) a tRNA having an anticodon complementary to a codon represented by M₁M₂C, a tRNA having an anticodon complementary to a codon represented by M₁M₂A, and an anticodon complementary to a codon represented by M₁M₂G;
      wherein M₁ and M₂ represent nucleosides for the first and second letters of the codon respectively, and each of M₁ and M₂ is independently selected from any of adenosine (A), guanosine (G), cytidine (C), and uridine (U); and
      wherein each of the three tRNAs in the above-mentioned (i) and (ii) is attached to an amino acid or an amino acid analog that is different from each other.
[4] The translation system of any one of [1] to [3], wherein at least three amino acids or amino acid analogs can be translated from a codon box constituted by M₁M₂U, M₁M₂C, M₁M₂A, and M₁M₂G.
[5] The translation system of any one of [1] to [4], wherein M₁ and M₂ are any one of the following:
   (i) M₁ is uridine (U) and M₂ is uridine (U);
   (ii) M₁ is uridine (U) and M₂ is adenosine (A);
   (iii) M₁ is uridine (U) and M₂ is guanosine (G);
   (iv) M₁ is cytidine (C) and M₂ is adenosine (A);
   (v) M₁ is cytidine (C) and M₂ is guanosine (G);
   (vi) M₁ is adenosine (A) and M₂ is uridine (U);
   (vii) M₁ is adenosine (A) and M₂ is cytidine (C);
   (viii) M₁ is adenosine (A) and M₂ is adenosine (A);
   (ix) M₁ is adenosine (A) and M₂ is guanosine (G); or
   (x) M₁ is guanosine (G) and M₂ is adenosine (A).
[6] The translation system of [5], wherein M₁ and M₂ are any one of the following:
   (i) M₁ is uridine (U) and M₂ is uridine (U);
   (ii) M₁ is uridine (U) and M₂ is guanosine (G);
   (iii) M₁ is cytidine (C) and M₂ is adenosine (A);
   (iv) M₁ is cytidine (C) and M₂ is guanosine (G);
   (v) M₁ is adenosine (A) and M₂ is adenosine (A);
   (vi) M₁ is adenosine (A) and M₂ is guanosine (G); or
   (vii) M₁ is guanosine (G) and M₂ is adenosine (A).
[7] The translation system of any one of [1] to [6], wherein the concentration of the tRNA included in the translation system per codon is any one of (i) 0.8 µM to 1000 µM, (ii) 1.6 µM to 500 µM, (iii) 3.2 µM to 250 µM, (iv) 6.4 µM to 150 µM, or (v) 10 µM to 100 µM.
[8] The translation system of any one of [1] to [7], wherein the amino acid is a natural amino acid or an unnatural amino acid.
[9] The translation system of any one of [1] to [8], wherein the tRNA is an initiator tRNA or an elongator tRNA.
[10] The translation system of any one of [1] to [9], wherein the tRNA is derived from a prokaryote or a eukaryote.
[11] The translation system of any one of [1] to [10], wherein the anticodon comprises one or more types of nucleosides selected from adenosine (A), guanosine (G), cytidine (C), and uridine (U).
[12] The translation system of any one of [1] to [11], wherein more than 20 types of amino acids or amino acid analogs can be translated from one genetic code table.
[13] The translation system of any one of [1] to [12], which is a cell-free translation system.
[14] The translation system of [13], which is a reconstituted cell-free translation system.
[15] The translation system of [13] or [14], comprising an *Escherichia coli*-derived ribosome.
[16] The translation system of any one of [1] to [15], wherein the tRNA has been attached to the amino acid or the amino acid analog outside the translation system.
[17] The translation system of any one of [1] to [16], wherein among the tRNAs, the tRNA having an anticodon complementary to a codon represented by M₁M₂A and the tRNA having an anticodon complementary to a codon represented by M₁M₂G have been attached to the amino acid or the amino acid analog outside the translation system.
[18] The translation system of any one of [1] to [17], wherein the tRNA is obtained by the pCpA method, the pdCpA method, a method using an artificial RNA catalyst (flexizyme), or a method using an aminoacyl-tRNA synthetase (ARS).
[19] A method for producing the translation system of any one of [1] to [18], comprising attaching the amino acid or the amino acid analog to a tRNA outside the translation system.
[20] The method of [19], wherein attaching the amino acid or the amino acid analog to a tRNA outside the translation system is carried out by the pCpA method, the pdCpA method, a method using an artificial RNA catalyst (flexizyme), or a method using an aminoacyl-tRNA synthetase (ARS).
[21] A method for producing a peptide, comprising translating a nucleic acid using the translation system of any one of [1] to [18] or a translation system obtained by the method of [19] or [20].
[22] The method of [21], wherein the peptide has a cyclic portion.
[23] A peptide produced by the method of [21] or [22].
[24] A method for producing a peptide library, comprising translating a nucleic acid library using the translation system of any one of [1] to [18] or a translation system obtained by the method of [19] or [20].
[25] A peptide library produced by the method of [24].
[26] A method for identifying a peptide having binding activity to a target molecule, comprising contacting the target molecule with the peptide library of [25].
[27] The translation system of any one of [1] to [18], wherein the anticodon does not contain lysidine.
[28] The translation system of any one of [1] to [18] and [27], wherein the anticodon does not contain a lysidine derivative.
[29] The translation system of any one of [1] to [18], [27], and [28], wherein the anticodon does not contain agmatidine.
[30] The translation system of any one of [1] to [18] and [27] to [29], wherein the anticodon does not contain an agmatidine derivative.
[31] The translation system of any one of [1] to [18] and [27] to [30], further comprising a nucleic acid comprising a codon represented by M₁M₂A and a codon represented by M₁M₂G.
[32] The translation system of [31], wherein the nucleic acid comprises (i) a codon represented by M₁M₂U, a codon represented by M₁M₂A, and a codon represented by M₁M₂G, or (ii) a codon represented by M₁M₂C, a codon represented by M₁M₂A, and a codon represented by M₁M₂G.

Furthermore, the present disclosure also encompasses the embodiments exemplified in [101] to [120] below:
[101] A method for producing a peptide, comprising translating a nucleic acid using a translation system,
   wherein the translation system comprises a tRNA having an anticodon complementary to a codon represented by M₁M₂A and a tRNA having an anticodon complementary to a codon represented by M₁M₂G, and
   M₁ and M₂ represent nucleosides for the first and second letters of the codon respectively, and each of M₁ and M₂ is independently selected from any of adenosine (A), guanosine (G), cytidine (C), and uridine (U), and
   wherein each of the two tRNAs is attached to an amino acid or an amino acid analog that is different from each other.
[102] The method of [101], wherein at least two amino acids or amino acid analogs can be translated from a codon box constituted by M₁M₂U, M₁M₂C, M₁M₂A, and M₁M₂G.
[103] A method for producing a peptide, comprising translating a nucleic acid using a translation system, wherein the translation system comprises
   (i) a tRNA having an anticodon complementary to a codon represented by M₁M₂U, a tRNA having an anticodon complementary to a codon represented by M₁M₂A, and a tRNA having an anticodon complementary to a codon represented by M₁M₂G, or
   (ii) a tRNA having an anticodon complementary to a codon represented by M₁M₂C, a tRNA having an anticodon complementary to a codon represented by M₁M₂A, and a tRNA having an anticodon complementary to a codon represented by M₁M₂G;
      wherein M₁ and M₂ represent nucleosides for the first and second letters of the codon respectively, and each of M₁ and M₂ is independently selected from any of adenosine (A), guanosine (G), cytidine (C), and uridine (U); and
      wherein each of the three tRNAs in the above-mentioned (i) and (ii) is attached to an amino acid or an amino acid analog that is different from each other.
[104] The method of any one of [101] to [103], wherein at least three amino acids or amino acid analogs can be translated from a codon box constituted by M₁M₂U, M₁M₂C, M₁M₂A, and M₁M₂G.
[105] The method of any one of [101] to [104], wherein M₁ and M₂ are any one of the following:
   (i) M₁ is uridine (U) and M₂ is uridine (U);
   (ii) M₁ is uridine (U) and M₂ is adenosine (A);
   (iii) M₁ is uridine (U) and M₂ is guanosine (G);
   (iv) M₁ is cytidine (C) and M₂ is adenosine (A);
   (v) M₁ is cytidine (C) and M₂ is guanosine (G);
   (vi) M₁ is adenosine (A) and M₂ is uridine (U);
   (vii) M₁ is adenosine (A) and M₂ is cytidine (C);
   (viii) M₁ is adenosine (A) and M₂ is adenosine (A);
   (ix) M₁ is adenosine (A) and M₂ is guanosine (G); or
   (x) M₁ is guanosine (G) and M₂ is adenosine (A).
[106] The method of [105], wherein M₁ and M₂ are any one of the following:
   (i) M₁ is uridine (U) and M₂ is uridine (U);
   (ii) M₁ is uridine (U) and M₂ is guanosine (G);
   (iii) M₁ is cytidine (C) and M₂ is adenosine (A);
   (iv) M₁ is cytidine (C) and M₂ is guanosine (G);
   (v) M₁ is adenosine (A) and M₂ is adenosine (A);
   (vi) M₁ is adenosine (A) and M₂ is guanosine (G); or
   (vii) M₁ is guanosine (G) and M₂ is adenosine (A).
[107] The method of any one of [101] to [106], wherein the concentration of the tRNA included in the translation system per codon is any one of (i) 0.8 µM to 1000 µM, (ii) 1.6 µM to 500 µM, (iii) 3.2 µM to 250 µM, (iv) 6.4 µM to 150 µM, or (v) 10 µM to 100 µM.
[108] The method of any one of [101] to [107], wherein the amino acid is a natural amino acid or an unnatural amino acid.
[109] The method of any one of [101] to [108], wherein the tRNA is an initiator tRNA or an elongator tRNA.
[110] The method of any one of [101] to [109], wherein the tRNA is derived from a prokaryote or a eukaryote.
[111] The method of any one of [101] to [110], wherein the anticodon comprises one or more types of nucleosides selected from adenosine (A), guanosine (G), cytidine (C), and uridine (U).
[112] The method of any one of [101] to [111], wherein more than 20 types of amino acids can be translated from one genetic code table.
[113] The method of any one of [101] to [112], wherein the translation system is a cell-free translation system.
[114] The method of [113], wherein the translation system is a reconstituted cell-free translation system.
[115] The method of [113] or [114], wherein the translation system comprises an *Escherichia coli*-derived ribosome.
[116] The method of any one of [101] to [115], wherein the tRNA has been attached to the amino acid or the amino acid analog outside the translation system.
[117] The method of any one of [101] to [116], wherein among the tRNAs, the tRNA having an anticodon complementary to a codon represented by M₁M₂A and the tRNA having an anticodon complementary to a codon represented by M₁M₂G have been attached to the amino acid or the amino acid analog outside the translation system.
[118] The method of any one of [101] to [117], wherein the tRNA is obtained by the pCpA method, the pdCpA method, a method using an artificial RNA catalyst (flexizyme), or a method using an aminoacyl-tRNA synthetase (ARS).
[119] The method of any one of [101] to [118], wherein the nucleic acid comprises a codon represented by M₁M₂A and a codon represented by M₁M₂G.
[120] The method of any one of [103] to [118], wherein the nucleic acid comprises (i) a codon represented by M₁M₂U, a codon represented by M₁M₂A, and a codon represented by M₁M₂G, or (ii) a codon represented by M₁M₂C, a codon represented by M₁M₂A, and a codon represented by M₁M₂G.

Furthermore, the present disclosure also encompasses the embodiments exemplified in [201] to [218] below:
[201] A kit or a composition for producing a peptide,
   which comprises a tRNA having an anticodon complementary to a codon represented by M₁M₂A and a tRNA having an anticodon complementary to a codon represented by M₁M₂G; wherein M₁ and M₂ represent nucleosides for the first and second letters of the codon respectively, and each of M₁ and M₂ is independently selected from any of adenosine (A), guanosine (G), cytidine (C), and uridine (U); and
   wherein each of the two tRNAs is attached to an amino acid or an amino acid analog that is different from each other.
[202] The method of [201], wherein at least two amino acids or amino acid analogs can be translated from a codon box constituted by M₁M₂U, M₁M₂C, M₁M₂A, and M₁M₂G.
[203] A kit or a composition for producing a peptide, comprising
   (i) a tRNA having an anticodon complementary to a codon represented by M₁M₂U, a tRNA having an anticodon complementary to a codon represented by M₁M₂A, and a tRNA having an anticodon complementary to a codon represented by M₁M₂G, or
   (ii) a tRNA having an anticodon complementary to a codon represented by M₁M₂C, a tRNA having an anticodon complementary to a codon represented by M₁M₂A, and an anticodon complementary to a codon represented by M₁M₂G;
      wherein M₁ and M₂ represent nucleosides for the first and second letters of the codon respectively, and each of M₁ and M₂ is independently selected from any of adenosine (A), guanosine (G), cytidine (C), and uridine (U); and
      wherein each of the three tRNAs in the above-mentioned (i) and (ii) is attached to an amino acid or an amino acid analog that is different from each other.
[204] The kit or composition of any one of [201] to [203], wherein at least three amino acids or amino acid analogs can be translated from a codon box constituted by M₁M₂U, M₁M₂C, M₁M₂A, and M₁M₂G;
[205] The kit or composition of any one of [201] to [204], wherein M₁ and M₂ are any one of the following:
   (i) M₁ is uridine (U) and M₂ is uridine (U);
   (ii) M₁ is uridine (U) and M₂ is adenosine (A);
   (iii) M₁ is uridine (U) and M₂ is guanosine (G);
   (iv) M₁ is cytidine (C) and M₂ is adenosine (A);
   (v) M₁ is cytidine (C) and M₂ is guanosine (G);
   (vi) M₁ is adenosine (A) and M₂ is uridine (U);
   (vii) M₁ is adenosine (A) and M₂ is cytidine (C);
   (viii) M₁ is adenosine (A) and M₂ is adenosine (A);
   (ix) M₁ is adenosine (A) and M₂ is guanosine (G); or
   (x) M₁ is guanosine (G) and M₂ is adenosine (A).
[206] The kit or composition of [205], wherein M₁ and M₂ are any one of the following:
   (i) M₁ is uridine (U) and M₂ is uridine (U);
   (ii) M₁ is uridine (U) and M₂ is guanosine (G);
   (iii) M₁ is cytidine (C) and M₂ is adenosine (A);
   (iv) M₁ is cytidine (C) and M₂ is guanosine (G);
   (v) M₁ is adenosine (A) and M₂ is adenosine (A);
   (vi) M₁ is adenosine (A) and M₂ is guanosine (G); or
   (vii) M₁ is guanosine (G) and M₂ is adenosine (A).
[207] The kit or composition of any one of [201] to [206], wherein the concentration of the tRNA per codon is any one of (i) 0.8 µM to 1000 µM, (ii) 1.6 µM to 500 µM, (iii) 3.2 µM to 250 µM, (iv) 6.4 µM to 150 µM, or (v) 10 µM to 100 µM.
[208] The kit or composition of any one of [201] to [207], wherein the amino acid is a natural amino acid or an unnatural amino acid.
[209] The kit or composition of any one of [201] to [208], wherein the tRNA is an initiator tRNA or an elongator tRNA.
[210] The kit or composition of any one of [201] to [209], wherein the tRNA is derived from a prokaryote or a eukaryote.
[211] The kit or composition of any one of [201] to [210], wherein the anticodon comprises one or more types of nucleotides selected from adenosine (A), guanosine (G), cytidine (C), and uridine (U).
[212] The kit or composition of any one of [201] to [211], wherein more than 20 types of amino acids can be translated from one genetic code table.
[213] The kit or composition of any one of [201] to [212], wherein the translation system is a cell-free translation system.
[214] The kit or composition of [213], wherein the translation system is a reconstituted cell-free translation system.
[215] The kit or composition of [213] or [214], wherein the translation system comprises an *Escherichia coli*-derived ribosome.
[216] The kit or composition of any one of [201] to [215], wherein the tRNA has been attached to the amino acid or the amino acid analog outside the translation system.
[217] The kit or composition of any one of [201] to [216], wherein among the tRNAs, the tRNA having an anticodon complementary to a codon represented by M₁M₂A and the tRNA having an anticodon complementary to a codon represented by M₁M₂G have been attached to the amino acid or the amino acid analog outside the translation system.
[218] The kit or composition of any one of [201] to [217], wherein the tRNA is obtained by the pCpA method, the pdCpA method, a method using an artificial RNA catalyst (flexizyme), or a method using an aminoacyl-tRNA synthetase (ARS).

### [Brief Description of Drawings]

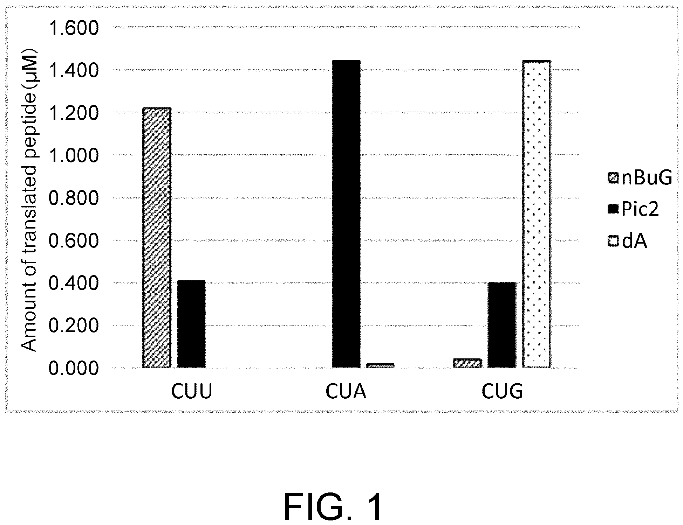

Fig. 1 is a graph showing the results of evaluating translation in terms of discrimination of three amino acids in a single codon box, as described in Examples 6 to 7. The codons evaluated are CUU, CUA, and CUG. The vertical axis of the graph shows the amount of translated peptide obtained when the translation was performed using each combination of the tRNAs and the mRNAs described below (see Table 6 for specific measurement values).

| | |
|---|---|
| tRNA: | Compound AAtR-1 (anticodon: aag; amino acid: nBuG) |
| | Compound AAtR-2 (anticodon: uag; amino acid: Pic2) |
| | Compound AAtR-3 (anticodon: cag; amino acid: dA) |
| mRNA: | mR-1 (containing the CUU codon) |
| | mR-2 (containing the CUA codon) |
| | mR-3 (containing the CUG codon) |

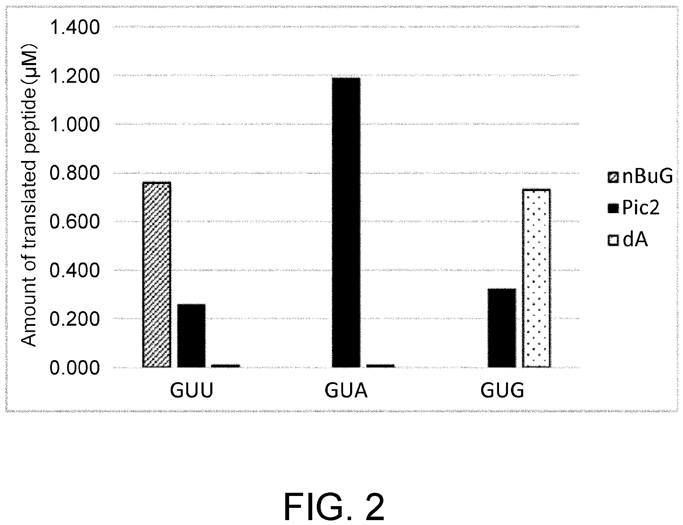

Fig. 2 is a graph showing the results of evaluating translation in terms of discrimination of three amino acids in a single codon box, as described in Examples 6 to 7. The codons evaluated are GUU, GUA, and GUG. The vertical axis of the graph shows the amount of translated peptide obtained when the translation was performed using each combination of the tRNAs and the mRNAs described below (see Table 7 for specific measurement values).

| | |
|---|---|
| tRNA: | Compound AAtR-4 (anticodon: aac; amino acid: nBuG) |
| | Compound AAtR-5 (anticodon: uac; amino acid: Pic2) |
| | Compound AAtR-6 (anticodon: cac; amino acid: dA) |
| mRNA: | mR-4 (containing the GUU codon) |
| | mR-5 (containing the GUA codon) |
| | mR-6 (containing the GUG codon) |

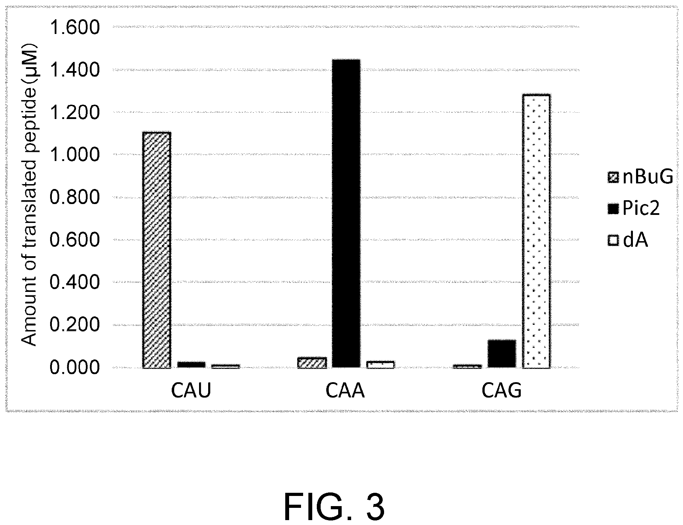

Fig. 3 is a graph showing the results of evaluating translation in terms of discrimination of three amino acids in a single codon box, as described in Examples 6 to 7. The codons evaluated are CAU, CAA, and CAG. The vertical axis of the graph shows the amount of translated peptide obtained when the translation was performed using each combination of the tRNAs and the mRNAs described below (see Table 8 for specific measurement values).

| | |
|---|---|
| tRNA: | Compound AAtR-7 (anticodon: aug; amino acid: nBuG) |
| | Compound AAtR-8 (anticodon: uug; amino acid: Pic2) |
| | Compound AAtR-9 (anticodon: cug; amino acid: dA) |
| mRNA: | mR-7 (containing the CAU codon) |
| | mR-8 (containing the CAA codon) |
| | mR-9 (containing the CAG codon) |

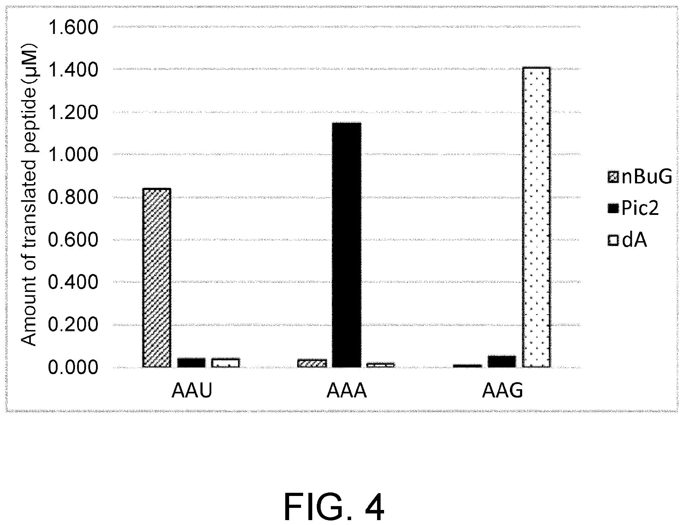

Fig. 4 is a graph showing the results of evaluating translation in terms of discrimination of three amino acids in a single codon box, as described in Examples 6 to 7. The codons evaluated are AAU, AAA, and AAG. The vertical axis of the graph shows the amount of translated peptide obtained when the translation was performed using each combination of the tRNAs and the mRNAs described below (see Table 9 for specific measurement values).

| | |
|---|---|
| tRNA: | Compound AAtR-10 (anticodon: auu; amino acid: nBuG) |
| | Compound AAtR-11 (anticodon: uuu; amino acid: Pic2) |
| | Compound AAtR-12 (anticodon: cuu; amino acid: dA) |
| mRNA: | mR-10 (containing the AAU codon) |
| | mR-11 (containing the AAA codon) |
| | mR-12 (containing the AAG codon) |

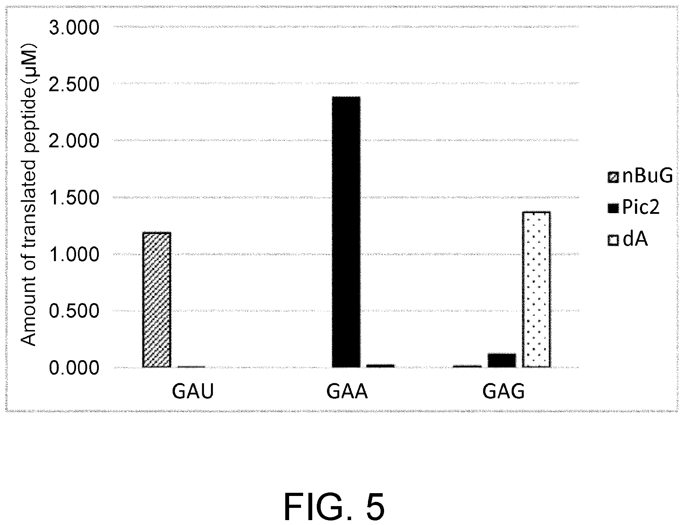

Fig. 5 is a graph showing the results of evaluating translation in terms of discrimination of three amino acids in a single codon box, as described in Examples 6 to 7. The codons evaluated are GAU, GAA, and GAG. The vertical axis of the graph shows the amount of translated peptide obtained when the translation was performed using each combination of the tRNAs and the mRNAs described below (see Table 10 for specific measurement values).

| | |
|---|---|
| tRNA: | Compound AAtR-13 (anticodon: auc; amino acid: nBuG) |
| | Compound AAtR-14 (anticodon: uuc; amino acid: Pic2) |
| | Compound AAtR-15 (anticodon: cuc; amino acid: dA) |
| mRNA: | mR-13 (containing the GAU codon) |
| | mR-14 (containing the GAA codon) |
| | mR-15 (containing the GAG codon) |

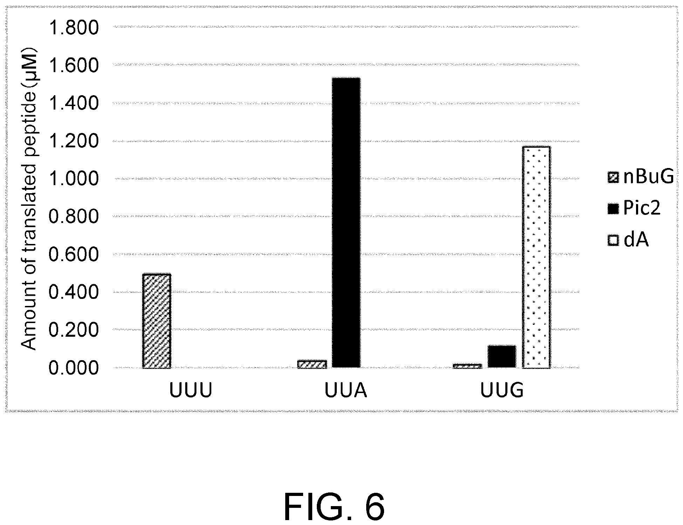

Fig. 6 is a graph showing the results of evaluating translation in terms of discrimination of three amino acids in a single codon box, as described in Examples 6 to 7. The codons evaluated are UUU, UUA, and UUG. The vertical axis of the graph shows the amount of translated peptide obtained when the translation was performed using each combination of the tRNAs and the mRNAs described below (see Table 11 for specific measurement values).

| | |
|---|---|
| tRNA: | Compound AAtR-16 (anticodon: aaa; amino acid: nBuG) |
| | Compound AAtR-17 (anticodon: uaa; amino acid: Pic2) |
| | Compound AAtR-18 (anticodon: caa; amino acid: dA) |
| mRNA: | mR-16 (containing the UUU codon) |
| | mR-17 (containing the UUA codon) |
| | mR-18 (containing the UUG codon) |

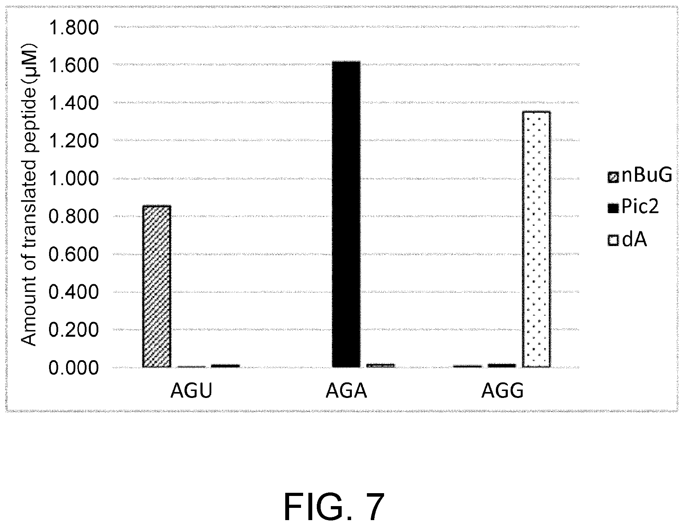

Fig. 7 is a graph showing the results of evaluating translation in terms of discrimination of three amino acids in a single codon box, as described in Examples 6 to 7. The codons evaluated are AGU, AGA, and AGG. The vertical axis of the graph shows the amount of translated peptide obtained when the translation was performed using each combination of the tRNAs and the mRNAs described below (see Table 12 for specific measurement values).

| | |
|---|---|
| tRNA: | Compound AAtR-19 (anticodon: acu; amino acid: nBuG) |
| | Compound AAtR-20 (anticodon: ucu; amino acid: Pic2) |
| | Compound AAtR-21 (anticodon: ccu; amino acid: dA) |
| mRNA: | mR-19 (containing the AGU codon) |
| | mR-20 (containing the AGA codon) |
| | mR-21 (containing the AGG codon) |

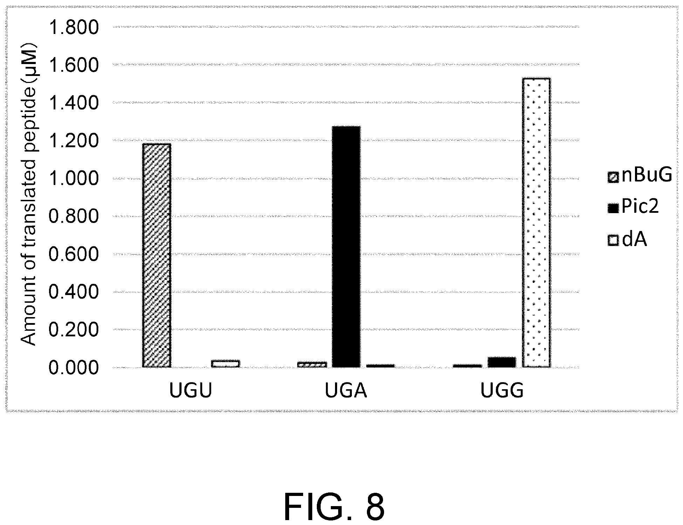

Fig. 8 is a graph showing the results of evaluating translation in terms of discrimination of three amino acids in a single codon box, as described in Examples 6 to 7. The codons evaluated are UGC, UGA, and UGG. The vertical axis of the graph shows the amount of translated peptide obtained when the translation was performed using each combination of the tRNAs and the mRNAs described below (see Table 13 for specific measurement values).

| | |
|---|---|
| tRNA: | Compound AAtR-22 (anticodon: gca; amino acid: nBuG) |
| | Compound AAtR-23 (anticodon: uca; amino acid: Pic2) |
| | Compound AAtR-24 (anticodon: cca; amino acid: dA) |
| mRNA: | mR-22 (containing the UGC codon) |
| | mR-23 (containing the UGA codon) |
| | mR-24 (containing the UGG codon) |

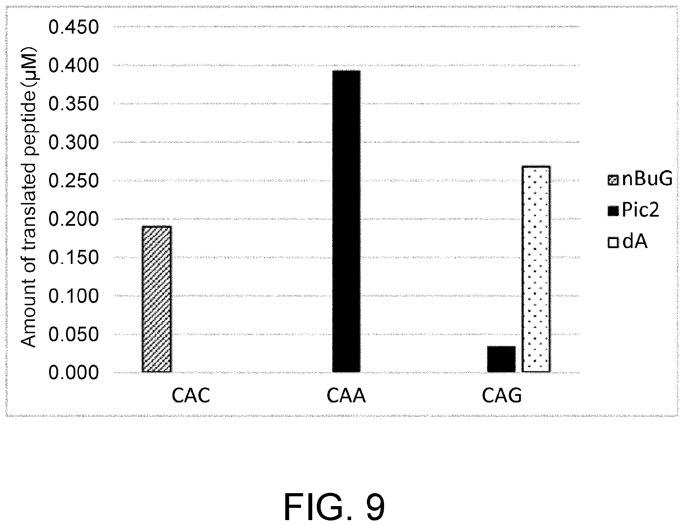

Fig. 9 is a graph showing the results of evaluating translation in terms of discrimination of three amino acids in a single codon box, as described in Examples 6 to 7. The codons evaluated are CAC, CAA, and CAG. The vertical axis of the graph shows the amount of translated peptide obtained when the translation was performed using each combination of the tRNAs and the mRNAs described below (see Table 14 for specific measurement values).

| | |
|---|---|
| tRNA: | Compound AAtR-26 (anticodon: gug; amino acid: nBuG) |
| | Compound AAtR-8 (anticodon: uug; amino acid: Pic2) |
| | Compound AAtR-9 (anticodon: cug; amino acid: dA) |
| mRNA: | mR-25 (containing the CAC codon) |
| | mR-8 (containing the CAA codon) |
| | mR-9 (containing the CAG codon) |

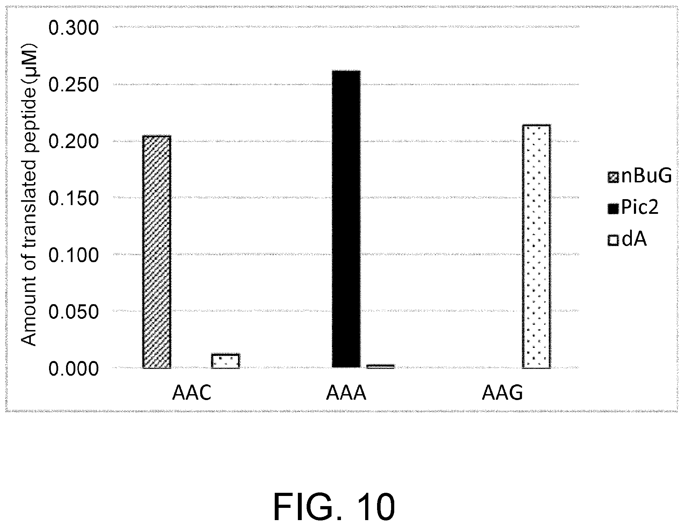

Fig. 10 is a graph showing the results of evaluating translation in terms of discrimination of three amino acids in a single codon box, as described in Examples 6 to 7. The codons evaluated are AAC, AAA, and AAG. The vertical axis of the graph shows the amount of translated peptide obtained when the translation was performed using each combination of the tRNAs and the mRNAs described below (see Table 15 for specific measurement values).

| | |
|---|---|
| tRNA: | Compound AAtR-27 (anticodon: guu; amino acid: nBuG) |
| | Compound AAtR-11 (anticodon: uuu; amino acid: Pic2) |
| | Compound AAtR-12 (anticodon: cuu; amino acid: dA) |
| mRNA: | mR-26 (containing the AAC codon) |
| | mR-11 (containing the AAA codon) |
| | mR-12 (containing the AAG codon) |

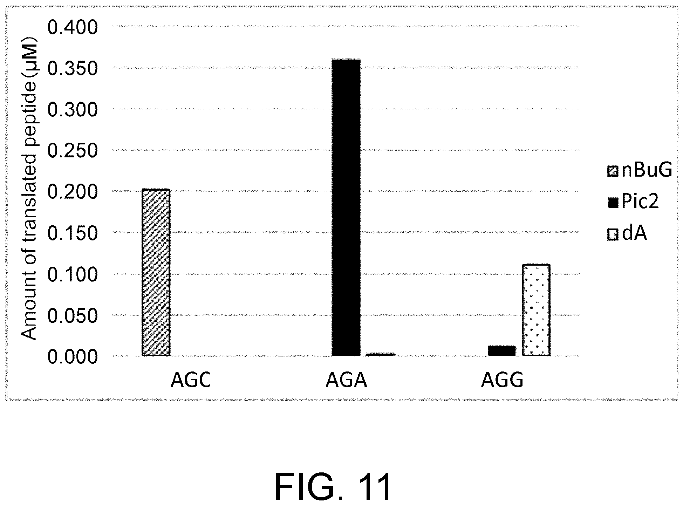

Fig. 11 is a graph showing the results of evaluating translation in terms of discrimination of three amino acids in a single codon box, as described in Examples 6 to 7. The codons evaluated are AGC, AGA, and AGG. The vertical axis of the graph shows the amount of translated peptide obtained when the translation was performed using each combination of the tRNAs and the mRNAs described below (see Table 16 for specific measurement values).

| | |
|---|---|
| tRNA: | Compound AAtR-28 (anticodon: gcu; amino acid: nBuG) |
| | Compound AAtR-20 (anticodon: ucu; amino acid: Pic2) |
| | Compound AAtR-21 (anticodon: ccu; amino acid: dA) |
| mRNA: | mR-29 (containing the AGC codon) |
| | mR-20 (containing the AGA codon) |
| | mR-21 (containing the AGG codon) |

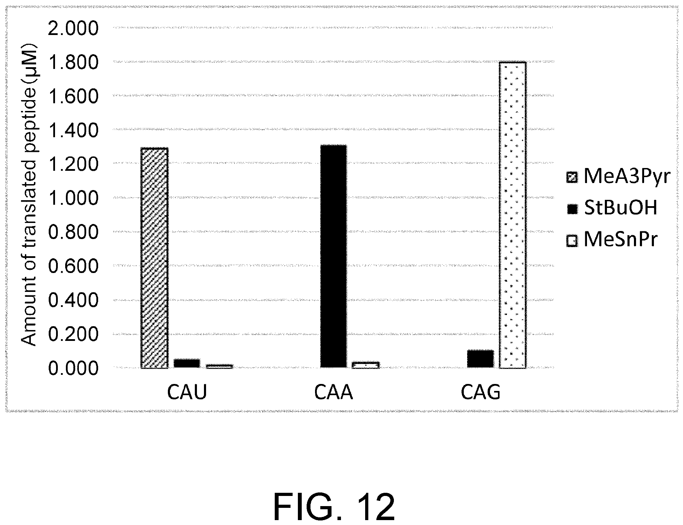

Fig. 12 is a graph showing the results of evaluating translation that discriminates three amino acids in a single codon box, as described in Examples 6 to 7. The codons evaluated are CAU, CAA, and CAG. The vertical axis of the graph shows the amount of translated peptide obtained when the translation was performed using each combination of the tRNAs and the mRNAs described below (see Table 17 for specific measurement values).

| | |
|---|---|
| tRNA: | Compound AAtR-29 (anticodon: aug; amino acid: MeA3Pyr) |
| | Compound AAtR-30 (anticodon: uug; amino acid: StBuOH) |
| | Compound AAtR-31 (anticodon: cug; amino acid: MeSnPr) |
| mRNA: | mR-7 (containing the CAU codon) |
| | mR-8 (containing the CAA codon) |
| | mR-9 (containing the CAG codon) |

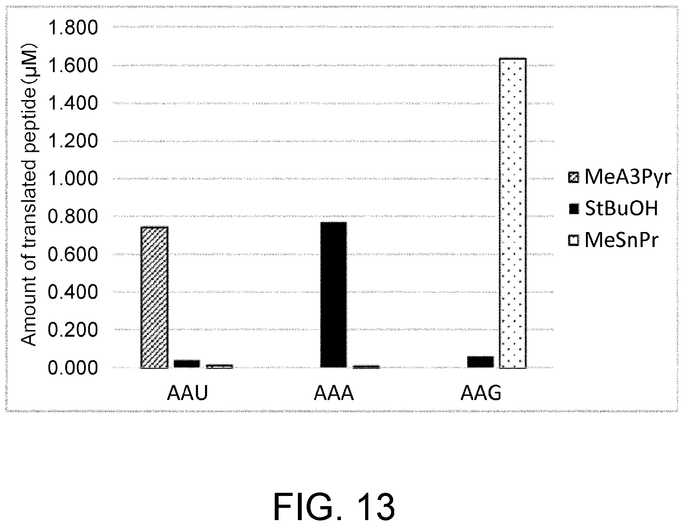

Fig. 13 is a graph showing the results of evaluating translation in terms of discrimination of three amino acids in a single codon box, as described in Examples 6 to 7. The codons evaluated are AAU, AAA, and AAG. The vertical axis of the graph shows the amount of translated peptide obtained when the translation was performed using each combination of the tRNAs and the mRNAs described below (see Table 18 for specific measurement values).

| | |
|---|---|
| tRNA: | Compound AAtR-32 (anticodon: auu; amino acid: MeA3Pyr) |
| | Compound AAtR-33 (anticodon: uuu; amino acid: StBuOH) |
| | Compound AAtR-34 (anticodon: cuu; amino acid: MeSnPr) |
| mRNA: | mR-10 (containing the AAU codon) |
| | mR-11 (containing the AAA codon) |
| | mR-12 (containing the AAG codon) |

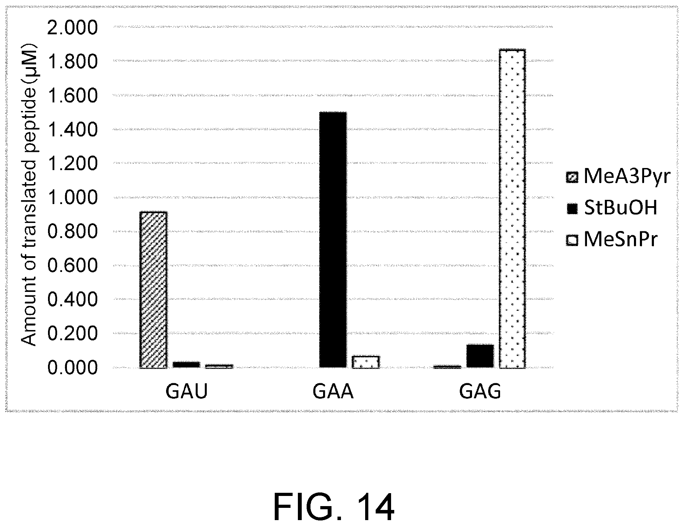

Fig. 14 is a graph showing the results of evaluating translation in terms of discrimination of three amino acids in a single codon box, as described in Examples 6 to 7. The codons evaluated are GAU, GAA, and GAG. The vertical axis of the graph shows the amount of translated peptide obtained when the translation was performed using each combination of the tRNAs and the mRNAs described below (see Table 19 for specific measurement values).

| | |
|---|---|
| tRNA: | Compound AAtR-35 (anticodon: auc; amino acid: MeA3Pyr) |
| | Compound AAtR-36 (anticodon: uuc; amino acid: StBuOH) |
| | Compound AAtR-37 (anticodon: cuc; amino acid: MeSnPr) |
| mRNA: | mR-13 (containing the GAU codon) |
| | mR-14 (containing the GAA codon) |
| | mR-15 (containing the GAG codon) |

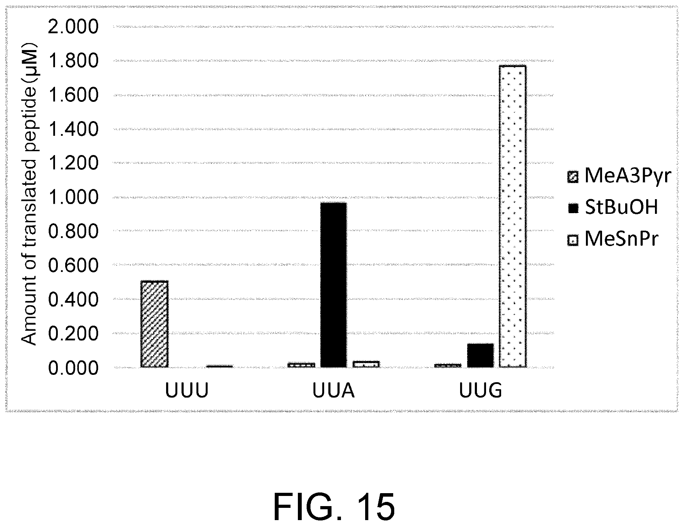

Fig. 15 is a graph showing the results of evaluating translation in terms of discrimination of three amino acids in a single codon box, as described in Examples 6 to 7. The codons evaluated are UUU, UUA, and UUG. The vertical axis of the graph shows the amount of translated peptide obtained when the translation was performed using each combination of the tRNAs and the mRNAs described below (see Table 20 for specific measurement values).

| | |
|---|---|
| tRNA: | Compound AAtR-38 (anticodon: aaa; amino acid: MeA3Pyr) |
| | Compound AAtR-39 (anticodon: uaa; amino acid: StBuOH) |
| | Compound AAtR-40 (anticodon: caa; amino acid: MeSnPr) |
| mRNA: | mR-16 (containing the UUU codon) |
| | mR-17 (containing the UUA codon) |
| | mR-18 (containing the UUG codon) |

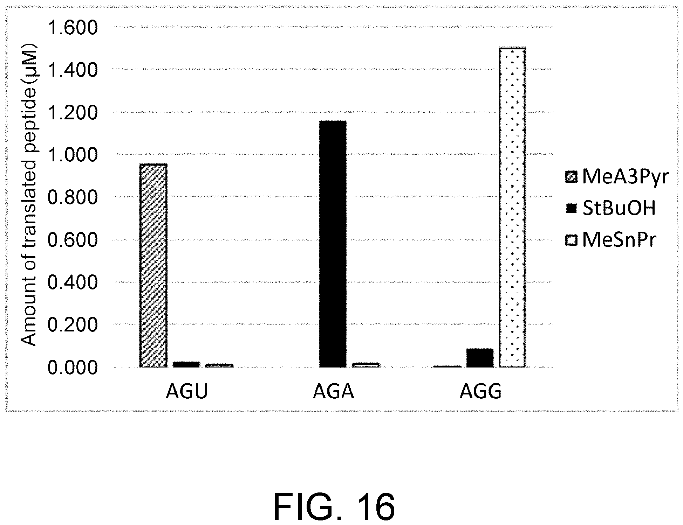

Fig. 16 is a graph showing the results of evaluating translation that discriminates three amino acids in a single codon box, as described in Examples 6 to 7. The codons evaluated are AGU, AGA, and AGG. The vertical axis of the graph shows the amount of translated peptide obtained when the translation was performed using each combination of the tRNAs and the mRNAs described below (see Table 21 for specific measurement values).

| | |
|---|---|
| tRNA: | Compound AAtR-41 (anticodon: acu; amino acid: MeA3Pyr) |
| | Compound AAtR-42 (anticodon: ucu; amino acid: StBuOH) |
| | Compound AAtR-43 (anticodon: ccu; amino acid: MeSnPr) |
| mRNA: | mR-19 (containing the AGU codon) |
| | mR-20 (containing the AGA codon) |
| | mR-21 (containing the AGG codon) |

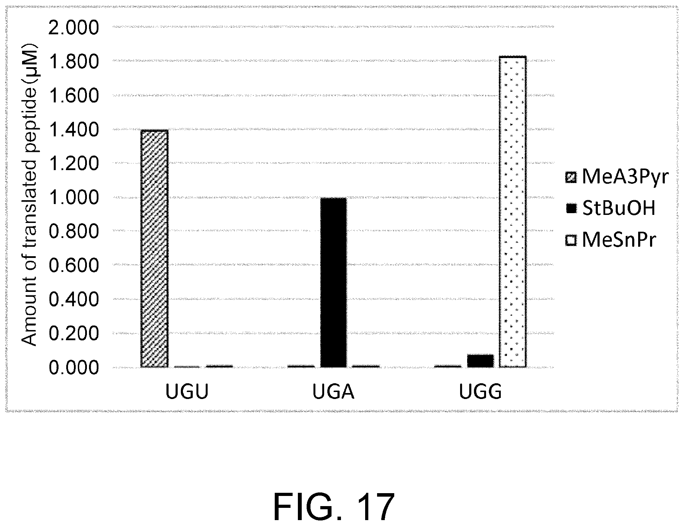

Fig. 17 is a graph showing the results of evaluating translation that discriminates three amino acids in a single codon box, as described in Examples 6 to 7. The codons evaluated are UGC, UGA, and UGG. The vertical axis of the graph shows the amount of translated peptide obtained when the translation was performed using each combination of the tRNAs and the mRNAs described below (see Table 22 for specific measurement values).

| | |
|---|---|
| tRNA: | Compound AAtR-44 (anticodon: gca; amino acid: MeA3Pyr) |
| | Compound AAtR-45 (anticodon: uca; amino acid: StBuOH) |
| | Compound AAtR-46 (anticodon: cca; amino acid: MeSnPr) |
| mRNA: | mR-22 (containing the UGC codon) |
| | mR-23 (containing the UGA codon) |
| | mR-24 (containing the UGG codon) |

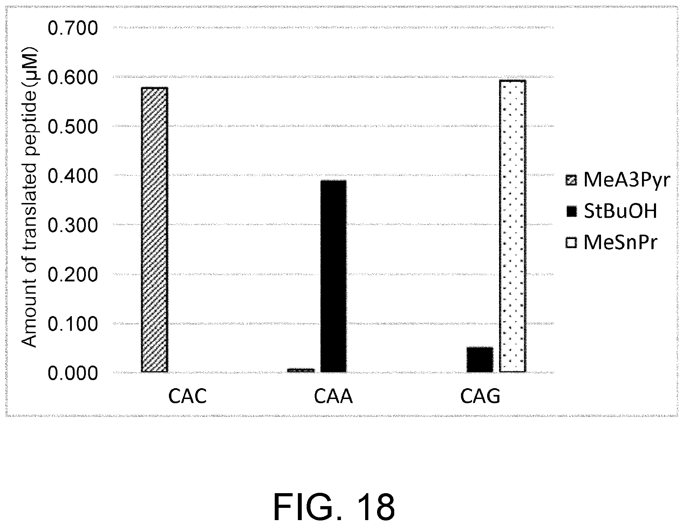

Fig. 18 is a graph showing the results of evaluating translation in terms of discrimination of three amino acids in a single codon box, as described in Examples 6 to 7. The codons evaluated are CAC, CAA, and CAG. The vertical axis of the graph shows the amount of translated peptide obtained when the translation was performed using each combination of the tRNAs and the mRNAs described below (see Table 23 for specific measurement values).

| | |
|---|---|
| tRNA: | Compound AAtR-47 (anticodon: gug; amino acid: MeA3Pyr) |
| | Compound AAtR-30 (anticodon: uug; amino acid: StBuOH) |
| | Compound AAtR-31 (anticodon: cug; amino acid: MeSnPr) |
| mRNA: | mR-25 (containing the CAC codon) |
| | mR-8 (containing the CAA codon) |
| | mR-9 (containing the CAG codon) |

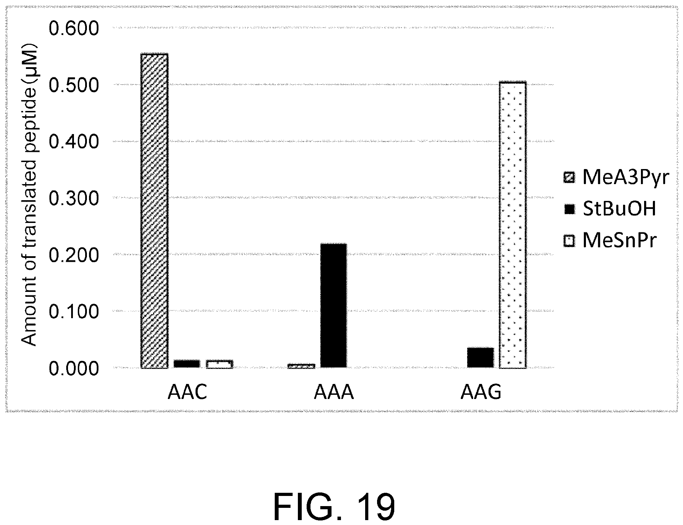

Fig. 19 is a graph showing the results of evaluating translation in terms of discrimination of three amino acids in a single codon box, as described in Examples 6 to 7. The codons evaluated are AAC, AAA, and AAG. The vertical axis of the graph shows the amount of translated peptide obtained when the translation was performed using each combination of the tRNAs and the mRNAs described below (see Table 24 for specific measurement values).

| | |
|---|---|
| tRNA: | Compound AAtR-48 (anticodon: guu; amino acid: MeA3Pyr) |
| | Compound AAtR-33 (anticodon: uuu; amino acid: StBuOH) |
| | Compound AAtR-34 (anticodon: cuu; amino acid: MeSnPr) |
| mRNA: | mR-26 (containing the AAC codon) |
| | mR-11 (containing the AAA codon) |
| | mR-12 (containing the AAG codon) |

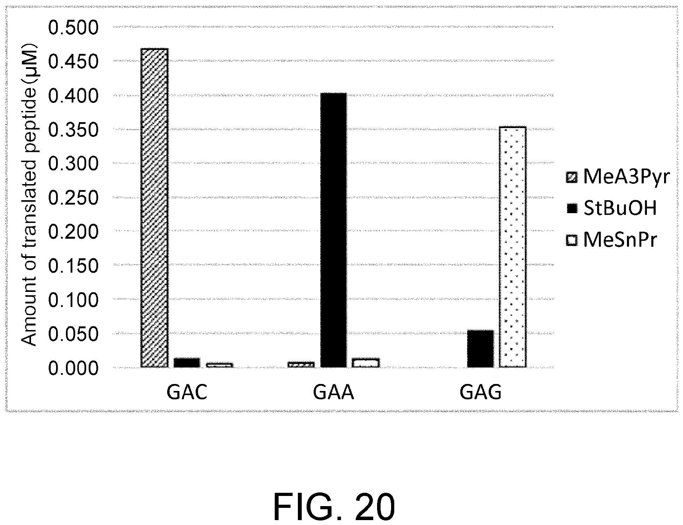

Fig. 20 is a graph showing the results of evaluating translation in terms of discrimination of three amino acids in a single codon box, as described in Examples 6 to 7. The codons evaluated are GAC, GAA, and GAG. The vertical axis of the graph shows the amount of translated peptide obtained when the translation was performed using each combination of the tRNAs and the mRNAs described below (see Table 25 for specific measurement values).

| | |
|---|---|
| tRNA: | Compound AAtR-49 (anticodon: guc; amino acid: MeA3Pyr) |
| | Compound AAtR-36 (anticodon: uuc; amino acid: StBuOH) |
| | Compound AAtR-37 (anticodon: cuc; amino acid: MeSnPr) |
| mRNA: | mR-27 (containing the GAC codon) |
| | mR-14 (containing the GAA codon) |
| | mR-15 (containing the GAG codon) |

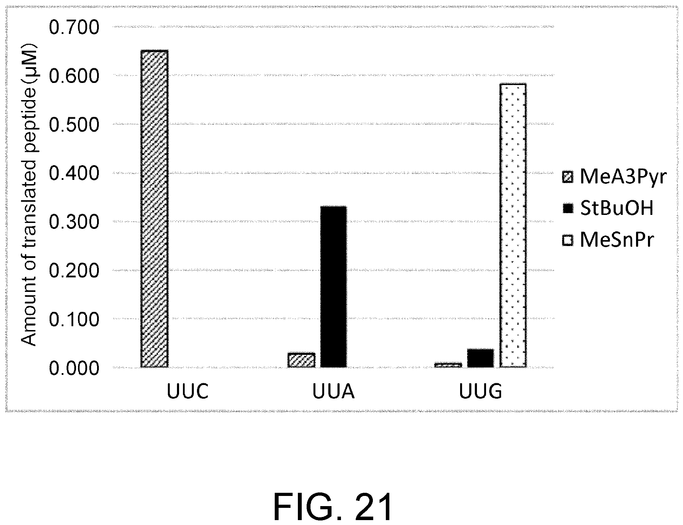

Fig. 21 is a graph showing the results of evaluating translation in terms of discrimination of three amino acids in a single codon box, as described in Examples 6 to 7. The codons evaluated are UUC, UUA, and UUG. The vertical axis of the graph shows the amount of translated peptide obtained when the translation was performed using each combination of the tRNAs and the mRNAs described below (see Table 26 for specific measurement values).

| | |
|---|---|
| tRNA: | Compound AAtR-50 (anticodon: gaa; amino acid: MeA3Pyr) |
| | Compound AAtR-39 (anticodon: uaa; amino acid: StBuOH) |
| | Compound AAtR-40 (anticodon: caa; amino acid: MeSnPr) |
| mRNA: | mR-28 (containing the UUC codon) |
| | mR-17 (containing the UUA codon) |
| | mR-18 (containing the UUG codon) |

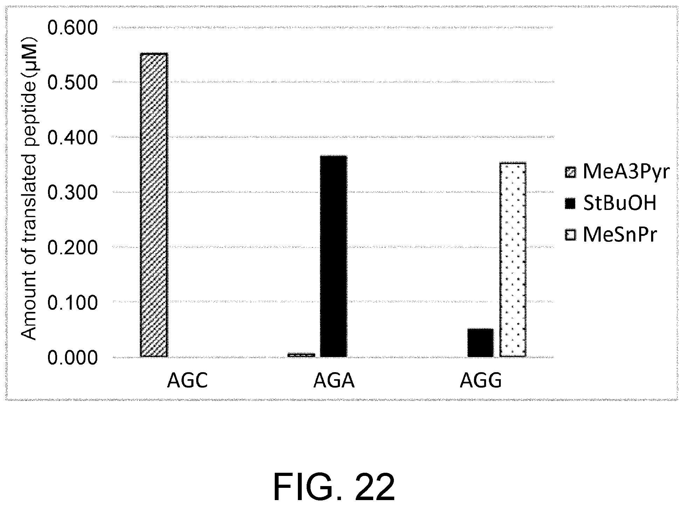

Fig. 22 is a graph showing the results of evaluating translation in terms of discrimination of three amino acids in a single codon box, as described in Examples 6 to 7. The codons evaluated are AGC, AGA, and AGG. The vertical axis of the graph shows the amount of translated peptide obtained when the translation was performed using each combination of the tRNAs and the mRNAs described below (see Table 27 for specific measurement values).

| | |
|---|---|
| tRNA: | Compound AAtR-51 (anticodon: gcu; amino acid: MeA3Pyr) |
| | Compound AAtR-42 (anticodon: ucu; amino acid: StBuOH) |
| | Compound AAtR-43 (anticodon: ccu; amino acid: MeSnPr) |
| mRNA: | mR-29 (containing the AGC codon) |
| | mR-20 (containing the AGA codon) |
| | mR-21 (containing the AGG codon) |

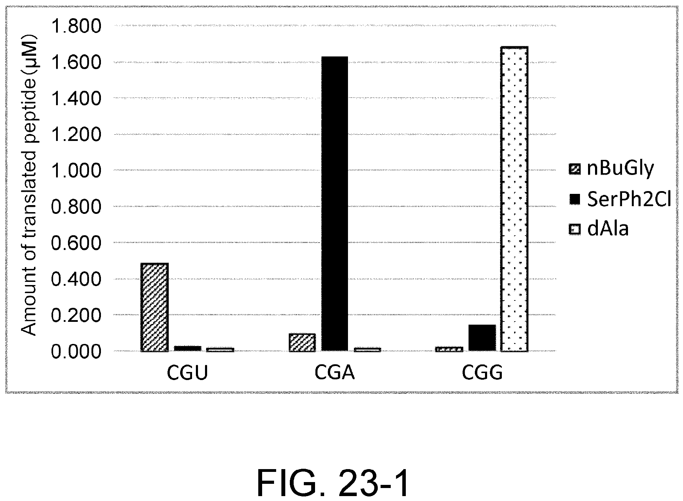

Fig. 23-1 is a graph showing the results of evaluating translation in terms of discrimination of three amino acids in a single codon box, as described in Examples 6 to 7. The codons evaluated are CGC, CGA, and CGG. The vertical axis of the graph shows the amount of translated peptide obtained when the translation was performed using each combination of the tRNAs and the mRNAs described below (see Table 28 for specific measurement values).

| | |
|---|---|
| tRNA: | Compound AAtR-57 (anticodon: gcg; amino acid: nBuG) |
| | Compound AAtR-58 (anticodon: ucg; amino acid: SPh2Cl) |
| | Compound AAtR-59 (anticodon: ccg; amino acid: dA) |
| mRNA: | mR-42 (containing the CGU codon) |
| | mR-43 (containing the CGA codon) |
| | mR-44 (containing the CGG codon) |

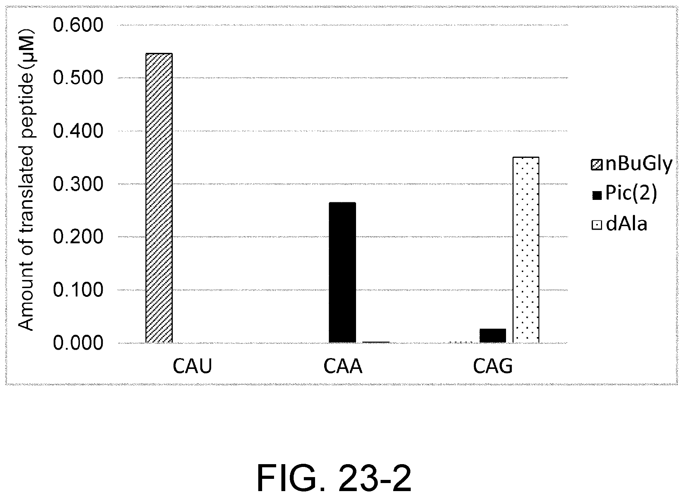

Fig. 23-2 is a graph showing the results of evaluating translation in terms of discrimination of three amino acids in a single codon box, as described in Examples 6 to 7. The codons evaluated are CAU, CAA, and CAG. The vertical axis of the graph shows the amount of translated peptide obtained when the translation was performed using each combination of the tRNAs and the mRNAs described below (see Table 28-2 for specific measurement values).

| | |
|---|---|
| tRNA: | Compound AAtR-60 (anticodon: aug; amino acid: nBuGly) |
| | Compound AAtR-61 (anticodon: uug; amino acid: Pic2) |
| | Compound AAtR-62 (anticodon: cug; amino acid: dA) |
| mRNA: | mR-7 (containing the CAU codon) |
| | mR-8 (containing the CAA codon) |
| | mR-9 (containing the CAG codon) |

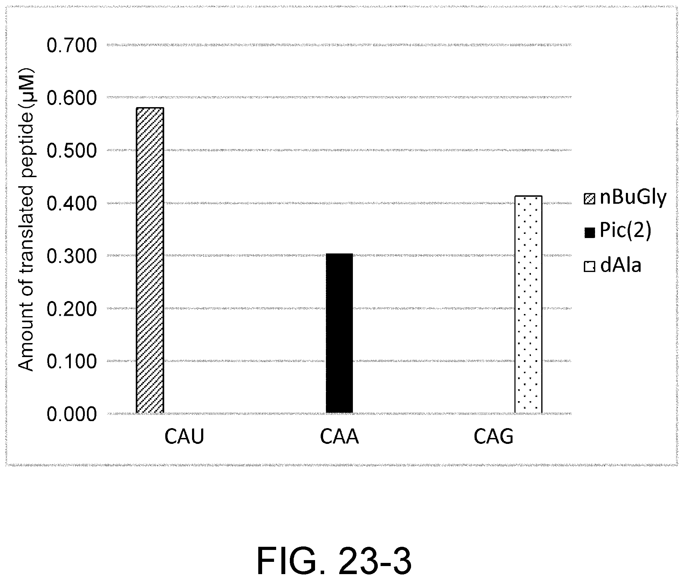

Fig. 23-3 is a graph showing the results of evaluating translation in terms of discrimination of three amino acids in a single codon box, as described in Examples 6 to 7. The codons evaluated are CAU, CAA, and CAG. The vertical axis of the graph shows the amount of translated peptide obtained when the translation was performed using each combination of the tRNAs and the mRNAs described below (see Table 28-3 for specific measurement values).

| | |
|---|---|
| tRNA: | Compound AAtR-63 (anticodon: aug; amino acid: nBuGly) |
| | Compound AAtR-64 (anticodon: uug; amino acid: Pic2) |
| | Compound AAtR-65 (anticodon: cug; amino acid: dA) |
| mRNA: | mR-7 (containing the CAU codon) |
| | mR-8 (containing the CAA codon) |
| | mR-9 (containing the CAG codon) |

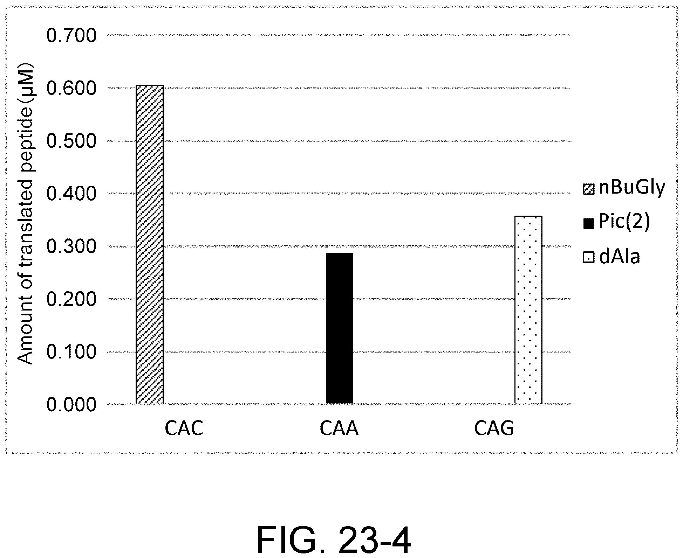

Fig. 23-4 is a graph showing the results of evaluating translation in terms of discrimination of three amino acids in a single codon box, as described in Examples 6 to 7. The codons evaluated are CAC, CAA, and CAG. The vertical axis of the graph shows the amount of translated peptide obtained when the translation was performed using each combination of the tRNAs and the mRNAs described below (see Table 28-4 for specific measurement values).

| | |
|---|---|
| tRNA: | Compound AAtR-66 (anticodon: gug; amino acid: nBuGly) |
| | Compound AAtR-67 (anticodon: uug; amino acid: Pic2) |
| | Compound AAtR-68 (anticodon: cug; amino acid: dA) |
| mRNA: | mR-25 (containing the CAC codon) |
| | mR-8 (containing the CAA codon) |
| | mR-9 (containing the CAG codon) |

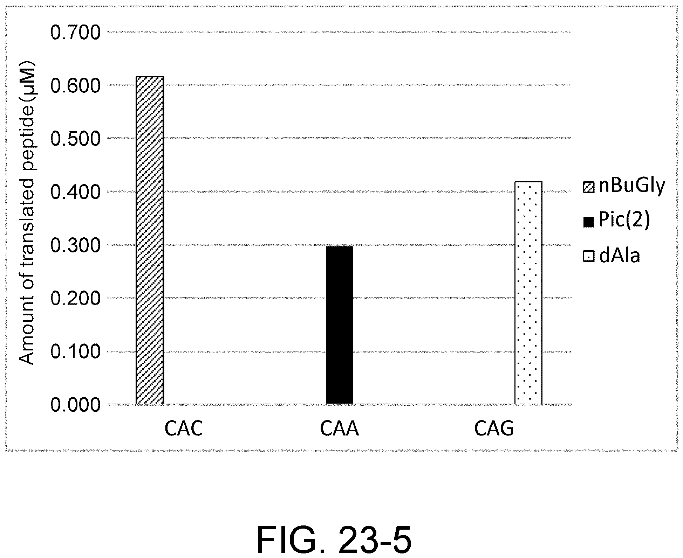

Fig. 23-5 is a graph showing the results of evaluating translation in terms of discrimination of three amino acids in a single codon box, as described in Examples 6 to 7. The codons evaluated are CAC, CAA, and CAG. The vertical axis of the graph shows the amount of translated peptide obtained when the translation was performed using each combination of the tRNAs and the mRNAs described below (see Table 28-5 for specific measurement values).

| | |
|---|---|
| tRNA: | Compound AAtR-69 (anticodon: gug; amino acid: nBuGly) |
| | Compound AAtR-70 (anticodon: uug; amino acid: Pic2) |
| | Compound AAtR-71 (anticodon: cug; amino acid: dA) |
| mRNA: | mR-25 (containing the CAC codon) |
| | mR-8 (containing the CAA codon) |
| | mR-9 (containing the CAG codon) |

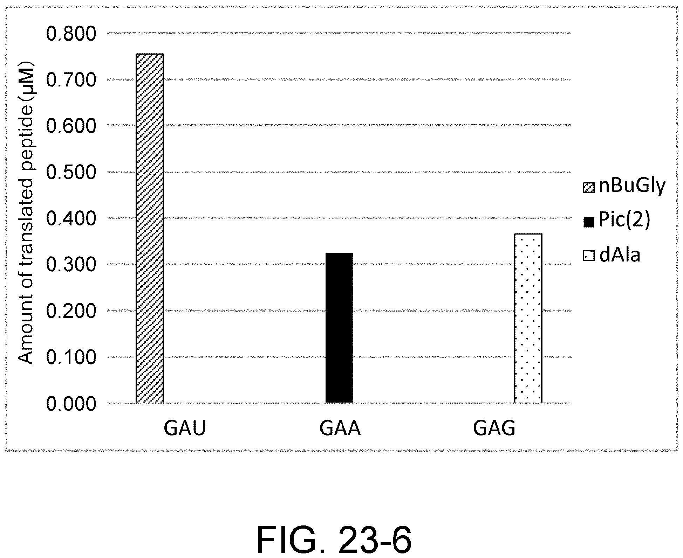

Fig. 23-6 is a graph showing the results of evaluating translation in terms of discrimination of three amino acids in a single codon box, as described in Examples 6 to 7. The codons evaluated are GAU, GAA, and GAG. The vertical axis of the graph shows the amount of translated peptide obtained when the translation was performed using each combination of the tRNAs and the mRNAs described below (see Table 28-6 for specific measurement values).

| | |
|---|---|
| tRNA: | Compound AAtR-72 (anticodon: auc; amino acid: nBuGly) |
| | Compound AAtR-73 (anticodon: uuc; amino acid: Pic2) |
| | Compound AAtR-74 (anticodon: cuc; amino acid: dA) |
| mRNA: | mR-13 (containing the GAU codon) |
| | mR-14 (containing the GAA codon) |
| | mR-15 (containing the GAG codon) |

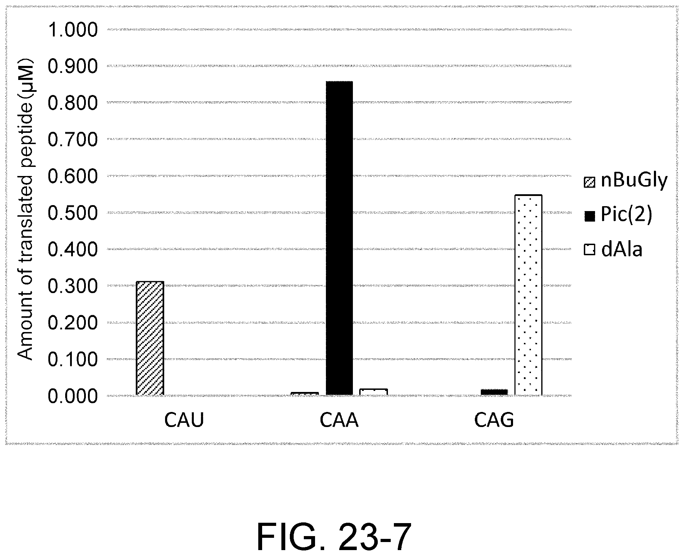

Fig. 23-7 is a graph showing the results of evaluating translation that discriminates three amino acids in a single codon box, as described in Examples 6 to 7. The codons evaluated are CAU, CAA, and CAG. The vertical axis of the graph shows the amount of translated peptide obtained when the translation was performed using each combination of the tRNAs and the mRNAs described below (see Table 28-7 for specific measurement values).

| | |
|---|---|
| tRNA: | Compound AAtR-75 (anticodon: aug; amino acid: MeA3Pr) |
| | Compound AAtR-76 (anticodon: uug; amino acid: StBuOH) |
| | Compound AAtR-77 (anticodon: cug; amino acid: MeSnPr) |
| mRNA: | mR-7 (containing the CAU codon) |
| | mR-8 (containing the CAA codon) |
| | mR-9 (containing the CAG codon) |

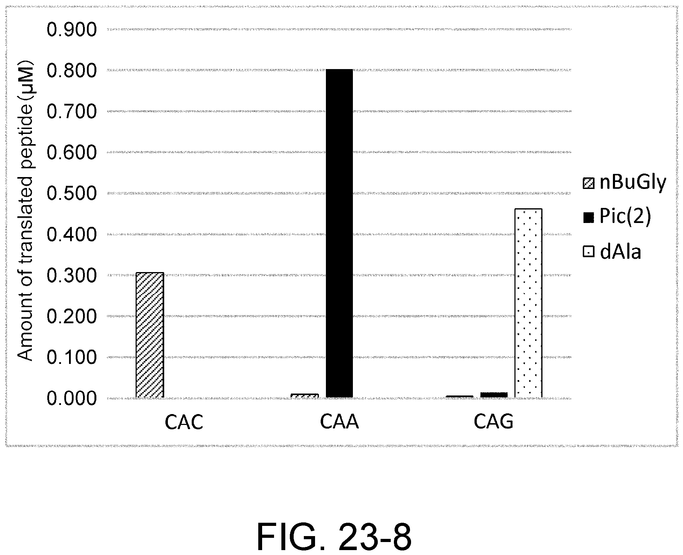

Fig. 23-8 is a graph showing the results of evaluating translation in terms of discrimination of three amino acids in a single codon box, as described in Examples 6 to 7. The codons evaluated are CAC, CAA, and CAG. The vertical axis of the graph shows the amount of translated peptide obtained when the translation was performed using each combination of the tRNAs and the mRNAs described below (see Table 28-8 for specific measurement values).

| | |
|---|---|
| tRNA: | Compound AAtR-78 (anticodon: gug; amino acid: MeA3Pr) |
| | Compound AAtR-79 (anticodon: uug; amino acid: StBuOH) |
| | Compound AAtR-80 (anticodon: cug; amino acid: MeSnPr) |
| mRNA: | mR-25 (containing the CAC codon) |
| | mR-8 (containing the CAA codon) |
| | mR-9 (containing the CAG codon) |

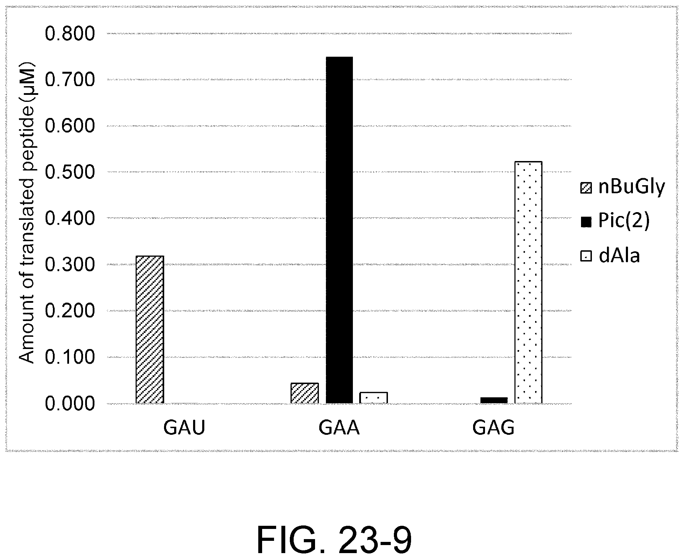

Fig. 23-9 is a graph showing the results of evaluating translation in terms of discrimination of three amino acids in a single codon box, as described in Examples 6 to 7. The codons evaluated are GAU, GAA, and GAG. The vertical axis of the graph shows the amount of translated peptide obtained when the translation was performed using each combination of the tRNAs and the mRNAs described below (see Table 28-9 for specific measurement values).

| | |
|---|---|
| tRNA: | Compound AAtR-81 (anticodon: auc; amino acid: MeA3Pr) |
| | Compound AAtR-82 (anticodon: uuc; amino acid: StBuOH) |
| | Compound AAtR-83 (anticodon: cuc; amino acid: MeSnPr) |
| mRNA: | mR-13 (containing the GAU codon) |
| | mR-14 (containing the GAA codon) |
| | mR-15 (containing the GAG codon) |

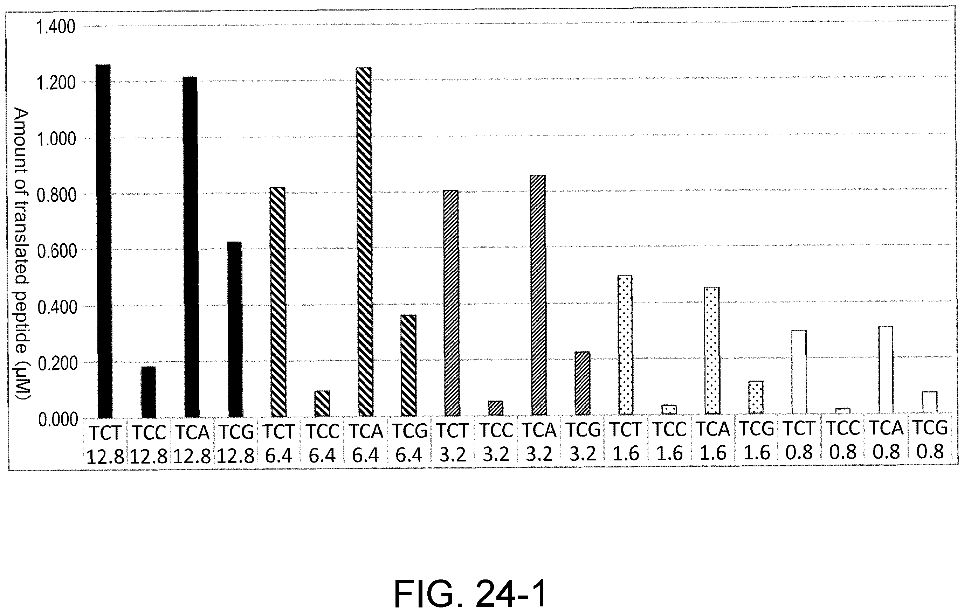

Fig. 24-1 is a graph showing the results of evaluating changes in the amounts of translated peptide of interest and cross-reading under conditions with different concentrations of aminoacylated tRNA, as described in Examples 6 to 7. The codons evaluated are UCU, UCC, UCA, and UCG. The vertical axis of the graph shows the amount of each translated peptide resulting from performing the translation using each combination of the tRNAs and the mRNAs described below (see Table 29-1 for specific measurement values).

| | | | |
|---|---|---|---|
| | tRNA: | Compound AAtR-52 (anticodon: uga; amino acid: MeHph) | |
| tRNA concentration: | | | 12.8 µM |
| | | | 6.4 µM |
| | | | 3.2 µM |
| | | | 1.6 µM |
| | | | 0.8 µM |
| mRNA: | mR-30 (containing the UCU codon) | | |
| | mR-31 (containing the UCC codon) | | |
| | mR-32 (containing the UCA codon) | | |
| | mR-33 (containing the UCG codon) | | |

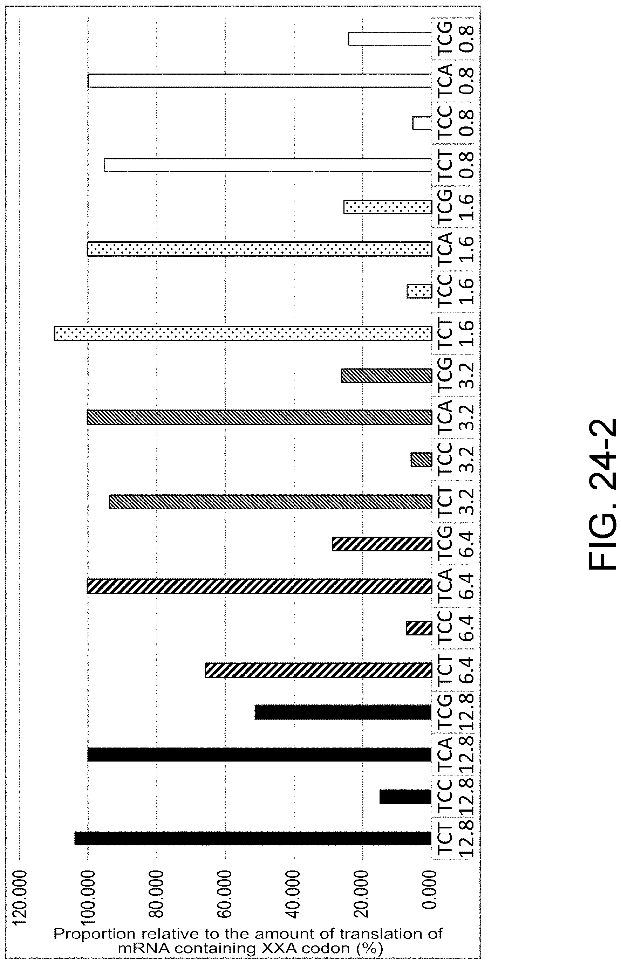

Fig. 24-2 is a graph showing the results of evaluating changes in the amounts of translated peptide of interest and cross-reading under conditions with different concentrations of aminoacylated tRNA, as described in Examples 6 to 7. The codons evaluated are UCU, UCC, UCA, and UCG. The vertical axis of the graph shows the ratio of [the amount of translated peptide of interest] to [the amount of each translated peptide] resulting from performing the translation using each combination of the tRNAs and the mRNAs described below (see Table 29-2 for specific measurement values).

| | | | |
|---|---|---|---|
| | tRNA: | Compound AAtR-52 (anticodon: uga; amino acid: MeHph) | |
| tRNA concentration: | | | 12.8 µM |
| | | | 6.4 µM |
| | | | 3.2 µM |
| | | | 1.6 µM |
| | | | 0.8 µM |
| mRNA: | mR-30 (containing the UCU codon) | | |
| | mR-31 (containing the UCC codon) | | |
| | mR-32 (containing the UCA codon) | | |
| | mR-33 (containing the UCG codon) | | |

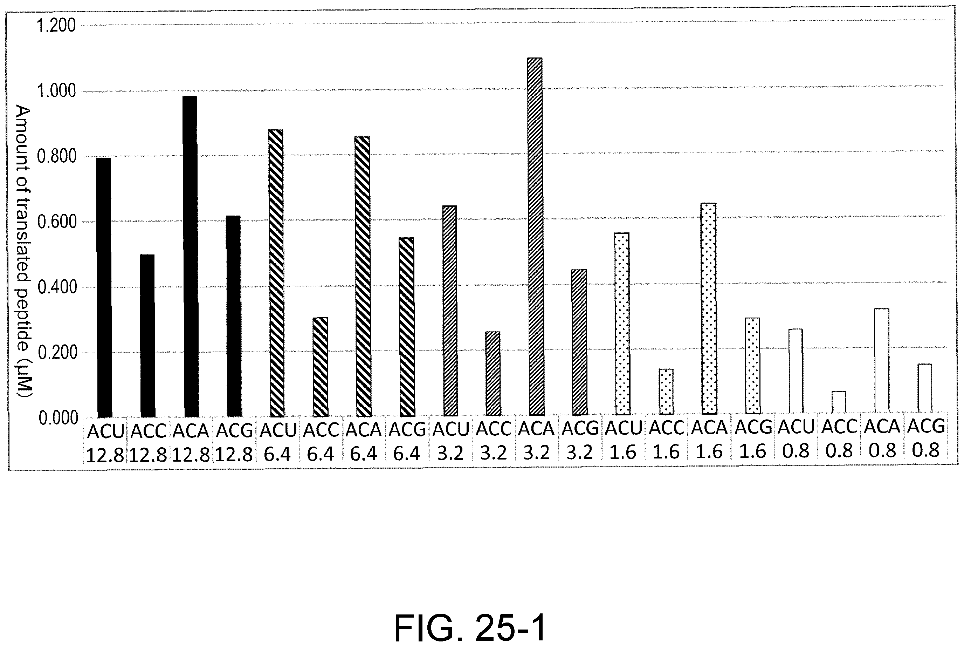

Fig. 25-1 is a graph showing the results of evaluating changes in the amounts of translated peptide of interest and cross-reading under conditions with different concentrations of aminoacylated tRNA, as described in Examples 6 to 7. The codons evaluated are ACU, ACC, ACA, and ACG. The vertical axis of the graph shows the amount of each translated peptide resulting from performing the translation using each combination of the tRNAs and the mRNAs described below (see Table 30-1 for specific measurement values).

| | | | |
|---|---|---|---|
| | tRNA: | Compound AAtR-53 (anticodon: ugu; amino acid: MeHph) | |
| tRNA concentration: | | | 12.8 µM |
| | | | 6.4 µM |
| | | | 3.2 µM |
| | | | 1.6 µM |
| | | | 0.8 µM |
| mRNA: | mR-34 (containing the ACU codon) | | |
| | mR-35 (containing the ACC codon) | | |
| | mR-36 (containing the ACA codon) | | |
| | mR-37 (containing the ACG codon) | | |

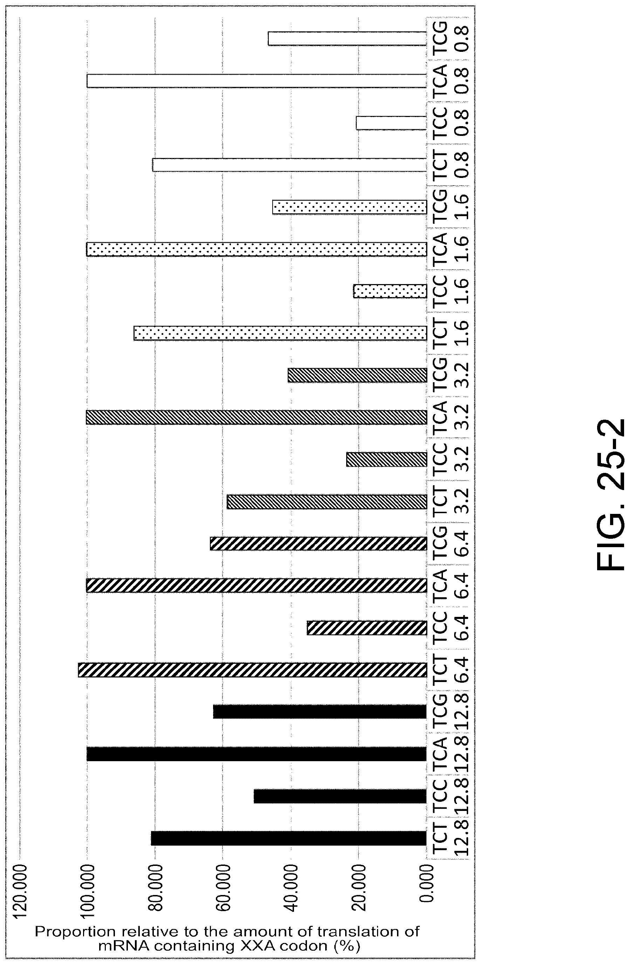

Fig. 25-2 is a graph showing the results of evaluating changes in the amounts of translated peptide of interest and cross-reading under conditions with different concentrations of aminoacylated tRNA, as described in Examples 6 to 7. The codons evaluated are ACU, ACC, ACA, and ACG. The vertical axis of the graph shows the ratio of [the amount of translated peptide of interest] to [the amount of each translated peptide] resulting from performing the translation using each combination of the tRNAs and the mRNAs described below (see Table 30-2 for specific measurement values.

| | | | |
|---|---|---|---|
| | tRNA: | Compound AAtR-53 (anticodon: ugu; amino acid: MeHph) | |
| tRNA concentration: | | | 12.8 µM |
| | | | 6.4 µM |
| | | | 3.2 µM |
| | | | 1.6 µM |
| | | | 0.8 µM |
| mRNA: | mR-34 (containing the ACU codon) | | |
| | mR-35 (containing the ACC codon) | | |
| | mR-36 (containing the ACA codon) | | |
| | mR-37 (containing the ACG codon) | | |

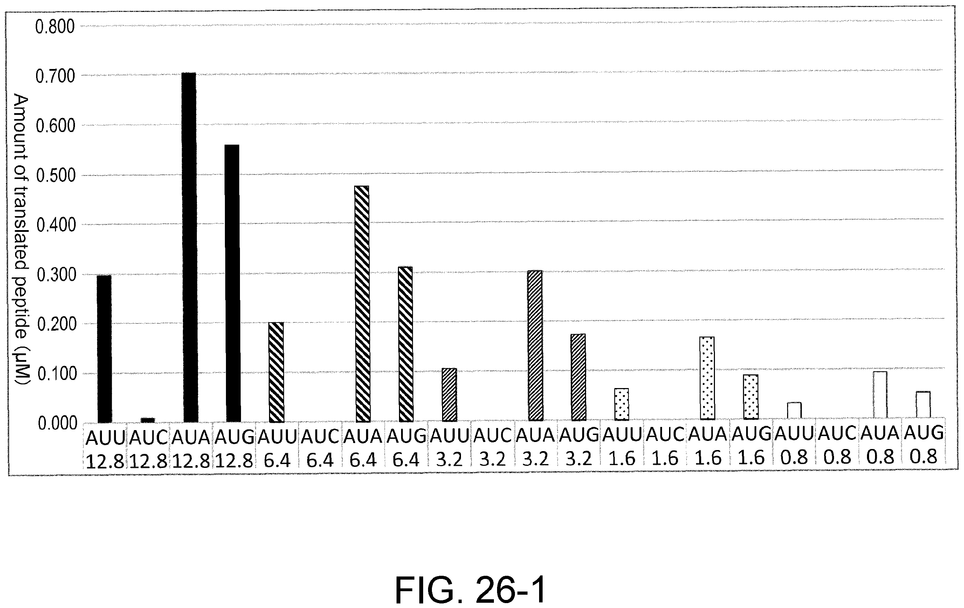

Fig. 26-1 is a graph showing the results of evaluating the amount of translated peptide of interest and cross-reading and their change under conditions with different concentrations of aminoacylated tRNA, as described in Examples 6 to 7. The codons evaluated are AUU, AUC, AUA, and AUG. The vertical axis of the graph shows the amount of each translated peptide resulting from performing the translation using each combination of the tRNAs and the mRNAs described below (see Table 31-1 for specific measurement values).

| | | |
|---|---|---|
| tRNA: | Compound AAtR-54 (anticodon: uau; amino acid: MeHph) | |
| tRNA concentration: | | 12.8 µM |
| | | 6.4 µM |
| | | 3.2 µM |
| | | 1.6 µM |
| | | 0.8 µM |
| mRNA: | mR-38 (containing the AUU codon) | |
| | mR-39 (containing the AUC codon) | |
| | mR-40 (containing the AUA codon) | |
| | mR-41 (containing the AUG codon) | |

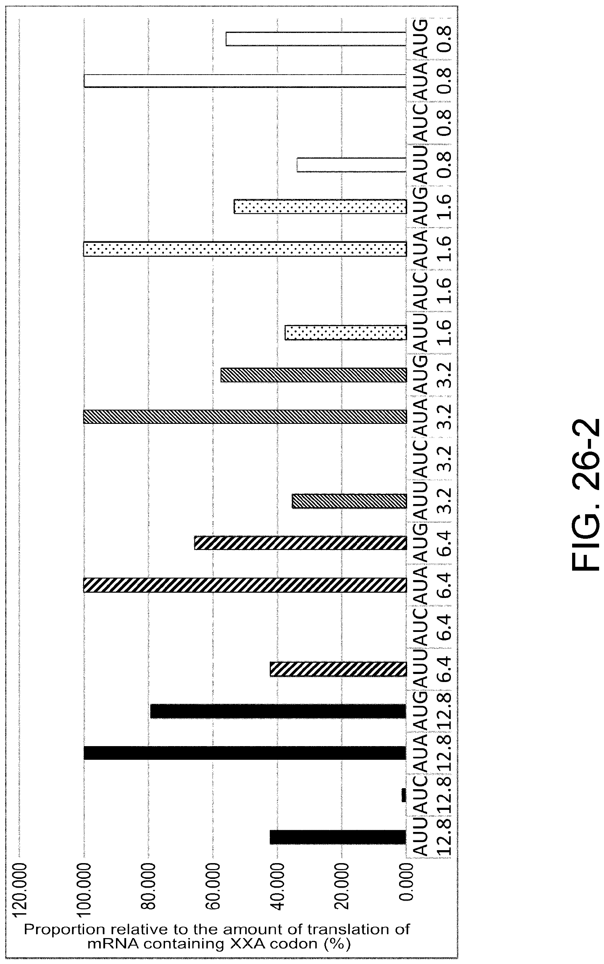

Fig. 26-2 is a graph showing the results of evaluating changes in the amounts of translated peptide of interest and cross-reading under conditions with different concentrations of aminoacylated tRNA, as described in Examples 6 to 7. The codons evaluated are AUU, AUC, AUA, and AUG. The vertical axis of the graph shows the ratio of [the amount of translated peptide of interest] to [the amount of each translated peptide] resulting from performing the translation using each combination of the tRNAs and the mRNAs described below (see Table 31-2 for specific measurement values).

| | | |
|---|---|---|
| tRNA: | Compound AAtR-54 (anticodon: uau; amino acid: MeHph) | |
| tRNA concentration: | | 12.8 µM |
| | | 6.4 µM |
| | | 3.2 µM |
| | | 1.6 µM |
| | | 0.8 µM |
| mRNA: | mR-38 (containing the AUU codon) | |
| | mR-39 (containing the AUC codon) | |
| | mR-40 (containing the AUA codon) | |
| | mR-41 (containing the AUG codon) | |

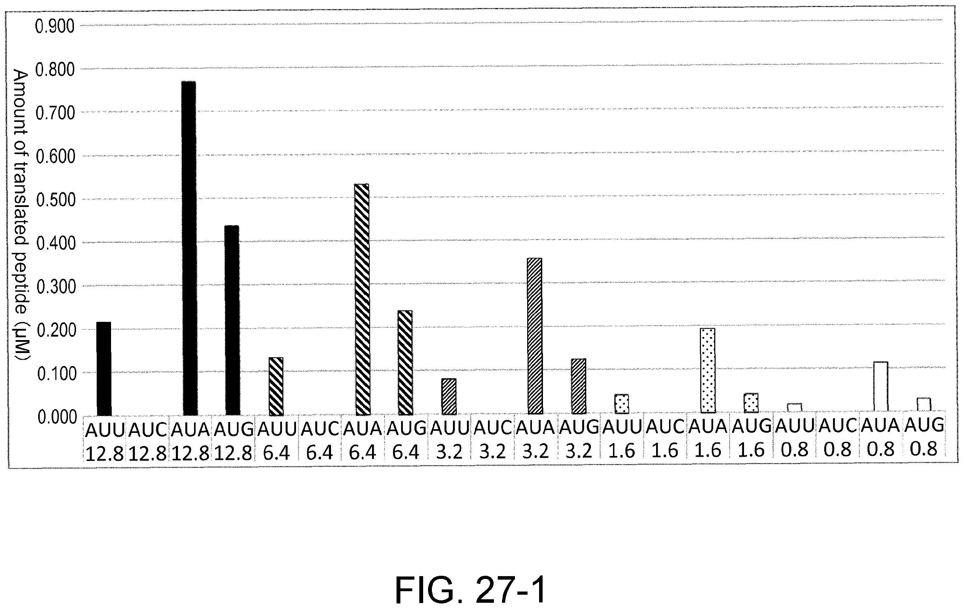

Fig. 27-1 is a graph showing the results of evaluating changes in the amounts of translated peptide of interest and cross-reading under conditions with different concentrations of aminoacylated tRNA, as described in Examples 6 to 7. The codons evaluated are AUU, AUC, AUA, and AUG. The vertical axis of the graph shows the amount of each translated peptide resulting from performing the translation using each combination of the tRNAs and the mRNAs described below (see Table 32-1 for specific measurement values).

| | | |
|---|---|---|
| tRNA: | Compound AAtR-55 (anticodon: uau; amino acid: MeHph) | |
| tRNA concentration: | | 12.8 µM |
| | | 6.4 µM |
| | | 3.2 µM |
| | | 1.6 µM |
| | | 0.8 µM |
| mRNA: | mR-38 (containing the AUU codon) | |
| | mR-39 (containing the AUC codon) | |
| | mR-40 (containing the AUA codon) | |
| | mR-41 (containing the AUG codon) | |

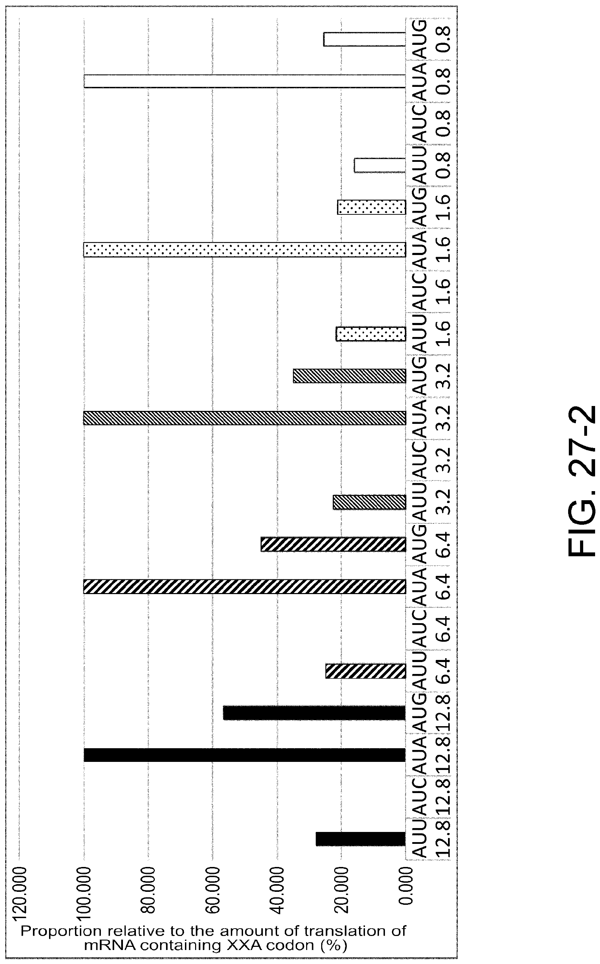

Fig. 27-2 is a graph showing the results of evaluating changes in the amounts of translated peptide of interest and cross-reading under conditions with different concentrations of aminoacylated tRNA, as described in Examples 6 to 7. The codons evaluated are AUU, AUC, AUA, and AUG. The vertical axis of the graph shows the ratio of [the amount of translated peptide of interest] to [the amount of each translated peptide] resulting from performing the translation using each combination of the tRNAs and the mRNAs described below (see Table 32-2 for specific measurement values).

| | | |
|---|---|---|
| tRNA: | Compound AAtR-55 (anticodon: uau; amino acid: MeHph) | |
| tRNA concentration: | | 12.8 µM |
| | | 6.4 µM |
| | | 3.2 µM |
| | | 1.6 µM |
| | | 0.8 µM |
| mRNA: | mR-38 (containing the AUU codon) | |
| | mR-39 (containing the AUC codon) | |
| | mR-40 (containing the AUA codon) | |
| | mR-41 (containing the AUG codon) | |

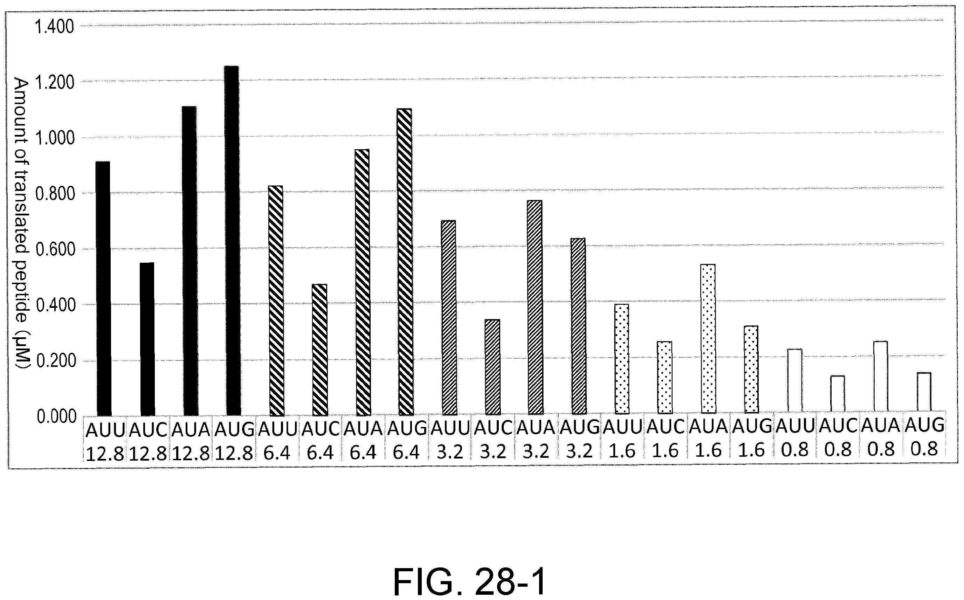

Fig. 28-1 is a graph showing the results of evaluating changes in the amounts of translated peptide of interest and cross-reading under conditions with different concentrations of aminoacylated tRNA, as described in Examples 6 to 7. The codons evaluated are AUU, AUC, AUA, and AUG. The vertical axis of the graph shows the amount of each translated peptide resulting from performing the translation using each combination of the tRNAs and the mRNAs described below (see Table 33-1 for specific measurement values).

| | | |
|---|---|---|
| tRNA: | | Compound AAtR-56 (anticodon: uau; amino acid: Ile) |
| tRNA concentration: | | 12.8 µM |
| | | 6.4 µM |
| | | 3.2 µM |
| | | 1.6 µM |
| | | 0.8 µM |
| mRNA: | mR-38 (containing the AUU codon) | |
| | mR-39 (containing the AUC codon) | |
| | mR-40 (containing the AUA codon) | |
| | mR-41 (containing the AUG codon) | |

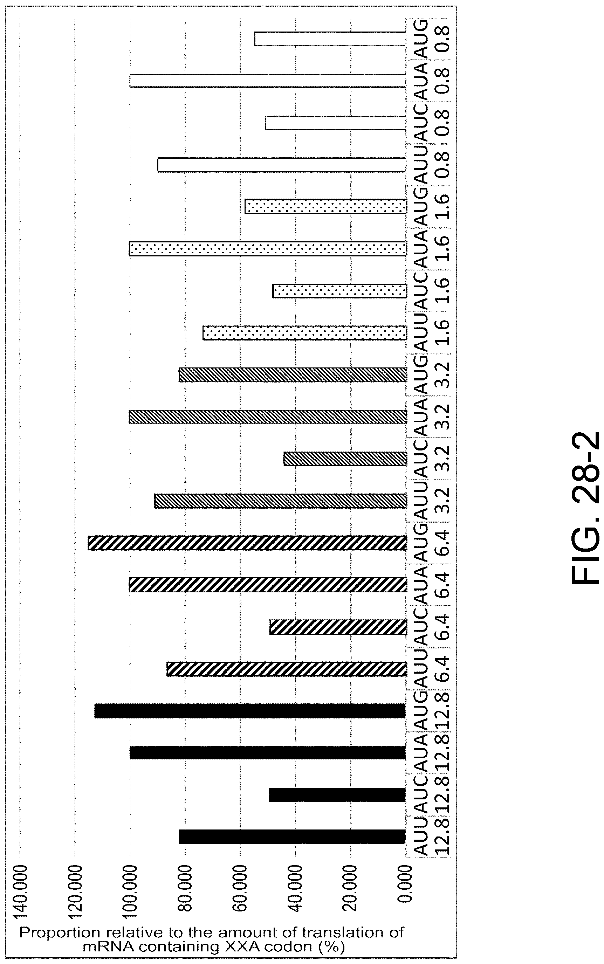

Fig. 28-2 is a graph showing the results of evaluating changes in the amounts of translated peptide of interest and cross-reading under conditions with different concentrations of aminoacylated tRNA, as described in Examples 6 to 7. The codons evaluated are AUU, AUC, AUA, and AUG. The vertical axis of the graph shows the ratio of [the amount of translated peptide of interest] to[the amount of each translated peptide] resulting from performing the translation using each combination of the tRNAs and the mRNAs described below (see Table 33-2 for specific measurement values).

| | | |
|---|---|---|
| tRNA: | Compound AAtR-56 (anticodon: uau; amino acid: Ile) | |
| tRNA concentration: | | 12.8 µM |
| | | 6.4 µM |
| | | 3.2 µM |
| | | 1.6 µM |
| | | 0.8 µM |
| mRNA: | mR-38 (containing the AUU codon) | |
| | mR-39 (containing the AUC codon) | |
| | mR-40 (containing the AUA codon) | |
| | mR-41 (containing the AUG codon) | |

### [Description of Embodiments]

### I. Definition

For the purpose of interpreting this specification, the following definitions will apply and whenever applicable, terms used in the singular will also include the plural, and vice versa. It is to be understood that the terminology used herein is for the purpose of describing particular embodiments only and is not intended to be limiting. If any of the following definitions conflict with any document incorporated herein by reference, the following definitions shall control.

"Codon" refers to a set of three nucleosides (triplet) that corresponds to each amino acid, when genetic information in a living body is translated to a protein. For DNA, four bases, adenine (A), guanine (G), cytosine (C), and thymine (T), are used. For mRNA, four bases, adenine (A), guanine (G), cytosine (C) and uracil (U), are used. The table showing the correspondence between each codon and amino acid is called the genetic code table or codon table, and 20 amino acids are assigned to 61 codons excluding the stop codon (Table 1). The genetic code table shown in Table 1 is used commonly for almost all eukaryote and prokaryote (eubacteria and archaea); therefore, it is called the standard genetic code table or the universal genetic code table. In the present disclosure, a genetic code table used for naturally-occurring organisms is referred to as the natural genetic code table, and it is distinguished from an artificially reprogrammed genetic code table (the correspondence between codons and amino acids is engineered). In the genetic code table, generally, four codons which are the same in the first and second letters and which differ only in the third letter are grouped into one box, and this group is called a codon box.

**[Table 1]**

| | U | | C | | A | | G | | |
|---|---|---|---|---|---|---|---|---|---|
| U | UUU | Phe | UCU | Ser | UAU | Tyr | UGU | Cys | U |
| | UUC | | UCC | | UAC | | UGC | | C |
| | UUA | Leu | UCA | | UAA | Stop | UGA | Stop | A |
| | UUG | | UCG | | UAG | | UGG | Trp | G |
| C | CUU | Leu | CCU | Pro | CAU | His | CGU | Arg | U |
| | CUC | | CCC | | CAC | | CGC | | C |
| | CUA | | CCA | | CAA | Gln | CGA | | A |
| | CUG | | CCG | | CAG | | CGG | | G |
| A | AUU | Ile | ACU | Thr | AAU | Asn | AGU | Ser | U |
| | AUC | | ACC | | AAC | | AGC | | C |
| | AUA | | ACA | | AAA | Lys | AGA | Arg | A |
| | AUG | Met | ACG | | AAG | | AGG | | G |
| G | GUU | Val | GCU | Ala | GAU | Asp | GGU | Gly | U |
| | GUC | | GCC | | GAC | | GGC | | C |
| | GUA | | GCA | | GAA | Glu | GGA | | A |
| | GUG | | GCG | | GAG | | GGG | | G |

In the present disclosure, a codon in mRNA may be expressed as "MiMzMs". Here, M₁, M₂, and M₃ represent the nucleosides for the first letter, the second letter, and the third letter of the codon, respectively.

"Anticodon" refers to three consecutive nucleosides on tRNA that correspond to a codon on the mRNA. Similar to mRNA, four bases, adenine (A), guanine (G), cytosine (C), and uracil (U), are used for the anticodon. Furthermore, modified bases obtained by modifying these bases may be used. When the codon is specifically recognized by the anticodon, the genetic information on the mRNA is read and translated into a protein. The codon sequence on the mRNA in the 5' to 3' direction and the anticodon sequence on the tRNA in the 5' to 3' direction bind complementarily; therefore, complementary nucleotide pairs are formed between the nucleosides for the first, second, and third letters of the codon, and the nucleosides for the third, second, and first letters of the anticodon, respectively.

In the present disclosure, an anticodon in tRNA may be represented by "N₁N₂N₃". Here, N₁, N₂, and N₃ represent the nucleosides for the first letter, second letter, and third letter of the anticodon, respectively. According to the tRNA numbering rule described below, N₁, N₂, and N₃ are numbered as positions 34, 35, and 36 of tRNA, respectively.

In the present disclosure, a combination of nucleic acids capable of forming thermodynamically stable base pairs is said to be "complementary" to each other. In addition to Watson-Crick base pairs such as adenosine and uridine (A-U), and guanosine and cytidine (G-C), combinations of nucleic acids forming non-Watson-Crick base pairs such as guanosine and uridine (G-U), inosine and uridine (I-U), inosine and adenosine (I-A), and inosine and cytidine (I-C) may also be included in the "complementary" nucleic acid combinations in the present disclosure. In particular, only Watson-Crick base pair formation is allowed between the first letter of the codon and the third letter of the anticodon, and between the second letter of the codon and the second letter of the anticodon, whereas there is some fluctuation in space (wobble) between the third letter of the codon and the first letter of the anticodon; therefore, formation of non-Watson-Crick base pair, such as those described above, may be permitted (wobble hypothesis).

"Messenger RNA (mRNA)" refers to an RNA that carries genetic information that can be translated into a protein. Genetic information is coded on mRNA as codons, and each of these codons corresponds to one among all 20 different amino acids. Protein translation begins at the initiation codon and ends at the stop codon. In principle, the initiation codon in eukaryotes is AUG, but in prokaryotes (eubacteria and archaea), GUG and UUG may also be used as initiation codons in addition to AUG. AUG is a codon that encodes methionine (Met), and in eukaryotes and archaea, translation is initiated directly from methionine. On the other hand, in eubacteria, only the initiation codon AUG corresponds to N-formylmethionine (fMet); therefore, translation is initiated from formylmethionine. There are three stop codons: UAA (ochre), UAG (amber), and UGA (opal). When the stop codon is recognized by a protein called a translation termination factor (release factor (RF)), the peptide chain synthesized up to that point is dissociated from the tRNA, and the translation process ends.

"Transfer RNA (tRNA)" refers to a short RNA of 100 bases or less that mediates peptide synthesis using mRNA as a template. In terms of secondary structure, it has a cloverleaf-like structure consisting of three stem loops (the D arm, the anticodon arm, and the T arm) and one stem (the acceptor stem). Depending on the tRNA, an additional variable loop may be included. The anticodon arm has a region consisting of three consecutive nucleosides called an anticodon, and the codon is recognized when the anticodon forms a base pair with the codon on the mRNA. Meanwhile, a nucleic acid sequenceconsisting of cytidine-cytidine-adenosine (CCA sequence) exists at the 3' end of tRNA, and an amino acid is added to the adenosine residue at the end (specifically, the hydroxyl group at position 2 or position 3 of the ribose of the adenosine residue and the carboxyl group of the amino acid form an ester bond). A tRNA to which an amino acid is added is called an aminoacyl tRNA. In the present disclosure, aminoacyl tRNA is also included in the definition of tRNA. Further, as described later, a method is known in which two terminal residues (C and A) are removed from the CCA sequence of tRNA and then this is used for the synthesis of aminoacyl-tRNA. Such a tRNA from which the CA sequence at the 3' end has been removed is also included in the definition of tRNA in the present disclosure. Addition of amino acids to tRNA is carried out by an enzyme called aminoacyl-tRNA synthetase (aaRS or ARS), in vivo. Usually, there is one aminoacyl-tRNA synthetase for each amino acid, and each aminoacyl-tRNA synthetase specifically recognizes only a specific tRNA as a substrate from multiple tRNAs; accordingly, correspondence between tRNAs and amino acids is strictly controlled.

Each nucleoside in tRNA is numbered according to the tRNA numbering rule (Sprinzl et al., Nucleic Acids Res (1998) 26: 148-153). For example, an anticodon is numbered as positions 34 to 36 and the CCA sequence is numbered as positions 74 to 76.

"Initiator tRNA" is a specific tRNA used at the start of mRNA translation. The initiator tRNA attached to the initiator amino acid is catalyzed by a translation initiation factor (IF), introduced into the ribosome, and binds to the initiation codon on the mRNA, thereby translation is initiated. Since AUG, which is a methionine codon, is generally used as an initiation codon, the initiator tRNA has an anticodon corresponding to AUG, and has methionine (formylmethionine for prokaryotes) attached to it as the initiator amino acid.

"Elongator tRNA" is tRNA used in the elongation reaction of the peptide chain in the translation process. In peptide synthesis, amino-acid-attached elongator tRNA is sequentially transported to the ribosome by the GTP-bound translation elongation factor (EF) EF-Tu/eEF-1, and this promotes the peptide chain elongation reaction.

The term "tRNA body" in the present disclosure refers to the main part of tRNA excluding the anticodon (positions 34 to 36) (the main part of the structure composed of nucleic acid). In some embodiments, the tRNA body of the present disclosure refers to positions 1 to 33 and 37 to 76 of tRNA. In other embodiments, the tRNA body of the present disclosure refers to positions 1 to 33 and 37 to 74 of tRNA.

"Lysidine" is a type of modified nucleoside and is also described as 2-lysylcytidine (k2C or L). Lysidine is used as the first letter nucleoside of the anticodon in tRNA corresponding to isoleucine (tRNA Ile2) in eubacteria. tRNA Ile2 is synthesized in the precursor state carrying the anticodon CAU, and then the cytidine (C) of the first letter of the anticodon is engineered (converted) to lysidine (k2C) by an enzyme called tRNA Ile-lysidine synthetase (TilS). As a result, tRNA Ile2 carrying the anticodon k2CAU is provided (Muramatsu et al., J Biol Chem (1988) 263: 9261-9267; and Suzuki et al., FEBS Lett (2010) 584: 272-277). It is known that the anticodon k2CAU specifically recognizes only the AUA codon of isoleucine. Moreover, it is believed that isoleucyl-tRNA synthetase recognizes tRNA Ile2 as a substrate and aminoacylation of (addition of isoleucine to) tRNA Ile2 occurs only when the anticodon is engineered to k2CAU.

"Agmatidine" is a type of modified nucleoside and is also referred to as 2-agmatinylcytidine (agm2C or Agm). Agmatidine is used as the first letter nucleoside of the anticodon in tRNA corresponding to isoleucine (tRNA Ile2) in archaea. tRNA Ile2 is synthesized in the precursor state carrying the anticodon CAU, and then the cytidine (C) of the first letter of the anticodon is engineered (converted) to agmatidine (agm2C) by an enzyme called tRNA Ile-agmatidine synthetase (TiaS). As a result, tRNAIle2 carrying the anticodon agm2CAU is provided (Ikeuchi et al., Nat Chem Biol (2010) 6(4): 277-282). It is known that the anticodon agm2CAU specifically recognizes only the AUA codon of isoleucine. Moreover, it is believed that isoleucyl-tRNA synthetase recognizes tRNA Ile2 as a substrate, and aminoacylation of (addition of isoleucine to) tRNA Ile2 occurs only when the anticodon is engineered to agm2CAU.

The term "cross-reading" in the present disclosure refers to the phenomena in which a certain aminoacyl tRNA recognizes a codon different from the codon it should recognize, thereby resulting in translation of an extra unintended amino acid. The level of translation of the unintended amino acid is not particularly limited, but usually refers to levels at which the orthogonality of translation is judged as being not sufficiently achieved.

In the present disclosure, "alkyl" is a monovalent group derived from an aliphatic hydrocarbon by removing one arbitrary hydrogen atom; it does not contain a hetero atom or an unsaturated carbon-carbon bond in the skeleton; and it has a subset of hydrocarbyl or hydrocarbon-group structures containing hydrogen and carbon atoms. The length of the carbon chain length, n, is in the range of 1 to 20. The examples of alkyl include C₁-C₁₀ alkyl, C₁-C₆ alkyl, and C₁-C₃ alkyl, and specific examples include methyl, ethyl, propyl, butyl, pentyl, hexyl, isopropyl, t-butyl, sec-butyl, 1-methylpropyl, 1,1-dimethylpropyl, 2,2-dimethylpropyl, 1,2-dimethylpropyl, 1,1,2-trimethylpropyl, 1,2,2-trimethylpropyl, 1,1,2,2-tetramethylpropyl, 1-methylbutyl, 2-methylbutyl, 3-methylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, isopentyl, and neopentyl.

In the present disclosure, "cycloalkyl" means a saturated or partially saturated cyclic monovalent aliphatic hydrocarbon group, and includes a monocyclic ring, a bicyclic ring, and a spiro ring. Examples of cycloalkyl include C₃-C₁₀ cycloalkyl, and specific examples include cyclopropyl, cyclobutyl, cyclopentyl, cyclohexyl, cycloheptyl, cyclooctyl, and bicyclo[2.2.1]heptyl.

In the present disclosure, "alkenyl" is a monovalent group having at least one double bond (two adjacent SP2 carbon atoms). Depending on the arrangement of double bonds and substituents (if present), the geometric configuration of the double bond can be entgegen (E) or zusammen (Z), and cis or trans configurations. It can be a straight chain or branched chain alkenyl, and includes a straight chain alkenyl containing an internal olefin. Examples of the alkenyl include C₂-C₁₀ alkenyl and C₂-C₆ alkenyl, and specific examples include vinyl, allyl, 1-propenyl, 2-propenyl, 1-butenyl, 2-butenyl (including cis and trans), 3-butenyl, pentenyl, and hexenyl.

In the present disclosure, "alkynyl" is a monovalent group having at least one triple bond (two adjacent SP carbon atoms). It can be a straight or branched chain alkynyl, and includes an internal alkylene. Examples of the alkynyl include C₂-C₁₀ alkynyl and C₂-C₆ alkynyl, and specific examples include ethynyl, 1-propynyl, propargyl, 3-butynyl, pentynyl, hexynyl, 3-phenyl-2-propinyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

In the present disclosure, "aryl" means a monovalent aromatic hydrocarbon ring. Examples of the aryl include C₆-C₁₀ aryl, and specific examples include phenyl and naphthyl (such as 1-naphthyl and 2-naphthyl).

In the present disclosure, "heteroaryl" means a monovalent aromatic ring group containing a hetero atom in the atoms constituting the ring, and may be partially saturated. The ring may be a monocyclic ring or a fused bicyclic ring (for example, a bicyclic heteroaryl formed by fusing with benzene or a monocyclic heteroaryl). The number of atoms constituting the ring is, for example, five to ten (5- to 10-membered heteroaryl). The number of heteroatoms contained in the ring-constituting atoms is, for example, one to five. Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzooxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

In the present disclosure, "arylalkyl (aralkyl)" is a group containing both aryl and alkyl, and means, for example, a group in which at least one hydrogen atom of the above-mentioned alkyl is substituted with aryl. Examples of the aralkyl include C₅-C₁₀ aryl C₁-C₆ alkyl, and specific examples include benzyl.

In the present disclosure, "alkylene" means a divalent group derived by further removing one arbitrary hydrogen atom from the above-mentioned "alkyl", and may be linear or branched. Examples of the straight chain alkylene include C₂-C₆ straight chain alkylene, C₄-C₅ straight chain alkylene and the like. Specific examples include -CH₂-, -(CH₂)₂-, -(CH₂)₃-, - (CH₂)₄-, -(CH₂)₅-, and -(CH₂)₆-. Examples of the branched alkylene include C₂-C₆ branched alkylene and C₄-C₅ branched alkylene. Specific examples include -CH(CH₃)CH₂-, -C(CH₃)₂-, - CH(CH₃)CH₂CH₂-, -C(CH₃)₂CH₂-, -CH₂CH(CH₃)CH₂-, CH₂C(CH₃)₂-, and -CH₂CH₂CH(CH₃)-.

In the present disclosure, "alkenylene" means a divalent group derived by further removing one arbitrary hydrogen atom from the above-mentioned "alkenyl", and may be linear or branched. Depending on the arrangement of double bonds and substituents (if present), it can take the form of entgegen (E) or zusammen (Z), and cis or trans configurations. Examples of the straight chain alkenylene include C₂-C₆ straight chain alkenylene and C₄-C₅ straight chain alkenylene. Specific examples include -CH=CH-, -CH=CHCH₂-, -CH₂CH=CH-, - CH=CHCH₂CH₂-, -CH₂CH=CHCH₂-, -CH₂CH₂CH=CH-, -CH=CHCH₂CH₂CH₂-, - CH₂CH=CHCH₂CH₂-, -CH₂CH₂CH=CHCH₂-, and -CH₂CH₂CH₂CH=CH-.

In the present disclosure, "arylene" means a divalent group derived by further removing one arbitrary hydrogen atom from the above-mentioned aryl. The ring may be a monocyclic ring or a fused ring. The number of atoms constituting the ring is not particularly limited, but is, for example, six to ten (C₆-C₁₀ arylene). Specific examples of arylene include phenylene and naphthylene.

In the present disclosure, "heteroarylene" means a divalent group derived by further removing one arbitrary hydrogen atom from the above-mentioned heteroaryl. The ring may be a monocyclic ring or a fused ring. The number of atoms constituting the ring is not particularly limited, but is, for example, five to ten (5- to 10-membered heteroarylene). As the heteroarylene, specific examples include pyrrolediyl, imidazoldiyl, pyrazolediyl, pyridinediyl, pyridazinediyl, pyrimidinediyl, pyrazinediyl, triazolediyl, triazinediyl, isoxazolediyl, oxazolediyl, oxadiazolediyl, isothiazolediyl, thiazolediyl, thiadiazolediyl, furandiyl, and thiophenediyl.

"Translation system" in the present disclosure is defined as a concept including both a method for translating a peptide and a composition and a kit for translating a peptide. The translation system usually contains as constituent components, ribosomes, translation factors, tRNAs, amino acids, aminoacyl-tRNA synthetase (aaRS), and factors necessary for peptide translation reactions such as ATP and GTP. The main types of translation systems include translation systems that utilize living cells and translation systems that utilize cell extract solutions (cell-free translation systems). As the translation system utilizing living cells, a known example is a system in which a desired aminoacyl-tRNA and mRNA are introduced into living cells such as Xenopus oocytes and mammalian cells by microinjection method or lipofection method to perform peptide translation (Nowak et al., Science (1995) 268: 439-442). Known examples of cell-free translation systems include translation systems that utilize extract solutions from *E. coli* (Chen et al., Methods Enzymol (1983) 101: 674-690), yeast (Gasior et al., J Biol Chem (1979) 254: 3965-3969), wheat germ (Erickson et al., Methods Enzymol (1983) 96: 38-50), rabbit reticulocytes (Jackson et al., Methods Enzymol (1983)96: 50-74), HeLa cells (Barton et al., Methods Enzymol (1996) 275: 35-57), or insect cells (Swerdel et al., Comp Biochem Physiol B (1989) 93: 803-806), etc. Such a translation system can be appropriately prepared by a method known to those skilled in the art or a similar method. The cell-free translation system also includes a translation system constructed by isolating and purifying each of the factors required for peptide translation and reconstituting them (reconstituted cell-free translation system) (Shimizu et al., Nat Biotech (2001) 19: 751-755). Reconstituted cell-free translation systems may usually include ribosomes, amino acids, tRNAs, aminoacyl-tRNA synthetases (aaRS), translation initiation factors (for example, IF1, IF2, and IF3), translation elongation factors (for example, EF-Tu, EF-Ts, and EF-G), translation termination factors (for example, RF1, RF2, and RF3), ribosome recycling factors (RRF), NTPs as energy sources, energy regeneration systems, and other factors required for translation. When the transcription reaction from DNA is also performed, RNA polymerase and the like may be further included. Various factors contained in the cell-free translation system can be isolated and purified by methods well known to those skilled in the art, and a reconstituted cell-free translation system can be appropriately constructed using them. Alternatively, a commercially available reconstituted cell-free translation system such as PUREfrex^{®}from Gene Frontier or PURExpress^{®}from New England BioLabs can be used. For a reconstituted cell-free translation system, a desired translation system can be constructed by reconstituting only the necessary components from among the translation system components.

An aminoacyl-tRNA is synthesized by a specific combination of amino acid, tRNA, and aminoacyl-tRNA synthetase, and it is used for peptide translation. Instead of the above-mentioned combination, aminoacyl-tRNA can be directly used as a constituent component of the translation system. In particular, when an amino acid that is difficult to aminoacylate with an aminoacyl-tRNA synthetase, such as an unnatural amino acid, is used for translation, it is desirable to use a tRNA which is aminoacylated in advance with an unnatural amino acid, as a constituent component.

The translation is started by adding mRNA to the translation system. An mRNA usually contains a sequence that encodes the peptide of interest, and may further include a sequence for increasing the efficiency of translation reaction (for example, a Shine-Dalgarno (SD) sequence in prokaryotes, or a Kozac sequence in eukaryotes). Pre-transcribed mRNA may be added directly to the system, or instead of mRNA, a template DNA containing a promoter and an RNA polymerase appropriate for the DNA (for example, T7 promoter and T7 RNA polymerase) can be added to the system, so that mRNA will be transcribed from the template DNA.

In the present specification, the sign "-" indicating a range of numerical values is meant to include the values on both sides of the sign, and for example, "A-B" means a range of numerical values that is A or more and B or less.

In the present specification, the meaning of the term "and/or" includes every combination where "and" and "or" are suitably combined. Specifically, for example, "A, B, and/or C" includes the following seven variations:
(i) A, (ii) B, (iii) C, (iv) A and B, (v) A and C, (vi) B and C, and (vii) A, B, and C.

### II. Translation systems and methods for producing translation systems

In one aspect, the present disclosure provides a translation system comprising a tRNA having an anticodon complementary to a codon represented by M₁M₂A and a tRNA having an anticodon complementary to a codon represented by M₁M₂G. In one aspect, in the present disclosure, M₁ and M₂ represent nucleosides for the first and second letters of the codon respectively, and each of M₁ and M₂ is independently selected from any of adenosine (A), guanosine (G), cytidine (C), and uridine (U). Each of the two tRNAs included in the translation system of the present disclosure may be an aminoacyl tRNA attached to an amino acid or an amino acid analog that is different from each other. The tRNAs in the present disclosure can selectively translate the codon represented by M₁M₂A and the codon represented by M₁M₂G. Therefore, by using a translation system comprising tRNAs of the present disclosure, at least two types of amino acids or amino acid analogs can be translated from a single codon box (the codon box is composed of the codon represented by M₁M₂U, the codon represented by M₁M₂C, the codon represented by M₁M₂A, and the codon represented by M₁M₂G).

The codons represented by M₁M₂A and the codons represented by M₁M₂G in the present disclosure include all combinations that can be specified by selecting any one of adenosine (A), guanosine (G), cytidine (C), and uridine (U) for M₁ and selecting any one of adenosine (A), guanosine (G), cytidine (C), and uridine (U) for M₂.

In this disclosure, the expression "selectively translate the codon" can be rephrased as "discriminate the codons" or "reduce cross-reading", and these phrases may be interpreted to have the same meaning.

In one aspect, the present disclosure provides a translation system comprising a tRNA having an anticodon complementary to a codon represented by M₁M₂U, a tRNA having an anticodon complementary to a codon represented by M₁M₂A, and a tRNA having an anticodon complementary to a codon represented by M₁M₂G. Furthermore, in one aspect, the present disclosure provides a translation system comprising a tRNA having an anticodon complementary to a codon represented by M₁M₂C, a tRNA having an anticodon complementary to a codon represented by M₁M₂A, and a tRNA having an anticodon complementary to a codon represented by M₁M₂G. In one aspect, in the present disclosure, M₁ and M₂ represent nucleosides for the first and second letters of the codon respectively, and each of M₁ and M₂ is independently selected from any of adenosine (A), guanosine (G), cytidine (C), and uridine (U). Each of the three tRNAs included the translation system of the present disclosure may be an aminoacyl tRNA attached to an amino acid or an amino acid analog that is different from each other. In one aspect, tRNAs in the present disclosure can selectively translate the codon represented by M₁M₂U, the codon represented by M₁M₂A, and the codon represented by M₁M₂G. Furthermore, in one aspect, tRNAs in the present disclosure can selectively translate the codon represented by M₁M₂C, the codon represented by M₁M₂A, and the codon represented by M₁M₂G. Therefore, by using a translation system comprising tRNAs of the present disclosure, at least three amino acids or amino acid analogs can be translated from a single codon box (the codon box is composed of the codon represented by M₁M₂U, the codon represented by M₁M₂C, the codon represented by M₁M₂A, and the codon represented by M₁M₂G).

The codons represented by M₁M₂U and the codons represented by M₁M₂C in the present disclosure include all combinations that can be specified by selecting any one of adenosine (A), guanosine (G), cytidine (C), and uridine (U) for M₁ and selecting any one of adenosine (A), guanosine (G), cytidine (C), and uridine (U) for M₂.

A translation system of the present disclosure comprises a plurality of different tRNAs, and a plurality of different amino acids or amino acid analogs can be translated from those tRNAs. In one aspect, the present disclosure provides compositions and kits for selectively translating codons, comprising a plurality of different tRNAs suitable for peptide translation. In another aspect, the present disclosure provides methods of selectively translating codons, comprising translating a nucleic acid in a translation system comprising a plurality of different tRNAs suitable for peptide translation. In certain aspects, the plurality of different tRNAs mentioned above include a tRNA of the present disclosure.

Combinations of codons that can be selectively translated in the present disclosure are, for example, the combination of a codon represented by M₁M₂A and a codon represented by M₁M₂G; the combination of a codon represented by M₁M₂U, a codon represented by M₁M₂A, and a codon represented by M₁M₂G; and the combination of a codon represented by M₁M₂C, a codon represented by M₁M₂A, and a codon represented by M₁M₂G.

In one embodiment, a translation system of the present disclosure may comprise a nucleic acid comprising one or more occurrences of each of the codon represented by M₁M₂A and the codon represented by M₁M₂G. In one embodiment, a translation system of the present disclosure may comprise (i) a nucleic acid comprising one or more occurrences of each of the codon represented by M₁M₂U, the codon represented by M₁M₂A, and the codon represented by M₁M₂G; or (ii) a nucleic acid comprising one or more occurrences of each of the codon represented by M₁M₂C, the codon represented by M₁M₂A, and the codon represented by M₁M₂G. In one embodiment, a translation system of the present disclosure may comprise a nucleic acid comprising one or more occurrences of each of the codon represented by M₁M₂A, the codon represented by M₁M₂G, the codon represented by M₁M₂U, and the codon represented by M₁M₂C. In one embodiment, a translation system of the present disclosure may comprise one or more such nucleic acids.

In one aspect, the present disclosure relates to methods for producing a translation system. A method for producing a translation system of the present disclosure comprises attaching an amino acid or an amino acid analog to a tRNA outside the translation system and/or artificially. In one aspect, the method for producing a translation system of the present disclosure further comprises a step of mixing the tRNA to which an amino acid or an amino acid analog has been attached with the translation system of the present disclosure.

The translation system usually contains as constituent components, ribosomes, translation factors, tRNAs, amino acids, aminoacyl-tRNA synthetase (aaRS), and factors necessary for peptide translation reactions such as ATP and GTP. Particularly for a reconstituted cell-free translation system, a desired translation system can be constructed by reconstituting only the necessary components from among the translation system components. Synthesis of aminoacyl tRNA (tRNA having an amino acid or an amino acid analog attached to it) in a translation system can be performed inside or outside the translation system. Alternatively, synthesis inside and outside the translation system may be used in combination.

In some embodiments, the tRNA having an anticodon complementary to a codon represented by M₁M₂A and the tRNA having an anticodon complementary to a codon represented by M₁M₂G of the present disclosure are attached to an amino acid or an amino acid analog outside the translation system. In some embodiments, the tRNA having an anticodon complementary to a codon represented by M₁M₂U, the tRNA having an anticodon complementary to a codon represented by M₁M₂A, and the tRNA having an anticodon complementary to a codon represented by M₁M₂G of the present disclosure are attached to an amino acid or an amino acid analog outside the translation system. In some embodiments, the tRNA having an anticodon complementary to a codon represented by M₁M₂C, the tRNA having an anticodon complementary to a codon represented by M₁M₂A, and the tRNA having an anticodon complementary to a codon represented by M₁M₂G of the present disclosure are attached to an amino acid or an amino acid analog outside the translation system. In further embodiments, tRNAs other than those described above that are included in the translation system may be attached to an amino acid or an amino acid analog inside the translation system or outside the translation system.

In the present disclosure, tRNAs are preferably those to which an amino acid or an amino acid analog has been attached artificially. tRNAs may be natural tRNAs derived from any organism (for example, *E. coli*), or artificially synthesized non-natural tRNAs having sequences different from the natural tRNA sequences. The tRNAs in the present disclosure may be "tRNAs synthesized by in vitro transcription".

In some embodiments, when a tRNA in the present disclosure is an engineered tRNA having a sequence different from natural tRNAs, that engineered tRNA can comprise at least one alteration selected from the following group in one or more nucleosides constituting the tRNA: (i) addition (adding any new nucleoside to an existing tRNA), (ii) deletion (deleting any nucleoside from an existing tRNA), (iii) substitution (substituting any nucleoside in an existing tRNA with another arbitrary nucleoside), (iv) insertion (adding a new arbitrary nucleoside between any two nucleosides in an existing tRNA), and (v) modification (changing a part of the structure (for example, the nucleotide or sugar portion) of any nucleoside in an existing tRNA to another structure). Engineering may be made to any structure of a tRNA (for example, the D arm, anticodon arm, T arm, acceptor stem, variable loop, and such). In some embodiments, the engineering of tRNA in the present disclosure is made to anticodons contained in anticodon arms. In a further embodiment, the engineering of tRNA in the present disclosure is made to at least one of the nucleosides for the first, second, and third letters of the anticodon. According to the nucleoside numbering rule in tRNA, nucleosides for the first, second, and third letters of the anticodon correspond to positions 34, 35, and 36 of tRNA, respectively. Herein, the nucleosides for the first, second, and third letters of the anticodon may be represented as N₁, N₂, and N₃, respectively. The number of nucleosides altered in the tRNA of the present disclosure can be any number not less than one. In some embodiments, the number of nucleosides altered in the tRNA of the present disclosure is 20 or less, 15 or less, 10 or less, 9 or less, 8 or less, 7 or less, 6 or less, 5 or less, 4 or less, 3 or less, 2 or less, or 1. In another embodiment, the nucleic acid sequence of the engineered tRNA has sequence identity of 80% or more, 85% or more, 90% or more, 91% or more, 92% or more, 93% or more, 94% or more, 95% or more, 96% or more, 97% or more, 98% or more, or 99% or more, as compared to the nucleic acid sequence before the engineering. In the present disclosure, "percent (%) sequence identity" with respect to a certain nucleotide sequence is defined as the percentage of bases in a candidate sequence that are identical with the bases in the reference nucleotide sequence, after aligning the sequences and introducing gaps, if necessary, to achieve the maximum percent sequence identity, and not considering any conservative substitutions as part of the sequence identity. Alignment for purposes of determining percent nucleotide sequence identity can be achieved by various methods that are within the skill in the art, for example, using publicly available computer software such as BLAST, BLAST-2, ALIGN, Megalign (DNASTAR) software, or GENETYX (registered trademark) (GENETYX Corporation). Those skilled in the art can determine appropriate parameters for aligning sequences, including any algorithms needed to achieve maximal alignment over the full length of the sequences being compared.

In some embodiments, engineering of tRNA in the present disclosure means substitution of one or more nucleosides constituting a tRNA. Regarding the types of nucleosides, a substituted nucleoside may be any nucleoside present in natural tRNAs or any nucleoside not present in natural tRNAs (an artificially synthesized nucleoside). In addition to the four typical nucleosides, adenosine, guanosine, cytidine and uridine, natural tRNAs include engineered forms obtained by modifying these four nucleosides (modified nucleosides). In some embodiments, the nucleoside present in natural tRNAs can be selected from among the following nucleosides: adenosine (A); cytidine (C); guanosine (G); uridine (U); 1-methyladenosine (m1A); 2-methyladenosine (m2A); N6-isopentenyladenosine (i6A); 2-methylthio-N6-isopentenyladenosine (ms2i6A); N6-methyladenosine (m6A); N6-threonylcarbamoyladenosine (t6A); N6-methyl-N6-threonylcarbamoyladenosine (m6t6A); 2-methylthio-N6-threonylcarbamoyladenosine (ms2t6A); 2'-O-methyladenosine (Am); inosine (I); 1-methylinosine (m1I); 2'-O-ribosyladenosine (phosphate) (Ar(p)); N6-(cis-hydroxyisopentenyl)adenosine (io6A); 2-thiocytidine (s2C); 2'-O-methylcytidine (Cm); N4-acetylcytidine (ac4C); 5-methylcytidine (m5C); 3-methylcytidine (m3C); lysidine (k2C); 5-formylcytidine (f5C); 2'-O-methyl-5-formylcytidine (f5Cm); agmatidine (agm2C); 2'-O-ribosylguanosine (phosphate) (Gr(p)); 1-methylguanosine (m1G); N2-methylguanosine (m2G); 2'-O-methylguanosine (Gm); N2, N2-dimethylguanosine(m22G); N2, N2, 2'-O-trimethylguanosine (m22Gm); 7-methylguanosine (m7G); archaeosine (G*); queuosine (Q); mannosylqueuosine (manQ); galactosylqueuosine (galQ); wybutosine (yW); peroxywybutosine (o2yW); 5-methylaminomethyluridine (mnm5U); 2-thiouridine (s2U); 2'-O-methyluridine (Um); 4-thiouridine (s4U); 5-carbamoylmethyluridine (ncm5U); 5-methoxycarbonylmethyluridine (mcm5U); 5-methylaminomethyl-2-thiouridine (mnm5s2U); 5-methoxycarbonylmethyl-2-thiouridine (mcm5s2U); uridine 5-oxyacetic acid (cmo5U); 5-methoxyuridine (mo5U); 5-carboxymethylaminomethyluridine (cmnm5U); 5-carboxymethylaminomethyl-2-thiouridine (cmnm5s2U); 3-(3-amino-3-carboxypropyl)uridine (acp3U); 5-(carboxyhydroxymethyl)uridinemethyl ester (mchm5U); 5-carboxymethylaminomethyl-2'-O-methyluridine (cmnm5Um); 5-carbamoylmethyl-2'-O-methyluridine (ncm5Um); dihydrouridine (D); pseudouridine (Ψ); 1-methylpseudouridine (m1Ψ); 2'-O-methylpseudouridine (Ψm); 5-methyluridine (m5U); 5-methyl-2-thiouridine (m5s2U); and 5, 2'-O-dimethyluridine (m5Um).

The tRNA engineered in the present disclosure can be appropriately selected from tRNAs having an arbitrary nucleic acid sequence. In some embodiments, the tRNA is any one of tRNA Ala, tRNA Arg, tRNA Asn, tRNA Asp, tRNA Cys, tRNA Gln, tRNA Glu, tRNA Gly, tRNA His, tRNA Ile, tRNA Leu, tRNA Lys, tRNA Met, tRNA Phe, tRNA Pro, tRNA Ser, tRNA Thr, tRNA Trp, tRNA Tyr, and tRNA Val. In addition to the above-mentioned 20 tRNAs, tRNA fMet, tRNA Sec (selenocysteine), tRNA Pyl (pyrrolysine), tRNA AsnE2 and the like may be used. In a particular embodiment, the tRNA is any one of tRNA Glu, tRNA Asp, tRNA AsnE2, tRNA(fMet), and tRNA(Ile). The term "tRNA body" is sometimes used to refer to the main part of tRNA (the main part of the structure composed of nucleic acid).

In addition, in the present disclosure, tRNA may be expressed as follows.
- "tRNA Xxx" or "tRNA(Xxx)"... indicates a tRNA (full length) corresponding to the amino acid Xxx (for example, tRNA Glu or tRNA(Glu)).
- "tRNA(Xxx)nnn"... indicates a tRNA corresponding to the amino acid Xxx, which is a tRNA (full length) having an anticodon sequence of nnn (for example, tRNA(Glu)uga or tRNA(Glu)Lga).
- "tRNA(Xxx)nnn-CA"... indicates a tRNA corresponding to the amino acid Xxx, which is a tRNA (the CA sequence at the 3' end has been removed) having an anticodon sequence of nnn (for example, tRNA(Glu )uga-CA and tRNA(Glu)Lga-CA).

In some embodiments, the tRNA of the present disclosure is an initiator tRNA or an elongator tRNA. The tRNA may be produced by engineering the initiator tRNA or the elongator tRNA, or the tRNA produced by the engineering may have a function as the initiator tRNA or the elongator tRNA. Whether or not a certain tRNA has a function as an initiator tRNA can be judged by observing whether the tRNA (i) is introduced into the ribosome via IF2, and (ii) whether the amino acid attached to the tRNA can be used as the initiator amino acid to start the peptide translation, when the tRNA is used in a translation system. Furthermore, whether or not a certain tRNA has a function as an elongator tRNA can be determined by observing whether the tRNA (i) is introduced into the ribosome via EF-Tu, and (ii) whether or not the amino acid attached to the tRNA can be incorporated into the peptide chain to extend the peptide chain, when the tRNA is used in a translation system.

In some embodiments, the tRNA of the present disclosure is a prokaryote-derived tRNA or a eukaryote-derived tRNA. A mutated tRNA may be produced by engineering a prokaryote-derived tRNA or a eukaryote-derived tRNA, and the tRNA produced by the engineering may have the highest nucleic acid sequence identity with the prokaryote-derived tRNA or the eukaryote-derived tRNA. Eukaryotes are further classified into animals, plants, fungi, and protists. The tRNA of the present disclosure may be, for example, a human-derived tRNA. Prokaryotes are further classified into eubacteria and archaea. Examples of eubacteria include *E*. *coli, Bacillus subtilis,* lactic acid bacteria, and *Desulfitobacterium hafniense.* Examples of archaea include extreme halophile, thermophile, or methane bacteria (for example, *Methanosarcina mazei, Methanosarcina barkeri,* and *Methanocaldococcusjannaschii*). The tRNA of the present disclosure may be, for example, tRNA derived from *E. coli, Desulfitobacterium hafniense,* or *Methanosarcina mazei.*

In some embodiments, the tRNA of the present disclosure may be one that does not have any one of lysidine (k2C), a lysidine derivative, agmatidine (agm2C), and an agmatidine derivative, at, for example, the first letter (N₁) of its anticodon. In a particular embodiment, the tRNA of the present disclosure may be one that has none of lysidine (k2C), a lysidine derivative, agmatidine (agm2C), and an agmatidine derivative, at, for example, the first letter (N₁) of the anticodon.

A tRNA can be synthesized, for example, by preparing a DNA encoding a desired tRNA gene, then placing an appropriate promoter such as T7, T3, or SP6 upstream of the DNA, and performing a transcription reaction with the DNA as a template using an RNA polymerase adapted to each promoter. Furthermore, tRNA can also be prepared by purification from biological materials. For example, tRNA can be recovered by preparing an extract from a material containing tRNA such as cells, and adding thereto a probe containing a sequence complementary to the nucleic acid sequence of tRNA. In this case, the material for the preparation may be cells transformed with an expression vector capable of expressing a desired tRNA. Usually, tRNAs synthesized by in vitro transcription only contain four typical nucleosides: adenosine, guanosine, cytidine, and uridine. On the other hand, tRNAs synthesized in cells may contain modified nucleosides resulting from modification of the typical nucleosides. Alternatively, tRNA can also be prepared by a method in which fragments synthesized by transcription or chemically synthesized fragments or such as described in the Examples below are ligated by an enzymatic reaction.

Aminoacyl-tRNAs can also be prepared by chemical and/or biological synthesis methods. For example, an aminoacyl-tRNA can be synthesized using an aminoacyl-tRNA synthetase (ARS) to attach an amino acid to a tRNA. The amino acid may be either natural amino acid or unnatural amino acid as long as it can serve as a substrate for ARS. Alternatively, a natural amino acid may be attached to a tRNA and then chemically modified. Furthermore, as there are many reports that introducing an amino acid mutation into ARSs enhanced their action on unnatural amino acids (see for example, WO2006/135096, WO2007/061136, WO2007/103307, WO2008/001947, WO2010/141851, and WO2015/120287), such mutated ARSs may be used to attach an amino acid to tRNA. In addition to the method using ARSs, aminoacyl-tRNAs can be synthesized by, for example, removing the CA sequence from the 3' end of tRNA, and ligating an aminoacylated pdCpA (a dinucleotide composed of deoxycytidine and adenosine) to it using RNA ligase (pdCpA method; Hecht et al., J Biol Chem (1978) 253: 4517-4520). A method using pCpA (a dinucleotide composed of cytidine and adenosine) instead of pdCpA is also known (pCpA method; Wang et al., ACS Chem Biol (2015)10: 2187-2192). Specifically, aminoacylated tRNAs can be prepared by using RNA ligase to ligate a pCpA-amino acid to a tRNA lacking the CA sequence at the 3' end. Furthermore, aminoacyl-tRNAs can also be synthesized by attaching an unnatural amino acid previously activated by esterification to a tRNA, using an artificial RNA catalyst (flexizyme) (WO2007/066627; WO2012/026566; H. Murakami et al., Chemistry & Biology, Vol. 10, 2003, 655-662; H. Murakami et al., Chemistry & Biology, Vol. 10, 2003, 1077-1084; H. Murakami et al., Nature Methods 3, 2006, 357-359; N. Niwa et al., Bioorganic & Medicinal Chemistry Letters 19, 2009, 3892-3894). Flexizymes are artificial RNA catalysts capable of linking amino acids or hydroxy acids to tRNA. The flexizymes in the present disclosure include a prototypical flexizyme (Fx), and its altered forms such as dinitrobenzyl flexizyme (dFx), enhanced flexizyme (eFx), and amino flexizyme (aFx).

In some embodiments, an amino acid or amino acid analog is attached to the tRNA of the present disclosure. The amino acid or amino acid analog is usually attached to the 3' end of the tRNA, or more specifically, to the adenosine residue of the CCA sequence at the 3' end. The specific type of the amino acid or amino acid analog attached to the tRNA can be appropriately selected from the amino acids or amino acid analogs mentioned below.

The amino acids in the present disclosure include α-amino acids, β-amino acids, and γ-amino acids. Regarding three-dimensional structures, both L-type amino acids and D-type amino acids are included. Furthermore, amino acids in the present disclosure include natural and unnatural amino acids. In a particular embodiment, the natural amino acids consist of the following 20α-amino acids: glycine (Gly), alanine (Ala), serine (Ser), threonine (Thr), valine (Val), leucine (Leu), isoleucine (Ile), phenylalanine (Phe), tyrosine (Tyr), tryptophan (Trp), histidine (His), glutamic acid (Glu), aspartic acid (Asp), glutamine (Gln), asparagine (Asn), cysteine (Cys), methionine (Met), lysine (Lys), arginine (Arg), and proline (Pro). Alternatively, the natural amino acids in the present disclosure may be those obtained by removing any one or more amino acids from the above-mentioned 20 amino acids. In one embodiment, the natural amino acids consist of 19 amino acids, excluding isoleucine. In one embodiment, the natural amino acids consist of 19 amino acids, excluding methionine. In a further embodiment, the natural amino acids consist of 18 amino acids, excluding isoleucine and methionine. Natural amino acids are usually L-type amino acids.

In the present disclosure, unnatural amino acids refer to all amino acids excluding the above-mentioned natural amino acids consisting of 20α-amino acids. Examples of unnatural amino acids include β-amino acids, γ-amino acids, D-type amino acids, α-amino acids whose side chains differ from natural amino acids, α,α-disubstituted amino acids, and amino acids whose main chain amino group has a substituent (N-substituted amino acids). The side chain of the unnatural amino acid is not particularly limited, but may have, for example, alkyl, alkenyl, alkynyl, aryl, heteroaryl, aralkyl, and cycloalkyl, in addition to the hydrogen atom. Further, in the case of an α,α-disubstituted amino acid, two side chains may form a ring. Furthermore, these side chains may have one or more substituents. In a particular embodiment, the substituents can be selected from any functional group containing a halogen atom, O atom, S atom, N atom, B atom, Si atom, or P atom. For example, in the present disclosure, "C₁-C₆ alkyl having halogen as a substituent" means a "C₁-C₆ alkyl" in which at least one hydrogen atom in an alkyl is substituted with a halogen atom, and specific examples include, trifluoromethyl, difluoromethyl, fluoromethyl, pentafluoroethyl, tetrafluoroethyl, trifluoroethyl, difluoroethyl, fluoroethyl, trichloromethyl, dichloromethyl, chloromethyl, pentachloroethyl, tetrachloroethyl, trichloroethyl, dichloroethyl, and chloroethyl. In addition, for example, "C₅-C₁₀ aryl C₁-C₆ alkyl having a substituent" means "C₅-C₁₀ aryl C₁-C₆ alkyl" in which at least one hydrogen atom in aryl and/or alkyl is substituted with a substituent. Furthermore, the meaning of the phrase "having two or more substituents" includes having a certain functional group (for example, a functional group containing an S atom) as a substituent, and the functional group has another substituent (for example, a substituent such as amino or halogen). For specific examples of unnatural amino acids, one can refer to WO2013/100132, WO2018/143145, and such.

The amino group of the main chain of the unnatural amino acid may be an unsubstituted amino group (NH₂ group) or a substituted amino group (NHR group). Here, R indicates an alkyl, alkenyl, alkynyl, aryl, heteroaryl, aralkyl, or cycloalkyl which optionally has a substituent. Further, like proline, the carbon chain attached to the N atom of the main chain amino group and the α-position carbon atom may form a ring. The substituent can be selected from any functional group containing a halogen atom, O atom, S atom, N atom, B atom, Si atom, or P atom. Examples of alkyl substitution of an amino group include N-methylation, N-ethylation, N-propylation, and N-butylation, and example of aralkyl substitution of an amino group include N-benzylation. Specific examples of an N-methylamino acid include N-methylalanine, N-methylglycine, N-methylphenylalanine, N-methyltyrosine, N-methyl-3-chlorophenylalanine, N-methyl-4-chlorophenylalanine, N-methyl-4-methoxyphenylalanine, N-methyl-4-thiazolealanine, N-methylhistidine, N-methylserine and N-methylaspartic acid.

Examples of a substituent containing a halogen atom include fluoro (-F), chloro (-Cl), bromo (-Br), and iodo (-I).

Examples of a substituent containing an O atom include hydroxyl (-OH), oxy (-OR), carbonyl (-C=O-R), carboxyl (-CO₂H), oxycarbonyl (-C=O-OR), carbonyloxy (-O-C=O-R), thiocarbonyl (-C=O-SR), carbonylthio (-S-C=O-R), aminocarbonyl (-C=O-NHR), carbonyl amino (-NH-C=O-R), oxycarbonyl amino (-NH-C=O-OR), sulfonyl amino (-NH-SO₂-R), aminosulfonyl (-SO₂-NHR), sulfamoyl amino (-NH-SO₂-NHR), thiocarboxyl (-C(=O)-SH), carboxyl carbonyl (-C(=O)-CO₂H).

Examples of oxy (-OR) include alkoxy, cycloalkoxy, alkenyloxy, alkynyloxy, aryloxy, heteroaryloxy, and aralkyloxy.

Examples of carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C=O-OR) include alkyloxycarbonyl, cycloalkyloxycarbonyl, alkenyloxycarbonyl, alkynyloxycarbonyl, aryloxycarbonyl, heteroaryloxy carbonyl, and aralkyloxycarbonyl.

Examples of carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Furthermore, the H atom attached to the N atom in -C=O-NHR may be substituted with a substituent selected from the group consisting of alkyl, cycloalkyl, alkenyl, alkynyl, aryl, heteroaryl, and aralkyl.

Examples of carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Furthermore, the H atom attached to the N atom in -NH-C=O-R may be substituted with a substituent selected from the group consisting of alkyl, cycloalkyl, alkenyl, alkynyl, aryl, heteroaryl, and aralkyl.

Examples of oxycarbonylamino (-NH-C=O-OR) include alkoxycarbonylamino, cycloalkoxycarbonylamino, alkenyloxycarbonylamino, alkynyloxy carbonylamino, aryloxycarbonylamino, heteroaryloxycarbonylamino, and aralkyloxycarbonylamino. Furthermore, the H atom attached to the N atom in -NH-C=O-OR may be substituted with a substituent selected from the group consisting of alkyl, cycloalkyl, alkenyl, alkynyl, aryl, heteroaryl, and aralkyl.

Examples of sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Furthermore, the H atom attached to the N atom in -NH-SO₂-R may be substituted with a substituent selected from the group consisting of alkyl, cycloalkyl, alkenyl, alkynyl, aryl, heteroaryl, and aralkyl.

Examples of aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Furthermore, the H atom attached to the N atom in -SO₂-NHR may be substituted with a substituent selected from the group consisting of alkyl, cycloalkyl, alkenyl, alkynyl, aryl, heteroaryl, and aralkyl.

Examples of sulfamoylamino (-NH-SO₂-NHR) include alkylsulfamoylamino, cycloalkylsulfamoylamino, alkenyl sulfamoyl amino, alkynyl sulfamoyl amino, arylsulfamoylamino, heteroaryl sulfamoyl amino, and aralkylsulfamoylamino. Furthermore, at least one of the two H atoms attached to the N atoms in -NH-SO₂-NHR may be substituted with a substituent selected from the group consisting of alkyl, cycloalkyl, alkenyl, alkynyl, aryl, heteroaryl, and aralkyl. When the two H atoms are both substituted, a substituent may each be independently selected, or these two substituents may form a ring.

Examples of a substituent containing an S atom include thiol (-SH), thio (-S-R), sulfinyl (-S=O-R), sulfonyl (-S(O)₂-R), and sulfo (-SO₃H).

Examples of thio (-S-R) include alkylthio, cycloalkylthio, alkenylthio, alkynylthio, arylthiol, heteroarylthio, and aralkylthio.

Examples of sulfinyl (-S=O-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl (-S(O)₂-R) include alkylsulfonyl, cycloalkylsulfonyl, alkenylsulfonyl, alkynylsulfonyl, arylsulfonyl, heteroarylsulfonyl, and aralkylsulfonyl.

Examples of a substituent containing an N atom include azide (-N₃), cyano (-CN), primary amino (-NH₂), secondary amino (-NH-R), tertiary amino (-NR(R')), amidino (-C(=NH)-NH₂), substituted amidino (-C(=NR)-NR'R"), guanidino (-NH=C(=NH)-NH₂), substituted guanidino (-NR-C(=NR‴)-NR'R"), and aminocarbonylamino (-NR-CO-NR'R").

Examples of the secondary amino (-NH-R) include alkylamino, cycloalkylamino, alkenylamino, alkynylamino, arylamino, heteroarylamino, and aralkylamino.

The two substituents R and R' on the N atom in the tertiary amino (-NR(R')) can each be independently selected from the group consisting of alkyl, cycloalkyl, alkenyl, alkynyl, aryl, heteroaryl, and aralkyl. Examples of the tertiary amino include, for example, alkyl(aralkyl)amino. These two substituents may form a ring.

The three substituents R, R', and R" on the N atom in the substituted amidino (-C(=NR)-NR'R") can each be independently selected from the group consisting of a hydrogen atom, alkyl, cycloalkyl, alkenyl, alkynyl, aryl, heteroaryl, and aralkyl. Examples of the substituted amidino include alkyl(aralkyl)(aryl)amidino. These substituents may together form a ring.

The four substituents R, R', R", and R'" on the N atom in the substituted guanidino (-NR-C(=NR‴)-NR'R") can each be independently selected from the group consisting of a hydrogen atom, alkyl, cycloalkyl, alkenyl, alkynyl, aryl, heteroaryl, and aralkyl. These substituents may together form a ring.

The three substituents R, R', and R" on the N atom in the aminocarbonylamino (-NR-CO-NR'R") can each be independently selected from the group consisting of a hydrogen atom, alkyl, cycloalkyl, alkenyl, alkynyl, aryl, heteroaryl, and aralkyl. These substituents may together form a ring.

Examples of a substituent containing a B atom include boryl (-BR(R')) and dioxyboryl (-B(OR)(OR')). The two substituents R and R' on the B atom can each be independently selected from the group consisting of a hydrogen atom, alkyl, cycloalkyl, alkenyl, alkynyl, aryl, heteroaryl, and aralkyl. These substituents may together form a ring.

In some embodiments, examples of the amino acids in the present disclosure include nBuG (2-(butylamino)acetic acid), Pic2 ((2S)-piperidine-2-carboxylic acid), dA ((2R)-2-aminopropanoic acid), MeA3Pyr ((2S)-2-(methylamino)-3-(3-pyridyl)propanoic acid), StBuOH ((2S)-3-(2-hydroxy-2-methyl-propoxy)-2-(methylamino)propanoic acid), MeSnPr ((2S)-2-(methylamino)-3-propoxy-propanoic acid), SPh2Cl ((2S)-2-amino-3-(2-chlorophenoxy)propanoic acid), MeHph ((2S)-2-(methylamino)-4-phenyl-butanoic acid), and Ile.

Examples of amino acid analogs in the present disclosure include hydroxycarboxylic acid (hydroxy acid). The hydroxycarboxylic acid includes α-hydroxycarboxylic acid, β-hydroxycarboxylic acid, and γ-hydroxycarboxylic acid. A side chain other than a hydrogen atom may be attached to the carbon at the α-position in the hydroxycarboxylic acid, as with amino acids. Regarding three-dimensional structures, both the L-type and D-type can be included. The structure of the side chain can be defined similarly to the side chain of the above-mentioned natural amino acid or unnatural amino acid. Examples of hydroxycarboxylic acids include hydroxyacetic acid, lactic acid, and phenyllactic acid.

The amino acid in the present disclosure may be a translatable amino acid, and the amino acid analog may be a translatable amino acid analog. As used herein, a "translatable" amino acid or amino acid analog (may be collectively referred to as an amino acid or the like) means amino acids and the like that can be incorporated into a peptide by translational synthesis (for example, using the translation system described in this disclosure). Whether a certain amino acid or the like is translatable can be confirmed by a translation synthesis experiment using a tRNA to which the amino acid or the like is attached. A reconstituted cell-free translation system may be used in the translation synthesis experiment (see for example, WO2013100132).

The unnatural amino acid or amino acid analog according to the present disclosure can be prepared by a conventionally known chemical synthesis method, a synthesis method described in the later-discussed Examples, or a synthesis method similar thereto.

In some embodiments, tRNAs of the present disclosure can discriminate between a codon represented by M₁M₂A and a codon represented by M₁M₂G, and translate these codons into different amino acids or amino acid analogs respectively. In some embodiments, tRNAs of the present disclosure can discriminate among a codon represented by M₁M₂U, a codon represented by M₁M₂A, and a codon represented by M₁M₂G, and translate these codons into different amino acids or amino acid analogs respectively. In some embodiments, tRNAs of the present disclosure can discriminate among a codon represented by M₁M₂C, a codon represented by M₁M₂A, and a codon represented by M₁M₂G, and translate these codons into different amino acids or amino acid analogs respectively. Here, the nucleoside of the first letter (Mi) and the nucleoside of the second letter (M₂) of the codon are each independently selected from any of adenosine (A), guanosine (G), cytidine (C), and uridine (U). In another embodiment, a tRNA of the present disclosure has an anticodon complementary to a specific codon represented by M₁M₂A, or a specific codon represented by M₁M₂G. In another embodiment, a tRNA of the present disclosure has an anticodon complementary to a specific codon represented by M₁M₂U, a specific codon represented by M₁M₂A, or a specific codon represented by M₁M₂G. In another embodiment, a tRNA of the present disclosure has an anticodon complementary to a specific codon represented by M₁M₂C, a specific codon represented by M₁M₂A, or a specific codon represented by M₁M₂G. In a further embodiment, the nucleoside of the third letter (N₃) and the nucleoside of the second letter (N₂) of the anticodon in a tRNA can be selected as nucleosides complementary to M₁ and Mz, respectively, and N₂ and N₃ may be each independently selected from any of adenosine (A), guanosine (G), cytidine (C), and uridine (U). Specifically, when M₂ (or Mi) is adenosine, N₂ (or N₃) is uridine. When M₂ (or Mi) is guanosine, N₂ (or N₃) is cytidine. When M₂ (or M₁) is cytidine, N₂ (or N₃) is guanosine. When M₂ (or Mi) is uridine, N₂ (or N₃) is adenosine.

In the context of the present disclosure, the embodiment "a certain tRNA is capable of translating a specific codon" essentially includes the embodiment "a certain tRNA has an anticodon complementary to the specific codon," and as long as the sequence of the anticodon on the tRNA is referred to, these expressions can be used interchangeably.

The nucleoside of the first letter (Mi) and the nucleoside of the second letter (M₂) of the codon translatable by a tRNA constituting the translation system of the present disclosure can be selected from the nucleoside of the first letter (Mi) and the nucleoside of the second letter (M₂) of codons constituting a specific codon box in the genetic code table, respectively. In a particular embodiment, the genetic code table is a standard genetic code table. In another embodiment, the genetic code table is the natural genetic code table.

In another embodiment, M₁ and M₂ of the present disclosure can be selected from a codon box in which a stop codon is assigned to a codon represented by M₁M₂A and an amino acid is assigned to a codon represented by M₁M₂G in the natural genetic code table. In another embodiment, M₁ and M₂ of the present disclosure can be selected from a codon box in which stop codons are assigned to both a codon represented by M₁M₂A and a codon represented by M₁M₂G in the natural genetic code table.

In some embodiments, a translation system in which the genetic code table is the same as the natural genetic code table is excluded from the translation system of the present disclosure. In some embodiments, a translation system in which the genetic code table is the same as the genetic code table shown in Table 1 is excluded from the translation system of the present disclosure.

In one embodiment, M₁ and M₂ can be selected from M₁ and M₂, respectively, in codons constituting a codon box in which a codon having A as the third letter and a codon having G as the third letter both encode the same amino acid. As an example, in the codon box whose codons are represented by UUM₃, the codon having A as the third letter (UUA) and the codon having G as the third letter (UUG) both encode the same amino acid (Leu); therefore, the nucleoside of the first letter (U) and the nucleoside of the second letter (U) in codons constituting this codon box can be selected as M₁ and M₂, respectively.

In one embodiment, M₁ and M₂ can be selected from M₁ and M₂, respectively, in codons constituting a codon box in which a codon having U as the third letter and a codon having A as the third letter both encode the same amino acid. As an example, in the codon box whose codons are represented by AUM₃, the codon having U as the third letter (AUU) and the codon having A as the third letter (AUA) both encode the same amino acid (Ile); therefore, the nucleoside of the first letter (A) and the nucleoside of the second letter (U) in codons constituting this codon box can be selected as M₁ and M₂, respectively.

In one embodiment, M₁ and M₂ can be selected from M₁ and M₂, respectively, in codons constituting a codon box in which a codon having A as the third letter and a codon having G as the third letter encode different amino acids from each other. As an example, in the codon box whose codons are represented by AUM₃, the codon having A as the third letter (AUA) and the codon having G as the third letter (AUG) encode different amino acids from each other (Ile and Met); therefore, the nucleoside of the first letter (A) and the nucleoside of the second letter (U) in codons constituting this codon box can be selected as M₁ and M₂, respectively.

In one embodiment, M₁ and M₂ can be selected from M₁ and M₂, respectively, in codons constituting a codon box in which a codon having A as the third letter and/or a codon having G as the third letter are stop codons. As an example, in the codon box whose codons are represented by UGM₃, the codon having A as the third letter (UGA) is a stop codon (opal); therefore, the nucleoside of the first letter (U) and the nucleoside of the second letter (G) in codons constituting this codon box can be selected as M₁ and M₂, respectively.

In further embodiments, M₁ and M₂ may be selected from M₁ and M₂, respectively, in codons constituting a codon box whose codons are represented by UUM₃. Specifically, the nucleoside of the first letter (U) and the nucleoside of the second letter (U) in the codons can be selected as M₁ and M₂, respectively.

In further embodiments, M₁ and M₂ may be selected from M₁ and M₂, respectively, in codons constituting a codon box whose codons are represented by UAMs. Specifically, the nucleoside of the first letter (U) and the nucleoside of the second letter (A) in the codons can be selected as M₁ and M₂, respectively.

In further embodiments, M₁ and M₂ may be selected from M₁ and M₂, respectively, in codons constituting a codon box whose codons are represented by UGM₃. Specifically, the nucleoside of the first letter (U) and the nucleoside of the second letter (G) in the codons can be selected as M₁ and M₂, respectively.

In further embodiments, M₁ and M₂ may be selected from M₁ and M₂, respectively, in codons constituting a codon box whose codons are represented by CAMs. Specifically, the nucleoside of the first letter (C) and the nucleoside of the second letter (A) in the codons can be selected as M₁ and M₂, respectively.

In further embodiments, M₁ and M₂ may be selected from M₁ and M₂, respectively, in codons constituting a codon box whose codons are represented by CGMs. Specifically, the nucleoside of the first letter (C) and the nucleoside of the second letter (G) in the codons can be selected as M₁ and M₂, respectively.

In further embodiments, M₁ and M₂ may be selected from M₁ and M₂, respectively, in codons constituting a codon box whose codons are represented by AUM₃. Specifically, the nucleoside of the first letter (A) and the nucleoside of the second letter (U) in the codons can be selected as M₁ and M₂, respectively.

In further embodiments, M₁ and M₂ may be selected from M₁ and M₂, respectively, in codons constituting a codon box whose codons are represented by ACM₃. Specifically, the nucleoside of the first letter (A) and the nucleoside of the second letter (C) in the codons can be selected as M₁ and M₂, respectively.

In further embodiments, M₁ and M₂ may be selected from M₁ and M₂, respectively, in codons constituting a codon box whose codons are represented by AAM₃. Specifically, the nucleoside of the first letter (A) and the nucleoside of the second letter (A) in the codons can be selected as M₁ and M₂, respectively.

In further embodiments, M₁ and M₂ may be selected from M₁ and M₂, respectively, in codons constituting a codon box whose codons are represented by AGM₃. Specifically, the nucleoside of the first letter (A) and the nucleoside of the second letter (G) in the codons can be selected as M₁ and M₂, respectively.

In further embodiments, M₁ and M₂ may be selected from M₁ and M₂, respectively, in codons constituting a codon box whose codons are represented by GAMs. Specifically, the nucleoside of the first letter (G) and the nucleoside of the second letter (A) in the codons can be selected as M₁ and M₂, respectively.

The nucleoside of the third letter (N₃) and the nucleoside of the second letter (N₂) of the anticodon in the tRNA of the present disclosure may be selected as nucleosides complementary to M₁ and M₂, respectively.

In one embodiment, N₃ and N₂ may be selected as nucleosides complementary to the nucleoside of the first letter (U) and the nucleoside of the second letter (U), respectively, in codons constituting a codon box whose codons are represented by UUM₃. Specifically, A can be selected as N₃ and A can be selected as N₂.

In one embodiment, N₃ and N₂ may be selected as nucleosides complementary to the nucleoside of the first letter (U) and the nucleoside of the second letter (A), respectively, in codons constituting a codon box whose codons are represented by UAM₃. Specifically, A can be selected as N₃ and U can be selected as N₂.

In one embodiment, N₃ and N₂ may be selected as nucleosides complementary to the nucleoside of the first letter (U) and the nucleoside of the second letter (G), respectively, in codons constituting a codon box whose codons are represented by UGM₃. Specifically, A can be selected as N₃ and C can be selected as N₂.

In one embodiment, N₃ and N₂ may be selected as nucleosides complementary to the nucleoside of the first letter (C) and the nucleoside of the second letter (A), respectively, in codons constituting a codon box whose codons are represented by CAMs. Specifically, G can be selected as N₃ and U can be selected as N₂.

In one embodiment, N₃ and N₂ may be selected as nucleosides complementary to the nucleoside of the first letter (C) and the nucleoside of the second letter (G), respectively, in codons constituting a codon box whose codons are represented by CGM₃. Specifically, G can be selected as N₃ and C can be selected as N₂.

In one embodiment, N₃ and N₂ may be selected as nucleosides complementary to the nucleoside of the first letter (A) and the nucleoside of the second letter (U), respectively, in codons constituting a codon box whose codons are represented by AUM₃. Specifically, U can be selected as N₃ and A can be selected as N₂.

In one embodiment, N₃ and N₂ may be selected as nucleosides complementary to the nucleoside of the first letter (A) and the nucleoside of the second letter (C), respectively, in codons constituting a codon box whose codons are represented by ACM₃. Specifically, U can be selected as N₃ and G can be selected as N₂.

In one embodiment, N₃ and N₂ may be selected as nucleosides complementary to the nucleoside of the first letter (A) and the nucleoside of the second letter (A), respectively, in codons constituting a codon box whose codons are represented by AAM₃. Specifically, U can be selected as N₃ and U can be selected as N₂.

In one embodiment, N₃ and N₂ may be selected as nucleosides complementary to the nucleoside of the first letter (A) and the nucleoside of the second letter (G), respectively, in codons constituting a codon box whose codons are represented by AGM₃. Specifically, U can be selected as N₃ and C can be selected as N₂.

In one embodiment, N₃ and N₂ may be selected as nucleosides complementary to the nucleoside of the first letter (G) and the nucleoside of the second letter (A), respectively, in codons constituting a codon box whose codons are represented by GAM₃. Specifically, C can be selected as N₃ and U can be selected as N₂.

In some embodiments, a tRNA having an anticodon complementary to a codon represented by M₁M₂A in the present disclosure can translate the codon represented by M₁M₂A selectively over other codons. The other codons may be codons different from the codon represented by M₁M₂A; for example, a codon represented by M₁M₂U, M₁M₂C, or M₁M₂G. In certain embodiments, the tRNA of the present disclosure having an anticodon complementary to a codon represented by M₁M₂A can translate the codon represented by M₁M₂A selectively over all the codons represented by M₁M₂U, M₁M₂C, and M₁M₂G.

In one embodiment of the present disclosure, "a tRNA can translate the M₁M₂A codon selectively" means that [the amount of translation on the M₁M₂A codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on another codon by the tRNA]. As an example, whether or not a certain tRNA can selectively translate the codon represented by CUA can be judged by whether [the amount of translation on the CUA codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUG codon by the tRNA].

Comparing the amount of translation of a specific codon (for example, M₁M₂A) and the amount of translation of another codon (for example, M₁M₂G) can be carried out by, for example, preparing a peptide-encoding mRNA that contains a M₁M₂A codon and another mRNA having the same nucleic acid sequence as the aforementioned mRNA except that the M₁M₂A codon has been replaced with a M₁M₂G codon, translating those two mRNAs under the same conditions, and comparing the amounts of two synthesized peptides obtained.

In another embodiment, a codon represented by M₁M₂A may be translated more selectively by a tRNA having an anticodon complementary to a codon represented by M₁M₂A in the present disclosure than by other tRNA. The other tRNA may be a tRNA having an anticodon complementary to a codon different from the codon represented by M₁M₂A, for example, a tRNA having an anticodon complementary to the M₁M₂U, M₁M₂C, or M₁M₂G codon. In a particular embodiment, the codon represented by M₁M₂A may be more selectively translated by the tRNA having an anticodon complementary to the codon represented by M₁M₂A in the present disclosure than by all of the tRNA having an anticodon complementary to the M₁M₂U codon, the tRNA having an anticodon complementary to the M₁M₂C codon, and the tRNA having an anticodon complementary to the M₁M₂G codon.

In one embodiment of the present disclosure, "a codon represented by M₁M₂A may be selectively translated by a tRNA" means that [the amount of translation on the M₁M₂A codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the M₁M₂A codon by other tRNAs]. As an example, whether or not the codon represented by CUA can be selectively translated by a certain tRNA can be judged by whether [the amount of translation on the CUA codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUA codon by a tRNA having an anticodon complementary to the CUG codon (for example, a tRNA having the CAG anticodon)].

A translation system of the present disclosure may have both of the above two characteristics. That is, in a particular embodiment, in the translation system of the present disclosure, (i) the tRNA having an anticodon complementary to a codon represented by M₁M₂A can translate the codon represented by M₁M₂A selectively over other codons, and (ii) the codon represented by M₁M₂A may be translated by the tRNA having an anticodon complementary to the codon represented by M₁M₂A more selectively than other tRNAs. When such a relationship is established, in the translation system of the present disclosure, the peptide translation using the tRNA having an anticodon complementary to the codon represented by M₁M₂A and the peptide translation using other tRNAs are in an independent relationship where they do not interact with each other; in other words, an orthogonal relationship. The translation system of the organisms in nature essentially has strict correspondences established between codons and amino acids; therefore, addition of a non-orthogonal tRNA to it may disturb these correspondences, and lead to a fatal effect on the function of the translation system. Therefore, in the translation system of the present disclosure, the orthogonality established between the tRNA having an anticodon complementary to the codon represented by M₁M₂A and other tRNAs may be one of the important features.

In one embodiment, the translation system in this disclosure comprises at least two tRNAs: (a) a tRNA having an anticodon complementary to a codon represented by M₁M₂A and (b) a tRNA having an anticodon complementary to a codon represented by M₁M₂G. The tRNA having an anticodon complementary to a codon represented by M₁M₂A and the tRNA having an anticodon complementary to a codon represented by M₁M₂G in the present disclosure may have the same nucleic acid sequence except for the anticodon, or may have different nucleic acid sequences. When the nucleic acid sequences other than the anticodon are the same, the physicochemical properties of these two tRNAs may be similar to each other; therefore, a translation system with more homogeneous and stable reactivity may be constructed.

In some embodiments, the tRNA having an anticodon complementary to a codon represented by M₁M₂G in the present disclosure can selectively translate the codons represented by M₁M₂G over other codons. The other codons may be codons different from the codons represented by M₁M₂G; for example, codons represented by M₁M₂U, M₁M₂C, or M₁M₂A. In certain embodiments, the tRNA having an anticodon complementary to a codon represented by M₁M₂G in the present disclosure can translate the codon represented by M₁M₂G selectively over all the codons represented by M₁M₂U, M₁M₂C, and M₁M₂A.

In one embodiment of the present disclosure, a certain tRNA can selectively translate the M₁M₂G codon means that [the amount of translation on the M₁M₂G codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the other codons by the tRNA]. As an example, whether or not a certain tRNA can selectively translate the codon represented by CUG can be judged by observing whether [the amount of translation on the CUG codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUA codon by the tRNA].

In another embodiment, the codon represented by M₁M₂G may be translated more selectively by a tRNA having an anticodon complementary to the codon represented by M₁M₂G in the present disclosure than by other tRNAs. The other tRNAs may be tRNAs having anticodons complementary to codons different from the codon represented by M₁M₂G, for example, a tRNA having an anticodon complementary to the M₁M₂U, M₁M₂C, or M₁M₂A codon. In a particular embodiment, the codon represented by M₁M₂G may be more selectively translated by the tRNA having an anticodon complementary to the codon represented by M₁M₂G in the present disclosure than by all of the tRNA having an anticodon complementary to the M₁M₂U codon, the tRNA having an anticodon complementary to the M₁M₂C codon, and the tRNA having an anticodon complementary to the M₁M₂A codon.

In one embodiment of the present disclosure, the codon represented by M₁M₂G may be selectively translated by a certain tRNA means that [the amount of translation on the M₁M₂G codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the M₁M₂G codon by other tRNAs]. As an example, whether or not the codon represented by CUG can be selectively translated by a certain tRNA can be judged by observing whether [the amount of translation on the CUG codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUG codon by a tRNA having an anticodon complementary to the CUA codon (for example, a tRNA that has the UAG anticodon].

A translation system of the present disclosure may have the above two characteristics in combination. That is, in a particular embodiment, in the translation system of the present disclosure, (i) the tRNA having an anticodon complementary to a codon represented by M₁M₂G can selectively translate the codon represented by M₁M₂G over other codons, and (ii) the codon represented by M₁M₂G may be translated by the tRNA having an anticodon complementary to the codon represented by M₁M₂G more selectively than other tRNAs. When such a relationship is established, in the translation system of the present disclosure, the peptide translation using the tRNA having an anticodon complementary to the codon represented by M₁M₂G and the peptide translation using other tRNAs are independent and do not interact with each other; in other words, they have an orthogonal relationship. In the translation system of the present disclosure, establishment of orthogonality between the tRNA having an anticodon complementary to the codon represented by M₁M₂A and other tRNAs may be one of the important features.

In a further embodiment, an amino acid attached to the tRNA having an anticodon complementary to the codon represented by M₁M₂A (hereinafter, this amino acid is also referred to as "amino acid-A") and an amino acid attached to the tRNA having an anticodon complementary to the codon represented by M₁M₂G (hereinafter, this amino acid is also referred to as "amino acid-G") in the present disclosure are different from each other. Regarding the tRNA having an anticodon complementary to the codon represented by M₁M₂A and the tRNA having an anticodon complementary to the codon represented by M₁M₂G in the present disclosure, when the above-mentioned orthogonal relationship is established, the M₁M₂A codon and amino acid-A, and the M₁M₂G codon and amino acid-G each have a one-to-one correspondence in the present translation system. That is, in the translation system of the present disclosure, two different amino acids can be translated from two codons, (i) M₁M₂A and (ii) M₁M₂G, in the same codon box.

In one embodiment, the translation system in the present disclosure comprises at least three tRNAs, which are (a) a tRNA having an anticodon complementary to the codon represented by M₁M₂A, (b) a tRNA having an anticodon complementary to the codon represented by M₁M₂G, and (c) a tRNA having an anticodon complementary to the codon represented by M₁M₂U. In the tRNA having an anticodon complementary to the codon represented by M₁M₂A, the tRNA having an anticodon complementary to the codon represented by M₁M₂G, and the tRNA having an anticodon complementary to the codon represented by M₁M₂U of the present disclosure, all of the nucleotide sequences other than the anticodon may be the same or different from each other. When the nucleotide sequences other than the anticodon are the same, the physicochemical properties of these three tRNAs may be similar to each other; therefore, a translation system with more homogeneous and stable reactivity may be constructed.

In some embodiments, the tRNA having an anticodon complementary to the codon represented by M₁M₂U in the present disclosure can selectively translate the codon represented by M₁M₂U over other codons. The other codons may be codons different from the codon represented by M₁M₂U; for example, a codon represented by either M₁M₂A or M₁M₂G. In a particular embodiment, the tRNA having an anticodon complementary to the codon represented by M₁M₂U in the present disclosure can translate the codon represented by M₁M₂U selectively over all the codons represented by M₁M₂A and M₁M₂G.

In one embodiment of the present disclosure, a certain tRNA can selectively translate the M₁M₂U codon means that [the amount of translation of the M₁M₂U codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation of the other codons by the tRNA]. As an example, whether or not a certain tRNA can selectively translate the codon represented by CUU can be judged by observing whether [the amount of translation of the CUU codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation of the CUA codon by the tRNA].

In another embodiment, the codon represented by M₁M₂U may be translated more selectively by a tRNA having an anticodon complementary to the codon represented by M₁M₂U in the present disclosure than by other tRNAs. The other tRNAs may be tRNAs having anticodons complementary to codons different from the codon represented by M₁M₂U, for example, a tRNA having an anticodon complementary to the M₁M₂A or M₁M₂G codon. In a particular embodiment, the codon represented by M₁M₂U may be more selectively translated by the tRNA having an anticodon complementary to the codon represented by M₁M₂U in the present disclosure than by all of the tRNA having an anticodon complementary to the M₁M₂A codon and the tRNA having an anticodon complementary to the M₁M₂G codon.

In one embodiment of the present disclosure, "the codon represented by M₁M₂U may be selectively translated by a certain tRNA" means that [the amount of translation of the M₁M₂U codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation of the M₁M₂U codon by other tRNAs]. As an example, whether or not the codon represented by CUU can be selectively translated by a certain tRNA can be judged by observing whether [the amount of translation of the CUU codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation of the CUU codon by a tRNA having an anticodon complementary to the CUA codon (for example, a tRNA that has the UAG anticodon)].

A translation system of the present disclosure may have the above two characteristics in combination. That is, in a particular embodiment, in the translation system of the present disclosure, (i) the tRNA having an anticodon complementary to the codon represented by M₁M₂U may selectively translate the codon represented by M₁M₂U over other codons, and (ii) the codon represented by M₁M₂U may be translated by the tRNA having an anticodon complementary to the codon represented by M₁M₂U more selectively than other tRNAs. When such a relationship is established, in the translation system of the present disclosure, the peptide translation using the tRNA having an anticodon complementary to the codon represented by M₁M₂U and the peptide translation using other tRNAs are independent and do not interact with each other; in other words, they have an orthogonal relationship. In the translation system of the present disclosure, establishment of orthogonality between the tRNA having an anticodon complementary to the codon represented by M₁M₂U and other tRNAs may be one of the important features.

In a further embodiment, an amino acid attached to the tRNA having an anticodon complementary to the codon represented by M₁M₂A ("amino acid-A"), an amino acid attached to the tRNA having an anticodon complementary to the codon represented by M₁M₂G ("amino acid-G"), and an amino acid attached to the tRNA having an anticodon complementary to the codon represented by M₁M₂U (hereinafter, this amino acid is also referred to as "amino acid-U") in the present disclosure, are different from one another. Regarding the tRNA having an anticodon complementary to the codon represented by M₁M₂A, the tRNA having an anticodon complementary to the codon represented by M₁M₂G, and the tRNA having an anticodon complementary to the codon represented by M₁M₂U in the present disclosure, when the above-mentioned orthogonal relationship is established, the M₁M₂A codon and amino acid-A, the M₁M₂G codon and amino acid-G, and the M₁M₂U codon and amino acid-U each have a one-to-one correspondence in the present translation system. That is, in the translation system of the present disclosure, three different amino acids can be translated from three codons, (i) M₁M₂A, (ii) M₁M₂G, and (iii) M₁M₂U, in the same codon box. Alternatively, in the translation system of the present disclosure, three different amino acids can be translated from a codon box composed of M₁M₂U, M₁M₂C, M₁M₂A, and M₁M₂G.

In one embodiment, the translation system in the present disclosure comprises at least three tRNAs, which are (a) a tRNA having an anticodon complementary to a codon represented by M₁M₂A, (b) a tRNA having an anticodon complementary to a codon represented by M₁M₂G, and (c) a tRNA having an anticodon complementary to a codon represented by M₁M₂C. The tRNA having an anticodon complementary to a codon represented by M₁M₂A, the tRNA having an anticodon complementary to a codon represented by M₁M₂G, and the tRNA having an anticodon complementary to a codon represented by M₁M₂C in the present disclosure may have the same nucleic acid sequence except for the anticodon, or they may have different nucleic acid sequences from one another. When the nucleic acid sequences other than the anticodon are the same, the physicochemical properties of these three tRNAs may be similar to each other; therefore, a translation system with more homogeneous and stable reactivity may be constructed.

In some embodiments, the tRNA having an anticodon complementary to a codon represented by M₁M₂C in the present disclosure can selectively translate the codon represented by M₁M₂C over other codons. The other codons may be codons different from the codon represented by M₁M₂C; for example, a codon represented by M₁M₂A or M₁M₂G. In a particular embodiment, the tRNA having an anticodon complementary to a codon represented by M₁M₂C of the present disclosure can translate the codon represented by M₁M₂C selectively over all the codons represented by M₁M₂A and M₁M₂G.

In one embodiment of the present disclosure, a certain tRNA can selectively translate the M₁M₂C codon means that [the amount of translation on the M₁M₂C codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the other codons by the tRNA]. As an example, whether or not a certain tRNA can selectively translate the codon represented by CUC can be judged by observing whether [the amount of translation on the CUC codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUA codon by the tRNA].

In another embodiment, the codon represented by M₁M₂C may be translated more selectively by a tRNA having an anticodon complementary to the codon represented by M₁M₂C in the present disclosure than by other tRNAs. The other tRNAs may be tRNAs having anticodons complementary to codons different from the codon represented by M₁M₂C, for example, a tRNA having an anticodon complementary to the M₁M₂A or M₁M₂G codon. In a particular embodiment, the codon represented by M₁M₂C may be more selectively translated by the tRNA having an anticodon complementary to the codon represented by M₁M₂C in the present disclosure than by all of the tRNA having an anticodon complementary to the M₁M₂A codon and the tRNA having an anticodon complementary to the M₁M₂G codon.

In one embodiment of the present disclosure, the codon represented by M₁M₂C may be selectively translated by a certain tRNA means that [the amount of translation on the M₁M₂C codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the M₁M₂C codon by other tRNAs]. As an example, whether or not the codon represented by CUC can be selectively translated by a certain tRNA can be judged by observing whether [the amount of translation on the CUC codon by the tRNA] is, for example, not less than twice, not less than 3 times, not less than 4 times, not less than 5 times, not less than 6 times, not less than 7 times, not less than 8 times, not less than 9 times, not less than 10 times, not less than 15 times, not less than 20 times, not less than 30 times, not less than 40 times, not less than 50 times, not less than 60 times, not less than 70 times, not less than 80 times, not less than 90 times, or not less than 100 times [the amount of translation on the CUC codon by a tRNA having an anticodon complementary to the CUA codon (for example, a tRNA that has the UAG anticodon].

A translation system of the present disclosure may have the above two characteristics in combination. That is, in a particular embodiment, in the translation system of the present disclosure, (i) the tRNA having an anticodon complementary to the codon represented by M₁M₂C can selectively translate the codon represented by M₁M₂C over other codons, and (ii) the codon represented by M₁M₂C may be translated by the tRNA having an anticodon complementary to the codon represented by M₁M₂C more selectively than other tRNAs. When such a relationship is established, in the translation system of the present disclosure, the peptide translation using the tRNA having an anticodon complementary to the codon represented by M₁M₂C and the peptide translation using other tRNAs are independent and do not interact with each other; in other words, they have an orthogonal relationship. In the translation system of the present disclosure, establishment of orthogonality between the tRNA having an anticodon complementary to the codon represented by M₁M₂C and other tRNAs may be one of the important features.

In a further embodiment, an amino acid attached to the tRNA having an anticodon complementary to the codon represented by M₁M₂A ("amino acid-A"), an amino acid attached to the tRNA having an anticodon complementary to the codon represented by M₁M₂G ("amino acid-G"), and an amino acid attached to the tRNA having an anticodon complementary to the codon represented by M₁M₂C (hereinafter, this amino acid is referred to as "amino acid-C") in the present disclosure are different from one another. Regarding the tRNA having an anticodon complementary to the codon represented by M₁M₂A, the tRNA having an anticodon complementary to the codon represented by M₁M₂G, and the tRNA having an anticodon complementary to the codon represented by M₁M₂C in the present disclosure, when the above-mentioned orthogonal relationship is established, the M₁M₂A codon and amino acid-A, the M₁M₂G codon and amino acid-G, and the M₁M₂C codon and amino acid-C each have a one-to-one correspondence in the present translation system. That is, in the translation system of the present disclosure, three different amino acids can be translated from three codons, (i) M₁M₂A, (ii) M₁M₂G, and (iii) M₁M₂C in the same codon box. Alternatively, in the translation system of the present disclosure, three different amino acids can be translated from a codon box composed of M₁M₂U, M₁M₂C, M₁M₂A, and M₁M₂G.

In some embodiments, an unnatural amino acid may be attached to at least one of the tRNA having an anticodon complementary to the codon represented by M₁M₂A, the tRNA having an anticodon complementary to the codon represented by M₁M₂G, and the tRNA having an anticodon complementary to the codon represented by M₁M₂C in the present disclosure.

In some embodiments, the tRNAs of the present disclosure may be assigned to codons that constitute at least one codon box in the genetic code table. In a further embodiment, the tRNAs of the present disclosure may be assigned to codons that constitute multiple codon boxes in the genetic code table. The multiple codon boxes may be, for example, 2, 3, 4, 5, 6, 7, 8, 9, 10, 11, 12, 13, 14, 15, or 16 codon boxes. To which codon box-constituting codon each tRNA will be assigned is determined by the nucleoside of the second letter (N₂) and the nucleoside of the third letter (N₃) of the anticodon carried by the tRNA. The tRNAs assigned to codons that constitute different codon boxes have different N₂ and N₃. Further, the tRNAs assigned to the codons constituting different codon boxes may have the same nucleic acid sequence except for the anticodon, or they may have different nucleic acid sequences from each other. When the nucleic acid sequences other than the anticodon are the same, the physicochemical properties of these tRNAs may be similar to each other; therefore, a translation system with more homogeneous and stable reactivity may be constructed.

In a particular embodiment, the tRNAs of the present disclosure may be assigned to codons that constitute at least one codon box selected from the following (i) to (x) in the genetic code table. In a further embodiment, the tRNAs of the present disclosure may be assigned to codons that constitute at least 2, 3, 4, 5, 6, 7, 8, 9, or 10 codon boxes selected from the following (i) to (x) in the genetic code table:
(i) a codon box whose codons are represented by UUM₃;
(ii) a codon box whose codons are represented by UAMs;
(iii) a codon box whose codons are represented by UGMs;
(iv) a codon box whose codons are represented by CAMs;
(v) a codon box whose codons are represented by CGMs;
(vi) a codon box whose codons are represented by AUMs;
(vii) a codon box whose codons are represented by ACM₃;
(viii) a codon box whose codons are represented by AAM₃;
(ix) a codon box whose codons are represented by AGM₃; and
(x) a codon box whose codons are represented by GAM₃.

In a particular embodiment, the tRNAs of the present disclosure may be assigned to codons that constitute at least one codon box selected from the following (i) to (vii) in the genetic code table. In a further embodiment, the tRNAs of the present disclosure may be assigned to codons that constitute at least 2, 3, 4, 5, 6, or 7 codon boxes selected from the following (i) to (vii) in the genetic code table:
(i) a codon box whose codons are represented by UUM₃;
(ii) a codon box whose codons are represented by UGM₃;
(iii) a codon box whose codons are represented by CAM₃;
(iv) a codon box whose codons are represented by CGM₃;
(v) a codon box whose codons are represented by AAM₃;
(vi) a codon box whose codons are represented by AGM₃; and
(vii) a codon box whose codons are represented by GAM₃.

In some embodiments, any tRNAs can be assigned to codons constituting the remaining codon boxes to which the tRNAs of the present disclosure are not assigned. Desirably, to the codons constituting the remaining codon boxes, a set of arbitrary tRNAs that can translate the respective codons into certain amino acids are assigned. Such a set of tRNAs may be natural tRNAs or artificially synthesized tRNAs.

In some embodiments, 1, 2, 3, 4, 5, 6, 7, 8, 9, 10, 11, 12, 13, 14, 15, 16, 17, 18, 19, or 20 types of amino acids or amino acid analogs can be translated from the translation system of the present disclosure. Alternatively, more than 20 amino acids or amino acid analogs can be translated by discriminating the M₁M₂A and M₁M₂G codons, the M₁M₂U, M₁M₂A, and M₁M₂G codons, or the M₁M₂C, M₁M₂A, and M₁M₂G codons in a single codon box using the tRNAs of the present disclosure. In a further embodiment, for example, 21, 22, 23, 24, 25, 26, 27, 28, 29, 30, 31, 32, 33, 34, 35, 36, 37, 38, 39, 40, 41, 42, 43, 44, 45, 46, 47, or 48 types of amino acids or amino acid analogs can be translated from the translation system of the present disclosure.

In some embodiments, the translation system of the present disclosure is a cell-free translation system. In a further embodiment, the translation system of the present disclosure is a reconstituted cell-free translation system. As the cell extract solution in the cell-free translation system and the factors required for peptide translation (for example, ribosome), those derived from various biological materials can be used. Examples of such biological materials include E. *coli,* yeast, wheat germ, rabbit reticulocytes, HeLa cells, and insect cells. In some embodiments, the cell-free translation system of the present disclosure includes E. coli-derived ribosomes.

In some embodiments, the translation system of the present disclosure may contain a tRNA per codon (tRNA corresponding to each codon) at a concentration within a range that can be specified by a lower limit selected from the group consisting of 0.8 µM, 1.6 µM, 2.4 µM, 3.2 µM, 4.0 µM, 4.8 µM, 5.6 µM, 6.4 µM, and 10 µM, and an upper limit selected from the group consisting of 100 µM, 150 µM, 200 µM, 250 µM, 300 µM, 350 µM, 400 µM, 450 µM, 500 µM, 550 µM, 600 µM, 650 µM, 700 µM, 750 µM, 800 µM, 850 µM, 900 µM, 950 µM, and 1000 µM; specifically, for example, at a concentration of 0.8-1000 µM, preferably 1.6-500 µM, more preferably 3.2-250 µM, even more preferably 6.4-150 µM, and particularly preferably 10-100 µM.

In a particular embodiment, codons in the translation system of the present disclosure are codons in which M₁ is uridine (U) and M₂ is uridine (U). That is, the tRNAs of the present disclosure can be assigned to a codon box whose codons are represented by UUM₃. Use of a translation system comprising the tRNAs of the present disclosure enables selective translation of two types of amino acids from the combination of UUA and UUG codons. Alternatively, three types of amino acids can be selectively translated from the combination of UUU, UUA, and UUG codons, or the combination of UUC, UUA, and UUG codons. To selectively translate these codons, artificially synthesized tRNAs (for example, tRNAs synthesized by transcription) are preferably used as the tRNAs of the present disclosure. In that case, in addition to using natural tRNA sequences, sequences derived from any tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

In a particular embodiment, codons in the translation system of the present disclosure are codons in which M₁ is uridine (U) and M₂ is adenosine (A). That is, the tRNAs of the present disclosure can be assigned to a codon box whose codons are represented by UAM₃. Use of a translation system comprising the tRNAs of the present disclosure enables selective translation of two types of amino acids from the combination of UAA and UAG codons. Alternatively, three types of amino acids can be selectively translated from the combination of UAU, UAA, and UAG codons, or the combination of UAC, UAA, and UAG codons. Artificially synthesized tRNAs (for example, tRNAs synthesized by transcription) are preferably used as the tRNAs of the present disclosure. In that case, in addition to using natural tRNA sequences, sequences derived from arbitrary tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside of the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

In a particular embodiment, codons in the translation system of the present disclosure are codons in which M₁ is uridine (U) and M₂ is guanosine (G). That is, the tRNAs of the present disclosure can be assigned to a codon box whose codons are represented by UGM₃. Use of a translation system comprising the tRNAs of the present disclosure enables selective translation of two types of amino acids from the combination of UGA and UGG codons. Alternatively, three types of amino acids can be selectively translated from the combination of UGU, UGA, and UGG codons, or the combination of UGC, UGA, and UGG codons. Artificially synthesized tRNAs (for example, tRNAs synthesized by transcription) are preferably used as the tRNAs of the present disclosure. In that case, in addition to using natural tRNA sequences, sequences derived from any tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

In a particular embodiment, codons in the translation system of the present disclosure are codons in which M₁ is cytidine (C) and M₂ is adenosine (A). That is, the tRNAs of the present disclosure can be assigned to a codon box whose codons are represented by CAM₃. Use of a translation system comprising the tRNAs of the present disclosure enables selective translation of two types of amino acids from the combination of CAA and CAG codons. Alternatively, three types of amino acids can be selectively translated from the combination of CAU, CAA, and CAG codons, or the combination of CAC, CAA, and CAG codons. Artificially synthesized tRNAs (for example, tRNAs synthesized by transcription) are preferably used as the tRNAs of the present disclosure. In that case, in addition to using natural tRNA sequences, sequences derived from any tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

In a particular embodiment, codons in the translation system of the present disclosure are codons in which M₁ is cytidine (C) and M₂ is guanosine (G). That is, the tRNAs of the present disclosure can be assigned to a codon box whose codons are represented by CGM₃. Use of a translation system comprising the tRNAs of the present disclosure enables selective translation of two types of amino acids from the combination of CGA and CGG codons. Alternatively, three types of amino acids can be selectively translated from the combination of CGU, CGA, and CGG codons, or the combination of CGC, CGA, and CGG codons. Artificially synthesized tRNAs (for example, tRNAs synthesized by transcription) are preferably used as the tRNAs of the present disclosure. In that case, in addition to using natural tRNA sequences, sequences derived from any tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

In a particular embodiment, codons in the translation system of the present disclosure are codons in which M₁ is adenosine (A) and M₂ is uridine (U). That is, the tRNAs of the present disclosure can be assigned to a codon box whose codons are represented by AUM₃. Use of a translation system comprising the tRNAs of the present disclosure enables selective translation of two types of amino acids from the combination of AUA and AUG codons. Alternatively, three types of amino acids can be selectively translated from the combination of AUU, AUA, and AUG codons, or the combination of AUC, AUA, and AUG codons. Artificially synthesized tRNAs (for example, tRNAs synthesized by transcription) are preferably used as the tRNAs of the present disclosure. In that case, in addition to using natural tRNA sequences, sequences derived from any tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

In a particular embodiment, codons in the translation system of the present disclosure are codons in which M₁ is adenosine (A) and M₂ is cytidine (C). That is, the tRNAs of the present disclosure can be assigned to a codon box whose codons are represented by ACM₃. Use of a translation system comprising the tRNAs of the present disclosure enables selective translation of two types of amino acids from the combination of ACA and ACG codons. Alternatively, three types of amino acids can be selectively translated from the combination of ACU, ACA, and ACG codons, or the combination of ACC, ACA, and ACG codons. Artificially synthesized tRNAs (for example, tRNAs synthesized by transcription) are preferably used as the tRNAs of the present disclosure. In that case, in addition to using natural tRNA sequences, sequences derived from any tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

In a particular embodiment, codons in the translation system of the present disclosure are codons in which M₁ is adenosine (A) and M₂ is adenosine (A). That is, the tRNAs of the present disclosure can be assigned to a codon box whose codons are represented by AAM₃. Use of a translation system comprising the tRNAs of the present disclosure enables selective translation of two types of amino acids from the combination of AAA and AAG codons. Alternatively, three types of amino acids can be selectively translated from the combination of AAU, AAA, and AAG codons, or the combination of AAC, AAA, and AAG codons. Artificially synthesized tRNAs (for example, tRNAs synthesized by transcription) are preferably used as the tRNAs of the present disclosure. In that case, in addition to using natural tRNA sequences, sequences derived from any tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

In a particular embodiment, codons in the translation system of the present disclosure are codons in which M₁ is adenosine (A) and M₂ is guanosine (G). That is, the tRNAs of the present disclosure can be assigned to a codon box whose codons are represented by AGM₃. Use of a translation system comprising the tRNAs of the present disclosure enables selective translation of two types of amino acids from the combination of AGA and AGG codons. Alternatively, three types of amino acids can be selectively translated from the combination of AGU, AGA, and AGG codons, or the combination of AGC, AGA, and AGG codons. Artificially synthesized tRNAs (for example, tRNAs synthesized by transcription) are preferably used as the tRNAs of the present disclosure. In that case, in addition to using natural tRNA sequences, sequences derived from any tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

In a particular embodiment, codons in the translation system of the present disclosure are codons in which M₁ is guanosine (G) and M₂ is adenosine (A). That is, the tRNAs of the present disclosure can be assigned to a codon box whose codons are represented by GAM₃. Use of a translation system comprising the tRNAs of the present disclosure enables selective translation of two types of amino acids from the combination of GAA and GAG codons. Alternatively, three types of amino acids can be selectively translated from the combination of GAU, GAA, and GAG codons, or the combination of GAC, GAA, and GAG codons. Artificially synthesized tRNAs (for example, tRNAs synthesized by transcription) are preferably used as the tRNAs of the present disclosure. In that case, in addition to using natural tRNA sequences, sequences derived from any tRNAs (for example, tRNA(Glu), tRNA(AsnE2), and tRNA(Asp)) can be used for the portions other than the anticodon, and a desired anticodon sequence can be ligated to those sequences. The tRNAs of the present disclosure, including the anticodon portions, are preferably composed only of the four nucleosides, adenosine (A), guanosine (G), cytidine (C), and uridine (U), and desirably do not contain other modified nucleosides. The translation system of the present disclosure is preferably a cell-free translation system, and particularly preferably a reconstituted cell-free translation system. For aminoacylation of each tRNA, attachment of an amino acid is preferably performed outside the translation system, and methods for such attachment are preferably, for example, the pCpA method, the pdCpA method, a method using an artificial RNA catalyst (flexizyme), or a method using an aminoacyl-tRNA synthetase (ARS). As the amino acid to be attached to each tRNA, either a natural amino acid or an unnatural amino acid may be used, but in view of the objective of this disclosure, use of an unnatural amino acid not included in the natural genetic code table is desirable. The concentration of a tRNA of this disclosure per codon in the translation system is preferably, for example, within the range of 0.8-1000 µM.

### III. Method, and composition and kit for producing a peptide

In one aspect, the present disclosure provides a method for producing a peptide, comprising translating a nucleic acid using the translation system of the present disclosure. Specifically, the present disclosure relates to a method for producing a peptide, comprising translating a nucleic acid in a translation system that comprises a tRNA having an anticodon complementary to a codon represented by M₁M₂A and a tRNA having an anticodon complementary to a codon represented by M₁M₂G. In one aspect, in the present disclosure, M₁ and M₂ represent nucleosides for the first and second letters of the codon respectively, and each of M₁ and M₂ is independently selected from any of adenosine (A), guanosine (G), cytidine (C), and uridine (U). In one aspect, each of the above-mentioned two tRNAs is attached to an amino acid or an amino acid analog that is different from each other. Furthermore, in one aspect, in the production method of the present disclosure, at least two types of amino acids or amino acid analogs can be translated from the codon represented by M₁M₂U, the codon represented by M₁M₂C, the codon represented by M₁M₂A, and the codon represented by M₁M₂G.

In one aspect, the present disclosure relates to a method for producing a peptide, comprising translating a nucleic acid in a translation system that comprises a tRNA having an anticodon complementary to a codon represented by M₁M₂U, a tRNA having an anticodon complementary to a codon represented by M₁M₂A, and a tRNA having an anticodon complementary to a codon represented by M₁M₂G. Furthermore, in one aspect, the present disclosure relates to a method for producing a peptide, comprising translating a nucleic acid in a translation system that comprises a tRNA having an anticodon complementary to a codon represented by M₁M₂C, a tRNA having an anticodon complementary to a codon represented by M₁M₂A, and a tRNA having an anticodon complementary to a codon represented by M₁M₂G. In one aspect, in the present disclosure, M₁ and M₂ represent nucleosides for the first and second letters of the codon respectively, and each of M₁ and M₂ is independently selected from any of adenosine (A), guanosine (G), cytidine (C), and uridine (U). In one aspect, each of the above-mentioned three tRNAs is attached to an amino acid or an amino acid analog that is different from each other. Furthermore, in one aspect, in the production method of the present disclosure, at least three types of amino acids or amino acid analogs can be translated from the codon represented by M₁M₂U, the codon represented by M₁M₂C, the codon represented by M₁M₂A, and the codon represented by M₁M₂G.

A nucleic acid of the present disclosure may comprise one or more occurrences of each of the codon represented by M₁M₂A and the codon represented by M₁M₂G. The method for producing a peptide in the present disclosure may comprise a process in which tRNAs of the present disclosure selectively translate (or discriminate) each of the codon represented by M₁M₂A and the codon represented by M₁M₂G that may be contained in the nucleic acid, once or multiple times. In one embodiment, in the method for producing a peptide in the present disclosure, one or more different nucleic acids comprising these codons may be translated.

A nucleic acid of the present disclosure may comprise one or more occurrences of each of the codon represented by M₁M₂U, the codon represented by M₁M₂A, and the codon represented by M₁M₂G. The method for producing a peptide in the present disclosure may comprise a process in which tRNAs of the present disclosure selectively translate (or discriminate) each of the codon represented by M₁M₂U, the codon represented by M₁M₂A, and the codon represented by M₁M₂G that may be contained in the nucleic acid, once or multiple times. In one embodiment, in the method for producing a peptide in the present disclosure, one or more different nucleic acids comprising these codons may be translated.

A nucleic acid of the present disclosure may comprise one or more occurrences of each of the codon represented by M₁M₂C, the codon represented by M₁M₂A, and the codon represented by M₁M₂G. The method for producing a peptide in the present disclosure may comprise a process in which tRNAs of the present disclosure selectively translate (or discriminate) each of the codon represented by M₁M₂C, the codon represented by M₁M₂A, and the codon represented by M₁M₂G that may be contained in the nucleic acid, once or multiple times. In one embodiment, in the method for producing a peptide in the present disclosure, one or more nucleic acids comprising these codons may be translated.

A nucleic acid of the present disclosure may comprise one or more occurrences of each of the codon represented by M₁M₂U, the codon represented by M₁M₂C, the codon represented by M₁M₂A, and the codon represented by M₁M₂G. The method for producing a peptide in the present disclosure may comprise a process in which tRNAs of the present disclosure selectively translate (or discriminate) each of the codon represented by M₁M₂U, the codon represented by M₁M₂C, the codon represented by M₁M₂A, and the codon represented by M₁M₂G that may be contained in the nucleic acid, once or multiple times. In one embodiment, in the method for producing a peptide in the present disclosure, one or more different nucleic acids comprising these codons may be translated.

In one aspect, the present disclosure relates to a composition and a kit for producing a peptide, that comprises a tRNA having an anticodon complementary to a codon represented by M₁M₂A and a tRNA having an anticodon complementary to a codon represented by M₁M₂G. In one aspect, in the present disclosure, M₁ and M₂ represent nucleosides for the first and second letters of the codon respectively, and each of M₁ and M₂ is independently selected from any of adenosine (A), guanosine (G), cytidine (C), and uridine (U). In one aspect, each of the above-mentioned two tRNAs is attached to an amino acid or an amino acid analog that is different from each other. Furthermore, in one aspect, by using the composition and kit of the present disclosure, at least two types of amino acids or amino acid analogs can be translated from the codon represented by M₁M₂U, the codon represented by M₁M₂C, the codon represented by M₁M₂A, and the codon represented by M₁M₂G.

In one aspect, the present disclosure relates to a composition and a kit for producing a peptide, that comprises a tRNA having an anticodon complementary to a codon represented by M₁M₂U, a tRNA having an anticodon complementary to a codon represented by M₁M₂A, and a tRNA having an anticodon complementary to a codon represented by M₁M₂G. Furthermore, in one aspect, the present disclosure relates to a composition and a kit for producing a peptide, that comprises a tRNA having an anticodon complementary to a codon represented by M₁M₂C, a tRNA having an anticodon complementary to a codon represented by M₁M₂A, and a tRNA having an anticodon complementary to a codon represented by M₁M₂G. In one aspect, in the present disclosure, M₁ and M₂ represent nucleosides for the first and second letters of the codon respectively, and each of M₁ and M₂ is independently selected from any of adenosine (A), guanosine (G), cytidine (C), and uridine (U). In one aspect, each of the above-mentioned three tRNAs is attached to an amino acid or an amino acid analog that is different from each other. Furthermore, in one aspect, in the production method of the present disclosure, at least three types of amino acids or amino acid analogs can be translated from the codon represented by M₁M₂U, the codon represented by M₁M₂C, the codon represented by M₁M₂A, and the codon represented by M₁M₂G.

The tRNAs constituting the translation system in the present disclosure can constitute a composition comprising a buffer, substances, and such commonly used for translating a nucleic acid. Furthermore, the tRNAs constituting the translation system of the present disclosure can be provided as a kit by packaging them in advance with various substances commonly used for peptide translation. Furthermore, the various substances contained in the kit may be in a powder or liquid form depending on their form of use. They may also be stored in an appropriate container, and used when appropriate.

In the present disclosure, the peptides of this disclosure may include compounds in which two or more amino acids are linked by an amide bond. In addition, the peptides of this disclosure may also include a compound in which amino acid analogs such as hydroxycarboxylic acid instead of amino acids are linked by an ester bond. The number of amino acids or amino acid analogs contained in the peptide is not particularly limited as long as it is 2 or more, for example, 2 or more, 3 or more, 4 or more, 5 or more, 6 or more, 7 or more, 8 or more, 9 or more, 10 or more, or 11 or more, and also 100 or less, 80 or less, 50 or less, 30 or less, 25 or less, 20 or less, 19 or less, 18 or less, 17 or less, 16 or less, 15 or less, 14 or less, 13 or less, or 12 or less. Alternatively, the number can be selected from 9, 10, 11, and 12.

In one aspect, a composition and a kit of the present disclosure may comprise a nucleic acid. In one embodiment, a nucleic acid constituting a composition and a kit in the present disclosure may comprise one or more occurrences of each of the codon represented by M₁M₂A and the codon represented by M₁M₂G. In one aspect, a nucleic acid constituting a composition and a kit of the present disclosure may comprise one or more occurrences of each of (i) the codon represented by M₁M₂U, the codon represented by M₁M₂A, and the codon represented by M₁M₂G, or (ii) the codon represented by M₁M₂C, the codon represented by M₁M₂A, and the codon represented by M₁M₂G. Furthermore, in one aspect, a nucleic acid constituting a composition and a kit of the present disclosure may comprise one or more occurrences of each of the codon represented by M₁M₂A, the codon represented by M₁M₂G, the codon represented by M₁M₂U, and the codon represented by M₁M₂C. In one embodiment, a composition and a kit of the present disclosure may comprise one or more such nucleic acids.

In one embodiment, the peptide of the present disclosure may contain N-substituted amino acids, and the number of N-substituted amino acids contained in the peptide may be, for example, 2, 3, 4, 5, 6, 7, 8, 9, or 10. In another embodiment, the peptide of the present disclosure may contain amino acids that are not N-substituted, and the number of N-unsubstituted amino acids may be, for example, 1, 2, 3, or 4. In a further embodiment, the peptide of the present disclosure may contain both N-substituted and N-unsubstituted amino acids.

In some embodiments, the peptide of the present disclosure may be a linear peptide or a peptide comprising a cyclic portion. A peptide comprising a cyclic portion means a peptide in which the main chain or side chain of an amino acid or amino acid analog existing on a peptide chain is attached to the main chain or side chain of another amino acid or amino acid analog existing on the same peptide chain to form a cyclic structure in the molecule. The peptide having a cyclic portion may be composed of only a cyclic portion, or may contain both a cyclic portion and a linear portion. The number of amino acids or amino acid analogs contained in the cyclic portion is, for example, 4 or more, 5 or more, 6 or more, 7 or more, 8 or more, or 9 or more, and 14 or less, 13 or less, 12 or less, or 11 or less. Alternatively, the number can be selected from 9, 10, and 11. The number of amino acids or amino acid analogs contained in the linear portion is, for example, 0 or more, and may be 8 or less, 7 or less, 6 or less, 5 or less, or 4 or less. Alternatively, the number can be selected from 0, 1, 2, and 3.

As the bond for forming the cyclic portion, for example, a peptide bond formed from an amino group and a carboxyl group can be used. In addition, an amide bond, disulfide bond, ether bond, thioether bond, ester bond, thioester bond, carbon-carbon bond, alkyl bond, alkenyl bond, phosphonate ether bond, azo bond, amine bond, C=N-C bond, lactam bridge, carbamoyl bond, urea bond, thiourea bond, thioamide bond, sulfinyl bond, sulfonyl bond, triazole bond, benzoxazole bond, and such formed from a combination of appropriate functional groups can be used. The carbon-carbon bond can be formed by a transition metal-catalyzed reaction such as a Suzuki reaction, a Heck reaction, and a Sonogashira reaction. In one embodiment, the peptides of the present disclosure contain at least one set of functional groups capable of forming the above-mentioned bond in the molecule. The formation of the cyclic portion may be performed by producing a linear peptide using the translation system of the present disclosure and then separately performing a reaction for linking the above-mentioned functional groups with each other. Regarding the synthesis of the peptide having a cyclic portion, one can refer to WO2013/100132, WO2012/026566, WO2012/033154, WO2012/074130, WO2015/030014, WO2018/052002, Comb Chem High Throughput Screen (2010)13: 75-87, Nat Chem Biol (2009) 5: 502-507, Nat Chem Biol (2009) 5: 888-90, Bioconjug Chem (2007) 18: 469-476, Chem Bio Chem (2009) 10: 787-798, Chem. Commun. (Camb) (2011) 47: 9946-9958, and such.

In some embodiments, the nucleic acid translated in the translation system of the present disclosure is mRNA. A peptide having a desired amino acid sequence may be encoded in an mRNA. By adding an mRNA to the translation system of the present disclosure, the mRNA can be translated into a peptide. On the other hand, when an RNA polymerase for transcribing DNA into mRNA is contained in the translation system, by adding the DNA to the translation system of the present disclosure, transcription of the DNA into mRNA can be performed in conjunction with translation of the mRNA into a peptide. The mRNA may at least contain a codon represented by M₁M₂A and a codon represented by M₁M₂G; a codon represented by M₁M₂U, a codon represented by M₁M₂A and a codon represented by M₁M₂G; or a codon represented by M₁M₂C, a codon represented by M₁M₂A and a codon represented by M₁M₂G

Methionine is usually present at the N-terminal of the translated peptide as an initiator amino acid, but some methods for introducing an amino acid other than methionine to the N-terminus have been reported. They may be used in combination with the methods for producing a peptide in the present disclosure. Examples of such a method include a method of translating a nucleic acid which is started from a desired amino acid by using an initiator tRNA which is aminoacylated with an amino acid other than methionine (initiation suppression). Particularly, the degree to which an exogenous amino acid may be tolerated is higher at the time of translation initiation than at the time of peptide chain elongation; therefore, at the N-terminal, even an amino acid having a structure largely different from that of a natural amino acid may be used (Goto & Suga, J Am Chem Soc (2009)131(14):5040-5041). Another method includes, for example, a method of translating a peptide starting from the second or subsequent codon by removing the initiator methionyl tRNA from the translation system or by replacing the initiator amino acid with an amino acid having low translation efficiency other than methionine (initiation read-through; skipping the start codon). Another method includes, for example, removing methionine at the N-terminus of the peptide by allowing enzymes such as peptide deformylase and methionine aminopeptidase to act (Meinnel et al., Biochimie (1993) 75: 1061-1075). A library of peptides starting from methionine is prepared, and the above enzyme is made to act on the peptide library to prepare a library of peptides starting from a random amino acid at N-terminus.

In another aspect, the present disclosure provides a peptide produced by the method for producing a peptide in the present disclosure. Peptides obtained by further chemically modifying the peptide produced by the method in the present disclosure are also included in the peptides provided by the present disclosure.

In one aspect, the present disclosure provides a method for producing a peptide library, comprising translating a nucleic acid library using the translation system in the present disclosure. By preparing a plurality of nucleic acid molecules each encoding a peptide and rich in nucleic acid sequence diversity, and then translating each of them into a peptide, a plurality of peptide molecules rich in amino acid sequence diversity can be produced. The size of the library is not particularly limited, and may be, for example, 10⁶ or more, 10⁷ or more, 10⁸ or more, 10⁹ or more, 10¹⁰ or more, 10¹¹ or more, 10¹² or more, 10¹³ or more, or 10¹⁴ or more. The nucleic acid may be DNA or RNA. RNA is usually mRNA. DNA is translated into a peptide via transcription into mRNA. Such a nucleic acid library can be prepared by a method known to those skilled in the art or a similar method. By using a mixed base at a desired position when synthesizing a nucleic acid library, a plurality of nucleic acid molecules rich in nucleic acid sequence diversity can be easily prepared. Examples of codons using mixed bases are, for example, NNN (where N represents a mixture of 4 bases, A, T, G, and C), NNW (where W represents a mixture of 2 bases, A and T), NNM (where W represents a mixture of two bases, A and C), NNK (where K represents a mixture of two bases, G and T), and NNS (where S represents a mixture of two bases, C and G). Alternatively, by limiting the base used in the third letter of the codon to any one of A, T, G, and C, a nucleic acid library in which only some specific amino acids are encoded can be synthesized. Furthermore, when a codon containing mixed bases is prepared, it is possible to arbitrarily adjust the appearance frequency of amino acids obtainable from the codon by mixing a plurality of bases at different ratios rather than in equal proportions. By taking a codon such as that mentioned above as one unit to prepare a plurality of different codon units, and then linking them in the desired order, a library in which the appearance position and appearance frequency of the contained amino acids are controlled can be designed.

In some embodiments, the peptide library in the present disclosure is a library in which peptides are displayed on nucleic acids (nucleic acid display library, or simply, display library). A display library is a library in which a phenotype and a genotype are associated with each other as a result of formation of a single complex by linking a peptide to a nucleic acid encoding that peptide. Examples of major display libraries include libraries prepared by the mRNA display method (Roberts and Szostak, Proc Natl Acad Sci USA (1997) 94: 12297-12302), in vitro virus method (Nemoto et al., FEBS Lett (1997) 414: 405-408), cDNA display method (Yamaguchi et al., Nucleic Acids Res (2009) 37: e108), ribosome display method (Mattheakis et al, Proc Natl Acad Sci USA (1994) 91: 9022-9026), covalent display method (Reiersen et.al., Nucleic Acids Res (2005) 33: e10), CIS display method (Odegrip et.al., Proc Natl Acad Sci USA (2004) 101: 2806-2810), and such. Alternatively, a library prepared by using the in vitro compartmentalization method (Tawfik and Griffiths, Nat Biotechnol (1998) 16: 652-656) can be mentioned as one embodiment of the display library.

In another aspect, the present disclosure provides a peptide library produced by the method for producing a peptide library in the present disclosure.

In one aspect, the present disclosure provides a method for identifying a peptide having binding activity to a target molecule, which comprises contacting the target molecule with a peptide library in the present disclosure. The target molecule is not particularly limited and can be appropriately selected from, for example, low molecular weight compounds, high molecular weight compounds, nucleic acids, peptides, proteins, sugars, and lipids. The target molecule may be a molecule existing outside the cell or a molecule existing inside the cell. Alternatively, it may be a molecule existing in the cell membrane, in which case any of the extracellular domain, the transmembrane domain, and the intracellular domain may be the target. In the step of contacting the target molecule with the peptide library, the target molecule is usually immobilized on some kind of solid-phase carrier (for example, a microtiter plate or microbeads). Then, by removing the peptides not attached to the target molecule and recovering only the peptides attached to the target molecule, the peptides having binding activity to the target molecule can be selectively concentrated (panning method). When the peptide library used is a nucleic acid display library, the recovered peptides have the nucleic acid encoding their respective genetic information attached to them; therefore, the nucleic acid sequence encoding the recovered peptide and the amino acid sequence can be readily identified by isolating and analyzing them. Furthermore, based on the obtained nucleic acid sequence or amino acid sequence, the identified peptides can be individually produced by chemical synthesis or gene recombination techniques.

In one aspect, the present disclosure provides a nucleic acid-peptide complex comprising a peptide and a nucleic acid encoding the peptide, wherein the complex has the following features:
(i) the nucleic acid sequence encoding the peptide comprises two codons, M₁M₂A and M₁M₂G; and
(ii) in the amino acid sequence of the peptide, the amino acid or amino acid analog corresponding to the M₁M₂A codon and the amino acid or amino acid analog corresponding to the M₁M₂G codon are different from each other.

Here, M₁ and M₂ represent the first and the second letters of a specific codon, respectively (however, the codons in which M₁ is A and M₂ is U are excluded).

In a further aspect, the present disclosure provides a nucleic acid-peptide complex comprising a peptide and a nucleic acid encoding the peptide, wherein the complex has the following features:
(i) the nucleic acid sequence encoding the peptide comprises three codons, M₁M₂U, M₁M₂A, and M₁M₂G; and
(ii) in the amino acid sequence of the peptide, the amino acid or amino acid analog corresponding to the M₁M₂U codon, the amino acid or amino acid analog corresponding to the M₁M₂A codon, and the amino acid or amino acid analog corresponding to the M₁M₂G codon are all different from each other.

Here, M₁ and M₂ represent the first and second letters of a specific codon, respectively.

In another aspect, the present disclosure provides a nucleic acid-peptide complex comprising a peptide and a nucleic acid encoding the peptide, wherein the complex has the following features:
(i) the nucleic acid sequence encoding the peptide comprises three codons, M₁M₂C, M₁M₂A, and M₁M₂G; and
(ii) in the amino acid sequence of the peptide, the amino acid or amino acid analog corresponding to the M₁M₂C codon, the amino acid or amino acid analog corresponding to the M₁M₂A codon, and the amino acid or amino acid analog corresponding to the M₁M₂G codon are all different from each other.

Here, M₁ and M₂ represent the first and second letters of a specific codon, respectively.

In some embodiments, the nucleic acid-peptide complex mentioned above may be contained in a peptide library (particularly a nucleic acid display library) as one of the elements constituting the library. In one embodiment, the present disclosure provides a library (peptide library or nucleic acid display library) comprising the nucleic acid-peptide complex in the present disclosure. In a particular embodiment, the nucleic acid-peptide complexes and libraries mentioned above may be prepared using the tRNAs in the present disclosure or the translation system in the present disclosure.

All prior art literatures cited in the present specification are incorporated herein by reference.

### [Examples]

The present invention is further illustrated by the following examples, but is not limited thereto.

The following abbreviations were used in the Examples.
AA: ammonium acetate
CH₂CN: cyanomethyl group
DBU: 1,8-diazabicyclo[5.4.0]-7-undecene
DCM: dichloromethane
DIC: N,N-diisopropylcarbodiimide
DIPEA: N,N-diisopropylethylamine
DMF: dimethylformamide
DMSO: dimethyl sulfoxide
FA: formic acid
Fmoc: 9-fluorenylmethyloxycarbonyl group
F-Pnaz: 4-(2-(4-fluorophenyl)acetamido)benzyloxycarbonyl group 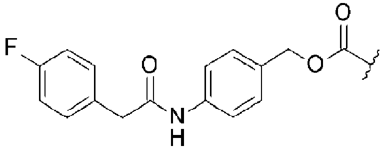
HFIP: 1,1,1,3,3,3-hexafluoro-2-propanol
MeCN: acetonitrile
NMP: N-methyl-2-pyrrolidone
TEA: triethylamine
TFA: trifluoroacetic acid
TFE: 2,2,2-trifluoroethanol
THF: tetrahydrofuran

The following abbreviations were used in this Example: Gly or G (glycine), Ile or I (isoleucine), Leu or L (leucine), Phe or F (phenylalanine), Pro or P (proline), Thr or T (threonine). In addition to these, the abbreviations shown in Table 2 were used.

**[Table 2]**

| Biocode | Structure | | Biocode | Structure |
|---|---|---|---|---|
| BdpFL -Phe | 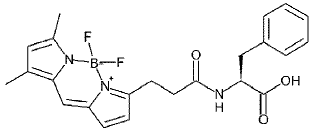 | | nBuG | 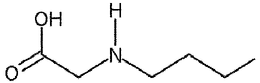 |
| MeA3Pyr | 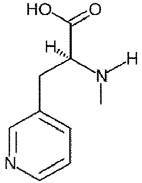 | | Pic2 | 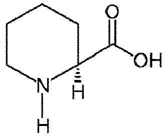 |
| StBuOH | 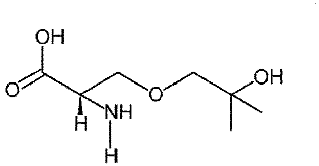 | | dA | 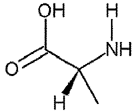 |
| MeSnPr | 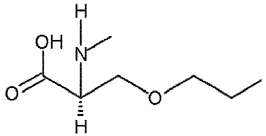 | | MeHph | 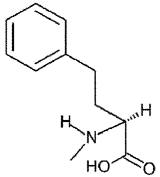 |
| SPh2Cl | 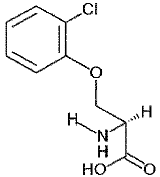 | | Thr (THP) | 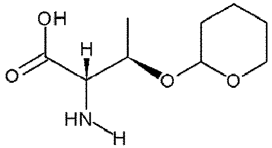 |

The LCMS analysis conditions are shown in Table 3 shown below.

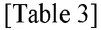

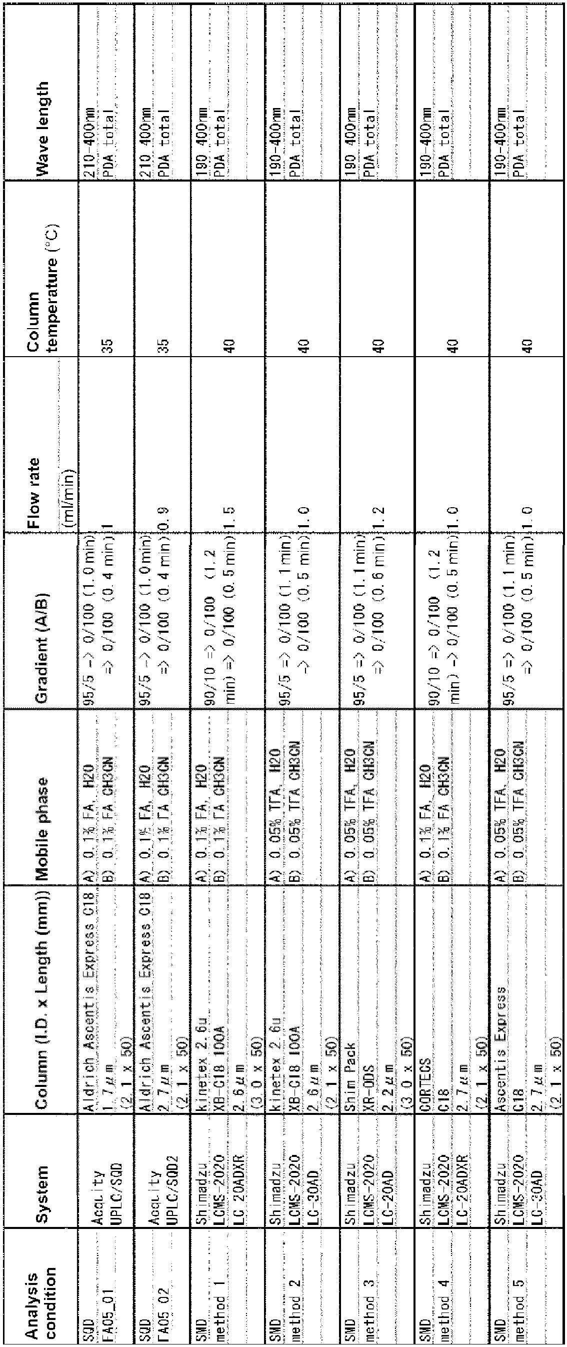

### Example 1. Synthesis of pCpA-amino acids to be used in a cell-free translation system

Aminoacylated pCpAs (SS14, SS15, SS16, SS48, SS49, SS50, and SS51) were synthesized according to the following scheme.

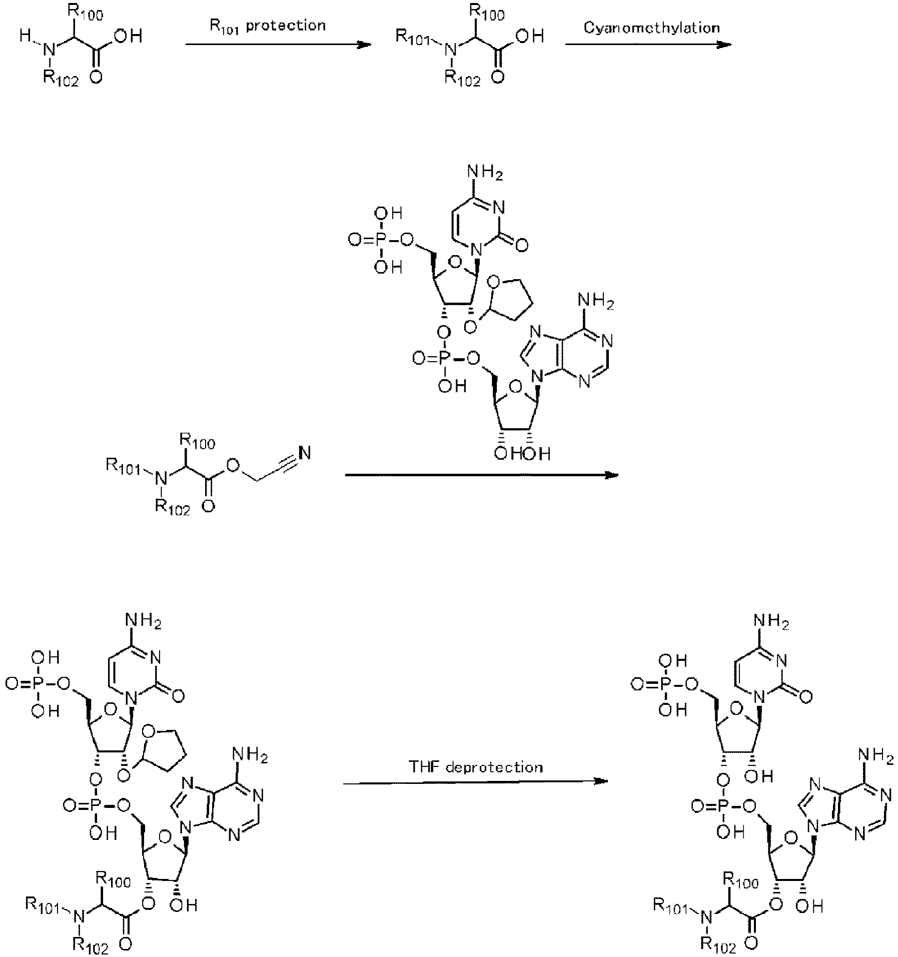

### Synthesis of (S)-1-(((4-(2-(4-fluorophenyl)acetamido)benzyl)oxy)carbonyl)piperidine-2-carboxylic acid (Compound SS17, F-Pnaz-Pic2-OH)

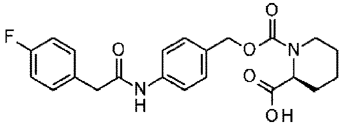

Under nitrogen atmosphere, DMF (330 µL) was added at room temperature to a mixture of (S)-piperidine-2-carboxylic acid (42.6 mg, 0.33 mmol) and (4-nitrophenyl)-4-(2-(4-fluorophenyl)acetamido)benzyl carbonate (Compound ts11) (140 mg, 0.44 mmol) synthesized by a method described in the patent literature (WO2018143145A1). After stirring this mixture at room temperature for five minutes, triethylamine (105.6 µL, 2.25 mmol) was added at 0°C. The reaction mixture was stirred at room temperature for 30 minutes, and then purified by reverse-phase silica gel column chromatography (0.1% aqueous formic acid solution/0.1% formic acid-acetonitrile solution) to obtain (S)-1-(((4-(2-(4-fluorophenyl)acetamido)benzyl)oxy)carbonyl)piperidine-2-carboxylic acid (Compound SS17, F-Pnaz-Pic2-OH) (92 mg, 67%).

LCMS (ESI) m/z = 413 (M-H)-

Retention time: 0.70 minutes (analysis condition SQDFA05_01)

### Synthesis of 1-(4-(2-(4-fluorophenyl)acetamido)benzyl) 2-(cyanomethyl) (S)-piperidine-1,2-dicarboxylate (Compound SS18, F-Pnaz-Pic2-OCH₂CN)

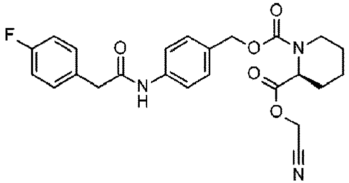

Under nitrogen atmosphere, ((S)-1-(((4-(2-(4-fluorophenyl)acetamido)benzyl)oxy)carbonyl)piperidine-2-carboxylic acid (Compound SS17, F-Pnaz-Pic2-OH) (30 mg, 0.072 mmol) and N-ethyl-isopropylpropan-2-amine (DIPEA) (20.23 µL, 0.116 mmol) were dissolved in acetonitrile (90 µL), 2-bromoacetonitrile (5.34 µL, 0.080 mmol) was added to this at 0°C, and the mixture was stirred at room temperature for two hours. The reaction solution was concentrated to obtain a crude product, 1-(4-(2-(4-fluorophenyl)acetamido)benzyl) 2-(cyanomethyl) (S)-piperidine-1,2-dicarboxylate (Compound SS18, F-Pnaz-Pic2-OCH₂CN). The obtained crude product was dissolved in acetonitrile (2.00 mL), and was directly used in the next step.

LCMS (ESI) m/z = 452 (M-H)-

Retention time: 0.79 minutes (analysis condition SQDFA05_01)

### Synthesis of 1-(4-(2-(4-fluorophenyl)acetamido)benzyl) 2-((2R,3S,4R,5R)-2-((((((2R,3S,4R,5R)-5-(4-amino-2-oxopyrimidin-1-(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl) (2S)-piperidine-1,2-dicarboxylate (Compound SS14, F-Pnaz-Pic2-pCpA)

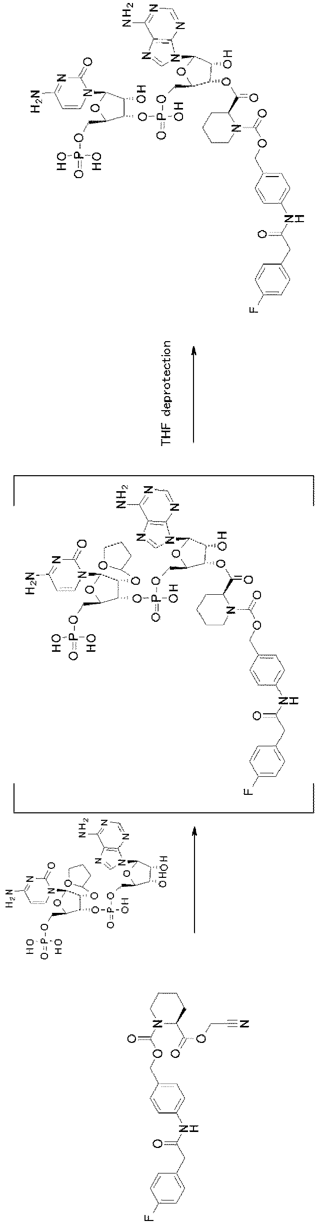

((2R,3R,4R, SR)-5-(4-amino-2-oxopyrimidin-1 (2H)-yl)-3-(((((2R,3 S,4R, SR)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (113 mg, 0.156 mmol) synthesized by a method described in the literature (Helv. Chim. Acta, 90, 297-310) was dissolved in Buffer A (40 mL), a solution of 1-(4-(2-(4-fluorophenyl)acetamido)benzyl) 2-(cyanomethyl) (S)-piperidine-1,2-dicarboxylate (Compound SS18, F-Pnaz-Pic2-OCH₂CN) (35.4 mg, 0.078 mmol) in acetonitrile (2.00 mL) was added, and the mixture was stirred at room temperature for 150 minutes. The reaction solution was cooled to 0°C, and then trifluoroacetic acid (2.00 mL) was added. The reaction solution was stirred at 0°C for 45 minutes, and then purified by reverse-phase silica gel column chromatography (0.05% aqueous trifluoroacetic acid solution/0.05% trifluoroacetic acid-acetonitrile) to obtain the title compound (Compound SS14, F-Pnaz-Pic2-pCpA) (6.0 mg, 7.3%).

LCMS (ESI) m/z = 1047.5 (M-H)-

Retention time: 0.50 minutes (analysis condition SQDFA05_01)

Buffer A was prepared as follows.

Acetic acid was added to an aqueous solution of N,N,N-trimethylhexadecan-1-aminium chloride (6.40 g, 20 mmol) and imidazole (6.81 g, 100 mmol) to give Buffer A (1L) of 20 mM N,N,N-trimethylhexadecan-1-aminium and 100 mM imidazole, pH8.

### Synthesis of O-(2-chlorophenyl)-N-(((4-(2-(4-fluorophenyl)acetamido)benzyl)oxy)carbonyl)-L-serine (Compound SS19, F-Pnaz-SPh2Cl-OH)

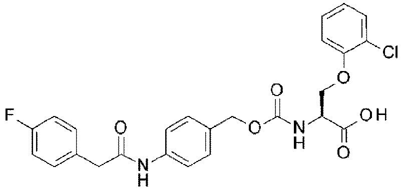

Under nitrogen atmosphere, DMSO (15 mL) and triethylamine (0.95 g, 9.42 mmol) were added at room temperature to a mixture of O-(2-chlorophenyl)-L-serine (Compound aa63) (1.25 g, 5.80 mmol) synthesized by a method described in the patent literature (WO2018225864) and (4-nitrophenyl)-4-(2-(4-fluorophenyl)acetamido)benzyl carbonate (Compound ts11) (2 g, 4.71 mmol) synthesized by a method described in the patent literature (WO2018143145A1). The reaction mixture was stirred at room temperature for 16 hours and then purified by reverse-phase silica gel column chromatography (0.1% aqueous formic acid solution/0.1% formic acid-acetonitrile solution) to obtain O-(2-chlorophenyl)-N-(((4-(2-(4-fluorophenyl)acetamido)benzyl)oxy)carbonyl)-L-serine (Compound SS19, F-Pnaz-SPh2Cl-OH) (1.8 g, 73%).

LCMS (ESI) m/z = 523 (M+Na)+

Retention time: 1.26 minutes (analysis condition SMD method 6)

### Synthesis of cyanomethyl O-(2-chlorophenyl)-N-(((4-(2-(4-fluorophenyl)acetamido)benzyl)oxy)karbonyl)-L-serinate (Compound SS20, F-Pnaz-SPh2Cl-OCH₂CN)

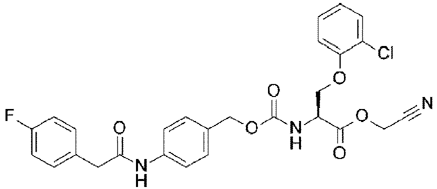

Under nitrogen atmosphere, O-(2-chlorophenyl)-N-(((4-(2-(4-fluorophenyl)acetamido)benzyl)oxy)carbonyl)-L-serine (Compound SS19, F-Pnaz-SPh2Cl-OH) (800 mg, 1.60 mmol) and N-ethyl-isopropylpropan-2-amine (DIPEA) (0.412 g, 3.19 mmol) were dissolved in DCM (15 mL), 2-bromoacetonitrile (760 mg, 6.34 mmol) was added at room temperature, and the mixture was stirred at room temperature for 16 hours. The reaction solution was concentrated and purified by reverse-phase silica gel column chromatography (0.1% aqueous formic acid solution/0.1% formic acid-acetonitrile solution) to obtain cyanomethyl O-(2-chlorophenyl)-N-(((4-(2-(4-fluorophenyl)acetamido)benzyl)oxy)carbonyl)-L-serinate (Compound SS20, F-Pnaz-SPh2Cl-OCH₂CN) (220 mg, 26%). The obtained product was dissolved in acetonitrile (5 mL), and used in the next step.

LCMS (ESI) m/z = 562 (M+Na)+

Retention time: 1.15 minutes (analysis condition SMD method 4)

### Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl O-(2-chlorophenyl)-N-(((4-(2-(4-fluorophenyl)acetamido)benzyl)oxy)carbonyl)-L-serinate (Compound SS15, F-Pnaz-SPh2Cl-pCpA)

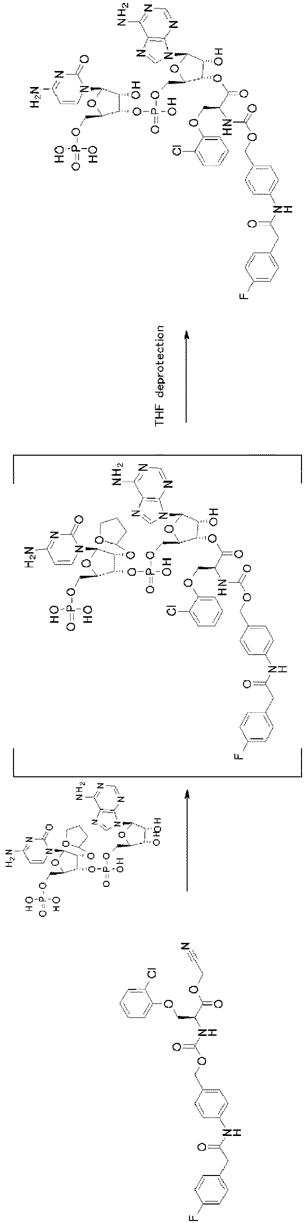

((2R,3R,4R, SR)-5-(4-amino-2-oxopyrimidin-1 (2H)-yl)-3-(((((2R,3 S,4R, SR)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (400 mg, 0.55 mmol) was dissolved in Buffer A (100 mL), a solution of cyanomethyl O-(2-chlorophenyl)-N-(((4-(2-(4-fluorophenyl)acetamido)benzyl)oxy)carbonyl)-L-serinate (Compound SS20, F-Pnaz-SPh2Cl-OCH₂CN) (220 mg, 0.41 mmol) in acetonitrile (5 mL) was added to it dropwise over 15 minutes or longer using a syringe pump, and this was stirred at room temperature for five minutes. Next, trifluoroacetic acid (2.3 mL) was added to the reaction solution. The reaction solution was freeze-dried, and then purified by reverse-phase silica gel column chromatography (0.05% aqueous trifluoroacetic acid solution/0.05% trifluoroacetic acid-acetonitrile) to obtain the title compound (Compound SS15, F-Pnaz-SPh2Cl-pCpA) (20.7 mg, 2%).

LCMS (ESI) m/z = 1133.4 (M-H)-

Retention time: 0.55 minutes (analysis condition SQDFA05_01)

### Synthesis of ((S)-2-(methylamino)-4-phenylbutanoic acid (Compound SS21, MeHph-OH)

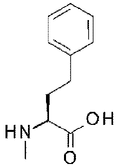

DCM (903 µL), water (903 µL), and piperidine (178 µL, 1.805 mmol) were added at room temperature to (S)-2-((((9H-fluoren-9-yl)methoxy)carbonyl)(methyl)amino)-4-phenylbutanoic acid (Compound aa11) (150 mg, 0.361 mmol) synthesized by a method described in the patent literature (WO2018225864). The reaction mixture was stirred at room temperature for 30 minutes and then purified by reverse-phase silica gel column chromatography (0.1% aqueous formic acid solution/0.1% formic acid-acetonitrile solution) to obtain ((S)-2-(methylamino)-4-phenylbutanoic acid (Compound SS21, MeHph-OH) (55 mg, 79%).

LCMS (ESI) m/z = 192 (M-H)-

Retention time: 0.15 minutes (analysis condition SQDFA05_02)

### Synthesis of (S)-2-((((4-(2-(4-fluoroi)henyl)acetamido)benzyl)oxy)carbonyl)(methyl)amino)-4-phenylbutanoic acid (Compound SS22, F-Pnaz-MeHph-OH)

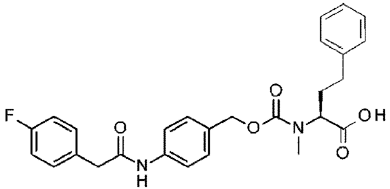

Under nitrogen atmosphere, DMSO (727 µL) was added at room temperature to a mixture of ((S)-2-(methylamino)-4-phenylbutanoic acid (Compound SS21, MeHph-OH) (35.1 mg, 0.182 mmol) and (4-nitrophenyl)-4-(2-(4-fluorophenyl)acetamido)benzyl carbonate (Compound ts11) (85 mg, 0.20 mmol) synthesized by a method described in the patent literature (WO2018143145A1). Triethylamine (76 µL, 0.545 mmol) was added at 50°C. The reaction mixture was stirred at 40°C for 16 hours, and then purified by reverse-phase silica gel column chromatography (0.1% aqueous formic acid solution/0.1% formic acid-acetonitrile solution) to obtain (S)-2-((((4-(2-(4-fluorophenyl)acetamido)benzyl)oxy)carbonyl)(methyl)amino)-4-phenylbutanoic acid (Compound SS22, F-Pnaz-MeHph-OH) (80 mg, 92%).

LCMS (ESI) m/z = 477 (M-H)-

Retention time: 0.85 minutes (analysis condition SQDFA05_02)

### Synthesis of cyanomethyl (S)-2-((((4-(2-(4-fluorophenyl)acetamido)benzyl)oxy)carbonyl)(methyl)amino)-4-phenylbutanoate (Compound SS23, F-Pnaz-MeHph-OCH₂CN)

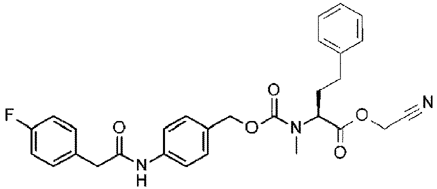

Under nitrogen atmosphere, acetonitrile (533 µL) was added at room temperature to a mixture of (S)-2-((((4-(2-(4-fluorophenyl)acetamido)benzyl)oxy)carbonyl)(methyl)amino)-4-phenylbutanoic acid (Compound SS22, F-Pnaz-MeHph-OH) (77 mg, 0.16 mmol) and N-ethyl-isopropylpropan-2-amine (DIPEA) (31 µL,0.176 mmol). Then, 2-bromoacetonitrile (86 µL, 1.280 mmol) was added at room temperature, and the reaction mixture was stirred at 40°C for one hour. The reaction solution was concentrated to obtain a crude product, cyanomethyl (S)-2-((((4-(2-(4-fluorophenyl)acetamido)benzyl)oxy)carbonyl)(methyl)amino)-4-phenylbutanoate (Compound SS23, F-Pnaz-MeHph-OCH₂CN). The obtained crude product was dissolved in acetonitrile (5.00 mL) and was directly used in the next step.

LCMS (ESI) m/z = 516 (M-H)-

Retention time: 0.92 minutes (analysis condition SQDFA05_02)

### Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-((((4-(2-(4-fluorophenyl)acetamido)benzyl)oxy)carbonyl)(methyl)amino)-4-phenylbutanoate (Compound SS16, F-Pnaz-MeHph-pCpA)

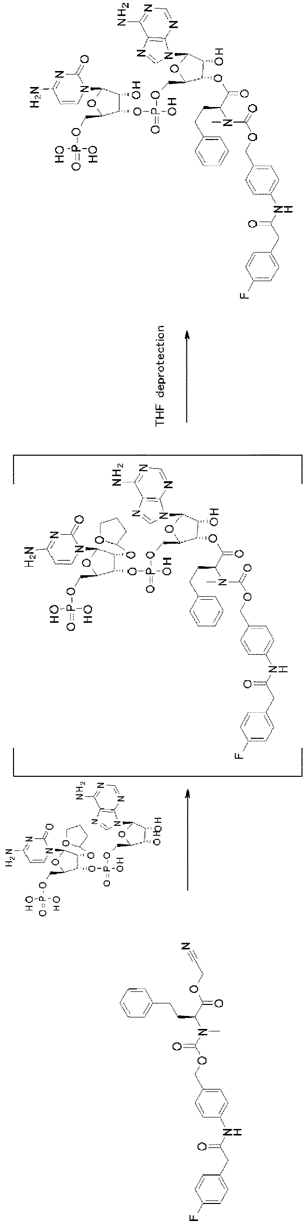

((2R,3R,4R,5R)-5-(4-amino-2-oxopyrimidin-1(2H)-yl)-3-(((((2R,3S,4R,SR)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (127 mg, 0.176 mmol) was dissolved in Buffer A (100 mL), a solution of cyanomethyl (S)-2-((((4-(2-(4-fluorophenyl)acetamido)benzyl)oxy)carbonyl)(methyl)amino)-4-phenylbutanoate (Compound SS23, F-Pnaz-MeHph-OCH₂CN) (83 mg, 0.16 mmol) in acetonitrile (5.00 mL) was added, and the mixture was stirred at room temperature for one hour. The reaction solution was cooled to 0°C, and then trifluoroacetic acid (5.00 mL) was added. The reaction solution was stirred at 0°C for one hour, and then purified by reverse-phase silica gel column chromatography (0.05% aqueous trifluoroacetic acid solution/0.05% trifluoroacetic acid-acetonitrile), and then further purified by reverse-phase silica gel column chromatography (0.1% aqueous formic acid solution/0.1% formic acid acetonitrile solution) to obtain the title compound (Compound SS16, F-Pnaz-MeHph-pCpA) (26 mg, 14.6%).

LCMS (ESI) m/z = 1111.5 (M-H)-

Retention time: 0.64 minutes (analysis condition SQDFA05_02)

The synthetic intermediate of Compound SS48 was synthesized according to the following scheme.

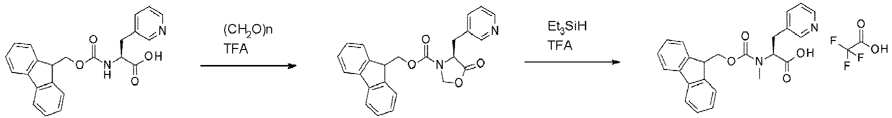

### Synthesis of (S)-2-((((9H-fluoren-9-yl)methoxy)carbonyl)(methyl)amino)-3-(pyridin-3-yl)propanoic acid compound 2,2,2-trifluoroacetic acid salt (Compound SS52, Fmoc-MeA3Pyr-OH·TFA

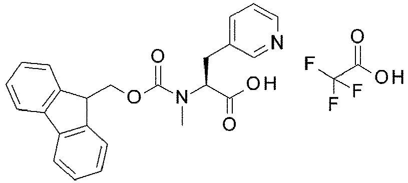

(S)-2-((((9H-fluoren-9-yl)methoxy)carbonyl)amino)-3-(pyridin-3-yl)propanoic acid (15 g, 38.62 mmol), trifluoroacetic acid (27 mL, 348 mmol), and paraformaldehyde ((CH₂O)ₙ) (3.48 g, 116 mmol) were suspended in toluene (50 mL), and this was stirred under nitrogen atmosphere at 40°C for 16 hours. After cooling to room temperature, the reaction solution was concentrated under reduced pressure. The residue was dissolved in DCM, and then washed with saturated aqueous sodium bicarbonate solution. The organic layer was dried over anhydrous sodium sulfate, then filtered, and concentrated under reduced pressure to remove the solvent, thus obtaining (9H-fluoren-9-yl)methyl (S)-5-oxo-4-(pyridin-3-ylmethyl)oxazolidin-3-carboxylate as a crude product.

The obtained crude product (9H-fluoren-9-yl) (S)-5-oxo-4-(pyridin-3-ylmethyl)oxazolidin-3-carboxylate (18 g, 44.95 mmol) was dissolved in dichloroethane (100 mL), and triethylsilane (Et₃SiH) (47 g, 404.20 mmol) and trifluoroacetic acid (100 mL) were added at room temperature. The reaction solution was stirred under nitrogen atmosphere at 70°C for 16 hours, and then concentrated under reduced pressure. The obtained residue was dissolved in isopropyl acetate, and then a mixed solution of t-butylmethyl ether and hexane (9:1) was added to it. The resulting solution was stirred at room temperature for 20 minutes, and then it was left to stand at 4°C for one hour. The precipitates that formed were collected by filtration, and they were washed with a cooled mixed solution of t-butylmethyl ether and hexane (9:1) to obtain (S)-2-((((9H-fluoren-9-yl)methoxy)carbonyl)(methyl)amino)-3-(pyridin-3-yl)propanoic acid compound 2,2,2-trifluoroacetic acid salt (Compound SS52, Fmoc-MeA3Pyr-OH·TFA) (19 g, 95%).

LCMS(ESI) m/z = 403 (M+H)+

Retention time: 0.77 minutes (analysis condition SMD method 1)

### Synthesis of (S)-2-(methylamino)-3-(pyridin-3-yl)propanoic acid (Compound SS53, MeA3Pyr-OH)

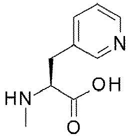

DMF (72 mL) and piperidine (28.5 mL) were added at room temperature to (S)-2-((((9H-fluoren-9-yl)methoxy)carbonyl)(methyl)amino)-3-(pyridin-3-yl)propanoic acid 2,2,2-trifluoroacetic acid salt (Compound SS52, Fmoc-MeA3Pyr-OH·TFA) (19 g, 47.21 mmol), and the reaction mixture was stirred at room temperature for three hours. Diethyl ether (140 mL) and hexane (280 mL) were added, and this was stirred at room temperature for another three hours. The precipitates that formed were collected by filtration to obtain (S)-2-(methylamino)-3-(pyridin-3-yl)propanoic acid (Compound SS53, MeA3Pyr-OH) (7 g) quantitatively.

LCMS(ESI) m/z = 181 (M+H)+

Retention time: 0.15 minutes (analysis condition SMD method 2)

### Synthesis of (S)-2-((((4-(2-(4-fluoroi)henyl)acetamido)benzyl)oxy)carbonyl)(methyl)amino)-3-(pyridin-3-yl)propanoic acid (Compound SS54, F-Pnaz-MeA3Pyr-OH)

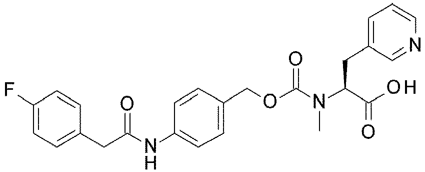

Under nitrogen atmosphere, DMSO (15 mL) and triethylamine (950 mg, 9.43 mmol) were added at room temperature to a mixture of (S)-2-(methylamino)-3-(pyridin-3-yl)propanoic acid (Compound SS53, MeA3Pyr-OH) (970 mg, 5.38 mmol) and (4-nitrophenyl)-4-(2-(4-fluorophenyl)acetamido)benzyl carbonate (Compound ts11) (2 g, 4.71 mmol) synthesized by a method described in the patent literature (WO2018143145A1). The reaction mixture was stirred at 40°C for 16 hours and then purified by reverse-phase silica gel column chromatography (0.1% aqueous formic acid solution/0.1% formic acid-acetonitrile solution) to obtain (S)-2-((((4-(2-(4-fluorophenyl)acetamido)benzyl)oxy)carbonyl)(methyl)amino)-3-(pyridin-3-yl)propanoic acid (Compound SS54, F-Pnaz-MeA3Pyr-OH) (1.0 g, 46%).

LCMS(ESI) m/z = 466 (M+H)+

Retention time: 0.98 minutes (analysis condition SMD method 3)

### Synthesis of cyanomethyl (S)-2-((((4-(2-(4-fluorophenyl)acetamido)benzyl)oxy)carbonyl)(methyl)amino)-3-(pyridin-3-yl)propanoate (Compound SS55, F-Pnaz-MeA3Pyr-OCH₂CN)

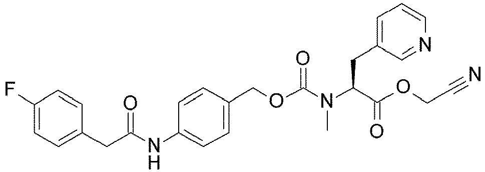

Under nitrogen atmosphere, a mixture of (S)-2-((((4-(2-(4-fluorophenyl)acetamido)benzyl)oxy)carbonyl)(methyl)amino)-3-(pyridin-3-yl)propanoic acid (Compound SS54, F-Pnaz-MeA3Pyr-OH) (800 mg, 1.72 mmol) and N-ethyl-isopropylpropan-2-amine (DIPEA) (444 mg, 3.44 mmol) were dissolved in DCM (20 mL), 2-bromoacetonitrile (818 mg, 6.82 mmol) was added at room temperature, and the mixture was stirred at room temperature for six hours. The reaction solution was concentrated and purified by normal-phase silica gel column chromatography (ethyl acetate/petroleum ether) to obtain cyanomethyl (S)-2-((((4-(2-(4-fluorophenyl)acetamido)benzyl)oxy)carbonyl)(methyl)amino)-3-(pyridin-3-yl)propanoate (Compound SS55, F-Pnaz-MeA3Pyr-OCH₂CN) (221 mg, 25%)

LCMS(ESI) m/z = 505 (M+H)+

Retention time: 0.83 minutes (analysis condition SMD method 4)

### Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (2S)-2-((((4-(2-(4-fluorophenyl)acetamido)benzyl)oxy)carbonyl)(methyl)amino)-3-(pyridin-3-yl)propanoate (Compound SS48, F-Pnaz-MeA3Pyr-pCpA)

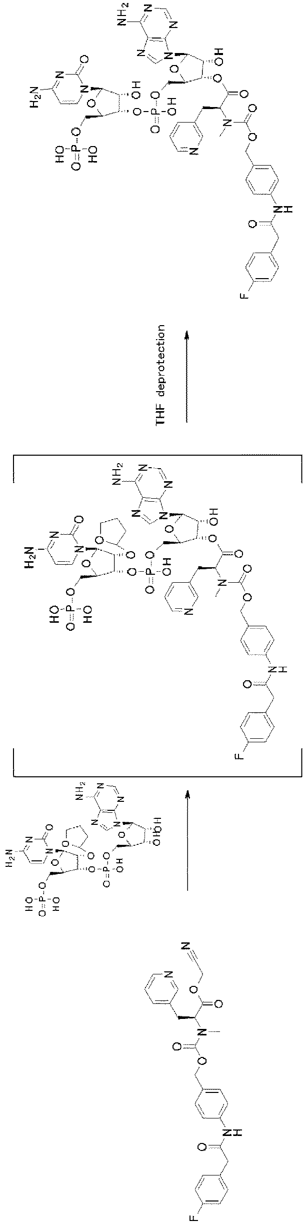

((2R,3R,4R, SR)-5-(4-amino-2-oxopyrimidin-1 (2H)-yl)-3-(((((2R,3 S,4R, SR)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (400 mg, 0.55 mmol) synthesized by a method described in the literature (Helv. Chim. Acta, 90, 297-310) was dissolved in Buffer A (100 mL), a solution of cyanomethyl (S)-2-((((4-(2-(4-fluorophenyl)acetamido)benzyl)oxy)carbonyl)(methyl)amino)-3-(pyridin-3-yl)propanoate (Compound SS55, F-Pnaz-MeA3Pyr-OCH₂CN) (146 mg, 0.29 mmol) in acetonitrile (5 mL) was added dropwise over 15 minutes or longer using a syringe pump, and the mixture was stirred at room temperature for one hour. Trifluoroacetic acid (2.3 mL) was added to the reaction solution, and after freeze-drying the reaction solution, this was purified by reverse-phase silica gel column chromatography (0.05% aqueous trifluoroacetic acid solution/0.05% trifluoroacetic acid-acetonitrile) to obtain the title compound (Compound SS48, F-Pnaz-MeA3Pyr-pCpA) (64.4 mg, 5%).

LCMS(ESI) m/z = 1098.5 (M-H)-

Retention time: 0.39 minutes (analysis condition SQDFA05_01)

### Synthesis of N-(((4-(2-(4-fluoroi)henyl)acetamido)benzyl)oxy)carbonyl)-O-(2-hydroxy-2-methylpropyl)-L-serine (Compound SS56, F-Pnaz-StBuOH-OH)

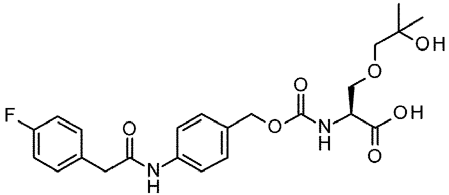

Under nitrogen atmosphere, DMSO (7 mL) and triethylamine (0.65 mL, 4.72 mmol) were added at room temperature to a mixture of O-(2-hydroxy-2-methylpropyl)-L-serine (0.5 g, 2.83 mmol) synthesized by a method described in the patent literature (WO2018225864) and (4-nitrophenyl)-4-(2-(4-fluorophenyl)acetamido)benzyl carbonate (Compound ts11) (1 g, 2.36 mmol) synthesized by a method described in the patent literature (WO2018143145A1). The reaction mixture was stirred at room temperature for 16 hours and then purified by reverse-phase silica gel column chromatography (0.1% aqueous formic acid solution/0.1% formic acid-acetonitrile solution) to obtain N-(((4-(2-(4-fluorophenyl)acetamido)benzyl)oxy)carbonyl)-O-(2-hydroxy-2-methylpropyl)-L-serine (Compound SS56, F-Pnaz-StBuOH-OH) (1 g, 92%).

LCMS(ESI) m/z = 485 (M+Na)+

Retention time: 0.80 minutes (analysis condition SMD method 5)

### Synthesis of cyanomethyl N-(((4-(2-(4-fluorophenyl)acetamido)benzyl)oxy)carbonyl)-O-(2-hydroxy-2-methylpropyl)-L-serinate (Compound SS57, F-Pnaz-StBuOH-OCH₂CN)

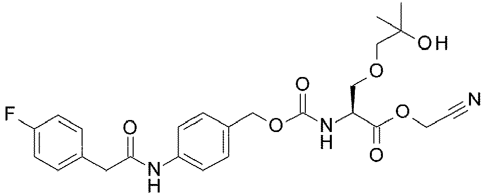

Under nitrogen atmosphere, N-(((4-(2-(4-fluorophenyl)acetamido)benzyl)oxy)carbonyl)-O-(2-hydroxy-2-methylpropyl)-L-serine (Compound SS56, F-Pnaz-StBuOH-OH) (1.2 g, 2.59 mmol) and N-ethyl-isopropylpropan-2-amine (DIPEA) (670 mg, 5.18 mmol) were dissolved in DCM (36 mL), 2-bromoacetonitrile (1.23 g, 10.25 mmol) was added at room temperature, and the mixture was stirred at room temperature for 48 hours. The reaction solution was concentrated and then purified by normal-phase silica gel column chromatography (ethyl acetate/petroleum ether) to obtain cyanomethyl N-(((4-(2-(4-fluorophenyl)acetamido)benzyl)oxy)carbonyl)-O-(2-hydroxy-2-methylpropyl)-L-serinate (Compound SS57, F-Pnaz-StBuOH-OCH2CN) (1 g, 77%).

LCMS(ESI) m/z = 524 (M+Na)+

Retention time: 0.97 minutes (analysis condition SMD method 4)

### Synthesis of (2R,3S,4R,SR)-2-((((((2R,3S,4R,SR)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(((4-(2-(4-fluorophenyl)acetamido)benzyl)oxy)carbonyl)-O-(2-hydroxy-2-methylpropyl)-L-serinate (Compound SS49, F-Pnaz-StBuOH-pCpA)

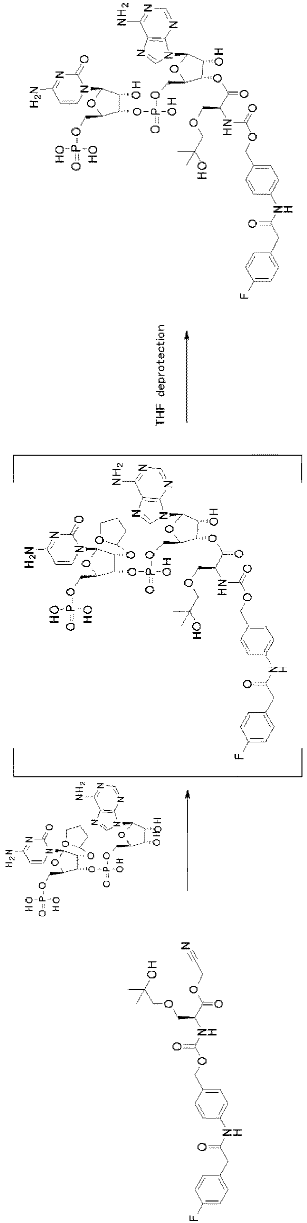

((2R,3R,4R, SR)-5-(4-amino-2-oxopyrimidin-1 (2H)-yl)-3-(((((2R,3 S,4R, SR)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (400 mg, 0.55 mmol) synthesized by a method described in the literature (Helv. Chim. Acta, 90, 297-310) was dissolved in Buffer A (100 mL), a solution of cyanomethyl N-(((4-(2-(4-fluorophenyl)acetamido)benzyl)oxy)carbonyl)-O-(2-hydroxy-2-methylpropyl)-L-serinate (Compound SS57, F-Pnaz-StBuOH-OCH2CN) (139 mg, 0.28 mmol) in acetonitrile (5 mL) was added dropwise over 15 minutes or longer using a syringe pump, and the mixture was stirred at room temperature for one hour. Trifluoroacetic acid (2.3 mL) was added to the reaction solution, and after freeze-drying the reaction solution, this was purified by reverse-phase silica gel column chromatography (0.05% aqueous trifluoroacetic acid solution/0.05% trifluoroacetic acid-acetonitrile) to obtain the title compound (Compound SS49, F-Pnaz-StBuOH-pCpA) (55.3 mg, 5%).

LCMS(ESI) m/z = 1095.4 (M-H)-

Retention time: 0.46 minutes (analysis condition SQDFA05_01)

### Synthesis of N-methyl-O-propyl-L-serine (Compound SS58, MeSnPr-OH)

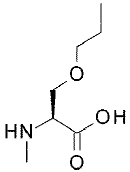

DMF (80 mL) and piperidine (30 mL) were added at room temperature to (S)-2-((((9H-fluoren-9-yl)methoxy)carbonyl)(methyl)amino)-4-phenylbutanoic acid (Compound aa52) (20 g, 52.16 mmol) synthesized by a method described in the patent literature (WO2018225864), and the reaction mixture was stirred at room temperature for three hours. Diethyl ether (160 mL) and hexane (500 mL) were added to the mixture, and this was stirred at room temperature for another three hours. The precipitates that formed were collected by filtration to obtain N-methyl-O-propyl-L-serine (Compound SS58, MeSnPr-OH) (7 g, 83%).

LCMS(ESI) m/z = 162 (M+H)+

Retention time: 0.34 minutes (analysis condition SMD method 2)

### Synthesis of N-(((4-(2-(4-fluorophenyl)acetamido)benzyl)oxy)carbonyl)-N-methyl-O-propyl-L-serine (Compound SS59, F-Pnaz-MeSnPr-OH)

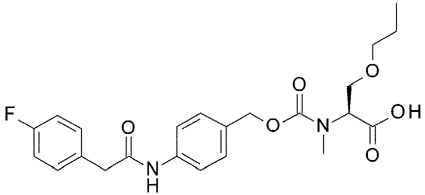

Under nitrogen atmosphere, DMSO (15 mL) and triethylamine (1.3 mL, 9.43 mmol) were added at room temperature to a mixture of N-methyl-O-propyl-L-serine (Compound SS58, MeSnPr-OH) (920 mg, 5.71 mmol) and (4-nitrophenyl)-4-(2-(4-fluorophenyl)acetamido)benzyl carbonate (Compound ts11) (2 g, 4.71 mmol) synthesized by a method described in the patent literature (WO2018143145A1). The reaction mixture was stirred at room temperature for 16 hours and then purified by reverse-phase silica gel column chromatography (0.1% aqueous formic acid solution/0.1% formic acid-acetonitrile solution) to obtain N-(((4-(2-(4-fluorophenyl)acetamido)benzyl)oxy)carbonyl)-N-methyl-O-propyl-L-serine (Compound SS59, F-Pnaz-MeSnPr-OH) (2 g, 95%).

LCMS(ESI) m/z = 469 (M+Na)+

Retention time: 1.23 minutes (analysis condition SMD method 3)

### Synthesis of cyanomethyl N-(((4-(2-(4-fluorophenyl)acetamido)benzyl)oxy)carbonyl)-N-methyl-O-propyl-L-serinate (Compound SS60, F-Pnaz-MeSnPr-OCH₂CN)

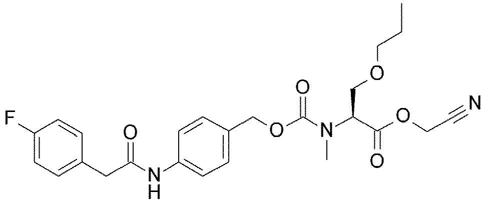

Under nitrogen atmosphere, a mixture of N-(((4-(2-(4-fluorophenyl)acetamido)benzyl)oxy)carbonyl)-N-methyl-O-propyl-L-serine (Compound SS59, F-Pnaz-MeSnPr-OH) (2.2 g, 4.93 mmol) and N-ethyl-isopropylpropan-2-amine (DIPEA) (1.28 g, 9.90 mmol) were dissolved in DCM (40 mL), 2-bromoacetonitrile (2.35 g, 19.59 mmol) was added at room temperature, and the mixture was stirred at room temperature for 48 hours. The reaction solution was concentrated and then purified by normal-phase silica gel column chromatography (ethyl acetate/petroleum ether) to obtain cyanomethyl N-(((4-(2-(4-fluorophenyl)acetamido)benzyl)oxy)carbonyl)-N-methyl-O-propyl-L-serinate (Compound SS60, F-Pnaz-MeSnPr-OCH₂CN) (2 g, 84%).

LCMS(ESI) m/z = 508 (M+Na)+

Retention time: 1.32 minutes (analysis condition SMD method 3)

### Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl N-(((4-(2-(4-fluorophenyl)acetamido)benzyl)oxy)carbonyl)-N-methyl-O-propyl-L-serinate (Compound SS50, F-Pnaz-MeSnPr-pCpA)

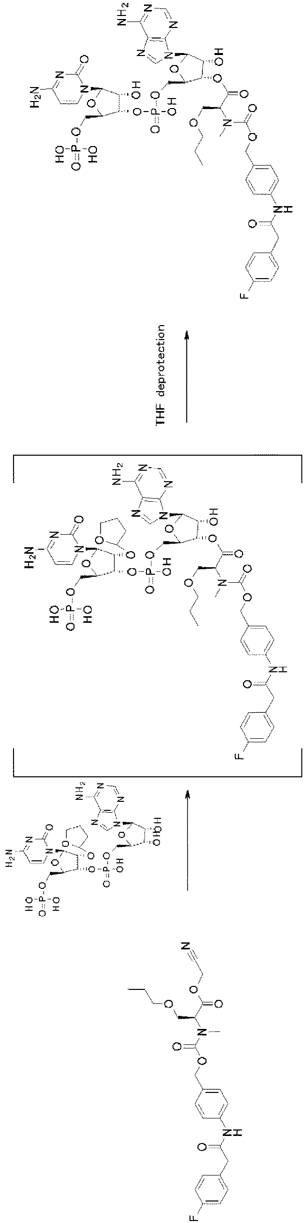

((2R,3R,4R, SR)-5-(4-amino-2-oxopyrimidin-1 (2H)-yl)-3-(((((2R,3 S,4R, SR)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (400 mg, 0.55 mmol) synthesized by a method described in the literature (Helv. Chim. Acta, 90, 297-310) was dissolved in Buffer A (100 mL), a solution of cyanomethyl N-(((4-(2-(4-fluorophenyl)acetamido)benzyl)oxy)carbonyl)-N-methyl-O-propyl-L-serinate (Compound SS60, F-Pnaz-MeSnPr-OCH₂CN) (135 mg, 0.28 mmol) in acetonitrile (5 mL) was added dropwise over 15 minutes or longer using a syringe pump, and the mixture was stirred at room temperature for four hours. Trifluoroacetic acid (2.3 mL) was added to the reaction solution, and after freeze-drying the reaction solution, this was purified by reverse-phase silica gel column chromatography (0.05% aqueous trifluoroacetic acid solution/0.05% trifluoroacetic acid-acetonitrile) to obtain the title compound (Compound SS50, F-Pnaz-MeSnPr-pCpA) (70.2 mg, 6%).

LCMS(ESI) m/z = 1079.5 (M-H)-

Retention time: 0.52 minutes (analysis condition SQDFA05_01)

### Synthesis of (((4-(2-(4-fluorophenyl)acetamido)benzyl)oxy)carbonyl)-L-isoleucine (Compound SS61, F-Pnaz-Ile-OH)

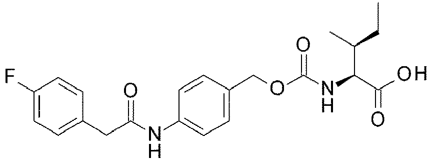

Under nitrogen atmosphere, DMSO (2 mL) and triethylamine (128 µL, 0.92 mmol) were added at room temperature to a mixture of L-isoleucine (52.5 mg, 0.40 mmol) and (4-nitrophenyl)-4-(2-(4-fluorophenyl)acetamido)benzyl carbonate (Compound ts11) (178 mg, 0.42 mmol) synthesized by a method described in the patent literature (WO2018143145A1). The reaction mixture was stirred at room temperature for 2.5 days and then purified by reverse-phase silica gel column chromatography (0.1% aqueous formic acid solution/0.1% formic acid-acetonitrile solution) to obtain (((4-(2-(4-fluorophenyl)acetamido)benzyl)oxy)carbonyl)-L-isoleucine (Compound SS61, F-Pnaz-Ile-OH) (125 mg, 75%).

LCMS(ESI) m/z = 415.4 (M-H)-

Retention time: 0.74 minutes (analysis condition SQDFA05_02)

### Synthesis of cyanomethyl (((4-(2-(4-fluorophenyl)acetamido)benzyl)oxy)carbonyl)-L-isoleucinate (Compound SS62, F-Pnaz-Ile-OCH₂CN)

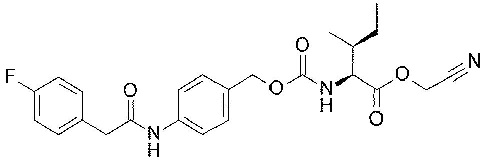

Under nitrogen atmosphere, (((4-(2-(4-fluorophenyl)acetamido)benzyl)oxy)carbonyl)-L-isoleucine (Compound SS61, F-Pnaz-Ile-OH) (42 mg, 0.1 mmol) and 2-bromoacetonitrile (13 µL, 0.200 mmol) were dissolved in acetonitrile (500 µL), N-ethyl-isopropylpropan-2-amine (DIPEA) (35 µL, 0.200 mmol) was added at room temperature, and the mixture was stirred at room temperature for 16 hours. The reaction solution was concentrated to obtain a crude product, cyanomethyl (((4-(2-(4-fluorophenyl)acetamido)benzyl)oxy)carbonyl)-L-isoleucinate (Compound SS62, F-Pnaz-Ile-OCH2CN). The obtained crude product was dissolved in acetonitrile (3.00 mL), and was directly used in the next step.

LCMS (ESI) m/z = 454 (M-H)-

Retention time: 0.83 minutes (analysis condition SQDFA05_02)

### Synthesis of (2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl (((4-(2-(4-fluorophenyl)acetamido)benzyl)oxy)carbonyl)-L-isoleucinate (Compound SS51, F-Pnaz-Ile-pCpA)

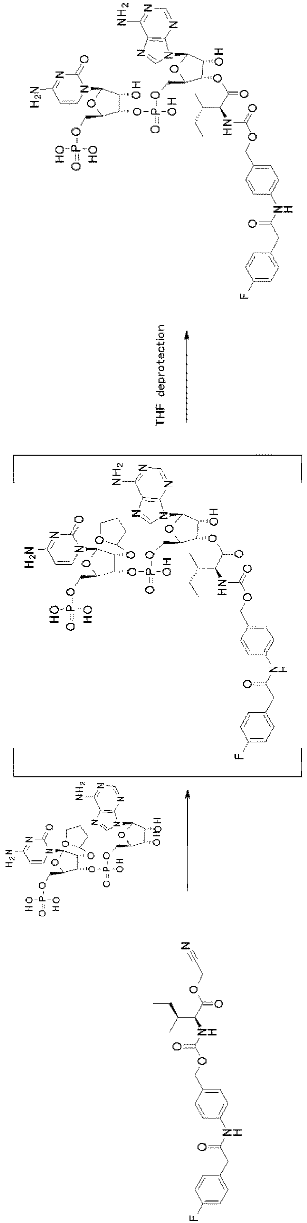

((2R,3R,4R, SR)-5-(4-amino-2-oxopyrimidin-1 (2H)-yl)-3-(((((2R,3 S,4R, SR)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (72.2 mg, 0.100 mmol) synthesized by a method described in the literature (Helv. Chim. Acta, 90, 297-310) was dissolved in Buffer A (60 mL), a solution of cyanomethyl (((4-(2-(4-fluorophenyl)acetamido)benzyl)oxy)carbonyl)-L-isoleucinate (Compound SS62, F-Pnaz-Ile-OCH2CN) (45.5 mg, 0.100 mmol) in acetonitrile (3.00 mL) was added, and the mixture was stirred at room temperature for 20 hours. The reaction solution was cooled to 0°C, and then trifluoroacetic acid (3.00 mL) was added. The reaction solution was stirred at room temperature for 30 minutes, and then purified by reverse-phase silica gel column chromatography (0.05% aqueous trifluoroacetic acid solution/0.05% trifluoroacetic acid-acetonitrile) to obtain the title compound (Compound SS51, F-Pnaz-Ile-pCpA) (12 mg, 11.4%).

LCMS(ESI) m/z = 1049.4 (M-H)-

Retention time: 0.54 minutes (analysis condition SQDFA05_02)

### Example 2. Synthesis of BdpFL-Phe-pCpA(MT01)

### Synthesis of (3-(5,5-difluoro-7,9-dimethyl-5H-4λ4,5λ4-dipyrrolo[1,2-c:2',1'-f][1,3,2]diazaborinin-3-yl)propanoyl)-L-phenylalanine (Compound MT02, BdpFL-Phe-OH)

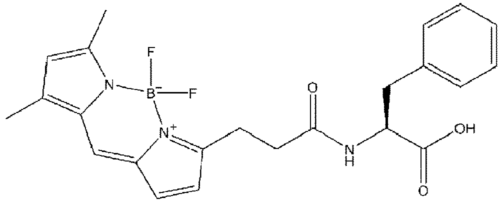

Under nitrogen atmosphere, DIC (0.128 mL, 0.822 mmol) was added at room temperature to a solution of 3-(2-carboxyethyl)-5,5-difluoro-7,9-dimethyl-5H-5λ4-dipyrrolo[1,2-c:2',1'-fJ[1,3,2]diazaborinin-4-ium (200 mg, 0.685 mmol) and 1-hydroxypyrrolidine-2,5-dione (87 mg, 0.753 mmol) in NMP (4.5 mL), and then the mixture was stirred at 40°C overnight. After the reaction solution was returned to room temperature, L-phenylalanine (113 mg, 0.685 mmol) and TEA (0.191 mL, 1.369 mmol) were added to it, and stirred at 40°C overnight. The reaction solution was purified by reverse-phase column chromatography (0.1 % FA MeCN/H₂O) to obtain (3-(5,5-difluoro-7,9-dimethyl-5H-4λ4,5λ4-dipyrrolo[1,2-c:2',1'-f][1,3,2]diazaborinin-3-yl)propanoyl)-L-phenylalanine (Compound MT02, BdpFL-Phe-OH) (102 mg, 34% yield).

LCMS (ESI) m/z = 438.3 (M-H)-

Retention time: 0.78 minutes (analysis condition SQDFA05_02)

### Synthesis of (3-(5,5-difluoro-7,9-dimethyl-5H-4λ4,5λ4-dipyrrolo[1,2-c:2',1'-f][1,3,2]diazaborinin-3-yl)propanoyl)-L-phenylalanine cyanomethyl ester (Compound MT03, BdpFL-Phe-OCH₂CN)

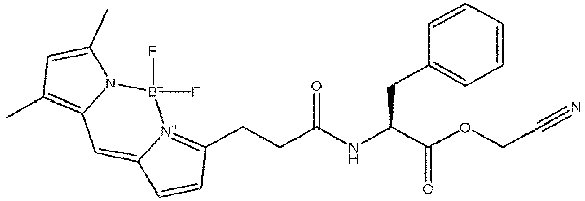

Under nitrogen atmosphere, (3-(5,5-difluoro-7,9-dimethyl-5H-4λ4,5λ4-dipyrrolo[1,2-c:2',1'-f][1,3,2]diazaborinin-3-yl)propanoyl)-L-phenylalanine (50 mg, 0.114 mmol) and N-ethyl-isopropylpropan-2-amine (DIPEA) (31.0 µL, 0.177 mmol) were dissolved in acetonitrile (500 µL), 2-bromoacetonitrile (12 µL, 0.177 mmol) was added at 0°C, and then the mixture was stirred at 40°C for three hours. The reaction solution was concentrated to obtain (3-(5,5-difluoro-7,9-dimethyl-5H-4λ4,5λ4-dipyrrolo[1,2-c:2',1'-f][1,3,2]diazaborinin-3-yl)propanoyl)-L-phenylalanine cyanomethyl ester (Compound MT03, BdpFL-Phe-OCHzCN) as a crude product. The obtained crude product was directly used in the next step.

LCMS (ESI) m/z = 477.3 (M-H)-

Retention time: 0.86 minutes (analysis condition SQDFA05_01)

### Synthesis of 3-(3-(((2S)-1-(((2R,3S,4R,5R)-2-((((((2R,3S,4R,5R)-5-(4-amino-2-oxopyrimidin-1(2H)-yl)-4-hydroxy-2-((phosphonooxy)methyl)tetrahydrofuran-3-yl)oxy)(hydroxy)phosphoryl)oxy)methyl)-5-(6-amino-9H-purin-9-yl)-4-hydroxytetrahydrofuran-3-yl)oxy)-1-oxo-3-phenylpropan-2-yl)amino)-3-oxopropyl)-5,5-difluoro-7,9-dimethyl-5H-5λ4-dipyrrolo[1,2-c:2',1'-f][1,3,2]diazaborinin-4-ium (Compound MT01, BdpFL-Phe-pCpA)

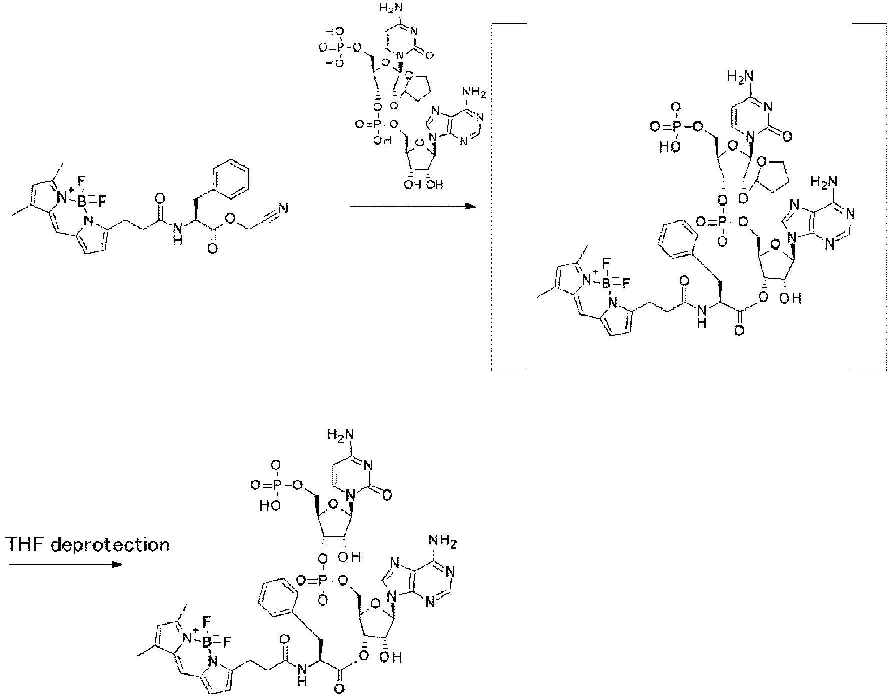

((2R,3R,4R,5R)-5-(4-amino-2-oxopyrimidin-1(2H)-yl)-3-(((((2R,3S,4R,5R)-5-(6-amino-9H-purin-9-yl)-3,4-dihydroxytetrahydrofuran-2-yl)methoxy)(hydroxy)phosphoryl)oxy)-4-((tetrahydrofuran-2-yl)oxy)tetrahydrofuran-2-yl)methyl dihydrogen phosphate (Compound pc01) (33.2 mg, 0.046 mmol) was dissolved in Buffer A (11.3 mL), a solution of (3-(5,5-difluoro-7,9-dimethyl-5H-4λ4,5λ4-dipyrrolo[1,2-c:2',1'-f][1,3,2]diazaborinin-3 -yl)propanoyl)-L-phenylalanine cyanomethyl ester (Compound MT03, BdpFL-Phe-OCHzCN) (11 mg, 0.023 mmol) in acetonitrile (0.13 mL) was added, and then the mixture was stirred at room temperature for 45 minutes. TFA (0.56 mL) was added to the reaction solution at 0°C and stirred for five minutes, and then stirred at room temperature for ten minutes. The reaction solution was purified by reverse-phase silica gel column chromatography (0.05% TFA MeCN/H₂O) to obtain the title compound (Compound MT01, BdpFL-Phe-pCpA) (2.1 mg, 8.5% yield).

LCMS (ESI) m/z = 1072.5 (M-H)-

Retention time: 0.56 minutes (analysis condition SQDFA05_02)

### Example 3. Synthesis of a peptide (LCT-67) having BdpFL at the N terminus, which is to be used as an authentic sample for LC/MS

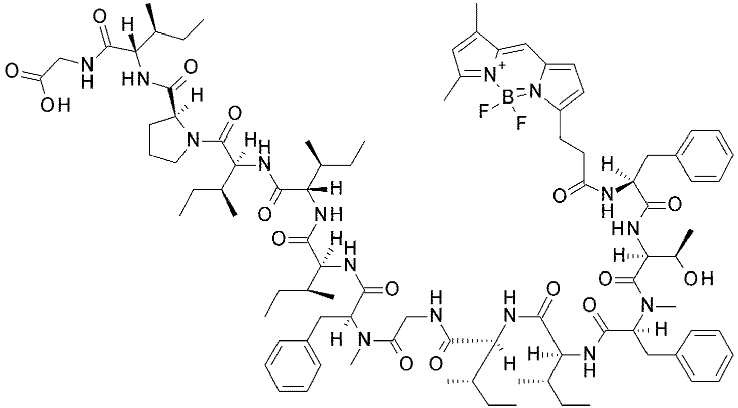

Using 2-chlorotrityl resin bearing Fmoc-Gly-OH (100 mg), and using Fmoc-Gly-OH, Fmoc-Thr(THP)-OH (aa01) synthesized by a method described in the patent literature (WO2018225864), Fmoc-Ile-OH, Fmoc-Leu-OH, Fmoc-Phe-OH, Fmoc-MePhe-OH, and Fmoc-Pro-OH as Fmoc amino acids, peptide elongation was performed on a peptide synthesizer (abbreviations of amino acids are described elsewhere in this specification). Peptide elongation was performed according to a peptide synthesis method using the Fmoc method (WO2013100132B2). After the peptide elongation, removal of the N-terminal Fmoc group was performed on the peptide synthesizer, and then the resin was washed with DCM.

TFE/DCM (1:1, v/v, 2 mL) was added to the resin and shaken for one hour, then the peptides were cleaved off from the resin. After completion of the reaction, the resin was removed by filtering the solution inside the tube through a column for synthesis, and the resin was washed twice with TFE/DCM (1:1, v/v, 1 mL). All of the extract solutions were mixed, DMF (2 mL) was added, and then the mixture was concentrated under reduced pressure. The obtained residue was dissolved in NMP (1 mL), 4,4-difluoro-5,7-dimethyl-4-bora-3a,4a-diaza-s-indacene-3-propionic acid N-succinimidyl ester (5 mg, 0.013 mmol) was added at room temperature, this mixture was stirred for 19 hours, then the reaction solution was subjected to reverse-phase silica gel column chromatography (0.1% FA MeCN/H₂O), and the fraction containing the intermediate was concentrated under reduced pressure. The obtained residue was dissolved in 5% TFA in DCM (2 mL) and stirred at room temperature for two hours. The reaction solution was concentrated under reduced pressure, and then the obtained residue was purified by reverse-phase silica gel column chromatography (0.1% FA MeCN/H₂O) to obtain the title compound (LCT-67) (13 mg). The amino acid sequence of LCT-67 is shown in SEQ ID NO: 223.

LCMS(ESI) m/z = 1751.2(M-H)-

Retention time: 0.97 minutes (analysis condition SQDFA05_02)

### Example 4. Synthesis of a peptide (LCT-12) having BdpFL at the N terminus, which is to be used as an authentic sample for LC/MS

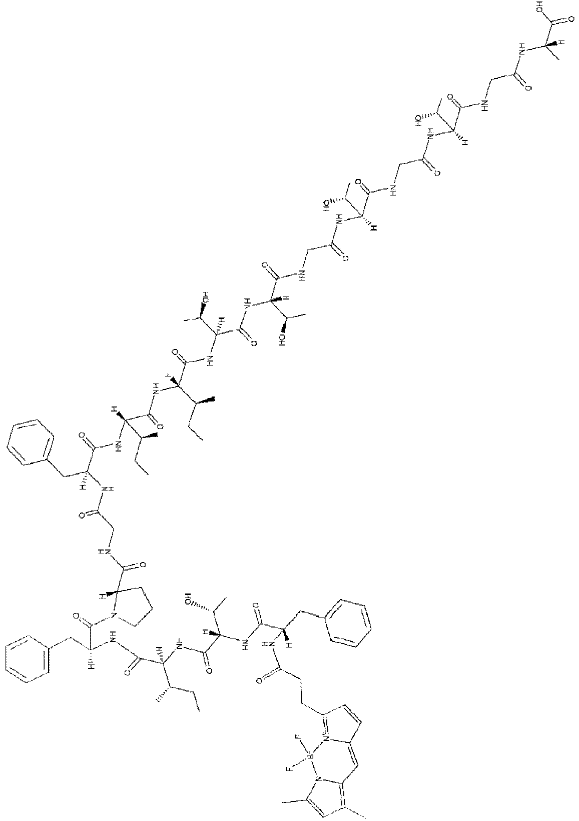

Using 2-chlorotrityl resin bearing Fmoc-Ala-OH (100 mg), and using Fmoc-Gly-OH, Fmoc-Thr(THP)-OH, Fmoc-Ile-OH, Fmoc-Phe-OH, and Fmoc-Pro-OH as Fmoc amino acids, peptide elongation was performed on a peptide synthesizer (abbreviations of amino acids are described elsewhere in this specification). Peptide elongation was performed according to a peptide synthesis method using the Fmoc method (WO2013100132B2). After the peptide elongation, removal of the N-terminal Fmoc group was performed on the peptide synthesizer, and then the resin was washed with DCM.

TFE/DCM (1:1, v/v, 2 mL) was added to the resin and shaken for one hour, then the peptides were cleaved off from the resin. After completion of the reaction, the resin was removed by filtering the solution inside the tube through a column for synthesis, and the resin was washed twice with TFE/DCM (1:1, v/v, 1 mL). All of the extracts were mixed, DMF (2 mL) was added, and then the mixture was concentrated under reduced pressure. The obtained residue was dissolved in NMP (0.5 mL), and one-fourth (125 µL) of it was used in the next reaction. To the peptide solution in NMP, BdpFL succinimide ester (140 µL) adjusted to 76.5 mM was added at room temperature, stirred overnight at 40°C, and then concentrated under reduced pressure. The obtained residue was dissolved in 0.05 M tetramethylammonium hydrogen sulfate in HFIP (1.2 mL, 0.060 mmol) and stirred at room temperature for two hours. The reaction solution was purified by reverse-phase silica gel column chromatography (0.1% FA MeCN/H₂O) to obtain the title compound (LCT-12) (0.3 mg). The amino acid sequence of LCT-12 is shown in SEQ ID NO: 222.

LCMS (ESI) m/z = 1972.9 (M-H)-

Retention time: 0.74 minutes (analysis condition SQDFA05_01)

### Example 5. Synthesis of aminoacyl tRNAs

From template DNAs (SEQ ID NO: 1 (D-1) to SEQ ID NO: 34 (D-34), SEQ ID NO: 35 (D-76) to SEQ ID NO: 37 (D-78) and SEQ ID NO: 224 (D-82) to SEQ ID NO: 241 (D-99)), tRNAs (SEQ ID NO: 38 (TR-1) to SEQ ID NO: 74 (TR-37) and SEQ ID NO: 242 (TR-38) to SEQ ID NO: 259 (TR-55)) were synthesized by in vitro transcription reaction using T7 RNA polymerase, and were purified by RNeasy kit (Qiagen).

Template DNA SEQ ID NO: 1 (D-1)
   DNA sequence: 
GGCGTAATACGACTCACTATAGTCCCCTTCGTCTAGAGGCCCAGGACACCGCCCTAA
GACGGCGGTAACAGGGGTTCGAATCCCCTAGGGGACGC
Template DNA SEQ ID NO: 2 (D-2)
   DNA sequence: 
GGCGTAATACGACTCACTATAGTCCCCTTCGTCTAGAGGCCCAGGACACCGCCCTTA
GACGGCGGTAACAGGGGTTCGAATCCCCTAGGGGACGC
Template DNA SEQ ID NO: 3 (D-3)
   DNA sequence: 
GGCGTAATACGACTCACTATAGTCCCCTTCGTCTAGAGGCCCAGGACACCGCCCTCA
GACGGCGGTAACAGGGGTTCGAATCCCCTAGGGGACGC
Template DNA SEQ ID NO: 4 (D-4)
   DNA sequence: 
GGCGTAATACGACTCACTATAGTCCCCTTCGTCTAGAGGCCCAGGACACCGCCCTAA
CACGGCGGTAACAGGGGTTCGAATCCCCTAGGGGACGC
Template DNA SEQ ID NO: 5 (D-5)
   DNA sequence: 
GGCGTAATACGACTCACTATAGTCCCCTTCGTCTAGAGGCCCAGGACACCGCCCTTA
CACGGCGGTAACAGGGGTTCGAATCCCCTAGGGGACGC
Template DNA SEQ ID NO: 6 (D-6)
   DNA sequence: 
GGCGTAATACGACTCACTATAGTCCCCTTCGTCTAGAGGCCCAGGACACCGCCCTCT
CACGGCGGTAACAGGGGTTCGAATCCCCTAGGGGACGC
Template DNA SEQ ID NO: 7 (D-7)
   DNA sequence: 
GGCGTAATACGACTCACTATAGTCCCCTTCGTCTAGAGGCCCAGGACACCGCCCTAT
GACGGCGGTAACAGGGGTTCGAATCCCCTAGGGGACGC
Template DNA SEQ ID NO: 8 (D-8)
   DNA sequence: 
GGCGTAATACGACTCACTATAGTCCCCTTCGTCTAGAGGCCCAGGACACCGCCCTTT
GACGGCGGTAACAGGGGTTCGAATCCCCTAGGGGACGC
Template DNA SEQ ID NO: 9 (D-9)
   DNA sequence: 
GGCGTAATACGACTCACTATAGTCCCCTTCGTCTAGAGGCCCAGGACACCGCCCTCT
GACGGCGGTAACAGGGGTTCGAATCCCCTAGGGGACGC
Template DNA SEQ ID NO: 10 (D-10)
   DNA sequence: 
GGCGTAATACGACTCACTATAGTCCCCTTCGTCTAGAGGCCCAGGACACCGCCCTAT
TACGGCGGTAACAGGGGTTCGAATCCCCTAGGGGACGC
Template DNA SEQ ID NO: 11 (D-11)
   DNA sequence: 
GGCGTAATACGACTCACTATAGTCCCCTTCGTCTAGAGGCCCAGGACACCGCCCTTT
TACGGCGGTAACAGGGGTTCGAATCCCCTAGGGGACGC
Template DNA SEQ ID NO: 12 (D-12)
   DNA sequence: 
GGCGTAATACGACTCACTATAGTCCCCTTCGTCTAGAGGCCCAGGACACCGCCCTCT
TACGGCGGTAACAGGGGTTCGAATCCCCTAGGGGACGC
Template DNA SEQ ID NO: 13 (D-13)
   DNA sequence: 
GGCGTAATACGACTCACTATAGTCCCCTTCGTCTAGAGGCCCAGGACACCGCCCTAT
CACGGCGGTAACAGGGGTTCGAATCCCCTAGGGGACGC
Template DNA SEQ ID NO: 14 (D-14)
   DNA sequence: 
GGCGTAATACGACTCACTATAGTCCCCTTCGTCTAGAGGCCCAGGACACCGCCCTTT
CACGGCGGTAACAGGGGTTCGAATCCCCTAGGGGACGC
Template DNA SEQ ID NO: 15 (D-15)
   DNA sequence: 
GGCGTAATACGACTCACTATAGTCCCCTTCGTCTAGAGGCCCAGGACACCGCCCTCT
CACGGCGGTAACAGGGGTTCGAATCCCCTAGGGGACGC
Template DNA SEQ ID NO: 16 (D-16)
   DNA sequence: 
GGCGTAATACGACTCACTATAGTCCCCTTCGTCTAGAGGCCCAGGACACCGCCCTAA
AACGGCGGTAACAGGGGTTCGAATCCCCTAGGGGACGC
Template DNA SEQ ID NO: 17 (D-17)
   DNA sequence: 
GGCGTAATACGACTCACTATAGTCCCCTTCGTCTAGAGGCCCAGGACACCGCCCTTA
AACGGCGGTAACAGGGGTTCGAATCCCCTAGGGGACGC
Template DNA SEQ ID NO: 18 (D-18)
   DNA sequence: 
GGCGTAATACGACTCACTATAGTCCCCTTCGTCTAGAGGCCCAGGACACCGCCCTCA
AACGGCGGTAACAGGGGTTCGAATCCCCTAGGGGACGC
Template DNA SEQ ID NO: 19 (D-19)
   DNA sequence: 
GGCGTAATACGACTCACTATAGTCCCCTTCGTCTAGAGGCCCAGGACACCGCCCTAC
TACGGCGGTAACAGGGGTTCGAATCCCCTAGGGGACGC
Template DNA SEQ ID NO: 20 (D-20)
   DNA sequence: 
GGCGTAATACGACTCACTATAGTCCCCTTCGTCTAGAGGCCCAGGACACCGCCCTTC
TACGGCGGTAACAGGGGTTCGAATCCCCTAGGGGACGC
Template DNA SEQ ID NO: 21 (D-21)
   DNA sequence: 
GGCGTAATACGACTCACTATAGTCCCCTTCGTCTAGAGGCCCAGGACACCGCCCTCC
TACGGCGGTAACAGGGGTTCGAATCCCCTAGGGGACGC
Template DNA SEQ ID NO: 22 (D-22)
   DNA sequence: 
GGCGTAATACGACTCACTATAGTCCCCTTCGTCTAGAGGCCCAGGACACCGCCCTGC
AACGGCGGTAACAGGGGTTCGAATCCCCTAGGGGACGC
Template DNA SEQ ID NO: 23 (D-23)
   DNA sequence: 
GGCGTAATACGACTCACTATAGTCCCCTTCGTCTAGAGGCCCAGGACACCGCCCTTC
AACGGCGGTAACAGGGGTTCGAATCCCCTAGGGGACGC
Template DNA SEQ ID NO: 24 (D-24)
   DNA sequence: 
GGCGTAATACGACTCACTATAGTCCCCTTCGTCTAGAGGCCCAGGACACCGCCCTCC
AACGGCGGTAACAGGGGTTCGAATCCCCTAGGGGACGC
Template DNA SEQ ID NO: 25 (D-25)
   DNA sequence: 
GGCGTAATACGACTCACTATAGGCGGGGTGGAGCAGCCTGGTAGCTCGTCGGGCTC
ATAACCCGAAGATCGTCGGTTCAAATCCGGCCCCCGCAA
Template DNA SEQ ID NO: 26 (D-26)
   DNA sequence: 
GGCGTAATACGACTCACTATAGTCCCCTTCGTCTAGAGGCCCAGGACACCGCCCTGT
GACGGCGGTAACAGGGGTTCGAATCCCCTAGGGGACGC
Template DNA SEQ ID NO: 27 (D-27)
   DNA sequence: 
GGCGTAATACGACTCACTATAGTCCCCTTCGTCTAGAGGCCCAGGACACCGCCCTGT
TACGGCGGTAACAGGGGTTCGAATCCCCTAGGGGACGC
Template DNA SEQ ID NO: 28 (D-28)
   DNA sequence: 
GGCGTAATACGACTCACTATAGTCCCCTTCGTCTAGAGGCCCAGGACACCGCCCTGT
CACGGCGGTAACAGGGGTTCGAATCCCCTAGGGGACGC
Template DNA SEQ ID NO: 29 (D-29)
   DNA sequence: 
GGCGTAATACGACTCACTATAGTCCCCTTCGTCTAGAGGCCCAGGACACCGCCCTGA
AACGGCGGTAACAGGGGTTCGAATCCCCTAGGGGACGC
Template DNA SEQ ID NO: 30 (D-30)
   DNA sequence: 
GGCGTAATACGACTCACTATAGTCCCCTTCGTCTAGAGGCCCAGGACACCGCCCTGC
TACGGCGGTAACAGGGGTTCGAATCCCCTAGGGGACGC
Template DNA SEQ ID NO: 31 (D-31)
   DNA sequence: 
GGCGTAATACGACTCACTATAGTCCCCTTCGTCTAGAGGCCCAGGACACCGCCCTTG
AACGGCGGTAACAGGGGTTCGAATCCCCTAGGGGACGC
Template DNA SEQ ID NO: 32 (D-32)
   DNA sequence: 
GGCGTAATACGACTCACTATAGTCCCCTTCGTCTAGAGGCCCAGGACACCGCCCTTG
TACGGCGGTAACAGGGGTTCGAATCCCCTAGGGGACGC
Template DNA SEQ ID NO: 33 (D-33)
   DNA sequence: 
GGCGTAATACGACTCACTATAGTCCCCTTCGTCTAGAGGCCCAGGACACCGCCCTTA
TACGGCGGTAACAGGGGTTCGAATCCCCTAGGGGACGC
Template DNA SEQ ID NO: 34 (D-34)
   DNA sequence: 
GGCGTAATACGACTCACTATAGGCCCCTTAGCTCAGTGGTTAGAGCAGGCGACTTAT
AATCGCTTGGTCGCTGGTTCAAGTCCAGCAGGGGCCAC
Template DNA SEQ ID NO: 35 (D-76)
   DNA sequence: 
GGCGTAATACGACTCACTATAGTCCCCTTCGTCTAGAGGCCCAGGACACCGCCCTGC
GACGGCGGTAACAGGGGTTCGAATCCCCTAGGGGACGC
Template DNA SEQ ID NO: 36 (D-77)
   DNA sequence: 
GGCGTAATACGACTCACTATAGTCCCCTTCGTCTAGAGGCCCAGGACACCGCCCTTC
GACGGCGGTAACAGGGGTTCGAATCCCCTAGGGGACGC
Template DNA SEQ ID NO: 37 (D-78)
   DNA sequence: 
GGCGTAATACGACTCACTATAGTCCCCTTCGTCTAGAGGCCCAGGACACCGCCCTCC
GACGGCGGTAACAGGGGTTCGAATCCCCTAGGGGACGC
Template DNA SEQ ID NO: 224 (D-82)
   DNA sequence: 
GGCGTAATACGACTCACTATAGGAGCGGTAGTTCAGTCGGTTAGAATACCTGCCTAU
GACGCAGGGGGTCGCGGGTTCGAGTCCCGTCCGTTCCGC
Template DNA SEQ ID NO: 225 (D-83)
   DNA sequence: 
GGCGTAATACGACTCACTATAGGAGCGGTAGTTCAGTCGGTTAGAATACCTGCTTAU
GATGCAGGGGGTCGCGGGTTCGAGTCCCGTCCGTTCCGC
Template DNA SEQ ID NO: 226 (D-84)
   DNA sequence: 
GGCGTAATACGACTCACTATAGGAGCGGTAGTTCAGTCGGTTAGAATACCTGCCTGU
GACGCAGGGGGTCGCGGGTTCGAGTCCCGTCCGTTCCGC
Template DNA SEQ ID NO: 227 (D-85)
   DNA sequence: 
GGCGTAATACGACTCACTATAGGAGCGGTAGTTCAGTCGGTTAGAATACCTGCTTGU
GATGCAGGGGGTCGCGGGTTCGAGTCCCGTCCGTTCCGC
Template DNA SEQ ID NO: 228 (D-86)
   DNA sequence: 
GGCGTAATACGACTCACTATAGGAGCGGTAGTTCAGTCGGTTAGAATACCTGCCTUU
GACGCAGGGGGTCGCGGGTTCGAGTCCCGTCCGTTCCGC
Template DNA SEQ ID NO: 229 (D-87)
   DNA sequence: 
GGCGTAATACGACTCACTATAGGAGCGGTAGTTCAGTCGGTTAGAATACCTGCTTUU
GATGCAGGGGGTCGCGGGTTCGAGTCCCGTCCGTTCCGC
Template DNA SEQ ID NO: 230 (D-88)
   DNA sequence: 
GGCGTAATACGACTCACTATAGGAGCGGTAGTTCAGTCGGTTAGAATACCTGCCTCU
GACGCAGGGGGTCGCGGGTTCGAGTCCCGTCCGTTCCGC
Template DNA SEQ ID NO: 231 (D-89)
   DNA sequence: 
GGCGTAATACGACTCACTATAGGAGCGGTAGTTCAGTCGGTTAGAATACCTGCTTCU
GATGCAGGGGGTCGCGGGTTCGAGTCCCGTCCGTTCCGC
Template DNA SEQ ID NO: 232 (D-90)
   DNA sequence: 
GGCGTAATACGACTCACTATAGGAGCGGTAGTTCAGTCGGTTAGAATACCTGCCTAU
CACGCAGGGGGTCGCGGGTTCGAGTCCCGTCCGTTCCGC
Template DNA SEQ ID NO: 233 (D-91)
   DNA sequence: 
GGCGTAATACGACTCACTATAGGAGCGGTAGTTCAGTCGGTTAGAATACCTGCCTUU
CACGCAGGGGGTCGCGGGTTCGAGTCCCGTCCGTTCCGC
Template DNA SEQ ID NO: 234 (D-92)
   DNA sequence: 
GGCGTAATACGACTCACTATAGGAGCGGTAGTTCAGTCGGTTAGAATACCTGCCTCU
CACGCAGGGGGTCGCGGGTTCGAGTCCCGTCCGTTCCGC
Template DNA SEQ ID NO: 235 (D-93)
   DNA sequence: 
GGCGTAATACGACTCACTATAGGCTCTGTAGTTCAGTCGGTAGAACGGCGGATTATG
ATTCCGTATGTCACTGGTTCGAGTCCAGTCAGAGCCGC
Template DNA SEQ ID NO: 236 (D-94)
   DNA sequence: 
GGCGTAATACGACTCACTATAGGCTCTGTAGTTCAGTCGGTAGAACGGCGGATTGTG
ATTCCGTATGTCACTGGTTCGAGTCCAGTCAGAGCCGC
Template DNA SEQ ID NO: 237 (D-95)
   DNA sequence: 
GGCGTAATACGACTCACTATAGGCTCTGTAGTTCAGTCGGTAGAACGGCGGATTTTG
ATTCCGTATGTCACTGGTTCGAGTCCAGTCAGAGCCGC
Template DNA SEQ ID NO: 238 (D-96)
   DNA sequence: 
GGCGTAATACGACTCACTATAGGCTCTGTAGTTCAGTCGGTAGAACGGCGGATTCTG
ATTCCGTATGTCACTGGTTCGAGTCCAGTCAGAGCCGC
Template DNA SEQ ID NO: 239 (D-97)
   DNA sequence: 
GGCGTAATACGACTCACTATAGGCTCTGTAGTTCAGTCGGTAGAACGGCGGACTATC
ACTCCGTATGTCACTGGTTCGAGTCCAGTCAGAGCCGC
Template DNA SEQ ID NO: 240 (D-98)
   DNA sequence: 
GGCGTAATACGACTCACTATAGGCTCTGTAGTTCAGTCGGTAGAACGGCGGACTTTC
ACTCCGTATGTCACTGGTTCGAGTCCAGTCAGAGCCGC
Template DNA SEQ ID NO: 241 (D-99)
   DNA sequence: 
GGCGTAATACGACTCACTATAGGCTCTGTAGTTCAGTCGGTAGAACGGCGGACTCTC
ACTCCGTATGTCACTGGTTCGAGTCCAGTCAGAGCCGC
tRNA SEQ ID NO: 38 (TR-1)
   tRNA(Glu)aag-CA RNA sequence: 
GUCCCCUUCGUCUAGAGGCCCAGGACACCGCCCUAAGACGGCGGUAACAGGGGUU
CGAAUCCCCUAGGGGACGC
tRNA SEQ ID NO: 39 (TR-2)
   tRNA(Glu)uag-CA RNA sequence: 
GUCCCCUUCGUCUAGAGGCCCAGGACACCGCCCUUAGACGGCGGUAACAGGGGUU
CGAAUCCCCUAGGGGACGC
tRNA SEQ ID NO: 40 (TR-3)
   tRNA(Glu)cag-CA RNA sequence: GUCCCCUUCGUCUAGAGGCCCAGGACACCGCCCUCAGACGGCGGUAACAGGGGUU CGAAUCCCCUAGGGGACGC
tRNA SEQ ID NO: 41 (TR-4)
   tRNA(Glu)aac-CA RNA sequence: 
GUCCCCUUCGUCUAGAGGCCCAGGACACCGCCCUAACACGGCGGUAACAGGGGUU
CGAAUCCCCUAGGGGACGC
tRNA SEQ ID NO: 42 (TR-5)
   tRNA(Glu)uac-CA RNA sequence: 
GUCCCCUUCGUCUAGAGGCCCAGGACACCGCCCUUACACGGCGGUAACAGGGGUU
CGAAUCCCCUAGGGGACGC
tRNA SEQ ID NO: 43 (TR-6)
   tRNA(Glu)cac-CA RNA sequence: 
GUCCCCUUCGUCUAGAGGCCCAGGACACCGCCCUCACACGGCGGUAACAGGGGUU
CGAAUCCCCUAGGGGACGC
tRNA SEQ ID NO: 44 (TR-7)
   tRNA(Glu)aug-CA RNA sequence: 
GUCCCCUUCGUCUAGAGGCCCAGGACACCGCCCUAUGACGGCGGUAACAGGGGUU
CGAAUCCCCUAGGGGACGC
tRNA SEQ ID NO: 45 (TR-8)
   tRNA(Glu)uug-CA RNA sequence: 
GUCCCCUUCGUCUAGAGGCCCAGGACACCGCCCUUUGACGGCGGUAACAGGGGUU
CGAAUCCCCUAGGGGACGC
tRNA SEQ ID NO: 46 (TR-9)
   tRNA(Glu)cug-CA RNA sequence: 
GUCCCCUUCGUCUAGAGGCCCAGGACACCGCCCUCUGACGGCGGUAACAGGGGUU
CGAAUCCCCUAGGGGACGC
tRNA SEQ ID NO: 47 (TR-10)
   tRNA(Glu)auu-CA RNA sequence: 
GUCCCCUUCGUCUAGAGGCCCAGGACACCGCCCUAUUACGGCGGUAACAGGGGUU
CGAAUCCCCUAGGGGACGC
tRNA SEQ ID NO: 48 (TR-11)
   tRNA(Glu)uuu-CA RNA sequence: 
GUCCCCUUCGUCUAGAGGCCCAGGACACCGCCCUUUUACGGCGGUAACAGGGGUU
CGAAUCCCCUAGGGGACGC
tRNA SEQ ID NO: 49 (TR-12)
   tRNA(Glu)cuu-CA RNA sequence: GUCCCCUUCGUCUAGAGGCCCAGGACACCGCCCUCUUACGGCGGUAACAGGGGUU CGAAUCCCCUAGGGGACGC
tRNA SEQ ID NO: 50 (TR-13)
   tRNA(Glu)auc-CA RNA sequence: 
GUCCCCUUCGUCUAGAGGCCCAGGACACCGCCCUAUCACGGCGGUAACAGGGGUU
CGAAUCCCCUAGGGGACGC
tRNA SEQ ID NO: 51 (TR-14)
   tRNA(Glu)uuc-CA RNA sequence: 
GUCCCCUUCGUCUAGAGGCCCAGGACACCGCCCUUUCACGGCGGUAACAGGGGUU
CGAAUCCCCUAGGGGACGC
tRNA SEQ ID NO: 52 (TR-15)
   tRNA(Glu)cuc-CA RNA sequence: 
GUCCCCUUCGUCUAGAGGCCCAGGACACCGCCCUCUCACGGCGGUAACAGGGGUU
CGAAUCCCCUAGGGGACGC
tRNA SEQ ID NO: 53 (TR-16)
   tRNA(Glu)aaa-CA RNA sequence: 
GUCCCCUUCGUCUAGAGGCCCAGGACACCGCCCUAAAACGGCGGUAACAGGGGUU
CGAAUCCCCUAGGGGACGC
tRNA SEQ ID NO: 54 (TR-17)
   tRNA(Glu)uaa-CA RNA sequence: 
GUCCCCUUCGUCUAGAGGCCCAGGACACCGCCCUUAAACGGCGGUAACAGGGGUU
CGAAUCCCCUAGGGGACGC
tRNA SEQ ID NO: 55 (TR-18)
   tRNA(Glu)caa-CA RNA sequence: 
GUCCCCUUCGUCUAGAGGCCCAGGACACCGCCCUCAAACGGCGGUAACAGGGGUU
CGAAUCCCCUAGGGGACGC
tRNA SEQ ID NO: 56 (TR-19)
   tRNA(Glu)acu-CA RNA sequence: 
GUCCCCUUCGUCUAGAGGCCCAGGACACCGCCCUACUACGGCGGUAACAGGGGUU
CGAAUCCCCUAGGGGACGC
tRNA SEQ ID NO: 57 (TR-20)
   tRNA(Glu)ucu-CA RNA sequence: 
GUCCCCUUCGUCUAGAGGCCCAGGACACCGCCCUUCUACGGCGGUAACAGGGGUU
CGAAUCCCCUAGGGGACGC
tRNA SEQ ID NO: 58 (TR-21)
   tRNA(Glu)ccu-CA RNA sequence: GUCCCCUUCGUCUAGAGGCCCAGGACACCGCCCUCCUACGGCGGUAACAGGGGUU CGAAUCCCCUAGGGGACGC
tRNA SEQ ID NO: 59 (TR-22)
   tRNA(Glu)gca-CA RNA sequence: 
GUCCCCUUCGUCUAGAGGCCCAGGACACCGCCCUGCAACGGCGGUAACAGGGGUU
CGAAUCCCCUAGGGGACGC
tRNA SEQ ID NO: 60 (TR-23)
   tRNA(Glu)uca-CA RNA sequence: 
GUCCCCUUCGUCUAGAGGCCCAGGACACCGCCCUUCAACGGCGGUAACAGGGGUU
CGAAUCCCCUAGGGGACGC
tRNA SEQ ID NO: 61 (TR-24)
   tRNA(Glu)cca-CA RNA sequence: 
GUCCCCUUCGUCUAGAGGCCCAGGACACCGCCCUCCAACGGCGGUAACAGGGGUU
CGAAUCCCCUAGGGGACGC
tRNA SEQ ID NO: 62 (TR-25)
   tRNA(fMet)cau-CA RNA sequence: 
GGCGGGGUGGAGCAGCCUGGUAGCUCGUCGGGCUCAUAACCCGAAGAUCGUCGGU
UCAAAUCCGGCCCCCGCAA
tRNA SEQ ID NO: 63 (TR-26)
   tRNA(Glu)gug-CA RNA sequence: 
GUCCCCUUCGUCUAGAGGCCCAGGACACCGCCCUGUGACGGCGGUAACAGGGGUU
CGAAUCCCCUAGGGGACGC
tRNA SEQ ID NO: 64 (TR-27)
   tRNA(Glu)guu-CA RNA sequence: 
GUCCCCUUCGUCUAGAGGCCCAGGACACCGCCCUGUUACGGCGGUAACAGGGGUU
CGAAUCCCCUAGGGGACGC
tRNA SEQ ID NO: 65 (TR-28)
   tRNA(Glu)guc-CA RNA sequence: 
GUCCCCUUCGUCUAGAGGCCCAGGACACCGCCCUGUCACGGCGGUAACAGGGGUU
CGAAUCCCCUAGGGGACGC
tRNA SEQ ID NO: 66 (TR-29)
   tRNA(Glu)gaa-CA RNA sequence: 
GUCCCCUUCGUCUAGAGGCCCAGGACACCGCCCUGAAACGGCGGUAACAGGGGUU
CGAAUCCCCUAGGGGACGC
tRNA SEQ ID NO: 67 (TR-30)
   tRNA(Glu)gcu-CA RNA sequence: GUCCCCUUCGUCUAGAGGCCCAGGACACCGCCCUGCUACGGCGGUAACAGGGGUU CGAAUCCCCUAGGGGACGC
tRNA SEQ ID NO: 68 (TR-31)
   tRNA(Glu)uga-CA RNA sequence: 
GUCCCCUUCGUCUAGAGGCCCAGGACACCGCCCUUGAACGGCGGUAACAGGGGUU
CGAAUCCCCUAGGGGACGC
tRNA SEQ ID NO: 69 (TR-32)
   tRNA(Glu)ugu-CA RNA sequence: 
GUCCCCUUCGUCUAGAGGCCCAGGACACCGCCCUUGUACGGCGGUAACAGGGGUU
CGAAUCCCCUAGGGGACGC
tRNA SEQ ID NO: 70 (TR-33)
   tRNA(Glu)uau-CA RNA sequence: 
GUCCCCUUCGUCUAGAGGCCCAGGACACCGCCCUUAUACGGCGGUAACAGGGGUU
CGAAUCCCCUAGGGGACGC
tRNA SEQ ID NO: 71 (TR-34)
   tRNA(Ile)uau-CA RNA sequence: 
GGCCCCUUAGCUCAGUGGUUAGAGCAGGCGACUUAUAAUCGCUUGGUCGCUGGU
UCAAGUCCAGCAGGGGCCAC
tRNA SEQ ID NO: 72 (TR-35)
   tRNA(Glu)gcg-CA RNA sequence: 
GUCCCCUUCGUCUAGAGGCCCAGGACACCGCCCUGCGACGGCGGUAACAGGGGUU
CGAAUCCCCUAGGGGACGC
tRNA SEQ ID NO: 73 (TR-36)
   tRNA(Glu)ucg-CA RNA sequence: 
GUCCCCUUCGUCUAGAGGCCCAGGACACCGCCCUUCGACGGCGGUAACAGGGGUU
CGAAUCCCCUAGGGGACGC
tRNA SEQ ID NO: 74 (TR-37)
   tRNA(Glu)ccg-CA RNA sequence: 
GUCCCCUUCGUCUAGAGGCCCAGGACACCGCCCUCCGACGGCGGUAACAGGGGUU
CGAAUCCCCUAGGGGACGC
tRNA SEQ ID NO: 242 (TR-38)
   tRNA(Asp)aug-CA RNA sequence: 
GGAGCGGUAGUUCAGUCGGUUAGAAUACCUGCCUAUGACGCAGGGGGUCGCGGG
UUCGAGUCCCGUCCGUUCCGC
tRNA SEQ ID NO: 243 (TR-39)
   tRNA(Asp)ccg-CA RNA sequence: GGAGCGGUAGUUCAGUCGGUUAGAAUACCUGCUUAUGAUGCAGGGGGUCGCGGG UUCGAGUCCCGUCCGUUCCGC
tRNA SEQ ID NO: 244 (TR-40)
   tRNA(Asp)gug-CA RNA sequence: 
GGAGCGGUAGUUCAGUCGGUUAGAAUACCUGCCUGUGACGCAGGGGGUCGCGGG
UUCGAGUCCCGUCCGUUCCGC
tRNA SEQ ID NO: 245 (TR-41)
   tRNA(Asp)gug-CA RNA sequence: 
GGAGCGGUAGUUCAGUCGGUUAGAAUACCUGCUUGUGAUGCAGGGGGUCGCGGG
UUCGAGUCCCGUCCGUUCCGC
tRNA SEQ ID NO: 246 (TR-42)
   tRNA(Asp)uug-CA RNA sequence: 
GGAGCGGUAGUUCAGUCGGUUAGAAUACCUGCCUUUGACGCAGGGGGUCGCGGG
UUCGAGUCCCGUCCGUUCCGC
tRNA SEQ ID NO: 247 (TR-43)
   tRNA(Asp)uug-CA RNA sequence: 
GGAGCGGUAGUUCAGUCGGUUAGAAUACCUGCUUUUGAUGCAGGGGGUCGCGGG
UUCGAGUCCCGUCCGUUCCGC
tRNA SEQ ID NO: 248 (TR-44)
   tRNA(Asp)cug-CA RNA sequence: 
GGAGCGGUAGUUCAGUCGGUUAGAAUACCUGCCUCUGACGCAGGGGGUCGCGGG
UUCGAGUCCCGUCCGUUCCGC
tRNA SEQ ID NO: 249 (TR-45)
   tRNA(Asp)cug-CA RNA sequence: 
GGAGCGGUAGUUCAGUCGGUUAGAAUACCUGCUUCUGAUGCAGGGGGUCGCGGG
UUCGAGUCCCGUCCGUUCCGC
tRNA SEQ ID NO: 250 (TR-46)
   tRNA(Asp)auc-CA RNA sequence: 
GGAGCGGUAGUUCAGUCGGUUAGAAUACCUGCCUAUCACGCAGGGGGUCGCGGG
UUCGAGUCCCGUCCGUUCCGC
tRNA SEQ ID NO: 251 (TR-47)
   tRNA(Asp)uuc-CA RNA sequence: 
GGAGCGGUAGUUCAGUCGGUUAGAAUACCUGCCUUUCACGCAGGGGGUCGCGGG
UUCGAGUCCCGUCCGUUCCGC
tRNA SEQ ID NO: 252 (TR-48)
   tRNA(Asp)cuc-CA RNA sequence: GGAGCGGUAGUUCAGUCGGUUAGAAUACCUGCCUCUCACGCAGGGGGUCGCGGG UUCGAGUCCCGUCCGUUCCGC
tRNA SEQ ID NO: 253 (TR-49)
   tRNA(AsnE2)aug-CA RNA sequence: 
GGCUCUGUAGUUCAGUCGGUAGAACGGCGGAUUAUGAUUCCGUAUGUCACUGGU
UCGAGUCCAGUCAGAGCCGC
tRNA SEQ ID NO: 254 (TR-50)
   tRNA(AsnE2)gug-CA RNA sequence: 
GGCUCUGUAGUUCAGUCGGUAGAACGGCGGAUUGUGAUUCCGUAUGUCACUGGU
UCGAGUCCAGUCAGAGCCGC
tRNA SEQ ID NO: 255 (TR-51)
   tRNA(AsnE2)uug-CA RNA sequence: 
GGCUCUGUAGUUCAGUCGGUAGAACGGCGGAUUUUGAUUCCGUAUGUCACUGGU
UCGAGUCCAGUCAGAGCCGC
tRNA SEQ ID NO: 256 (TR-52)
   tRNA(AsnE2)cug-CA RNA sequence: 
GGCUCUGUAGUUCAGUCGGUAGAACGGCGGAUUCUGAUUCCGUAUGUCACUGGU
UCGAGUCCAGUCAGAGCCGC
tRNA SEQ ID NO: 257 (TR-53)
   tRNA(AsnE2)auc-CA RNA sequence: 
GGCUCUGUAGUUCAGUCGGUAGAACGGCGGACUAUCACUCCGUAUGUCACUGGU
UCGAGUCCAGUCAGAGCCGC
tRNA SEQ ID NO: 258 (TR-54)
   tRNA(AsnE2)uuc-CA RNA sequence: 
GGCUCUGUAGUUCAGUCGGUAGAACGGCGGACUUUCACUCCGUAUGUCACUGGU
UCGAGUCCAGUCAGAGCCGC
tRNA SEQ ID NO: 259 (TR-55)
   tRNA(AsnE2)cuc-CA RNA sequence :

GGCUCUGUAGUUCAGUCGGUAGAACGGCGGACUCUCACUCCGUAUGUCACUGGUU
CGAGUCCAGUCAGAGCCGC

### Preparation of a mixed aminoacyl tRNA solution using aminoacyl pCpA

In order to prepare Compound AAtR-1, a reaction solution was prepared by adding Nuclease free water to adjust the solution to 25 µM transcribed tRNA(Glu)aag-CA (SEQ ID NO: 38 (TR-1)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of Compound ts14 described in WO2018143145A1 and synthesized by a method described in the patent literature (WO2018143145A1)), and ligation reaction was performed at 15°C for 45 minutes. It should be noted that before adding T4 RNA ligase and aminoacylated pCpA, the reaction solution was heated at 95°C for two minutes and then left at room temperature for five minutes to refold the tRNA in advance. Hereafter, all aminoacylated pCpAs starting with the notation "ts" or "TS" are compounds described in WO2018143145A1 and synthesized by a method described in WO2018143145A1.

Similarly, aminoacylated pCpA (SS14) was ligated to the transcribed tRNA(Glu)uag-CA (SEQ ID NO: 39 (TR-2)) by the method described above to prepare Compound AAtR-2.

Similarly, aminoacylated pCpA (TS24) was ligated to the transcribed tRNA(Glu)cag-CA (SEQ ID NO: 40 (TR-3)) by the method described above to prepare Compound AAtR-3.

Similarly, aminoacylated pCpA (ts14) was ligated to the transcribed tRNA(Glu)aac-CA (SEQ ID NO: 41 (TR-4)) by the method described above to prepare Compound AAtR-4.

Similarly, aminoacylated pCpA (SS14) was ligated to the transcribed tRNA(Glu)uac-CA (SEQ ID NO: 42 (TR-5)) by the method described above to prepare Compound AAtR-5.

Similarly, aminoacylated pCpA (TS24) was ligated to the transcribed tRNA(Glu)cac-CA (SEQ ID NO: 43 (TR-6)) by the method described above to prepare Compound AAtR-6.

Similarly, aminoacylated pCpA (ts14) was ligated to the transcribed tRNA(Glu)aug-CA (SEQ ID NO: 44 (TR-7)) by the method described above to prepare Compound AAtR-7.

Similarly, aminoacylated pCpA (SS14) was ligated to the transcribed tRNA(Glu)uug-CA (SEQ ID NO: 45 (TR-8)) by the method described above to prepare Compound AAtR-8.

Similarly, aminoacylated pCpA (TS24) was ligated to the transcribed tRNA(Glu)cug-CA (SEQ ID NO: 46 (TR-9)) by the method described above to prepare Compound AAtR-9.

Similarly, aminoacylated pCpA (ts14) was ligated to the transcribed tRNA(Glu)auu-CA (SEQ ID NO: 47 (TR-10)) by the method described above to prepare Compound AAtR-10.

Similarly, aminoacylated pCpA (SS14) was ligated to the transcribed tRNA(Glu)uuu-CA (SEQ ID NO: 48 (TR-11)) by the method described above to prepare Compound AAtR-11.

Similarly, aminoacylated pCpA (TS24) was ligated to the transcribed tRNA(Glu)cuu-CA (SEQ ID NO: 49 (TR-12)) by the method described above to prepare Compound AAtR-12.

Similarly, aminoacylated pCpA (ts14) was ligated to the transcribed tRNA(Glu)auc-CA (SEQ ID NO: 50 (TR-13)) by the method described above to prepare Compound AAtR-13.

Similarly, aminoacylated pCpA (SS14) was ligated to the transcribed tRNA(Glu)uuc-CA (SEQ ID NO: 51 (TR-14)) by the method described above to prepare Compound AAtR-14.

Similarly, aminoacylated pCpA (TS24) was ligated to the transcribed tRNA(Glu)cuc-CA (SEQ ID NO: 52 (TR-15)) by the method described above to prepare Compound AAtR-15.

Similarly, aminoacylated pCpA (ts14) was ligated to the transcribed tRNA(Glu)aaa-CA (SEQ ID NO: 53 (TR-16)) by the method described above to prepare Compound AAtR-16.

Similarly, aminoacylated pCpA (SS14) was ligated to the transcribed tRNA(Glu)uaa-CA (SEQ ID NO: 54 (TR-17)) by the method described above to prepare Compound AAtR-17.

Similarly, aminoacylated pCpA (TS24) was ligated to the transcribed tRNA(Glu)caa-CA (SEQ ID NO: 55 (TR-18)) by the method described above to prepare Compound AAtR-18.

Similarly, aminoacylated pCpA (ts14) was ligated to the transcribed tRNA(Glu)acu-CA (SEQ ID NO: 56 (TR-19)) by the method described above to prepare Compound AAtR-19.

Similarly, aminoacylated pCpA (SS14) was ligated to the transcribed tRNA(Glu)ucu-CA (SEQ ID NO: 57 (TR-20)) by the method described above to prepare Compound AAtR-20.

Similarly, aminoacylated pCpA (TS24) was ligated to the transcribed tRNA(Glu)ccu-CA (SEQ ID NO: 58 (TR-21)) by the method described above to prepare Compound AAtR-21.

Similarly, aminoacylated pCpA (ts14) was ligated to the transcribed tRNA(Glu)gca-CA (SEQ ID NO: 59 (TR-22)) by the method described above to prepare Compound AAtR-22.

Similarly, aminoacylated pCpA (SS14) was ligated to the transcribed tRNA(Glu)uca-CA (SEQ ID NO: 60 (TR-23)) by the method described above to prepare Compound AAtR-23.

Similarly, aminoacylated pCpA (TS24) was ligated to the transcribed tRNA(Glu)cca-CA (SEQ ID NO: 61 (TR-24)) by the method described above to prepare Compound AAtR-24.

Similarly, aminoacylated pCpA (ts14) was ligated to the transcribed tRNA(Glu)gug-CA (SEQ ID NO: 63 (TR-26)) by the method described above to prepare Compound AAtR-26.

Similarly, aminoacylated pCpA (ts14) was ligated to the transcribed tRNA(Glu)guu-CA (SEQ ID NO: 64 (TR-27)) by the method described above to prepare Compound AAtR-27.

Similarly, aminoacylated pCpA (ts14) was ligated to the transcribed tRNA(Glu)gcu-CA (SEQ ID NO: 67 (TR-30)) by the method described above to prepare Compound AAtR-28.

Similarly, aminoacylated pCpA (SS48) was ligated to the transcribed tRNA(Glu)aug-CA (SEQ ID NO: 44 (TR-7)) by the method described above to prepare Compound AAtR-29.

Similarly, aminoacylated pCpA (SS49) was ligated to the transcribed tRNA(Glu)uug-CA (SEQ ID NO: 45 (TR-8)) by the method described above to prepare Compound AAtR-30.

Similarly, aminoacylated pCpA (SS50) was ligated to the transcribed tRNA(Glu)cug-CA (SEQ ID NO: 46 (TR-9)) by the method described above to prepare Compound AAtR-31.

Similarly, aminoacylated pCpA (SS48) was ligated to the transcribed tRNA(Glu)auu-CA (SEQ ID NO: 47 (TR-10)) by the method described above to prepare Compound AAtR-32.

Similarly, aminoacylated pCpA (SS49) was ligated to the transcribed tRNA(Glu)uuu-CA (SEQ ID NO: 48 (TR-11)) by the method described above to prepare Compound AAtR-33.

Similarly, aminoacylated pCpA (SS50) was ligated to the transcribed tRNA(Glu)cuu-CA (SEQ ID NO: 49 (TR-12)) by the method described above to prepare Compound AAtR-34.

Similarly, aminoacylated pCpA (SS48) was ligated to the transcribed tRNA(Glu)auc-CA (SEQ ID NO: 50 (TR-13)) by the method described above to prepare Compound AAtR-35.

Similarly, aminoacylated pCpA (SS49) was ligated to the transcribed tRNA(Glu)uuc-CA (SEQ ID NO: 51 (TR-14)) by the method described above to prepare Compound AAtR-36.

Similarly, aminoacylated pCpA (SS50) was ligated to the transcribed tRNA(Glu)cuc-CA (SEQ ID NO: 52 (TR-15)) by the method described above to prepare Compound AAtR-37.

Similarly, aminoacylated pCpA (SS48) was ligated to the transcribed tRNA(Glu)aaa-CA (SEQ ID NO: 53 (TR-16)) by the method described above to prepare Compound AAtR-38.

Similarly, aminoacylated pCpA (SS49) was ligated to the transcribed tRNA(Glu)uaa-CA (SEQ ID NO: 54 (TR-17)) by the method described above to prepare Compound AAtR-39.

Similarly, aminoacylated pCpA (SS50) was ligated to the transcribed tRNA(Glu)caa-CA (SEQ ID NO: 55 (TR-18)) by the method described above to prepare Compound AAtR-40.

Similarly, aminoacylated pCpA (SS48) was ligated to the transcribed tRNA(Glu)acu-CA (SEQ ID NO: 56 (TR-19)) by the method described above to prepare Compound AAtR-41.

Similarly, aminoacylated pCpA (SS49) was ligated to the transcribed tRNA(Glu)ucu-CA (SEQ ID NO: 57 (TR-20)) by the method described above to prepare Compound AAtR-42.

Similarly, aminoacylated pCpA (SS50) was ligated to the transcribed tRNA(Glu)ccu-CA (SEQ ID NO: 58 (TR-21)) by the method described above to prepare Compound AAtR-43.

Similarly, aminoacylated pCpA (SS48) was ligated to the transcribed tRNA(Glu)gca-CA (SEQ ID NO: 59 (TR-22)) by the method described above to prepare Compound AAtR-44.

Similarly, aminoacylated pCpA (SS49) was ligated to the transcribed tRNA(Glu)uca-CA (SEQ ID NO: 60 (TR-23)) by the method described above to prepare Compound AAtR-45.

Similarly, aminoacylated pCpA (SS50) was ligated to the transcribed tRNA(Glu)cca-CA (SEQ ID NO: 61 (TR-24)) by the method described above to prepare Compound AAtR-46.

Similarly, aminoacylated pCpA (SS48) was ligated to the transcribed tRNA(Glu)gug-CA (SEQ ID NO: 63 (TR-26)) by the method described above to prepare Compound AAtR-47.

Similarly, aminoacylated pCpA (SS48) was ligated to the transcribed tRNA(Glu)guu-CA (SEQ ID NO: 64 (TR-27)) by the method described above to prepare Compound AAtR-48.

Similarly, aminoacylated pCpA (SS48) was ligated to the transcribed tRNA(Glu)guc-CA (SEQ ID NO: 65 (TR-28)) by the method described above to prepare Compound AAtR-49.

Similarly, aminoacylated pCpA (SS48) was ligated to the transcribed tRNA(Glu)gaa-CA (SEQ ID NO: 66 (TR-29)) by the method described above to prepare Compound AAtR-50.

Similarly, aminoacylated pCpA (SS48) was ligated to the transcribed tRNA(Glu)gcu-CA (SEQ ID NO: 67 (TR-30)) by the method described above to prepare Compound AAtR-51.

Similarly, aminoacylated pCpA (SS16) was ligated to the transcribed tRNA(Glu)uga-CA (SEQ ID NO: 68 (TR-31)) by the method described above to prepare Compound AAtR-52.

Similarly, aminoacylated pCpA (SS16) was ligated to the transcribed tRNA(Glu)ugu-CA (SEQ ID NO: 69 (TR-32)) by the method described above to prepare Compound AAtR-53.

Similarly, aminoacylated pCpA (SS16) was ligated to the transcribed tRNA(Glu)uau-CA (SEQ ID NO: 70 (TR-33)) by the method described above to prepare Compound AAtR-54.

Similarly, aminoacylated pCpA (SS16) was ligated to the transcribed tRNA(Ile)uau-CA (SEQ ID NO: 71 (TR-34)) by the method described above to prepare Compound AAtR-55.

Similarly, aminoacylated pCpA (SS51) was ligated to the transcribed tRNA(Ile)uau-CA (SEQ ID NO: 71 (TR-34)) by the method described above to prepare Compound AAtR-56.

Similarly, aminoacylated pCpA (ts14) was ligated to the transcribed tRNA(Glu)gcg-CA (SEQ ID NO: 72 (TR-35)) by the method described above to prepare Compound AAtR-57.

Similarly, aminoacylated pCpA (SS15) was ligated to the transcribed tRNA(Glu)ucg-CA (SEQ ID NO: 73 (TR-36)) by the method described above to prepare Compound AAtR-58.

Similarly, aminoacylated pCpA (TS24) was ligated to the transcribed tRNA(Glu)ccg-CA (SEQ ID NO: 74 (TR-37)) by the method described above to prepare Compound AAtR-59.

Similarly, aminoacylated pCpA (ts14) was ligated to the transcribed tRNA(Asp)aug-CA (SEQ ID NO: 242 (TR-38)) by the method described above to prepare Compound AAtR-60.

Similarly, aminoacylated pCpA (SS14) was ligated to the transcribed tRNA(Asp)uug-CA (SEQ ID NO: 246 (TR-42)) by the method described above to prepare Compound AAtR-61.

Similarly, aminoacylated pCpA (TS24) was ligated to the transcribed tRNA(Asp)cug-CA (SEQ ID NO: 248 (TR-44)) by the method described above to prepare Compound AAtR-62.

Similarly, aminoacylated pCpA (ts14) was ligated to the transcribed tRNA(Asp)aug-CA (SEQ ID NO: 243 (TR-39)) by the method described above to prepare Compound AAtR-63.

Similarly, aminoacylated pCpA (SS14) was ligated to the transcribed tRNA(Asp)uug-CA (SEQ ID NO: 247 (TR-43)) by the method described above to prepare Compound AAtR-64.

Similarly, aminoacylated pCpA (TS24) was ligated to the transcribed tRNA(Asp)cug-CA (SEQ ID NO: 249 (TR-45)) by the method described above to prepare Compound AAtR-65.

Similarly, aminoacylated pCpA (ts14) was ligated to the transcribed tRNA(Asp)gug-CA (SEQ ID NO: 244 (TR-40)) by the method described above to prepare Compound AAtR-66.

Similarly, aminoacylated pCpA (SS14) was ligated to the transcribed tRNA(Asp)uug-CA (SEQ ID NO: 246 (TR-42)) by the method described above to prepare Compound AAtR-67.

Similarly, aminoacylated pCpA (TS24) was ligated to the transcribed tRNA(Asp)cug-CA (SEQ ID NO: 248 (TR-44)) by the method described above to prepare Compound AAtR-68.

Similarly, aminoacylated pCpA (ts14) was ligated to the transcribed tRNA(Asp)gug-CA (SEQ ID NO: 245 (TR-41)) by the method described above to prepare Compound AAtR-69.

Similarly, aminoacylated pCpA (SS14) was ligated to the transcribed tRNA(Asp)uug-CA (SEQ ID NO: 247 (TR-43)) by the method described above to prepare Compound AAtR-70.

Similarly, aminoacylated pCpA (TS24) was ligated to the transcribed tRNA(Asp)cug-CA (SEQ ID NO: 249 (TR-45)) by the method described above to prepare Compound AAtR-71.

Similarly, aminoacylated pCpA (ts14) was ligated to the transcribed tRNA(Asp)auc-CA (SEQ ID NO: 250 (TR-46)) by the method described above to prepare Compound AAtR-72.

Similarly, aminoacylated pCpA (SS14) was ligated to the transcribed tRNA(Asp)uuc-CA (SEQ ID NO: 251 (TR-47)) by the method described above to prepare Compound AAtR-73.

Similarly, aminoacylated pCpA (TS24) was ligated to the transcribed tRNA(Asp)cuc-CA (SEQ ID NO: 252 (TR-48)) by the method described above to prepare Compound AAtR-74.

Similarly, aminoacylated pCpA (SS48) was ligated to the transcribed tRNA(AsnE2)aug-CA (SEQ ID NO: 253 (TR-49)) by the method described above to prepare Compound AAtR-75.

Similarly, aminoacylated pCpA (SS49) was ligated to the transcribed tRNA(AsnE2)uug-CA (SEQ ID NO: 255 (TR-51)) by the method described above to prepare Compound AAtR-76.

Similarly, aminoacylated pCpA (SS50) was ligated to the transcribed tRNA(AsnE2)cug-CA (SEQ ID NO: 256 (TR-52)) by the method described above to prepare Compound AAtR-77.

Similarly, aminoacylated pCpA (SS48) was ligated to the transcribed tRNA(AsnE2)gug-CA (SEQ ID NO: 254 (TR-50)) by the method described above to prepare Compound AAtR-78.

Similarly, aminoacylated pCpA (SS49) was ligated to the transcribed tRNA(AsnE2)uug-CA (SEQ ID NO: 255 (TR-51)) by the method described above to prepare Compound AAtR-79.

Similarly, aminoacylated pCpA (SS50) was ligated to the transcribed tRNA(AsnE2)cug-CA (SEQ ID NO: 256 (TR-52)) by the method described above to prepare Compound AAtR-80.

Similarly, aminoacylated pCpA (SS48) was ligated to the transcribed tRNA(AsnE2)auc-CA (SEQ ID NO: 257 (TR-53)) by the method described above to prepare Compound AAtR-81.

Similarly, aminoacylated pCpA (SS49) was ligated to the transcribed tRNA(AsnE2)uuc-CA (SEQ ID NO: 258 (TR-54)) by the method described above to prepare Compound AAtR-82.

Similarly, aminoacylated pCpA (SS50) was ligated to the transcribed tRNA(AsnE2)cuc-CA (SEQ ID NO: 259 (TR-55)) by the method described above to prepare Compound AAtR-83.

Following addition of 0.3 M sodium acetate to each of the solutions that have undergone the ligation reaction, phenol-chloroform solution was added. At this stage, the respective ligation products were subjected to phenol-chloroform extraction and ethanol precipitation in a mixed state by mixing them or in a single state, and then recovered.

Specifically, 0.3 M sodium acetate was added to three ligation products of Compound AAtR-1, Compound AAtR-2, and Compound AAtR-3, and phenol-chloroform solution was added. These solutions were mixed in equal amounts and subjected to phenol-chloroform extraction and ethanol precipitation to prepare a mixed aminoacylated tRNA solution (mixed solution of Compound AAtR-1, Compound AAtR-2, and Compound AAtR-3).

Similarly, 0.3 M sodium acetate was added to three ligation products of Compound AAtR-4, Compound AAtR-5, and Compound AAtR-6, and phenol-chloroform solution was added. These solutions were mixed in equal amounts and subjected to phenol-chloroform extraction and ethanol precipitation to prepare a mixed aminoacylated tRNA solution (mixed solution of Compound AAtR-4, Compound AAtR-5, and Compound AAtR-6).

Similarly, 0.3 M sodium acetate was added to three ligation products of Compound AAtR-7, Compound AAtR-8, and Compound AAtR-9, and phenol-chloroform solution was added. These solutions were mixed in equal amounts and subjected to phenol-chloroform extraction and ethanol precipitation to prepare a mixed aminoacylated tRNA solution (mixed solution of Compound AAtR-7, Compound AAtR-8, and Compound AAtR-9).

Similarly, 0.3 M sodium acetate was added to three ligation products of Compound AAtR-10, Compound AAtR-11, and Compound AAtR-12, and phenol-chloroform solution was added. These solutions were mixed in equal amounts and subjected to phenol-chloroform extraction and ethanol precipitation to prepare a mixed aminoacylated tRNA solution (mixed solution of Compound AAtR-10, Compound AAtR-11, and Compound AAtR-12).

Similarly, 0.3 M sodium acetate was added to three ligation products of Compound AAtR-13, Compound AAtR-14, and Compound AAtR-15, and phenol-chloroform solution was added. These solutions were mixed in equal amounts and subjected to phenol-chloroform extraction and ethanol precipitation to prepare a mixed aminoacylated tRNA solution (mixed solution of Compound AAtR-13, Compound AAtR-14, and Compound AAtR-15).

Similarly, 0.3 M sodium acetate was added to three ligation products of Compound AAtR-16, Compound AAtR-17, and Compound AAtR-18, and phenol-chloroform solution was added. These solutions were mixed in equal amounts andsubjected to phenol-chloroform extraction and ethanol precipitation to prepare a mixed aminoacylated tRNA solution (mixed solution of Compound AAtR-16, Compound AAtR-17, and Compound AAtR-18).

Similarly, 0.3 M sodium acetate was added to three ligation products of Compound AAtR-19, Compound AAtR-20, and Compound AAtR-21, and phenol-chloroform solution was added. These solutions were mixed in equal amounts and subjected to phenol-chloroform extraction and ethanol precipitation to prepare a mixed aminoacylated tRNA solution (mixed solution of Compound AAtR-19, Compound AAtR-20, and Compound AAtR-21).

Similarly, 0.3 M sodium acetate was added to three ligation products of Compound AAtR-22, Compound AAtR-23, and Compound AAtR-24, and phenol-chloroform solution was added. These solutions were mixed in equal amounts and subjected to phenol-chloroform extraction and ethanol precipitation to prepare a mixed aminoacylated tRNA solution (mixed solution of Compound AAtR-22, Compound AAtR-23, and Compound AAtR-24).

Similarly, 0.3 M sodium acetate was added to three ligation products of Compound AAtR-26, Compound AAtR-8, and Compound AAtR-9 and phenol-chloroform solution was added. These solutions were mixed in equal amounts and subjected to phenol-chloroform extraction and ethanol precipitation to prepare a mixed aminoacylated tRNA solution (mixed solution of Compound AAtR-26, Compound AAtR-8, and Compound AAtR-9).

Similarly, 0.3 M sodium acetate was added to three ligation products of Compound AAtR-27, Compound AAtR-11, and Compound AAtR-12 and phenol-chloroform solution was added. These solutions were mixed in equal amounts and subjected to phenol-chloroform extraction and ethanol precipitation to prepare a mixed aminoacylated tRNA solution (mixed solution of Compound AAtR-27, Compound AAtR-11, and Compound AAtR-12).

Similarly, 0.3 M sodium acetate was added to three ligation products of Compound AAtR-28, Compound AAtR-20, and Compound AAtR-21, and phenol-chloroform solution was added. These solutions were mixed in equal amounts and subjected to phenol-chloroform extraction and ethanol precipitation to prepare a mixed aminoacylated tRNA solution (mixed solution of Compound AAtR-28, Compound AAtR-20, and Compound AAtR-21).

Similarly, 0.3 M sodium acetate was added to three ligation products of Compound AAtR-29, Compound AAtR-30, and Compound AAtR-31, and phenol-chloroform solution was added. These solutions were mixed in equal amounts and subjected to phenol-chloroform extraction and ethanol precipitation to prepare a mixed aminoacylated tRNA solution (mixed solution of Compound AAtR-29, Compound AAtR-30, and Compound AAtR-31).

Similarly, 0.3 M sodium acetate was added to three ligation products of Compound AAtR-32, Compound AAtR-33, and Compound AAtR-34, and phenol-chloroform solution was added. These solutions were mixed in equal amounts and subjected to phenol-chloroform extraction and ethanol precipitation to prepare a mixed aminoacylated tRNA solution (mixed solution of Compound AAtR-32, Compound AAtR-33, and Compound AAtR-34).

Similarly, 0.3 M sodium acetate was added to three ligation products of Compound AAtR-35, Compound AAtR-36, and Compound AAtR-37, and phenol-chloroform solution was added. These solutions were mixed in equal amounts and subjected to phenol-chloroform extraction and ethanol precipitation to prepare a mixed aminoacylated tRNA solution (mixed solution of Compound AAtR-35, Compound AAtR-36, and Compound AAtR-37).

Similarly, 0.3 M sodium acetate was added to three ligation products of Compound AAtR-38, Compound AAtR-39, and Compound AAtR-40, and phenol-chloroform solution was added. These solutions were mixed in equal amounts and subjected to phenol-chloroform extraction and ethanol precipitation to prepare a mixed aminoacylated tRNA solution (mixed solution of Compound AAtR-38, Compound AAtR-39, and Compound AAtR-40).

Similarly, 0.3 M sodium acetate was added to three ligation products of Compound AAtR-41, Compound AAtR-42, and Compound AAtR-43, and phenol-chloroform solution was added. These solutions were mixed in equal amounts and subjected to phenol-chloroform extraction and ethanol precipitation to prepare a mixed aminoacylated tRNA solution (mixed solution of Compound AAtR-41, Compound AAtR-42, and Compound AAtR-43).

Similarly, 0.3 M sodium acetate was added to three ligation products of Compound AAtR-44, Compound AAtR-45, and Compound AAtR-46, and phenol-chloroform solution was added. These solutions were mixed in equal amounts and subjected to phenol-chloroform extraction and ethanol precipitation to prepare a mixed aminoacylated tRNA solution (mixed solution of Compound AAtR-44, Compound AAtR-45, and Compound AAtR-46).

Similarly, 0.3 M sodium acetate was added to three ligation products of Compound AAtR-47, Compound AAtR-30, and Compound AAtR-31, and phenol-chloroform solution wwas added. These solutions were mixed in equal amounts and subjected to phenol-chloroform extraction and ethanol precipitation to prepare a mixed aminoacylated tRNA solution (mixed solution of Compound AAtR-47, Compound AAtR-30, and Compound AAtR-31).

Similarly, 0.3 M sodium acetate was added to three ligation products of Compound AAtR-48, Compound AAtR-33, and Compound AAtR-34, and phenol-chloroform solution was added. These solutions were mixed in equal amounts and subjected to phenol-chloroform extraction and ethanol precipitation to prepare a mixed aminoacylated tRNA solution (mixed solution of Compound AAtR-48, Compound AAtR-33, and Compound AAtR-34).

Similarly, 0.3 M sodium acetate was added to three ligation products of Compound AAtR-49, Compound AAtR-36, and Compound AAtR-37, and phenol-chloroform solution was added. These solutions were mixed in equal amounts and subjected to phenol-chloroform extraction and ethanol precipitation to prepare a mixed aminoacylated tRNA solution (mixed solution of Compound AAtR-49, Compound AAtR-36, and Compound AAtR-37).

Similarly, 0.3 M sodium acetate was added to three ligation products of Compound AAtR-50, Compound AAtR-39, and Compound AAtR-40, and phenol-chloroform solution was added. These solutions were mixed in equal amounts and subjected to phenol-chloroform extraction and ethanol precipitation to prepare a mixed aminoacylated tRNA solution (mixed solution of Compound AAtR-50, Compound AAtR-39, and Compound AAtR-40).

Similarly, 0.3 M sodium acetate was added to three ligation products of Compound AAtR-51, Compound AAtR-42, and Compound AAtR-43, and phenol-chloroform solution was added. These solutions were mixed in equal amounts and subjected to phenol-chloroform extraction and ethanol precipitation to prepare a mixed aminoacylated tRNA solution (mixed solution of Compound AAtR-51, Compound AAtR-42, and Compound AAtR-43).

Similarly, 0.3 M sodium acetate was added to two ligation products of Compound AAtR-57 and Compound AAtR-59, and phenol-chloroform solution was added. These solutions were mixed in equal amounts and subjected to phenol-chloroform extraction and ethanol precipitation to prepare a mixed aminoacylated tRNA solution (mixed solution of Compound AAtR-57 and Compound AAtR-59).

Similarly, 0.3 M sodium acetate was added to three ligation products of Compound AAtR-60, Compound AAtR-61, and Compound AAtR-62, and phenol-chloroform solution was added. These solutions were mixed in equal amounts and subjected to phenol-chloroform extraction and ethanol precipitation to prepare a mixed aminoacylated tRNA solution (mixed solution of Compound AAtR-60, Compound AAtR-61, and Compound AAtR-62).

Similarly, 0.3 M sodium acetate was added to three ligation products of Compound AAtR-63, Compound AAtR-64, and Compound AAtR-65, and phenol-chloroform solution was added. These solutions were mixed in equal amounts and subjected to phenol-chloroform extraction and ethanol precipitation to prepare a mixed aminoacylated tRNA solution (mixed solution of Compound AAtR-63, Compound AAtR-64, and Compound AAtR-65).

Similarly, 0.3 M sodium acetate was added to three ligation products of Compound AAtR-66, Compound AAtR-67, and Compound AAtR-68, and phenol-chloroform solution was added. These solutions were mixed in equal amounts and subjected to phenol-chloroform extraction and ethanol precipitation to prepare a mixed aminoacylated tRNA solution (mixed solution of Compound AAtR-66, Compound AAtR-67, and Compound AAtR-68).

Similarly, 0.3 M sodium acetate was added to three ligation products of Compound AAtR-69, Compound AAtR-70, and Compound AAtR-71, and phenol-chloroform solution was added. These solutions were mixed in equal amounts and subjected to phenol-chloroform extraction and ethanol precipitation to prepare a mixed aminoacylated tRNA solution (mixed solution of Compound AAtR-69, Compound AAtR-70, and Compound AAtR-71).

Similarly, 0.3 M sodium acetate was added to three ligation products of Compound AAtR-72, Compound AAtR-73, and Compound AAtR-74, and phenol-chloroform solution was added. These solutions were mixed in equal amounts and subjected to phenol-chloroform extraction and ethanol precipitation to prepare a mixed aminoacylated tRNA solution (mixed solution of Compound AAtR-72, Compound AAtR-73, and Compound AAtR-74).

Similarly, 0.3 M sodium acetate was added to three ligation products of Compound AAtR-75, Compound AAtR-76, and Compound AAtR-77, and phenol-chloroform solution was added. These solutions were mixed in equal amounts and subjected to phenol-chloroform extraction and ethanol precipitation to prepare a mixed aminoacylated tRNA solution (mixed solution of Compound AAtR-75, Compound AAtR-76, and Compound AAtR-77).

Similarly, 0.3 M sodium acetate was added to three ligation products of Compound AAtR-78, Compound AAtR-79, and Compound AAtR-80, and phenol-chloroform solution was added. These solutions were mixed in equal amounts and subjected to phenol-chloroform extraction and ethanol precipitation to prepare a mixed aminoacylated tRNA solution (mixed solution of Compound AAtR-78, Compound AAtR-79, and Compound AAtR-80).

Similarly, 0.3 M sodium acetate was added to three ligation products of Compound AAtR-81, Compound AAtR-82, and Compound AAtR-83, and phenol-chloroform solution was added. These solutions were mixed in equal amounts and subjected to phenol-chloroform extraction and ethanol precipitation to prepare a mixed aminoacylated tRNA solution (mixed solution of Compound AAtR-81, Compound AAtR-82, and Compound AAtR-83).

The following compounds were purified and prepared without mixing.

Sodium acetate was added at 0.3 M to the ligation product, Compound AAtR-52, and after adding a phenol-chloroform solution, this solution was subjected to phenol-chloroform extraction and ethanol precipitation to prepare Compound AAtR-52.

Sodium acetate was added at 0.3 M to the ligation product of Compound AAtR-53, and after adding a phenol-chloroform solution, this solution was subjected to phenol-chloroform extraction and ethanol precipitation to prepare Compound AAtR-53.

Sodium acetate was added at 0.3 M to the ligation product of Compound AAtR-54, and after adding a phenol-chloroform solution, this solution was subjected to phenol-chloroform extraction and ethanol precipitation to prepare Compound AAtR-54.

Sodium acetate was added at 0.3 M to the ligation product of Compound AAtR-55, and after adding a phenol-chloroform solution, this solution was subjected to phenol-chloroform extraction and ethanol precipitation to prepare Compound AAtR-55.

Sodium acetate was added at 0.3 M to the ligation product of Compound AAtR-56, and after adding a phenol-chloroform solution, this solution was subjected to phenol-chloroform extraction and ethanol precipitation to prepare Compound AAtR-56.

Sodium acetate was added at 0.3 M to the ligation product of Compound AAtR-58, and after adding a phenol-chloroform solution, this solution was subjected to phenol-chloroform extraction and ethanol precipitation to prepare Compound AAtR-58.

### Preparation of initiator aminoacyl tRNA using aminoacyl pCpA

A reaction solution was prepared by adding Nuclease free water to adjust the solution to 25 µM transcribed tRNA(fMet)cau-CA (SEQ ID NO: 62 (TR-25)), 50 mM HEPES-KOH pH7.5, 20 mM MgCl₂, 1 mM ATP, 0.6 unit/µL T4 RNA ligase (New England Biolabs), and 0.25 mM aminoacylated pCpA (a DMSO solution of MT01), and ligation reaction was performed at 15°C for 45 minutes. It should be noted that before adding T4 RNA ligase and aminoacylated pCpA, the reaction solution was heated at 95°C for two minutes and then left to stand at room temperature for five minutes to refold the tRNA in advance.

Sodium acetate was added at 0.3 M to the ligation reaction solution, phenol-chloroform extraction was performed, and initiator aminoacyl tRNA (Compound AAtR-25) was recovered by ethanol precipitation.

The initiator aminoacylated tRNA was dissolved in 1 mM sodium acetate immediately before addition to the translation mixture.

Compound AAtR-1 SEQ ID NO: 75 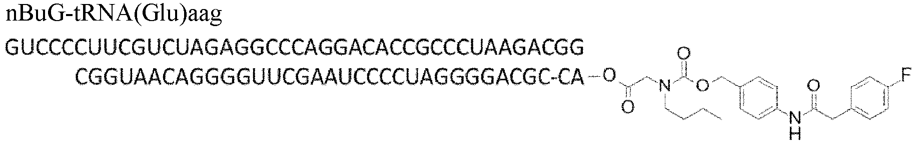
Compound AAtR-2 SEQ ID NO: 76 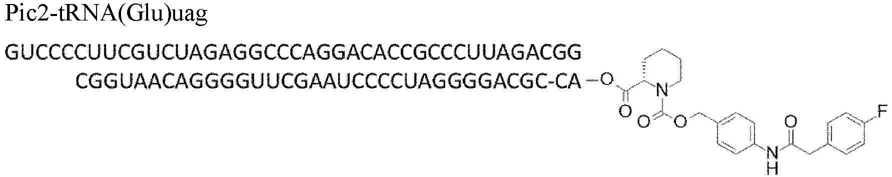
Compound AAtR-3 SEQ ID NO: 77 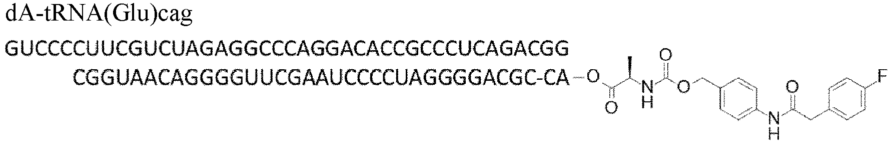
Compound AAtR-4 SEQ ID NO: 78 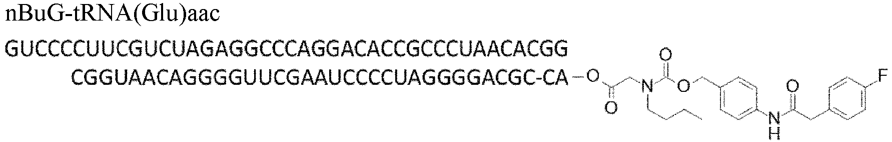
Compound AAtR-5 SEQ ID NO: 79 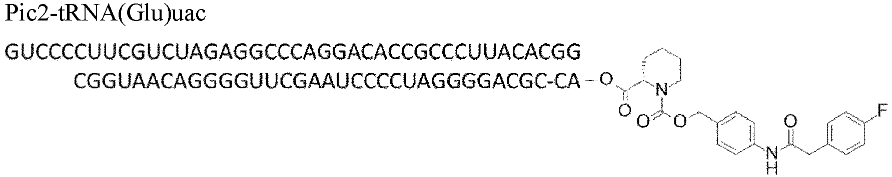
Compound AAtR-6 SEQ ID NO: 80 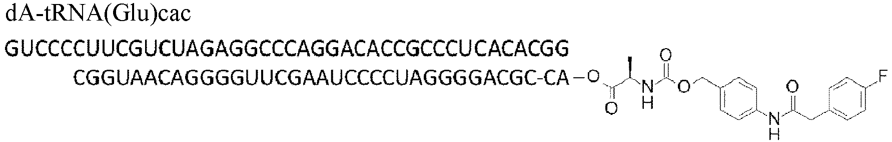
Compound AAtR-7 SEQ ID NO: 81 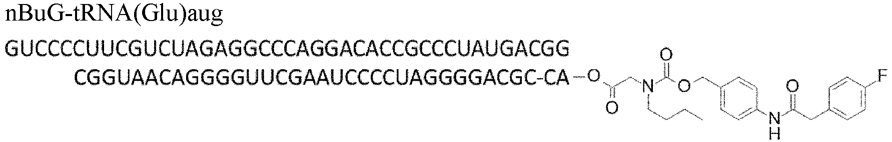
Compound AAtR-8 SEQ ID NO: 82 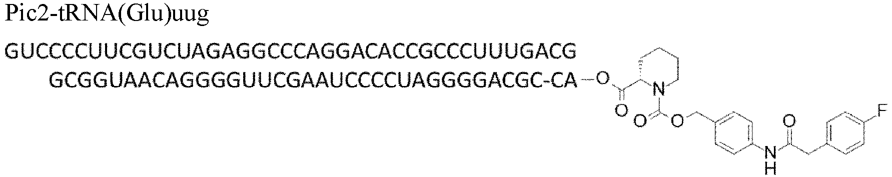
Compound AAtR-9 SEQ ID NO: 83 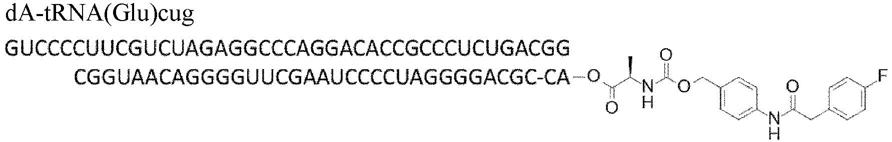
Compound AAtR-10 SEQ ID NO: 84 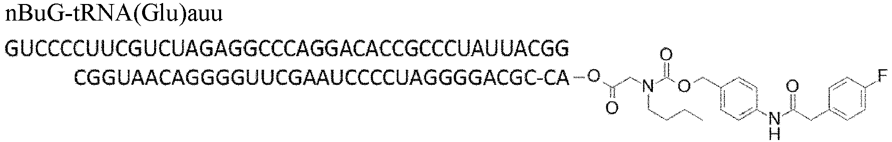
Compound AAtR-11 SEQ ID NO: 85 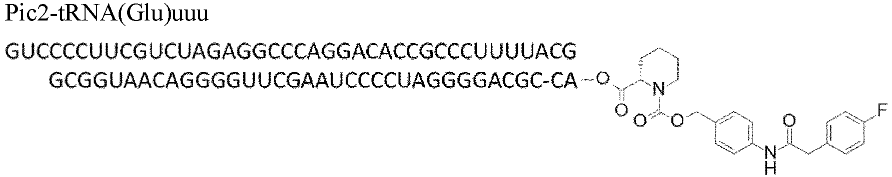
Compound AAtR-12 SEQ ID NO: 86 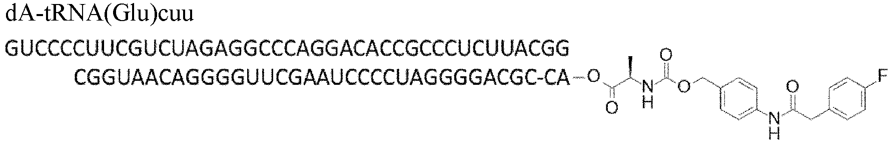
Compound AAtR-13 SEQ ID NO: 87 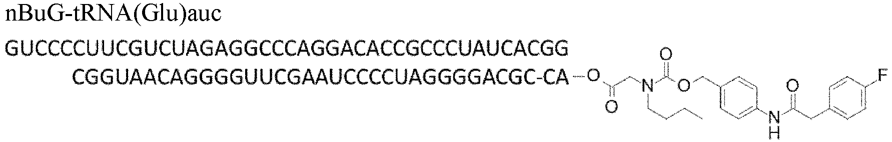
Compound AAtR-14 SEQ ID NO: 88 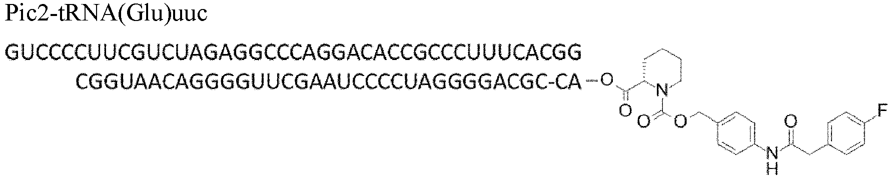
Compound AAtR-15 SEQ ID NO: 89 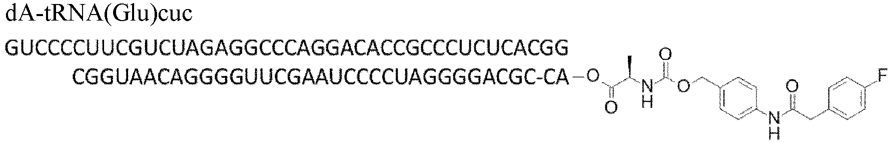
Compound AAtR-16 SEQ ID NO: 90 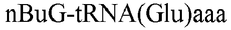 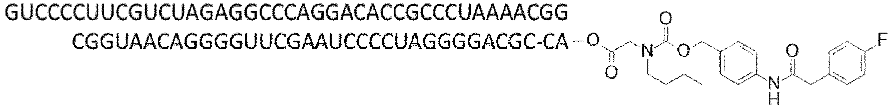
Compound AAtR-17 SEQ ID NO: 91 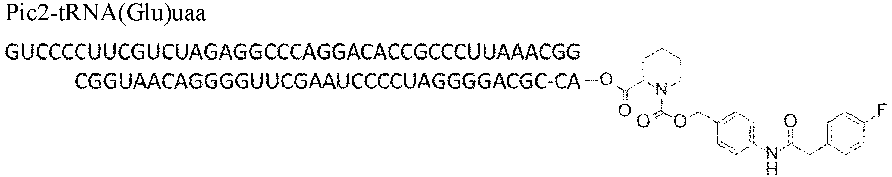
Compound AAtR-18 SEQ ID NO: 92 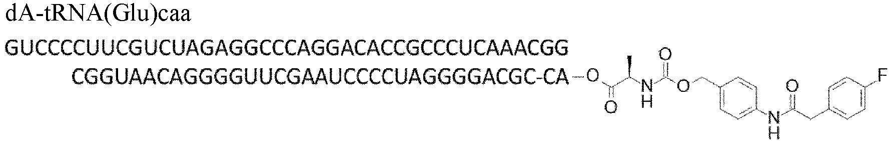
Compound AAtR-19 SEQ ID NO: 93 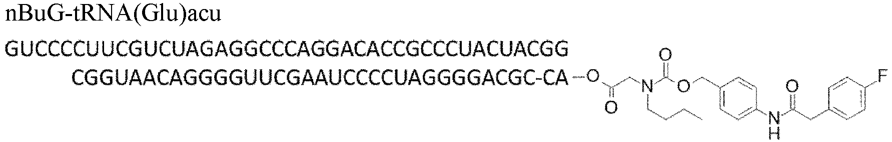
Compound AAtR-20 SEQ ID NO: 94 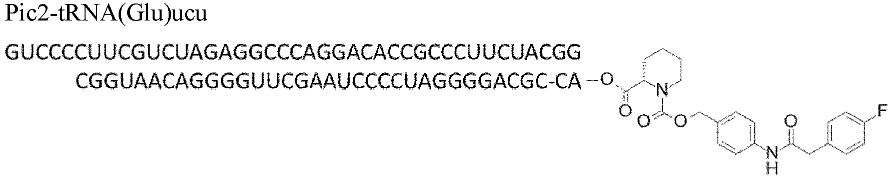
Compound AAtR-21 SEQ ID NO: 95 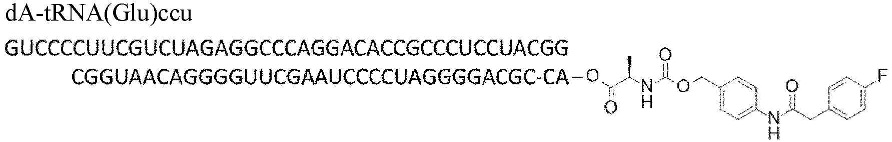
Compound AAtR-22 SEQ ID NO: 96 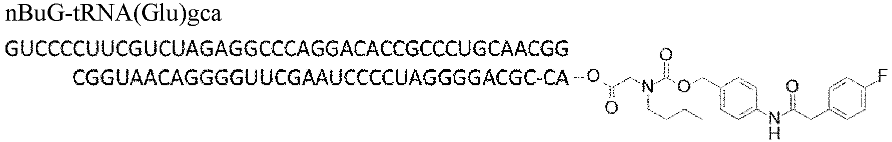
Compound AAtR-23 SEQ ID NO: 97 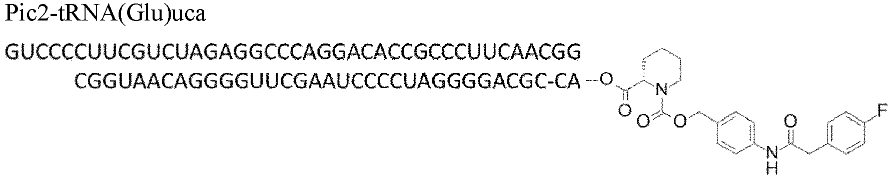
Compound AAtR-24 SEQ ID NO: 98 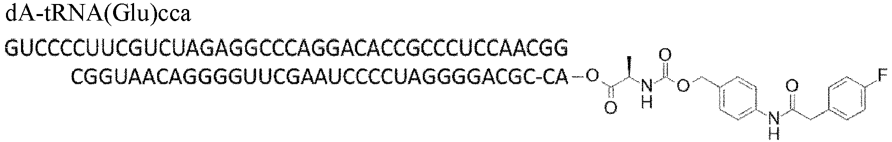
Compound AAtR-25 SEQ ID NO: 99 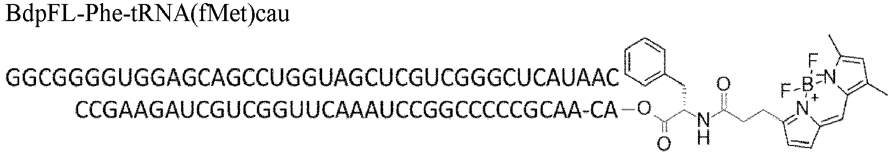
Compound AAtR-26 SEQ ID NO: 100 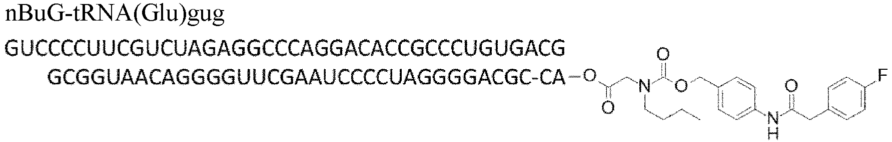
Compound AAtR-27 SEQ ID NO: 101 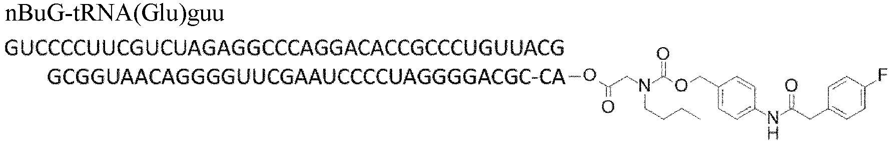
Compound AAtR-28 SEQ ID NO: 102 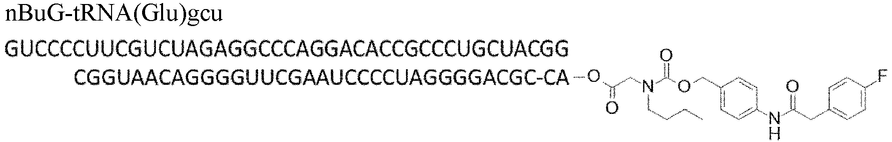
Compound AAtR-29 SEQ ID NO: 103 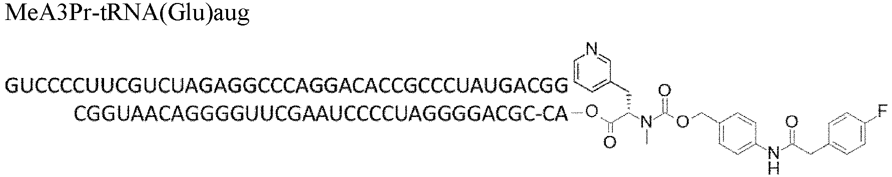
Compound AAtR-30 SEQ ID NO: 104 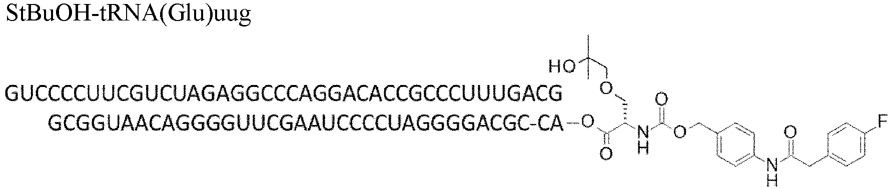
Compound AAtR-31 SEQ ID NO: 105 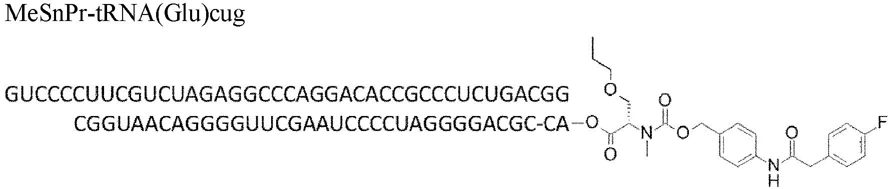
Compound AAtR-32 SEQ ID NO: 106 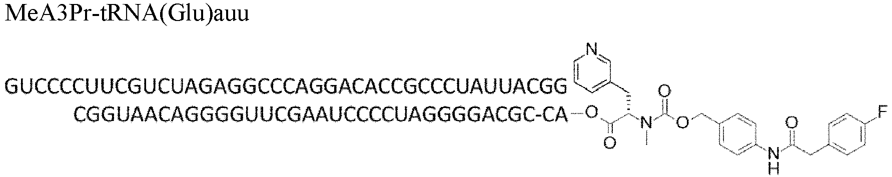
Compound AAtR-33 SEQ ID NO: 107 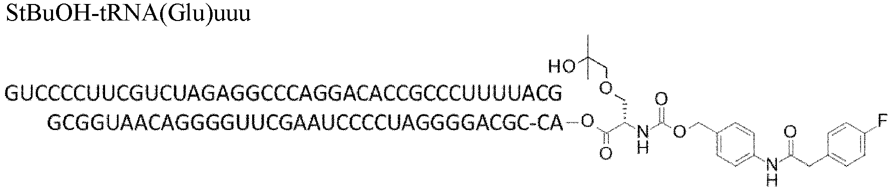
Compound AAtR-34 SEQ ID NO: 108 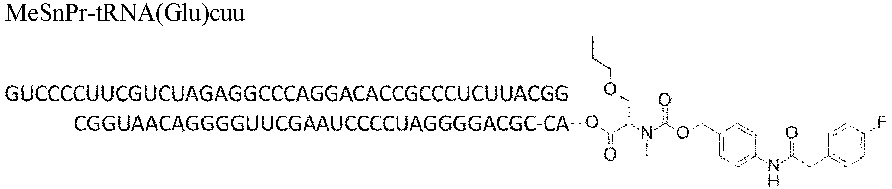
Compound AAtR-35 SEQ ID NO: 109 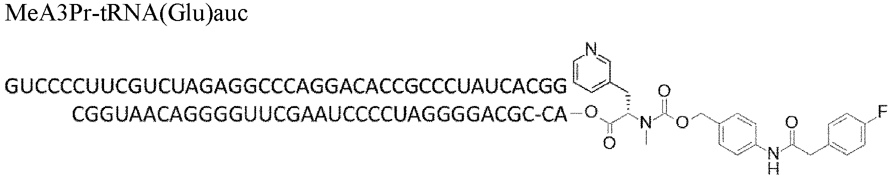
Compound AAtR-36 SEQ ID NO: 110 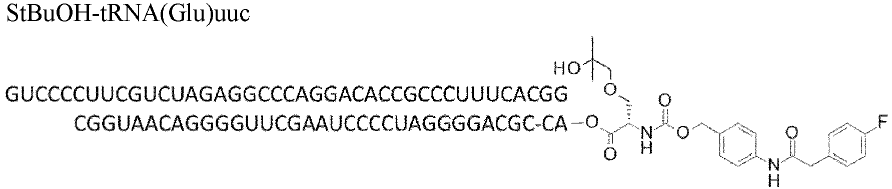
Compound AAtR-37 SEQ ID NO: 111 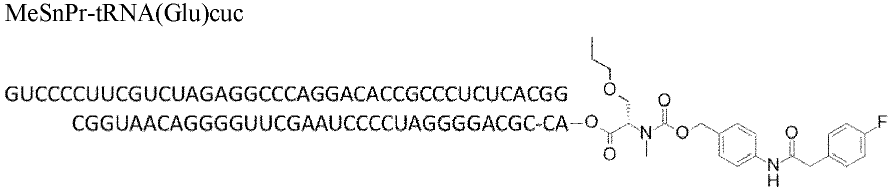
Compound AAtR-38 SEQ ID NO: 112 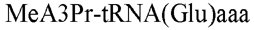 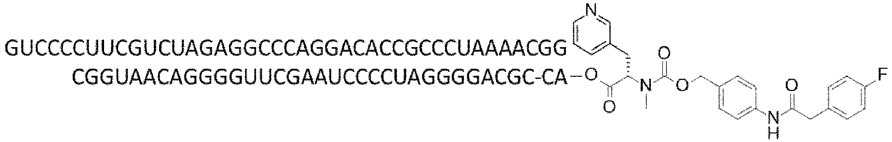
Compound AAtR-39 SEQ ID NO: 113 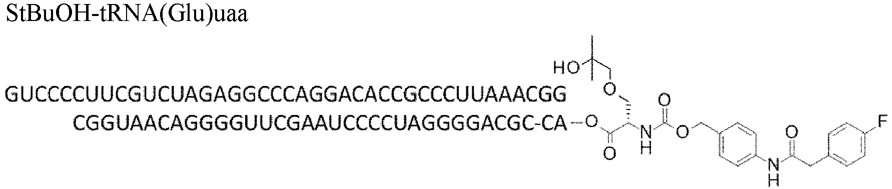
Compound AAtR-40 SEQ ID NO: 114 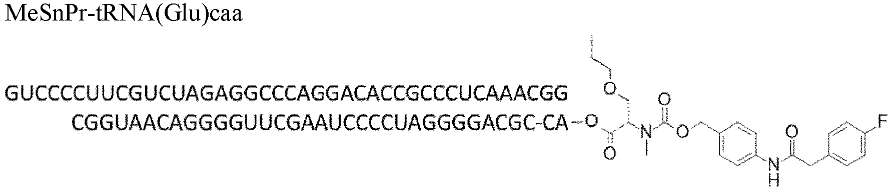
Compound AAtR-41 SEQ ID NO: 115 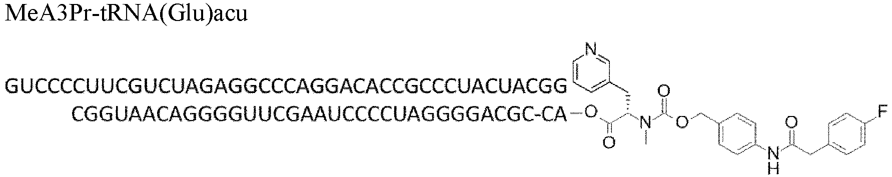
Compound AAtR-42 SEQ ID NO: 116 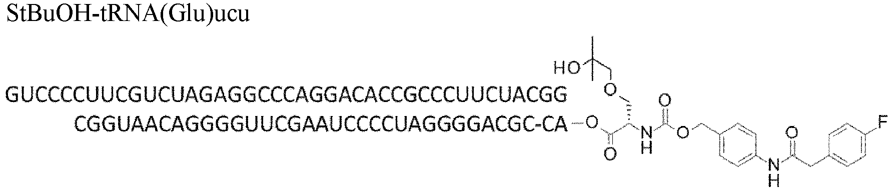
Compound AAtR-43 SEQ ID NO: 117 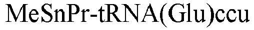 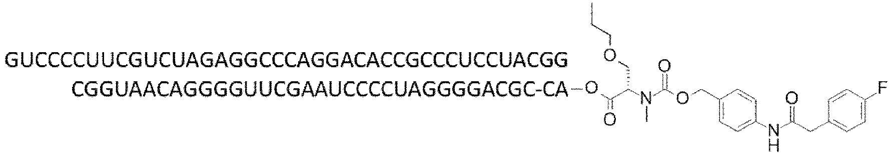
Compound AAtR-44 SEQ ID NO: 118 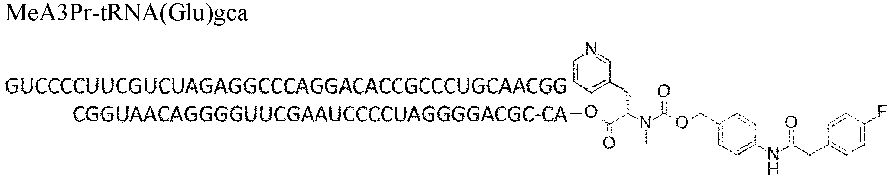
Compound AAtR-45 SEQ ID NO: 119 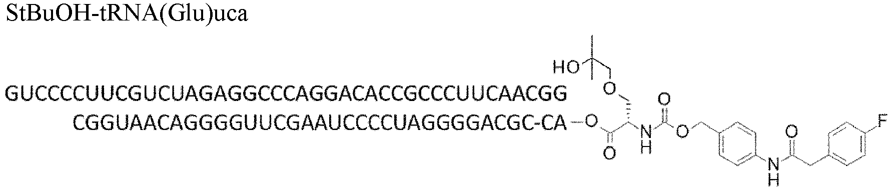
Compound AAtR-46 SEQ ID NO: 120 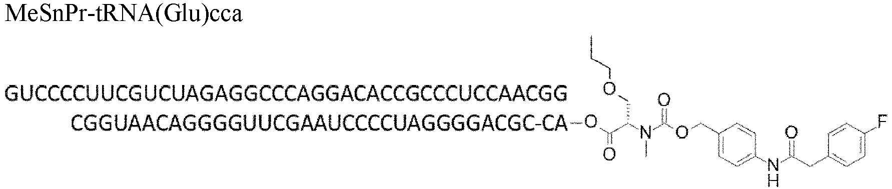
Compound AAtR-47 SEQ ID NO: 121 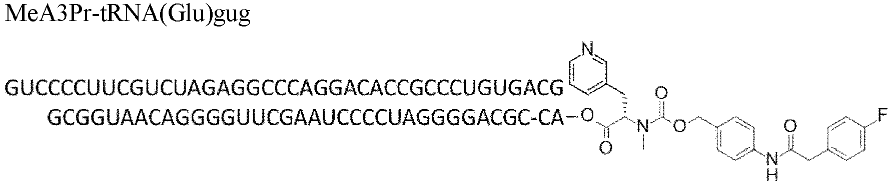
Compound AAtR-48 SEQ ID NO: 122 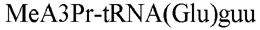 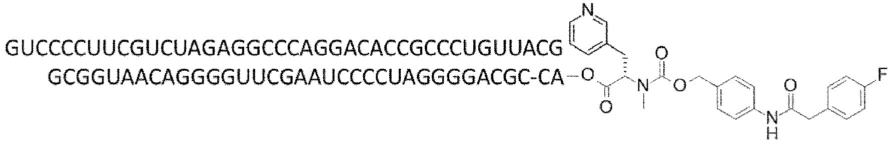
Compound AAtR-49 SEQ ID NO: 123 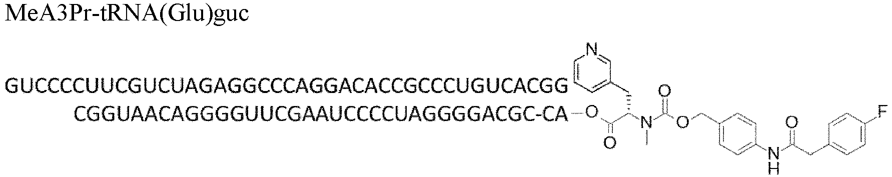
Compound AAtR-50 SEQ ID NO: 124 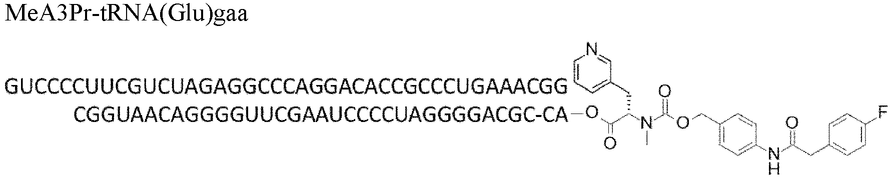
Compound AAtR-51 SEQ ID NO: 125 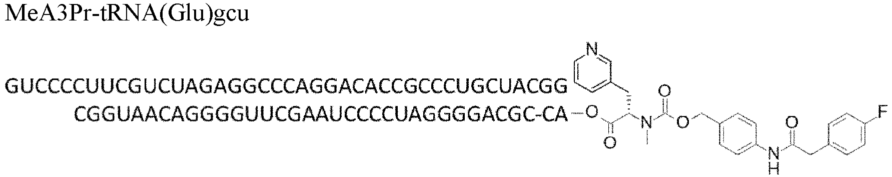
Compound AAtR-52 SEQ ID NO: 126 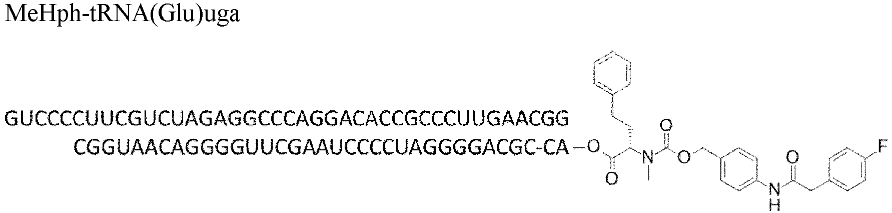
Compound AAtR-53 SEQ ID NO: 127 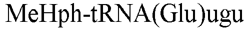 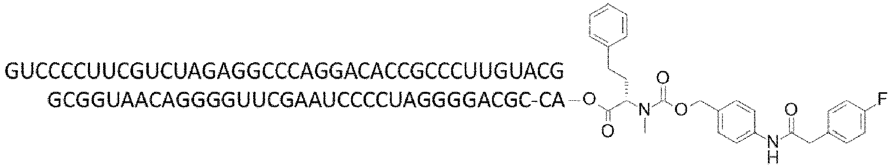
Compound AAtR-54 SEQ ID NO: 128 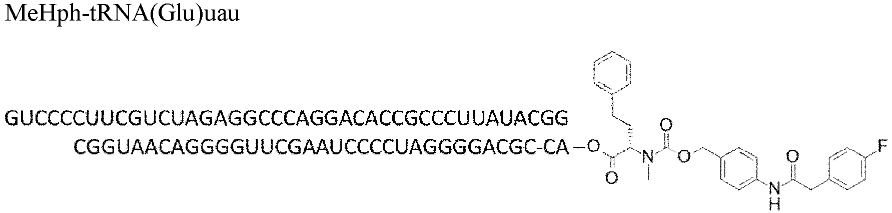
Compound AAtR-55 SEQ ID NO: 129 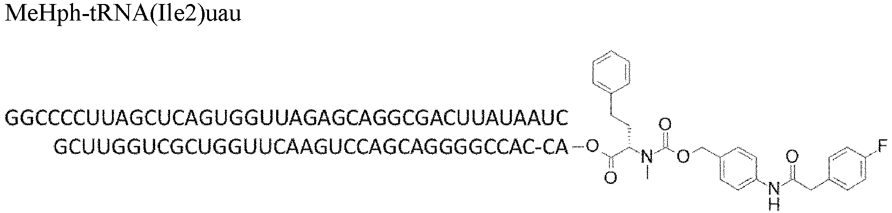
Compound AAtR-56 SEQ ID NO: 130 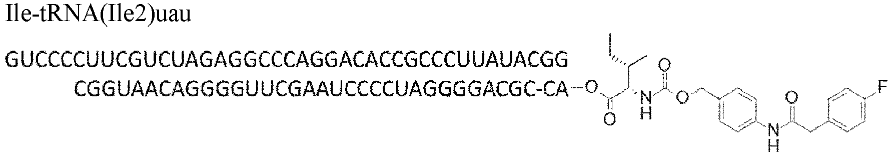
Compound AAtR-57 SEQ ID NO: 131 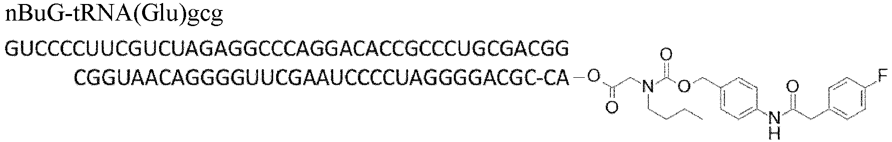
Compound AAtR-58 SEQ ID NO: 132 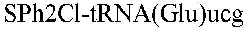 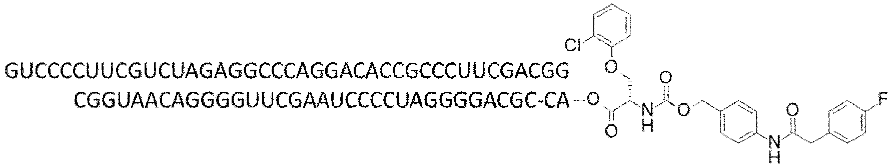
Compound AAtR-59 SEQ ID NO: 133 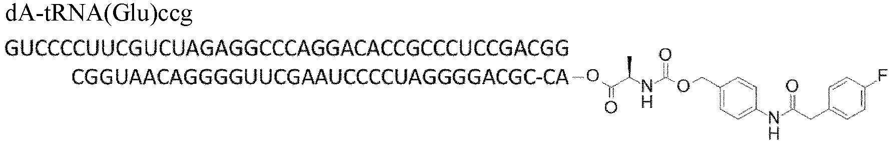
Compound AAtR-60 SEQ ID NO: 260 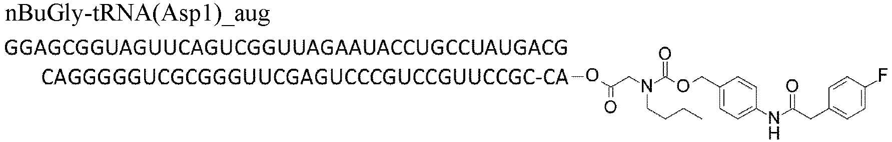
Compound AAtR-61 SEQ ID NO: 261 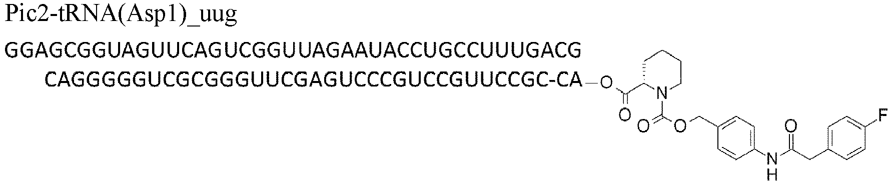
Compound AAtR-62 SEQ ID NO: 262 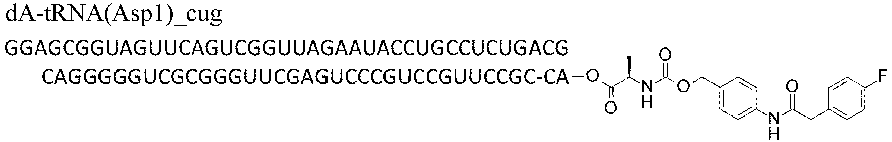
Compound AAtR-63 SEQ ID NO: 263 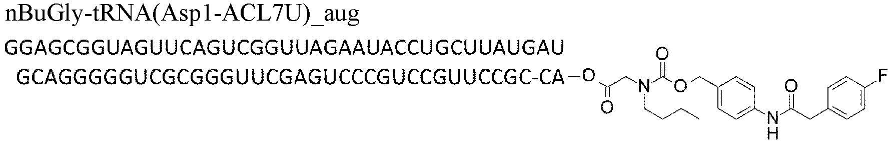
Compound AAtR-64 SEQ ID NO: 264 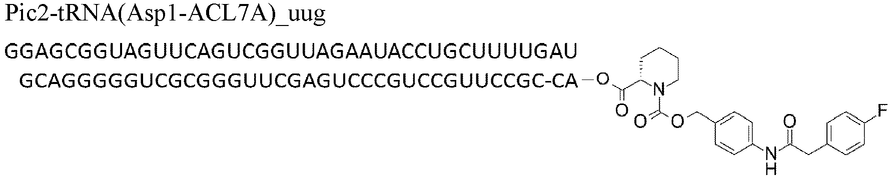
Compound AAtR-65 SEQ ID NO: 265 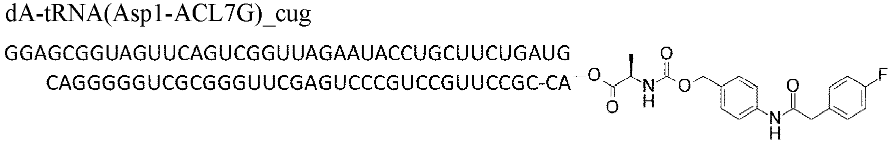
Compound AAtR-66 SEQ ID NO: 266 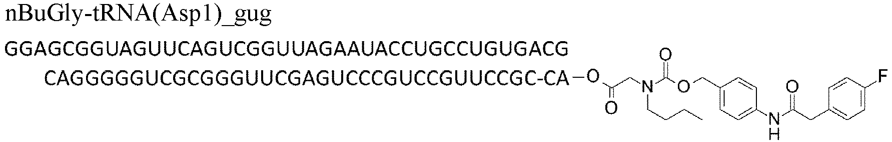
Compound AAtR-67 SEQ ID NO: 267 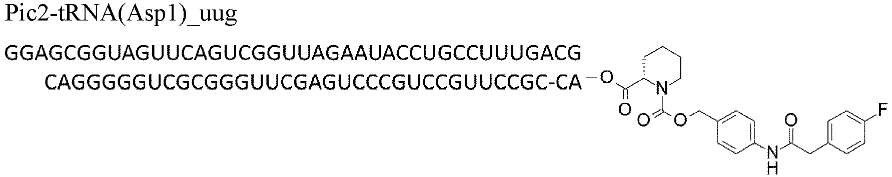
Compound AAtR-68 SEQ ID NO: 268 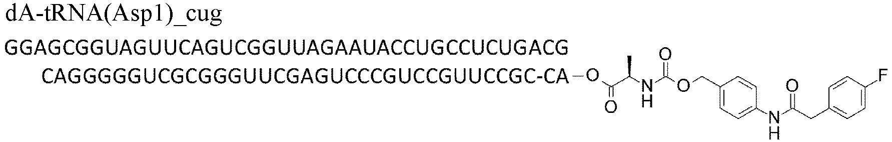
Compound AAtR-69 SEQ ID NO: 269 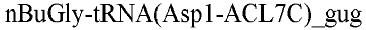 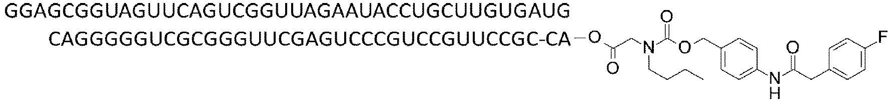
Compound AAtR-70 SEQ ID NO: 270 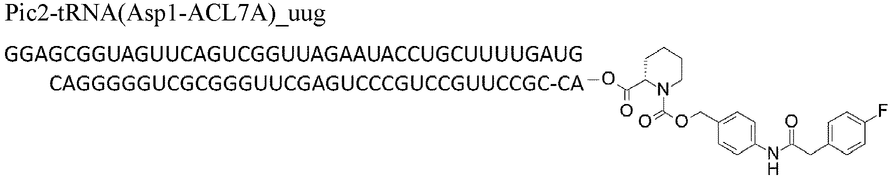
Compound AAtR-71 SEQ ID NO: 271 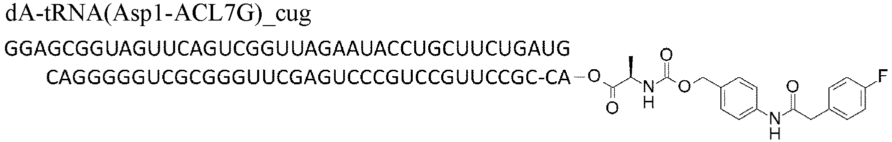
Compound AAtR-72 SEQ ID NO: 272 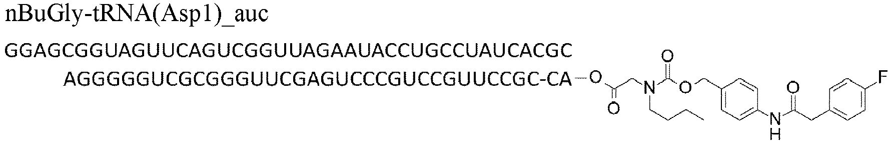
Compound AAtR-73 SEQ ID NO: 273 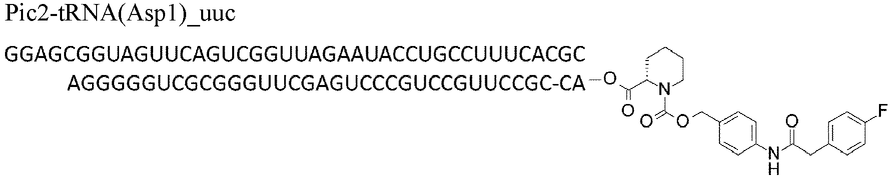
Compound AAtR-74 SEQ ID NO: 274 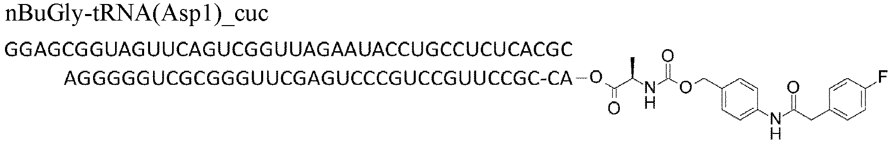
Compound AAtR-75 SEQ ID NO: 275 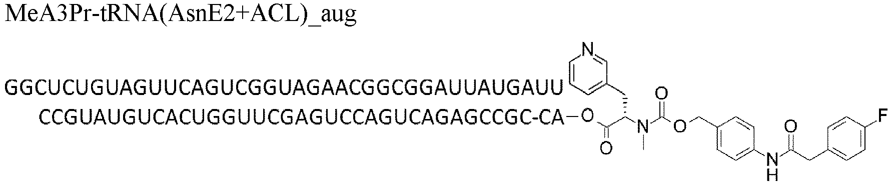
Compound AAtR-76 SEQ ID NO: 276 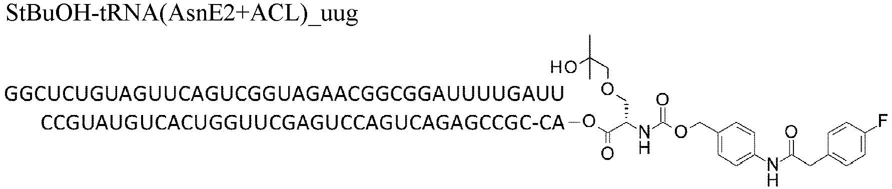
Compound AAtR-77 SEQ ID NO: 277 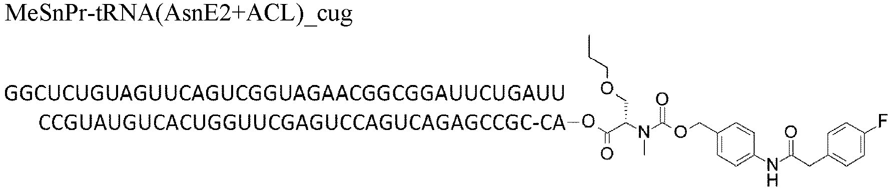
Compound AAtR-78 SEQ ID NO: 278 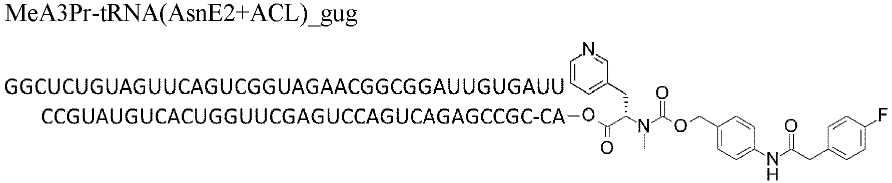
Compound AAtR-79 SEQ ID NO: 279 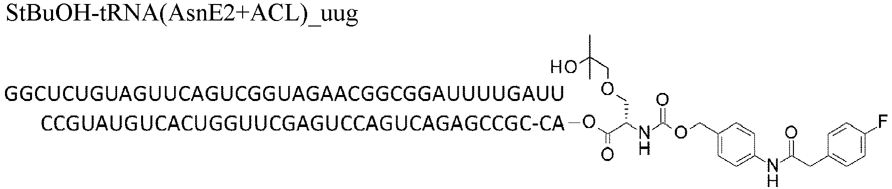
Compound AAtR-80 SEQ ID NO: 280 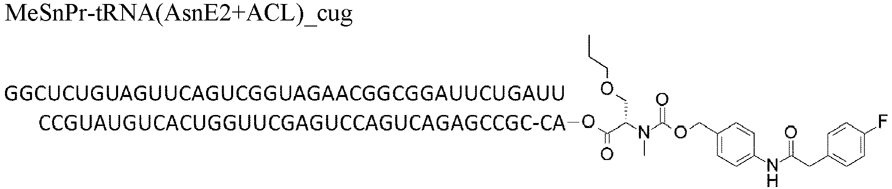
Compound AAtR-81 SEQ ID NO: 281 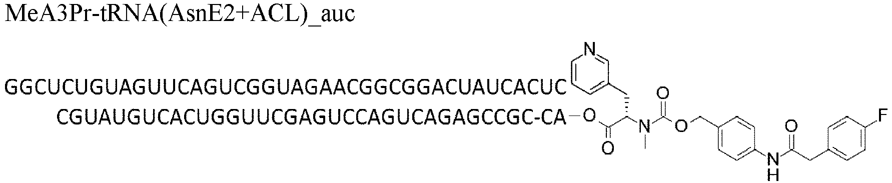
Compound AAtR-82 SEQ ID NO: 282 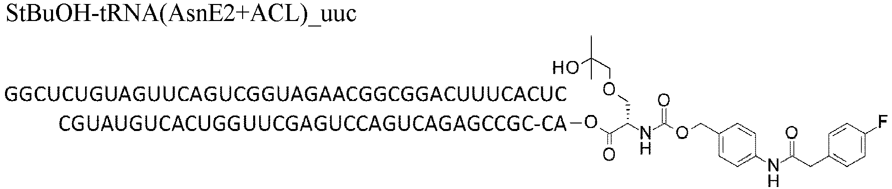
Compound AAtR-83 SEQ ID NO: 283

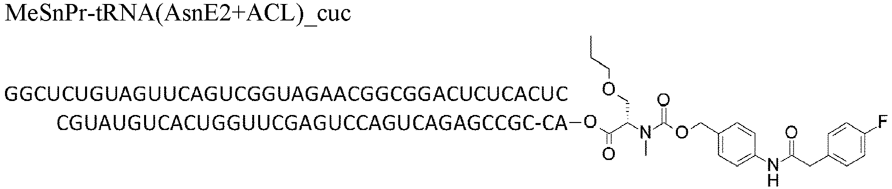

### Example 6. Translational synthesis of peptides

Next, an experiment was performed to confirm the discrimination of three amino acids in one codon box in the presence of three aminoacylated tRNAs. Specifically, template mRNAs containing any one of three codons in the same codon box and having the same sequence for the rest of the sequences (template mRNAs of SEQ ID NO: 134 (mR-1) to SEQ ID NO: 162 (mR-29) and SEQ ID NO: 219 (mR-42) to SEQ ID NO: 221 (mR-44)) were translated using a mixed aminoacylated tRNA solution to translate peptide compounds. In specific codon boxes, discrimination between the wobble codons and further simultaneous discrimination of another codon were successfully achieved. Amino acids were tested in two combinations. In both conditions, the discrimination of three amino acids in one codon box was shown to be possible in specific codon boxes.

The translation system used was PURE system, a prokaryote-derived reconstituted cell-free protein synthesis system.

Specifically, the translation was carried out as follows: 1 µM template mRNA (SEQ ID NO: 134 (mR-1), SEQ ID NO: 135 (mR-2), or SEQ ID NO: 136 (mR-3)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-1, Compound AAtR-2, and Compound AAtR-3) was added at 30 µM to the translation reaction mixture, and left at 37°C for one hour.

Cell-free translations were also performed on other sequences (SEQ ID NO: 137 (mR-4), SEQ ID NO: 138 (mR-5), or SEQ ID NO: 139 (mR-6)).

Specifically, the translation was carried out as follows: 1 µM template mRNA (SEQ ID NO: 137 (mR-4), SEQ ID NO: 138 (mR-5), or SEQ ID NO: 139 (mR-6)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-4, Compound AAtR-5, and Compound AAtR-6) was added at 30 µM to the translation reaction mixture, and left at 37°C for one hour.

Cell-free translations were also performed on other sequences (SEQ ID NO: 140 (mR-7), SEQ ID NO: 141 (mR-8), or SEQ ID NO: 142 (mR-9)).

Specifically, the translation was carried out as follows: 1 µM template mRNA (SEQ ID NO: 140 (mR-7), SEQ ID NO: 141 (mR-8), or SEQ ID NO: 142 (mR-9)), a group of natural amino acids encoded in the respective template mRNAs at 0.25 mM respectively, and initiator aminoacylated tRNA (Compound AAtR-18) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 54 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-7, Compound AAtR-8, and Compound AAtR-9; mixed solution of Compound AAtR-29, Compound AAtR-30, and Compound AAtR-31; mixed solution of Compound AAtR-60, Compound AAtR-61, and Compound AAtR-62; mixed solution of Compound AAtR-63, Compound AAtR-64, and Compound AAtR-65; or mixed solution of Compound AAtR-75, Compound AAtR-76, and Compound AAtR-77) was added at 30 µM to the translation reaction mixture, and left at 37°C for one hour.

Cell-free translations were also performed on other sequences (SEQ ID NO: 143 (mR-10), SEQ ID NO: 144 (mR-11), or SEQ ID NO: 145 (mR-12)).

Specifically, the translation was carried out as follows: 1 µM template mRNA (SEQ ID NO: 143 (mR-10), SEQ ID NO: 144 (mR-11), or SEQ ID NO: 145 (mR-12)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 54 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, 0.09 µM ThrRS), and a mixed aminoacylated tRNA solution (mixed solution of Compound AAtR-10, Compound AAtR-11, and Compound AAtR-12; or mixed solution of Compound AAtR-32, Compound AAtR-33, and Compound AAtR-34) was added at 30 µM to the translation reaction mixture, and left at 37°C for one hour.

Cell-free translations were also performed on other sequences (SEQ ID NO: 146 (mR-13), SEQ ID NO: 147 (mR-14), and SEQ ID NO: 148 (mR-15)).

Specifically, the translation was carried out as follows: 1 µM template mRNA (SEQ ID NO: 146 (mR-13), SEQ ID NO: 147 (mR-14), or SEQ ID NO: 148 (mR-15)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 49 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM I1eRS, 0.68 µM PheRS, 0.16 µM ProRS, and 0.09 µM ThrRS), and a mixed aminoacylated tRNA solution (mixed solution of Compound AAtR-13, Compound AAtR-14, and Compound AAtR-15; mixed solution of Compound AAtR-72, Compound AAtR-73, and Compound AAtR-74; or mixed solution of Compound AAtR-81, Compound AAtR-82, and Compound AAtR-83) was added at 30 µM to the translation reaction mixture, and this was left to stand at 37°C for one hour.

Cell-free translations were also performed on other sequences (SEQ ID NO: 149 (mR-16), SEQ ID NO: 150 (mR-17), and SEQ ID NO: 151 (mR-18)).

Specifically, the translation was carried out as follows: 1 µM template mRNA (SEQ ID NO: 149 (mR-16), SEQ ID NO: 150 (mR-17), SEQ ID NO: 151 (mR-18)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 54 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM I1eRS, 0.68 µM PheRS, 0.16 µM ProRS, and 0.02 µM ValRS), and a mixed aminoacylated tRNA solution (mixed solution of Compound AAtR-16, Compound AAtR-17, and Compound AAtR-18; or mixed solution of Compound AAtR-38, Compound AAtR-39, and Compound AAtR-40) was added at 30 µM to the translation reaction mixture, and this was left to stand at 37°C for one hour.

Cell-free translations were also performed on other sequences (SEQ ID NO: 152 (mR-19), SEQ ID NO: 153 (mR-20), and SEQ ID NO: 154 (mR-21)).

Specifically, the translation was carried out as follows: 1 µM template mRNA (SEQ ID NO: 152 (mR-19), SEQ ID NO: 153 (mR-20), or SEQ ID NO: 154 (mR-21)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-19, Compound AAtR-20, and Compound AAtR-21; or mixed solution of Compound AAtR-41, Compound AAtR-42, and Compound AAtR-43) was added at 30 µM to the translation reaction mixture, and this was left to stand at 37°C for one hour.

Cell-free translations were also performed on other sequences (SEQ ID NO: 155 (mR-22), SEQ ID NO: 156 (mR-23), and SEQ ID NO: 157 (mR-24)).

Specifically, the translation was carried out as follows: 1 µM template mRNA (SEQ ID NO: 155 (mR-22), SEQ ID NO: 156 (mR-23), or SEQ ID NO: 157 (mR-24)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.25 µM RF1, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 34.6 µM EF-Ts, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-22, Compound AAtR-23, and Compound AAtR-24; or mixed solution of Compound AAtR-44, Compound AAtR-45, and Compound AAtR-46) was added at 30 µM to the translation reaction mixture, and this was left to stand at 37°C for one hour.

Cell-free translations were also performed on other sequences (SEQ ID NO: 158 (mR-25), SEQ ID NO: 141 (mR-8), and SEQ ID NO: 142 (mR-9)).

Specifically, the translation was carried out as follows: 1 µM template mRNA (SEQ ID NO: 158 (mR-25), SEQ ID NO: 141 (mR-8), or SEQ ID NO: 142 (mR-9)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 3 µM Ala1BtRNA, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-26, Compound AAtR-8, and Compound AAtR-9; mixed solution of Compound AAtR-47, Compound AAtR-30, and Compound AAtR-31; mixed solution of Compound AAtR-66, Compound AAtR-67, and Compound AAtR-68; mixed solution of Compound AAtR-69, Compound AAtR-70, and Compound AAtR-71; or mixed solution of Compound AAtR-78, Compound AAtR-79, and Compound AAtR-80) was added at 30 µM to the translation reaction mixture, and this was left to stand at 37°C for one hour.

Cell-free translations were also performed on other sequences (SEQ ID NO: 159 (mR-26), SEQ ID NO: 144 (mR-11), and SEQ ID NO: 145 (mR-12)).

Specifically, the translation was carried out as follows: 1 µM template mRNA (SEQ ID NO: 159 (mR-26), SEQ ID NO: 144 (mR-11), or SEQ ID NO: 145 (mR-12)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 3 µM Ala1BtRNA, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-27, Compound AAtR-11, and Compound AAtR-12; or mixed solution of Compound AAtR-48, Compound AAtR-33, and Compound AAtR-34) was added at 30 µM to the translation reaction mixture, and this was left to stand at 37°C for one hour.

Cell-free translations were also performed on other sequences (SEQ ID NO: 162 (mR-29), SEQ ID NO: 153 (mR-20), and SEQ ID NO: 154 (mR-21)).

Specifically, the translation was carried out as follows: 1 µM template mRNA (SEQ ID NO: 162 (mR-29), SEQ ID NO: 153 (mR-20), or SEQ ID NO: 154 (mR-21)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 3 µM Ala1BtRNA, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-28, Compound AAtR-20, and Compound AAtR-21; or mixed solution of Compound AAtR-51, Compound AAtR-42, and Compound AAtR-43) was added at 30 µM to the translation reaction mixture, and this was left to stand at 37°C for one hour.

Cell-free translations were also performed on other sequences (SEQ ID NO: 146 (mR-13), SEQ ID NO: 147 (mR-14), and SEQ ID NO: 148 (mR-15)).

Specifically, the translation was carried out as follows: 1 µM template mRNA (SEQ ID NO: 146 (mR-13), SEQ ID NO: 147 (mR-14), or SEQ ID NO: 148 (mR-15)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 54 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-35, Compound AAtR-36, and Compound AAtR-37) was added at 30 µM to the translation reaction mixture, and this was left to stand at 37°C for one hour.

Cell-free translations were also performed on other sequences (SEQ ID NO: 152 (mR-19), SEQ ID NO: 153 (mR-20), and SEQ ID NO: 154 (mR-21)).

Specifically, the translation was carried out as follows: 1 µM template mRNA (SEQ ID NO: 152 (mR-19), SEQ ID NO: 153 (mR-20), or SEQ ID NO: 154 (mR-21)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 54 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM I1eRS, 0.68 µM PheRS, 0.16 µM ProRS, and 0.09 µM ThrRS), and a mixed aminoacylated tRNA solution (mixed solution of Compound AAtR-41, Compound AAtR-42, and Compound AAtR-43) was added at 30 µM to the translation reaction mixture, and this was left to stand at 37°C for one hour.

Cell-free translations were also performed on other sequences (SEQ ID NO: 155 (mR-22), SEQ ID NO: 156 (mR-23), and SEQ ID NO: 157 (mR-24)).

Specifically, the translation was carried out as follows: 1 µM template mRNA (SEQ ID NO: 155 (mR-22), SEQ ID NO: 156 (mR-23), or SEQ ID NO: 157 (mR-24)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.25 µM RF 1, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 34.6 µM EF-Ts, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-44, Compound AAtR-45, and Compound AAtR-46) was added at 30 µM to the translation reaction mixture, and this was left to stand at 37°C for one hour.

Cell-free translations were also performed on other sequences (SEQ ID NO: 160 (mR-27), SEQ ID NO: 147 (mR-14), and SEQ ID NO: 148 (mR-15)).

Specifically, the translation was carried out as follows: 1 µM template mRNA (SEQ ID NO: 160 (mR-27), SEQ ID NO: 147 (mR-14), or SEQ ID NO: 148 (mR-15)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 3 µM Ala1BtRNA, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-49, Compound AAtR-36, and Compound AAtR-37) was added at 30 µM to the translation reaction mixture, and this was left to stand at 37°C for one hour.

Cell-free translations were also performed on other sequences (SEQ ID NO: 161 (mR-28), SEQ ID NO: 150 (mR-17), and SEQ ID NO: 151 (mR-18)).

Specifically, the translation was carried out as follows: 1 µM template mRNA (SEQ ID NO: 161 (mR-28), SEQ ID NO: 150 (mR-17), SEQ ID NO: 151 (mR-18)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM I1eRS, 0.68 µM PheRS, 0.16 µM ProRS, and 0.02 µM ValRS), and a mixed aminoacylated tRNA solution (mixed solution of Compound AAtR-50, Compound AAtR-39, and Compound AAtR-40) was added at 30 µM to the translation reaction mixture, and this was left to stand at 37°C for one hour.

Cell-free translations were also performed on other sequences (SEQ ID NO: 219 (mR-42), SEQ ID NO: 220 (mR-43), and SEQ ID NO: 221 (mR-44)).

Specifically, the translation was carried out as follows: 1 µM template mRNA (SEQ ID NO: 219 (mR-42), SEQ ID NO: 220 (mR-43), or SEQ ID NO: 221 (mR-44)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 3 µM Ala1BtRNA, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 49 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and a mixed aminoacylated tRNA solution (mixed solution of Compound AAtR-57 and Compound AAtR-59) was added at 20 µM, and aminoacylated tRNA (Compound AAtR-58) was added at 20 µM to the translation reaction mixture, and this was left to stand at 37°C for one hour.

Next, to examine whether discrimination efficiency in a codon box differs depending on the concentration of an aminoacylated tRNA, the proportion of cross-reading of a codon other than the XXA codon in the codon box was determined by the amount of translation under conditions where the only aminoacylated tRNA present in the translation system is that having an anticodon for XXA. Specifically, template mRNAs containing any one of four codons in the same codon box and having the same sequence for the rest of the sequences (template mRNAs of SEQ ID NO: 163 (mR-30) to SEQ ID NO: 174 (mR-41)) were translated by adding an aminoacylated tRNA having an anticodon for the XXA codon (Compound AAtR-52, Compound AAtR-53, Compound AAtR-54, Compound AAtR-55, or Compound AAtR-56) at different concentration conditions to translationally synthesize peptide compounds.

It was shown that discrimination rate within a codon box changes depending on the concentration of aminoacyl tRNA. Furthermore, this phenomenon was shown to be independent of tRNA body sequences and the amino acids charged.

The translation system used was PURE system, a prokaryote-derived reconstituted cell-free protein synthesis system. Specifically, the translation was carried out as follows: 1 µM template mRNA (SEQ ID NO: 163 (mR-30), SEQ ID NO: 164 (mR-31), SEQ ID NO: 165 (mR-32), SEQ ID NO: 166 (mR-33)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 3 µM Ala1BtRNA 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, 0.16 µM ProRS, and 0.09 µM ThrRS), and an aminoacylated tRNA (Compound AAtR-52) was added at 0.8 µM or 1.6 µM or 3.2 µM, 6.4 µM or 12.8 µM to the translation reaction mixture, and left at 37°C for one hour.

Similar examinations were performed on other codon boxes.

Specifically, examination was carried out as follows: 1 µM template mRNA (SEQ ID NO: 167 (mR-34), SEQ ID NO: 168 (mR-35), SEQ ID NO: 169 (mR-36), SEQ ID NO: 170 (mR-37)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 3 µM Ala1BtRNA, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.4 µM IleRS, 0.68 µM PheRS, and 0.16 µM ProRS), and aminoacylated tRNA (Compound AAtR-53) was added at 0.8 µM, 1.6 µM, 3.2 µM, 6.4 µM, or 12.8 µM to the translation reaction mixture, and this was left to stand at 37°C for one hour.

Similar examinations were performed on other codon boxes.

Specifically, the examination was carried out as follows: 1 µM template mRNA (SEQ ID NO: 171 (mR-38), SEQ ID NO: 172 (mR-39), SEQ ID NO: 173 (mR-40), SEQ ID NO: 174 (mR-41)), a group of natural amino acids encoded in the respective template mRNAs at 0.25 mM respectively, and initiator aminoacylated tRNA (Compound AAtR-25) at 10 µM were added to a translation solution (1 mM GTP, 1 mM ATP, 20 mM phosphocreatine, 50 mM HEPES-KOH pH7.6, 100 mM potassium acetate, 10 mM magnesium acetate, 2 mM spermidine, 1 mM dithiothreitol, 3 µM Ala1BtRNA, 1.5 mg/mL E. coli MRE600 (RNase-negative)-derived tRNA (Roche), 0.26 µM EF-G, 0.24 µM RF2, 0.17 µM RF3, 0.5 µM RRF, 4 µg/mL creatine kinase, 3 µg/mL myokinase, 2 unit/mL inorganic pyrophosphatase, 1.1 µg/mL nucleoside diphosphate kinase, 2.7 µM IF1, 0.4 µM IF2, 1.5 µM IF3, 40 µM EF-Tu, 35 µM EF-Ts, 1 µM EF-P-Lys, 0.4 unit/µL RNasein Ribonuclease inhibitor (Promega, N2111), 1.2 µM ribosome, 0.5 mM PGA, 0.09 µM GlyRS, 0.68 µM LeuRS, 0.68 µM PheRS, and 0.16 µM ProRS), and aminoacylated tRNA (Compound AAtR-54, Compound AAtR-55, or Compound AAtR-56) was added at 0.8 µM, 1.6 µM, 3.2 µM, 6.4 µM, or 12.8 µM to the translation reaction mixture, and this was left to stand at 37°C for one hour.

The template mRNA, the expected translated peptide compound, and the molecular weight of the peptide are shown in Table 4 below.

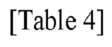

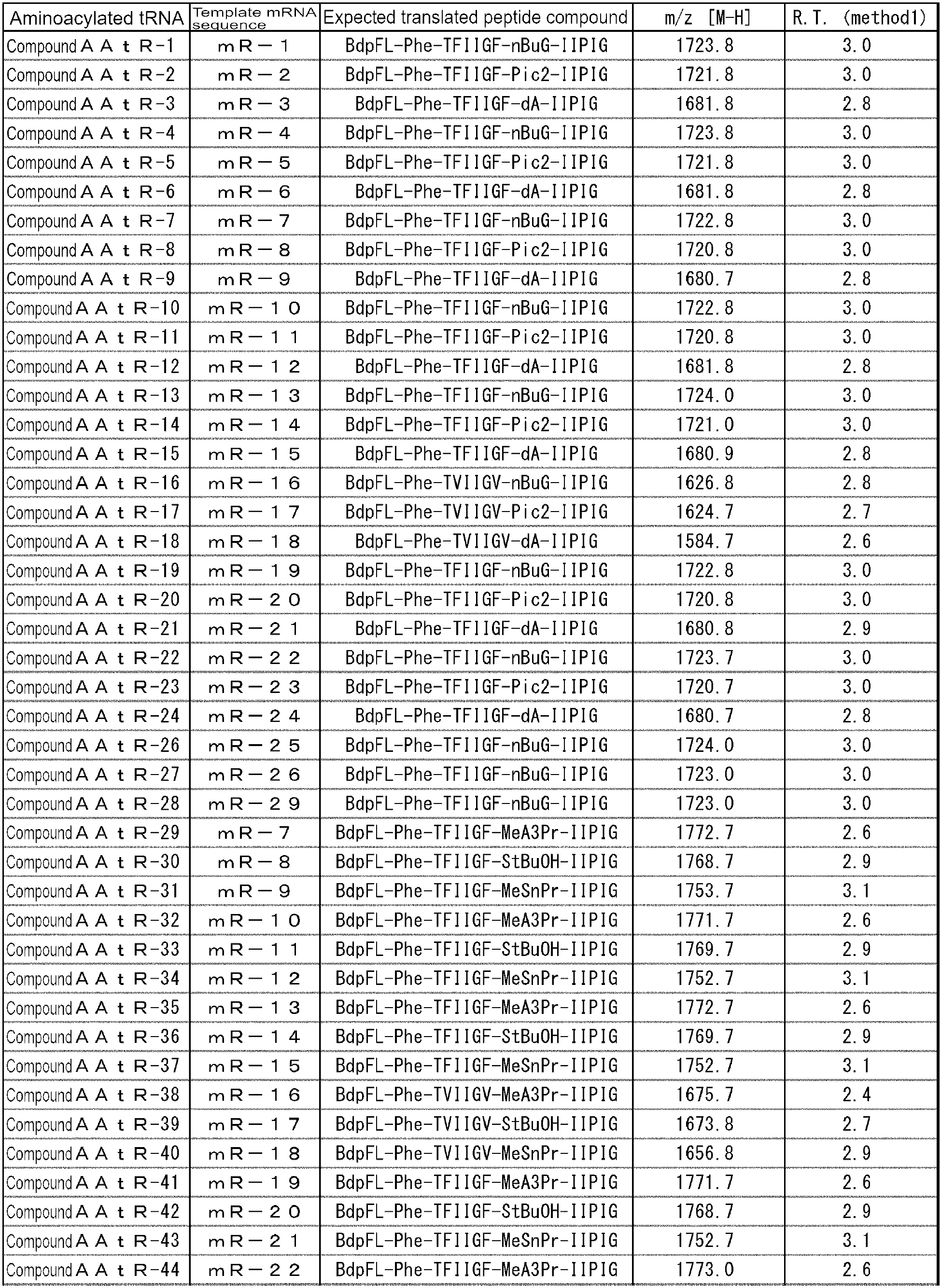

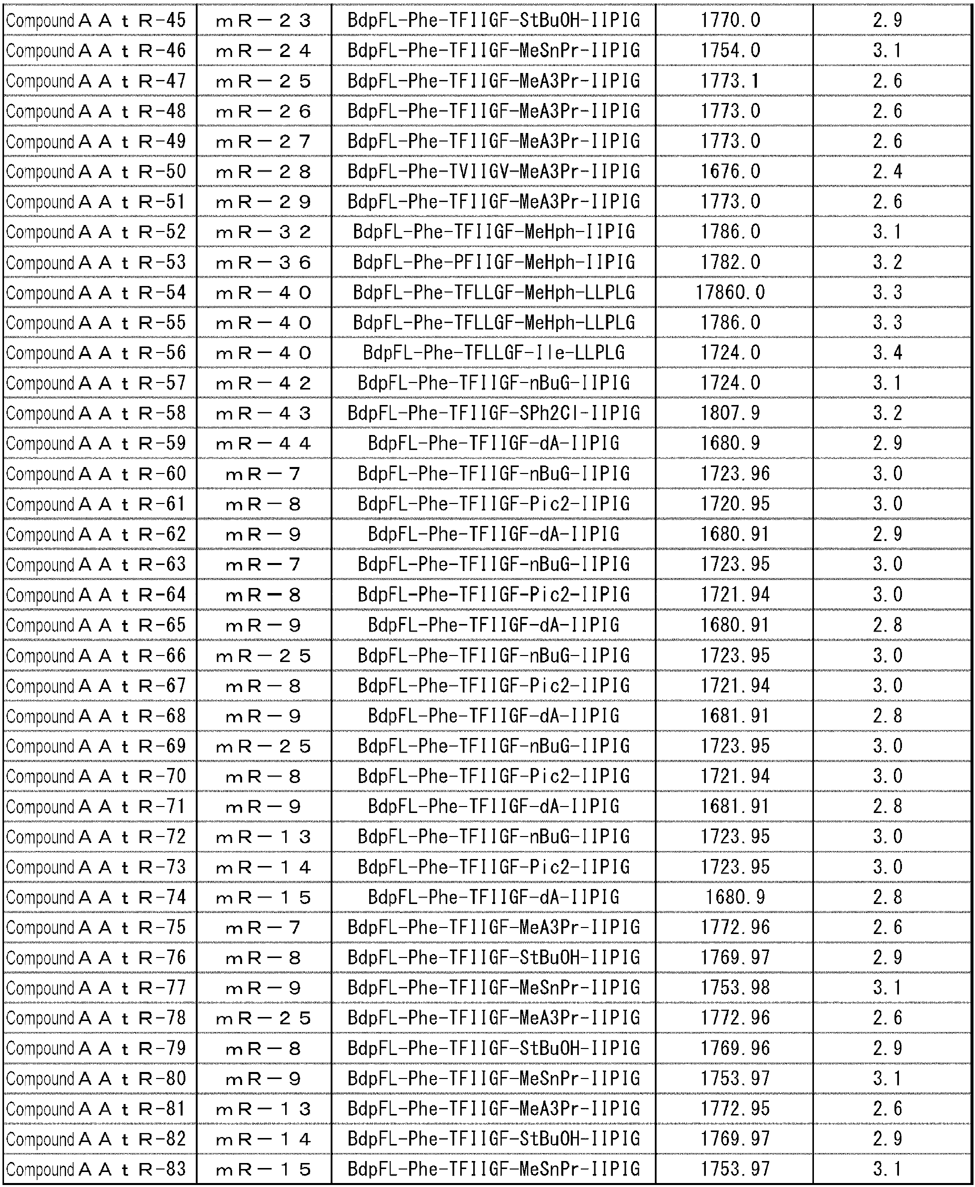

From the template DNAs (SEQ ID NO: 175 (D-35) to SEQ ID NO: 215 (D-75) and SEQ ID NO: 216 (D-79) to SEQ ID NO: 218 (D-81)), template mRNAs (SEQ ID NO: 134 (mR-1) to SEQ ID NO: 174 (mR-41) and SEQ ID NO: 219 (mR-42) to SEQ ID NO: 221 (mR-44)) were synthesized by in vitro transcription reaction using RiboMAX Large Scale RNA production System T7 (Promega, P1300), and then purified by RNeasy Mini kit (Qiagen).

Template DNA SEQ ID NO: 175 (D-35)
   DNA sequence: 
GGCGTAATACGACTCACTATAGGGTTAACTTTAAGAAGGAGATATACATATGACTTT
TATTATTGGTTTTCTTATTATTCCGATTGGTTAAGCTTCG
Template DNA SEQ ID NO: 176 (D-36)
   DNA sequence: 
GGCGTAATACGACTCACTATAGGGTTAACTTTAAGAAGGAGATATACATATGACTTT
TATTATTGGTTTTCTAATTATTCCGATTGGTTAAGCTTCG
Template DNA SEQ ID NO: 177 (D-37)
   DNA sequence: 
GGCGTAATACGACTCACTATAGGGTTAACTTTAAGAAGGAGATATACATATGACTTT
TATTATTGGTTTTCTGATTATTCCGATTGGTTAAGCTTCG
Template DNA SEQ ID NO: 178 (D-38)
   DNA sequence: 
GGCGTAATACGACTCACTATAGGGTTAACTTTAAGAAGGAGATATACATATGACTTT
TATTATTGGTTTTGTTATTATTCCGATTGGTTAAGCTTCG
Template DNA SEQ ID NO: 179 (D-39)
   DNA sequence: 
GGCGTAATACGACTCACTATAGGGTTAACTTTAAGAAGGAGATATACATATGACTTT
TATTATTGGTTTTGTAATTATTCCGATTGGTTAAGCTTCG
Template DNA SEQ ID NO: 180 (D-40)
   DNA sequence: 
GGCGTAATACGACTCACTATAGGGTTAACTTTAAGAAGGAGATATACATATGACTTT
TATTATTGGTTTTGTGATTATTCCGATTGGTTAAGCTTCG
Template DNA SEQ ID NO: 181 (D-41)
   DNA sequence: 
GGCGTAATACGACTCACTATAGGGTTAACTTTAAGAAGGAGATATACATATGACTTT
TATTATTGGTTTTCATATTATTCCGATTGGTTAAGCTTCG
Template DNA SEQ ID NO: 182 (D-42)
   DNA sequence: 
GGCGTAATACGACTCACTATAGGGTTAACTTTAAGAAGGAGATATACATATGACTTT
TATTATTGGTTTTCAAATTATTCCGATTGGTTAAGCTTCG
Template DNA SEQ ID NO: 183 (D-43)
   DNA sequence: 
GGCGTAATACGACTCACTATAGGGTTAACTTTAAGAAGGAGATATACATATGACTTT
TATTATTGGTTTTCAGATTATTCCGATTGGTTAAGCTTCG
Template DNA SEQ ID NO: 184 (D-44)
   DNA sequence: 
GGCGTAATACGACTCACTATAGGGTTAACTTTAAGAAGGAGATATACATATGACTTT
TATTATTCTATTTAATATTATTCCGATTCTATAAGCTTCG
Template DNA SEQ ID NO: 185 (D-45)
   DNA sequence: 
GGCGTAATACGACTCACTATAGGGTTAACTTTAAGAAGGAGATATACATATGACTTT
TATTATTCTATTTAAAATTATTCCGATTCTATAAGCTTCG
Template DNA SEQ ID NO: 186 (D-46)
   DNA sequence: 
GGCGTAATACGACTCACTATAGGGTTAACTTTAAGAAGGAGATATACATATGACTTT
TATTATTCTATTTAAGATTATTCCGATTCTATAAGCTTCG
Template DNA SEQ ID NO: 187 (D-47)
   DNA sequence: 
GGCGTAATACGACTCACTATAGGGTTAACTTTAAGAAGGAGATATACATATGACTTT
TATTATTGGTTTTCATATTATTCCGATTGGTTAAGCTTCG
Template DNA SEQ ID NO: 188 (D-48)
   DNA sequence: 
GGCGTAATACGACTCACTATAGGGTTAACTTTAAGAAGGAGATATACATATGACTTT
TATTATTGGTTTTCAAATTATTCCGATTGGTTAAGCTTCG
Template DNA SEQ ID NO: 189 (D-49)
   DNA sequence: 
GGCGTAATACGACTCACTATAGGGTTAACTTTAAGAAGGAGATATACATATGACTTT
TATTATTGGTTTTCAGATTATTCCGATTGGTTAAGCTTCG
Template DNA SEQ ID NO: 190 (D-50)
   DNA sequence: 
GGCGTAATACGACTCACTATAGGGTTAACTTTAAGAAGGAGATATACATATGACTGT
TATTATTGGTGTTTTTATTATTCCGATTGGTTAAGCTTCG
Template DNA SEQ ID NO: 191 (D-51)
   DNA sequence: 
GGCGTAATACGACTCACTATAGGGTTAACTTTAAGAAGGAGATATACATATGACTGT
TATTATTGGTGTTTTAATTATTCCGATTGGTTAAGCTTCG
Template DNA SEQ ID NO: 192 (D-52)
   DNA sequence: 
GGCGTAATACGACTCACTATAGGGTTAACTTTAAGAAGGAGATATACATATGACTGT
TATTATTGGTGTTTTGATTATTCCGATTGGTTAAGCTTCG
Template DNA SEQ ID NO: 193 (D-53)
   DNA sequence: 
GGCGTAATACGACTCACTATAGGGTTAACTTTAAGAAGGAGATATACATATGACTTT
TATTATTGGTTTTAGTATTATTCCGATTGGTTAAGCTTCG
Template DNA SEQ ID NO: 194 (D-54)
   DNA sequence: 
GGCGTAATACGACTCACTATAGGGTTAACTTTAAGAAGGAGATATACATATGACTTT
TATTATTGGTTTTAGAATTATTCCGATTGGTTAAGCTTCG
Template DNA SEQ ID NO: 195 (D-55)
   DNA sequence: 
GGCGTAATACGACTCACTATAGGGTTAACTTTAAGAAGGAGATATACATATGACTTT
TATTATTGGTTTTAGGATTATTCCGATTGGTTAAGCTTCG
Template DNA SEQ ID NO: 196 (D-56)
   DNA sequence: 
GGCGTAATACGACTCACTATAGGGTTAACTTTAAGAAGGAGATATACATATGACTTT
TATTATTCTATTTTGCATTATTCCGATTCTATAAGCTTCG
Template DNA SEQ ID NO: 197 (D-57)
   DNA sequence: 
GGCGTAATACGACTCACTATAGGGTTAACTTTAAGAAGGAGATATACATATGACTTT
TATTATTCTATTTTGAATTATTCCGATTCTATAAGCTTCG
Template DNA SEQ ID NO: 198 (D-58)
   DNA sequence: 
GGCGTAATACGACTCACTATAGGGTTAACTTTAAGAAGGAGATATACATATGACTTT
TATTATTCTATTTTGGATTATTCCGATTCTATAAGCTTC
Template DNA SEQ ID NO: 199 (D-59)
   DNA sequence: 
GGCGTAATACGACTCACTATAGGGTTAACTTTAAGAAGGAGATATACATATGACTTT
TATTATTGGTTTTCACATTATTCCGATTGGTTAAGCTTCG
Template DNA SEQ ID NO: 200 (D-60)
   DNA sequence: 
GGCGTAATACGACTCACTATAGGGTTAACTTTAAGAAGGAGATATACATATGACTTT
TATTATTGGTTTTAACATTATTCCGATTGGTTAAGCTTCG
Template DNA SEQ ID NO: 201 (D-61)
   DNA sequence: 
GGCGTAATACGACTCACTATAGGGTTAACTTTAAGAAGGAGATATACATATGACTTT
TATTATTCTATTTGACATTATTCCGATTCTATAAGCTTCG
Template DNA SEQ ID NO: 202 (D-62)
   DNA sequence: 
GGCGTAATACGACTCACTATAGGGTTAACTTTAAGAAGGAGATATACATATGACTGT
TATTATTGGTGTTTTCATTATTCCGATTGGTTAAGCTTCG
Template DNA SEQ ID NO: 203 (D-63)
   DNA sequence: 
GGCGTAATACGACTCACTATAGGGTTAACTTTAAGAAGGAGATATACATATGACTTT
TATTATTCTATTTAGCATTATTCCGATTCTATAAGCTTC
Template DNA SEQ ID NO: 204 (D-64)
   DNA sequence: 
GGCGTAATACGACTCACTATAGGGTTAACTTTAAGAAGGAGATATACATATGACTTT
TATTATTGGTTTTTCTATTATTCCGATTGGTTAAGCTTCG
Template DNA SEQ ID NO: 205 (D-65)
   DNA sequence: 
GGCGTAATACGACTCACTATAGGGTTAACTTTAAGAAGGAGATATACATATGACTTT
TATTATTGGTTTTTCCATTATTCCGATTGGTTAAGCTTCG
Template DNA SEQ ID NO: 206 (D-66)
   DNA sequence: 
GGCGTAATACGACTCACTATAGGGTTAACTTTAAGAAGGAGATATACATATGACTTT
TATTATTGGTTTTTCAATTATTCCGATTGGTTAAGCTTCG
Template DNA SEQ ID NO: 207 (D-67)
   DNA sequence: 
GGCGTAATACGACTCACTATAGGGTTAACTTTAAGAAGGAGATATACATATGACTTT
TATTATTGGTTTTTCGATTATTCCGATTGGTTAAGCTTCG
Template DNA SEQ ID NO: 208 (D-68)
   DNA sequence: 
GGCGTAATACGACTCACTATAGGGTTAACTTTAATAAGGAGATATAAATATGCCGTT
TATTATTGGTTTTACTATTATTCCGATTGGTTAAGCTTCG
Template DNA SEQ ID NO: 209 (D-69)
   DNA sequence: 
GGCGTAATACGACTCACTATAGGGTTAACTTTAATAAGGAGATATAAATATGCCGTT
TATTATTGGTTTTACCATTATTCCGATTGGTTAAGCTTCG
Template DNA SEQ ID NO: 210 (D-70)
   DNA sequence: 
GGCGTAATACGACTCACTATAGGGTTAACTTTAATAAGGAGATATAAATATGCCGTT
TATTATTGGTTTTACAATTATTCCGATTGGTTAAGCTTCG
Template DNA SEQ ID NO: 211 (D-71)
   DNA sequence: 
GGCGTAATACGACTCACTATAGGGTTAACTTTAATAAGGAGATATAAATATGCCGTT
TATTATTGGTTTTACGATTATTCCGATTGGTTAAGCTTCG
Template DNA SEQ ID NO: 212 (D-72)
   DNA sequence: 
GGCGTAATACGACTCACTATAGGGTTAACTTTAAGAAGGAGATATACATATGACTTT
TCTACTAGGTTTTATTCTACTACCGCTAGGTTAAGCTTCG
Template DNA SEQ ID NO: 213 (D-73)
   DNA sequence: 
GGCGTAATACGACTCACTATAGGGTTAACTTTAAGAAGGAGATATACATATGACTTT
TCTACTAGGTTTTATCCTACTACCGCTAGGTTAAGCTTCG
Template DNA SEQ ID NO: 214 (D-74)
   DNA sequence: 
GGCGTAATACGACTCACTATAGGGTTAACTTTAAGAAGGAGATATACATATGACTTT
TCTACTAGGTTTTATACTACTACCGCTAGGTTAAGCTTCG
Template DNA SEQ ID NO: 215 (D-75)
   DNA sequence: 
GGCGTAATACGACTCACTATAGGGTTAACTTTAAGAAGGAGATATACATATGACTTT
TCTACTAGGTTTTATGCTACTACCGCTAGGTTAAGCTTCG
Template DNA SEQ ID NO: 216 (D-79)
   DNA sequence: 
GGCGTAATACGACTCACTATAGGGTTAACTTTAAGAAGGAGATATACATATGACTTT
TATTATTGGTTTTCGTATTATTCCGATTGGTTAAGCTTCG
Template DNA SEQ ID NO: 217 (D-80)
   DNA sequence: 
GGCGTAATACGACTCACTATAGGGTTAACTTTAAGAAGGAGATATACATATGACTTT
TATTATTGGTTTTCGAATTATTCCGATTGGTTAAGCTTCG
Template DNA SEQ ID NO: 218 (D-81)
   DNA sequence: 
GGCGTAATACGACTCACTATAGGGTTAACTTTAAGAAGGAGATATACATATGACTTT
TATTATTGGTTTTCGGATTATTCCGATTGGTTAAGCTTCG
Template mRNA SEQ ID NO: 134 (mR-1)
   RNA sequence: 
GGGUUAACUUUAAGAAGGAGAUAUACAUAUGACUUUUAUUAUUGGUUUUCUUAU
UAUUCCGAUUGGUUAAGCUUCG
Template mRNA SEQ ID NO: 135 (mR-2)
   RNA sequence: 
GGGUUAACUUUAAGAAGGAGAUAUACAUAUGACUUUUAUUAUUGGUUUUCUAAU
UAUUCCGAUUGGUUAAGCUUCG
Template mRNA SEQ ID NO: 136 (mR-3)
   RNA sequence: 
GGGUUAACUUUAAGAAGGAGAUAUACAUAUGACUUUUAUUAUUGGUUUUCUGAU
UAUUCCGAUUGGUUAAGCUUCG
Template mRNA SEQ ID NO: 137 (mR-4)
   RNA sequence: 
GGGUUAACUUUAAGAAGGAGAUAUACAUAUGACUUUUAUUAUUGGUUUUGUUAU
UAUUCCGAUUGGUUAAGCUUCG
Template mRNA SEQ ID NO: 138 (mR-5)
   RNA sequence: 
GGGUUAACUUUAAGAAGGAGAUAUACAUAUGACUUUUAUUAUUGGUUUUGUAAU
UAUUCCGAUUGGUUAAGCUUCG
Template mRNA SEQ ID NO: 139 (mR-6)
   RNA sequence: 
GGGUUAACUUUAAGAAGGAGAUAUACAUAUGACUUUUAUUAUUGGUUUUGUGAU
UAUUCCGAUUGGUUAAGCUUCG
Template mRNA SEQ ID NO: 140 (mR-7)
   RNA sequence: 
GGGUUAACUUUAAGAAGGAGAUAUACAUAUGACUUUUAUUAUUGGUUUUCAUAU
UAUUCCGAUUGGUUAAGCUUCG
Template mRNA SEQ ID NO: 141 (mR-8)
   RNA sequence: 
GGGUUAACUUUAAGAAGGAGAUAUACAUAUGACUUUUAUUAUUGGUUUUCAAAU
UAUUCCGAUUGGUUAAGCUUCG
Template mRNA SEQ ID NO: 142 (mR-9)
   RNA sequence: 
GGGUUAACUUUAAGAAGGAGAUAUACAUAUGACUUUUAUUAUUGGUUUUCAGAU
UAUUCCGAUUGGUUAAGCUUCG
Template mRNA SEQ ID NO: 143 (mR-10)
   RNA sequence: 
GGGUUAACUUUAAGAAGGAGAUAUACAUAUGACUUUUAUUAUUCUAUUUAAUAU
UAUUCCGAUUCUAUAAGCUUCG
Template mRNA SEQ ID NO: 144 (mR-11)
   RNA sequence: 
GGGUUAACUUUAAGAAGGAGAUAUACAUAUGACUUUUAUUAUUCUAUUUAAAAU
UAUUCCGAUUCUAUAAGCUUCG
Template mRNA SEQ ID NO: 145 (mR-12)
   RNA sequence: 
GGGUUAACUUUAAGAAGGAGAUAUACAUAUGACUUUUAUUAUUCUAUUUAAGAU
UAUUCCGAUUCUAUAAGCUUCG
Template mRNA SEQ ID NO: 146 (mR-13)
   RNA sequence: 
GGGUUAACUUUAAGAAGGAGAUAUACAUAUGACUUUUAUUAUUGGUUUUGAUAU
UAUUCCGAUUGGUUAAGCUUCG
Template mRNA SEQ ID NO: 147 (mR-14)
   RNA sequence: 
GGGUUAACUUUAAGAAGGAGAUAUACAUAUGACUUUUAUUAUUGGUUUUGAAAU
UAUUCCGAUUGGUUAAGCUUCG
Template mRNA SEQ ID NO: 148 (mR-15)
   RNA sequence: 
GGGUUAACUUUAAGAAGGAGAUAUACAUAUGACUUUUAUUAUUGGUUUUGAGAU
UAUUCCGAUUGGUUAAGCUUCG
Template mRNA SEQ ID NO: 149 (mR-16)
   RNA sequence: 
GGGUUAACUUUAAGAAGGAGAUAUACAUAUGACUGUUAUUAUUGGUGUUUUUAU
UAUUCCGAUUGGUUAAGCUUCG
Template mRNA SEQ ID NO: 150 (mR-17)
   RNA sequence: 
GGGUUAACUUUAAGAAGGAGAUAUACAUAUGACUGUUAUUAUUGGUGUUUUAAU
UAUUCCGAUUGGUUAAGCUUCG
Template mRNA SEQ ID NO: 151 (mR-18)
   RNA sequence: 
GGGUUAACUUUAAGAAGGAGAUAUACAUAUGACUGUUAUUAUUGGUGUUUUGAU
UAUUCCGAUUGGUUAAGCUUCG
Template mRNA SEQ ID NO: 152 (mR-19)
   RNA sequence: 
GGGUUAACUUUAAGAAGGAGAUAUACAUAUGACUUUUAUUAUUGGUUUUAGUAU
UAUUCCGAUUGGUUAAGCUUCG
Template mRNA SEQ ID NO: 153 (mR-20)
   RNA sequence: 
GGGUUAACUUUAAGAAGGAGAUAUACAUAUGACUUUUAUUAUUGGUUUUAGAAU
UAUUCCGAUUGGUUAAGCUUCG
Template mRNA SEQ ID NO: 154 (mR-21)
   RNA sequence: 
GGGUUAACUUUAAGAAGGAGAUAUACAUAUGACUUUUAUUAUUGGUUUUAGGAU
UAUUCCGAUUGGUUAAGCUUCG
Template mRNA SEQ ID NO: 155 (mR-22)
   RNA sequence: 
GGGUUAACUUUAAGAAGGAGAUAUACAUAUGACUUUUAUUAUUCUAUUUUGCAU
UAUUCCGAUUCUAUAAGCUUCG
Template mRNA SEQ ID NO: 156 (mR-23)
   RNA sequence: 
GGGUUAACUUUAAGAAGGAGAUAUACAUAUGACUUUAUUAUUCUAUUUGAAU
UAUUCCGAUUCUAUAAGCUUCG
Template mRNA SEQ ID NO: 157 (mR-24)
   RNA sequence: 
GGGUUAACUUUAAGAAGGAGAUAUACAUAUGACUUUUAUUAUUCUAUUUUGGAU
UAUUCCGAUUCUAUAAGCUUCG
Template mRNA SEQ ID NO: 158 (mR-25)
   RNA sequence: 
GGGUUAACUUUAAGAAGGAGAUAUACAUAUGACUUUUAUUAUUGGUUUUCACAU
UAUUCCGAUUGGUUAAGCUUCG
Template mRNA SEQ ID NO: 159 (mR-26)
   RNA sequence: 
GGGUUAACUUUAAGAAGGAGAUAUACAUAUGACUUUUAUUAUUGGUUUUAACAU
UAUUCCGAUUGGUUAAGCUUCG
Template mRNA SEQ ID NO: 160 (mR-27)
   RNA sequence: 
GGGUUAACUUUAAGAAGGAGAUAUACAUAUGACUUUUAUUAUUGGUUUUGACAU
UAUUCCGAUUGGUUAAGCUUCG
Template mRNA SEQ ID NO: 161 (mR-28)
   RNA sequence: 
GGGUUAACUUUAAGAAGGAGAUAUACAUAUGACUGUUAUUAUUGGUGUUUUCAU
UAUUCCGAUUGGUUAAGCUUCG
Template mRNA SEQ ID NO: 162 (mR-29)
   RNA sequence: 
GGGUUAACUUUAAGAAGGAGAUAUACAUAUGACUUUUAUUAUUCUAUUUAGCAU
UAUUCCGAUUCUAUAAGCUUCG
Template mRNA SEQ ID NO: 163 (mR-30)
   RNA sequence: 
GGGUUAACUUUAAGAAGGAGAUAUACAUAUGACUUUUAUUAUUGGUUUUUCUAU
UAUUCCGAUUGGUUAAGCUUCG
Template mRNA SEQ ID NO: 164 (mR-31)
   RNA sequence: 
GGGUUAACUUUAAGAAGGAGAUAUACAUAUGACUUUUAUUAUUGGUUUUUCCAU
UAUUCCGAUUGGUUAAGCUUCG
Template mRNA SEQ ID NO: 165 (mR-32)
   RNA sequence: 
GGGUUAACUUUAAGAAGGAGAUAUACAUAUGACUUUUAUUAUUGGUUUUUCAAU
UAUUCCGAUUGGUUAAGCUUCG
Template mRNA SEQ ID NO: 166 (mR-33)
   RNA sequence: 
GGGUUAACUUUAAGAAGGAGAUAUACAUAUGACUUUUAUUAUUGGUUUUUCGAU
UAUUCCGAUUGGUUAAGCUUCG
Template mRNA SEQ ID NO: 167 (mR-34)
   RNA sequence: 
GGGUUAACUUUAAUAAGGAGAUAUAAAUAUGCCGUUUAUUAUUGGUUUUACUAU
UAUUCCGAUUGGUUAAGCUUCG
Template mRNA SEQ ID NO: 168 (mR-35)
   RNA sequence: 
GGGUUAACUUUAAUAAGGAGAUAUAAAUAUGCCGUUUAUUAUUGGUUUUACCAU
UAUUCCGAUUGGUUAAGCUUCG
Template mRNA SEQ ID NO: 169 (mR-36)
   RNA sequence: 
GGGUUAACUUUAAUAAGGAGAUAUAAAUAUGCCGUUUAUUAUUGGUUUUACAAU
UAUUCCGAUUGGUUAAGCUUCG
Template mRNA SEQ ID NO: 170 (mR-37)
   RNA sequence: 
GGGUUAACUUUAAUAAGGAGAUAUAAAUAUGCCGUUUAUUAUUGGUUUUACGAU
UAUUCCGAUUGGUUAAGCUUCG
Template mRNA SEQ ID NO: 171 (mR-38)
   RNA sequence: 
GGGUUAACUUUAAGAAGGAGAUAUACAUAUGACUUUUCUACUAGGUUUUAUUCU
ACUACCGCUAGGUUAAGCUUCG
Template mRNA SEQ ID NO: 172 (mR-39)
   RNA sequence: 
GGGUUAACUUUAAGAAGGAGAUAUACAUAUGACUUUUCUACUAGGUUUUAUCCU
ACUACCGCUAGGUUAAGCUUCG
Template mRNA SEQ ID NO: 173 (mR-40)
   RNA sequence: 
GGGUUAACUUUAAGAAGGAGAUAUACAUAUGACUUUUCUACUAGGUUUUAUACU
ACUACCGCUAGGUUAAGCUUCG
Template mRNA SEQ ID NO: 174 (mR-41)
   RNA sequence: 
GGGUUAACUUUAAGAAGGAGAUAUACAUAUGACUUUUCUACUAGGUUUUAUGCU
ACUACCGCUAGGUUAAGCUUCG
Template mRNA SEQ ID NO: 219 (mR-42)
   RNA sequence: 
GGGUUAACUUUAAGAAGGAGAUAUACAUAUGACUUUUAUUAUUGGUUUUCGUAU
UAUUCCGAUUGGUUAAGCUUCG
Template mRNA SEQ ID NO: 220 (mR-43)
   RNA sequence: 
GGGUUAACUUUAAGAAGGAGAUAUACAUAUGACUUUUAUUAUUGGUUUUCGAAU
UAUUCCGAUUGGUUAAGCUUCG
Template mRNA SEQ ID NO: 221 (mR-44)
   RNA sequence :

GGGUUAACUUUAAGAAGGAGAUAUACAUAUGACUUUUAUUAUUGGUUUUCGGAU
UAUUCCGAUUGGUUAAGCUUCG

### Example 7. Analysis of the translated peptides

The unnatural peptide translation solutions prepared in Example 6 were diluted ten-fold, and then analyzed using a LC-FLR-MS system. The amount of translated peptide was evaluated from the analysis data by identifying the retention time of the target translated peptide from the MS data, and quantifying the fluorescence peak at the relevant retention time. In the quantitative evaluation, the LCT12 synthesized in Example 4 or the LCT067 synthesized in Example 3 was used as a standard to prepare a calibration curve, and the content was calculated by relative quantification. The LC-MS was analyzed according to the conditions shown in Table 5 below by selecting the optimum conditions according to the sample of interest.

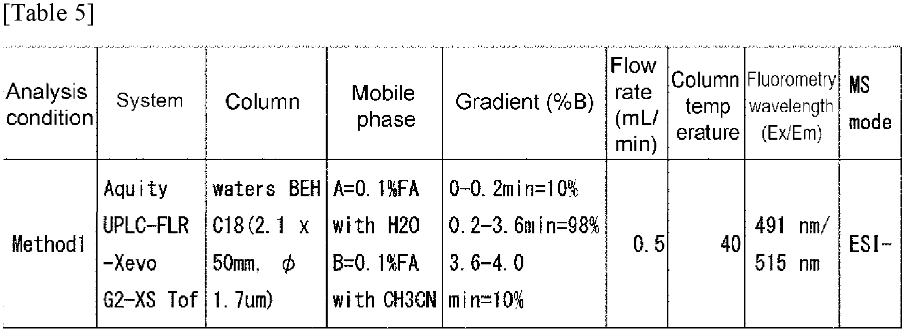

The results of evaluation are shown in Tables 6 to 33 and Figures 1 to 28. Among the examined codon boxes, translation into an amino acid other than the intended amino acid by cross-reading was somewhat large in number for codon boxes CUX and GUX, and sufficient discrimination effects were not observed (Table 6 and Table 7, and Fig. 1 and Fig. 2). On the other hand, in each of the other codon boxes, specific amino acid translation corresponding to three codons was observed, and sufficient discrimination effect was observed. Furthermore, it was confirmed that similar effects are obtained even when the tRNA body (the portion of the sequence other than the anticodon) was changed. (Table 8 to Table 28-9, and Fig. 3 to Fig. 23-9). Furthermore, it was suggested that although increasing the concentration of the tRNA added to the translation system increases both the amount of translated peptide of interest and cross-reading, their rate of increase may be different from each other (Table 29 to Table 33, and Fig. 24 to Fig. 28). Accordingly, it was considered possible to set optimum conditions for tRNA concentrations (conditions that may result in more efficient translation of the peptide of interest compared to cross-reading).

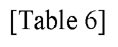

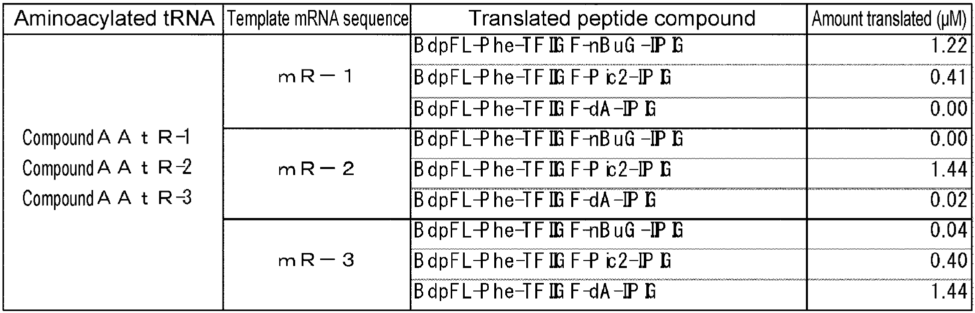

This is a table showing the results of evaluating translation in terms of discrimination of three amino acids in a single codon box (evaluated codons: CUU, CUA, and CUG).

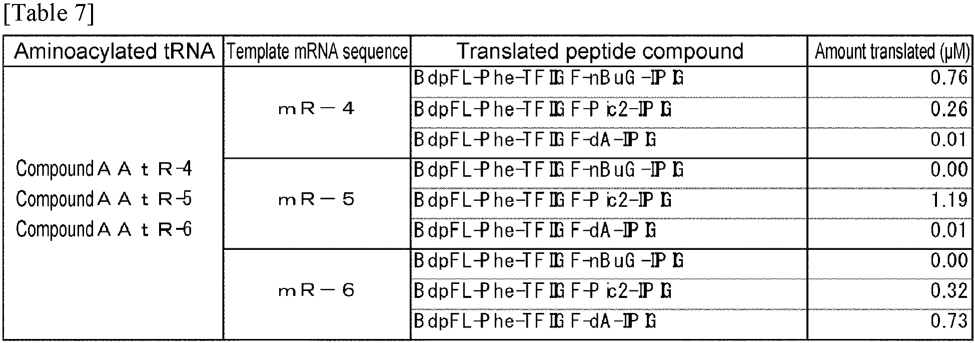

This is a table showing the results of evaluating translation in terms of discrimination of three amino acids in a single codon box (evaluated codons: GUU, GUA, and GUG).

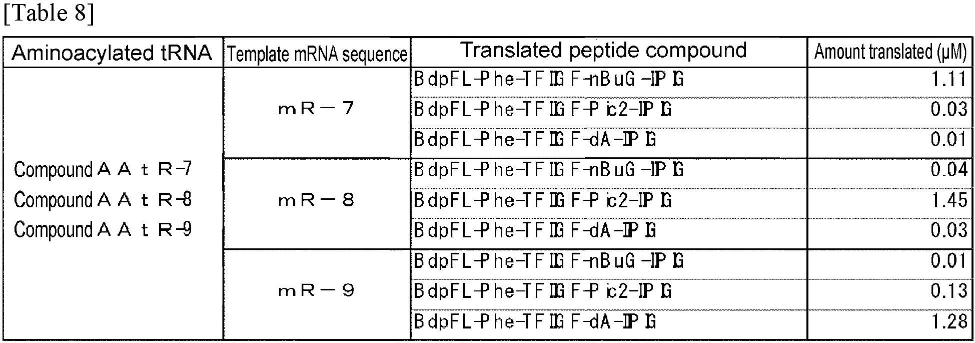

This is a table showing the results of evaluating translation in terms of discrimination of three amino acids in a single codon box (evaluated codons: CAU, CAA, and CAG).

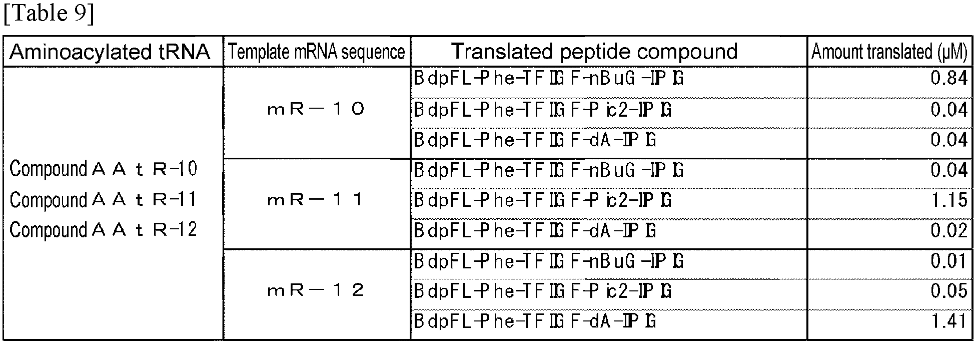

This is a table showing the results of evaluating translation in terms of discrimination of three amino acids in a single codon box (evaluated codons: AAU, AAA, and AAG).

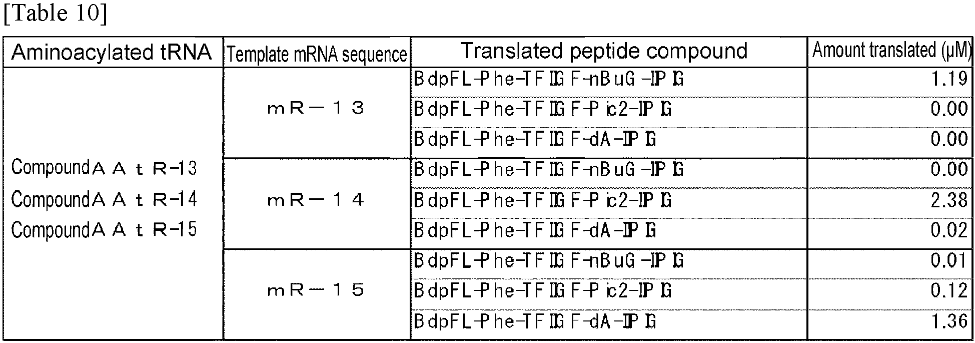

This is a table showing the results of evaluating translation in terms of discrimination of three amino acids in a single codon box (evaluated codons: GAU, GAA, and GAG).

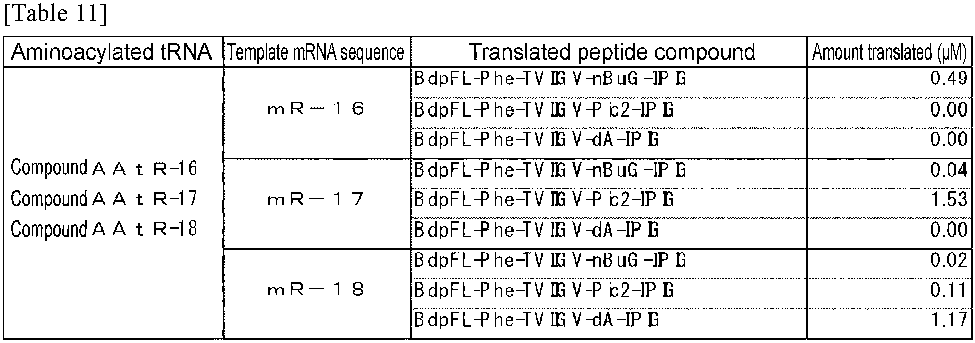

This is a table showing the results of evaluating translation in terms of discrimination of three amino acids in a single codon box (evaluated codons: UUU, UUA, and UUG).

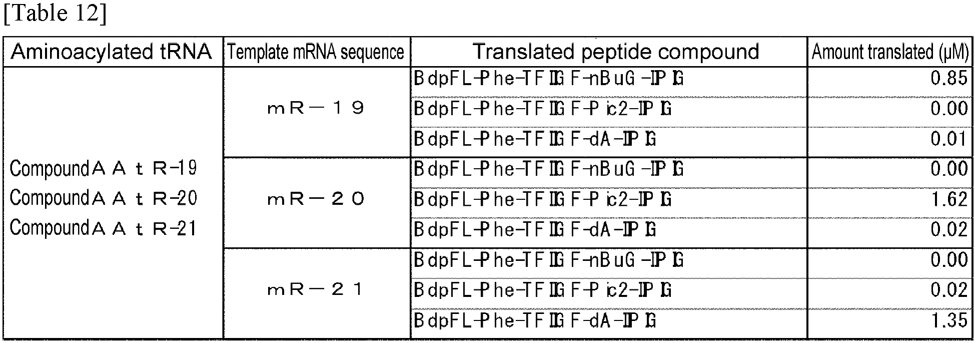

This is a table showing the results of evaluating translation in terms of discrimination of three amino acids in a single codon box (evaluated codons: AGU, AGA, and AGG).

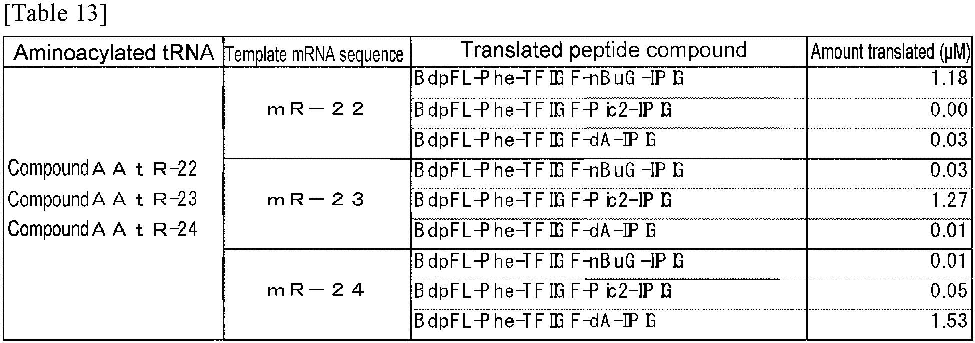

This is a table showing the results of evaluating translation in terms of discrimination of three amino acids in a single codon box (evaluated codons: UGC, UGA, and UGG).

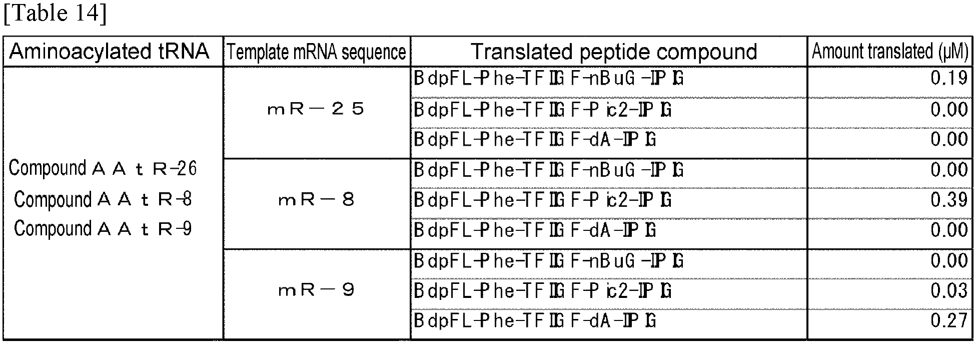

This is a table showing the results of evaluating translation in terms of discrimination of three amino acids in a single codon box (evaluated codons: CAC, CAA, and CAG).

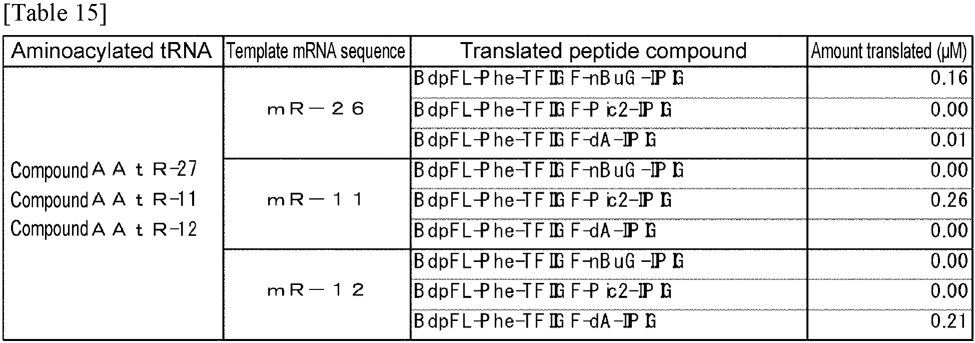

This is a table showing the results of evaluating translation in terms of discrimination of three amino acids in a single codon box (evaluated codons: AAC, AAA, and AAG).

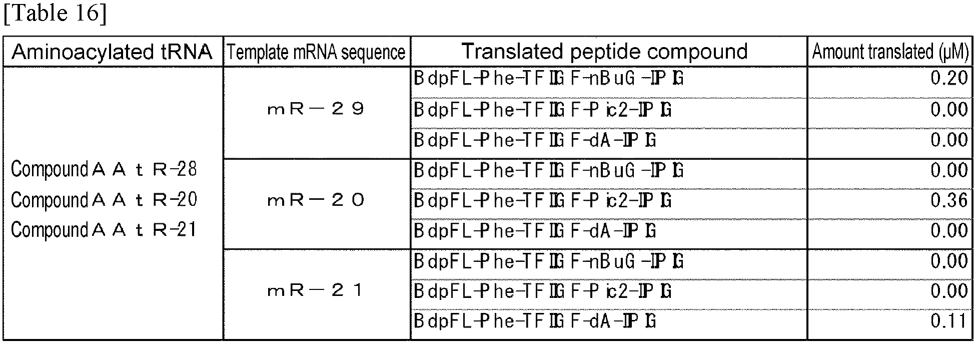

This is a table showing the results of evaluating translation in terms of discrimination of three amino acids in a single codon box (evaluated codons: AGC, AGA, and AGG).

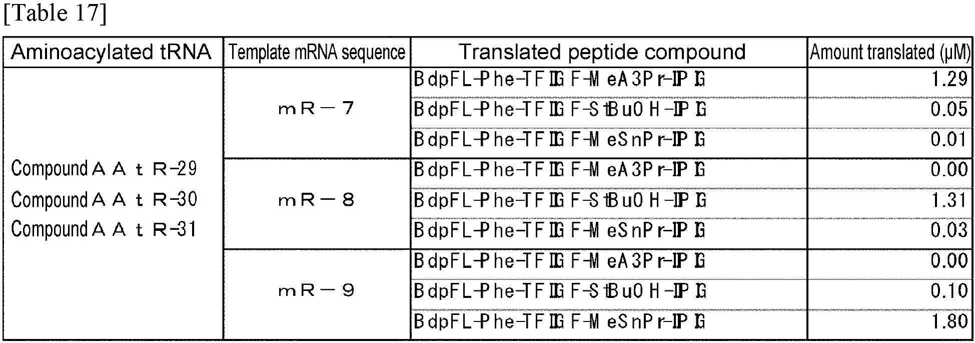

This is a table showing the results of evaluating translation in terms of discrimination of three amino acids in a single codon box (evaluated codons: CAU, CAA, and CAG).

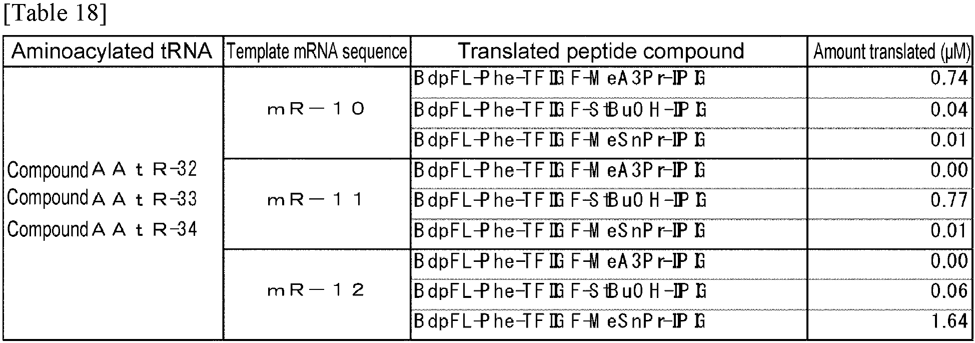

This is a table showing the results of evaluating translation in terms of discrimination of three amino acids in a single codon box (evaluated codons: AAU, AAA, and AAG).

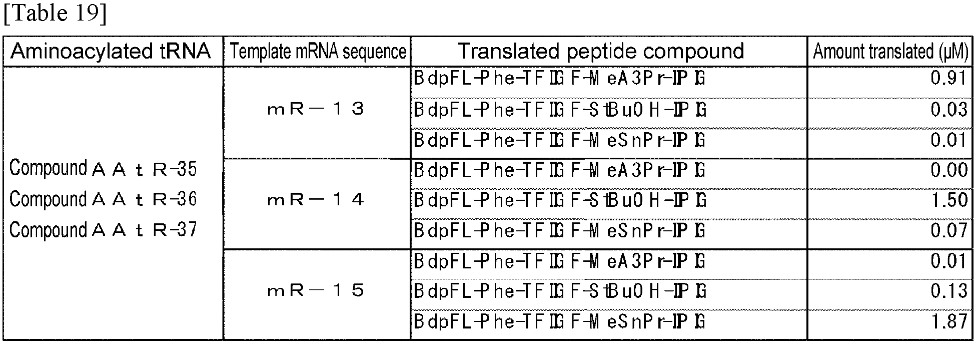

This is a table showing the results of evaluating translation in terms of discrimination of three amino acids in a single codon box (evaluated codons: GAU, GAA, and GAG).

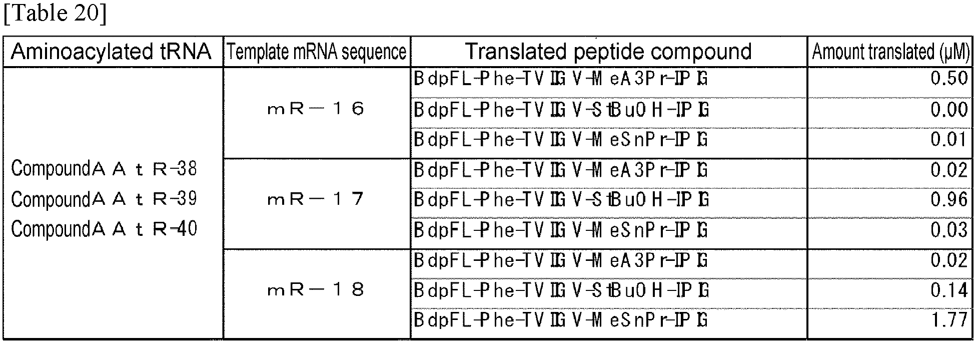

This is a table showing the results of evaluating translation in terms of discrimination of three amino acids in a single codon box (evaluated codons: UUU, UUA, and UUG).

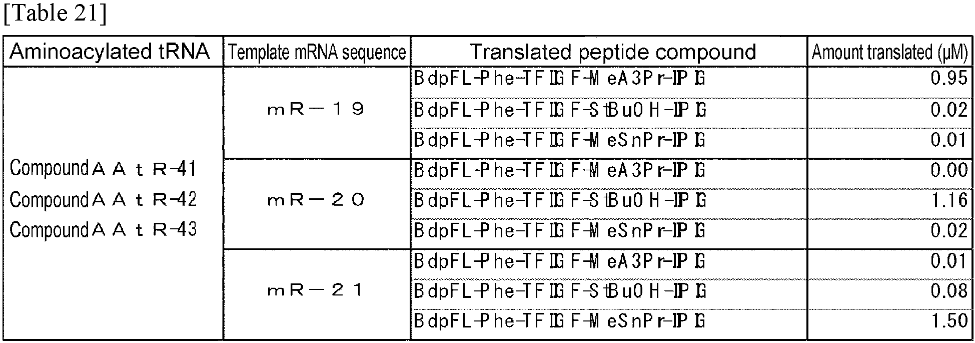

This is a table showing the results of evaluating translation in terms of discrimination of three amino acids in a single codon box (evaluated codons: AGU, AGA, and AGG).

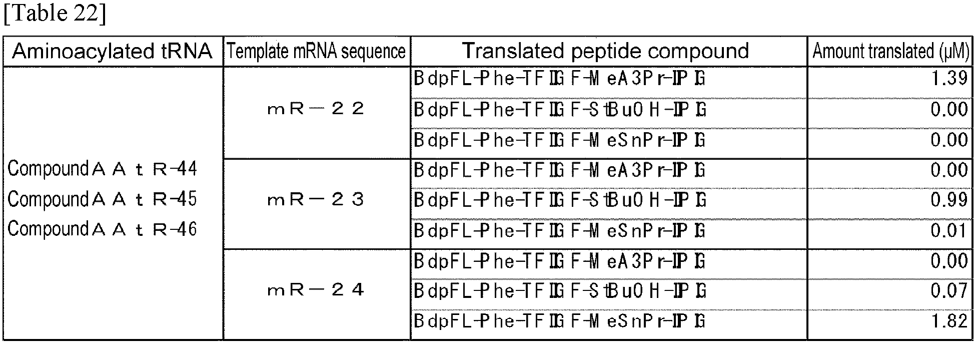

This is a table showing the results of evaluating translation in terms of discrimination of three amino acids in a single codon box (evaluated codons: UGC, UGA, and UGG).

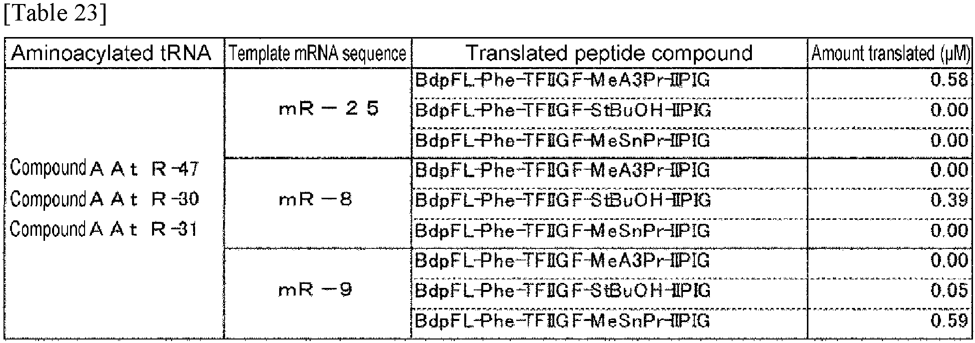

This is a table showing the results of evaluating translation in terms of discrimination of three amino acids in a single codon box (evaluated codons: CAC, CAA, and CAG).

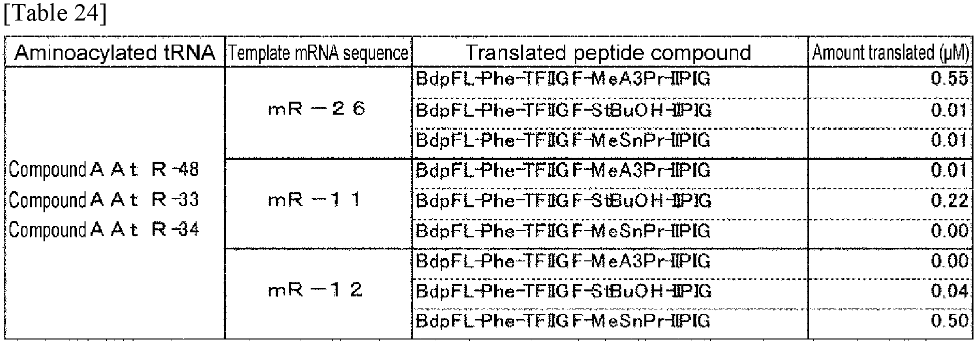

This is a table showing the results of evaluating translation in terms of discrimination of three amino acids in a single codon box (evaluated codons: AAC, AAA, and AAG).

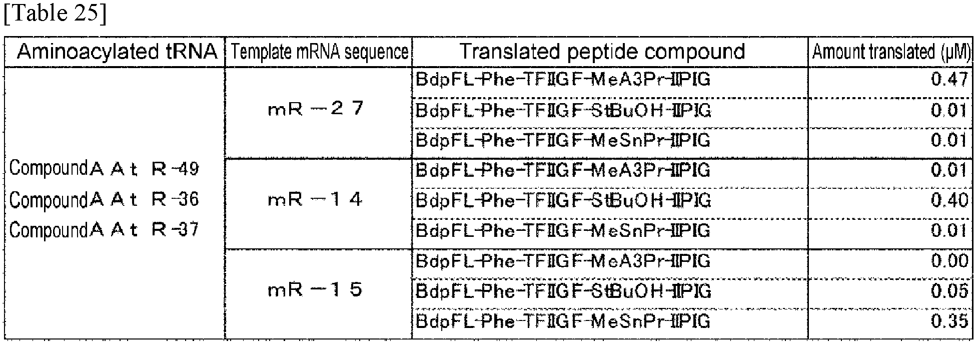

This is a table showing the results of evaluating translation in terms of discrimination of three amino acids in a single codon box (evaluated codons: GAC, GAA, and GAG).

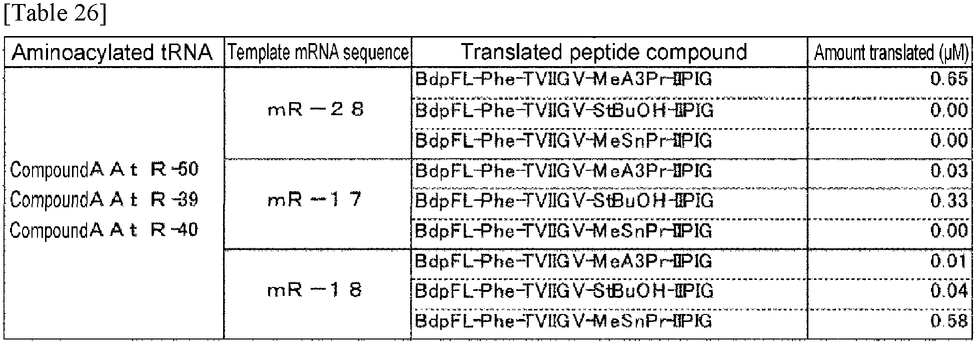

This is a table showing the results of evaluating translation in terms of discrimination of three amino acids in a single codon box (evaluated codons: UUC, UUA, and UUG).

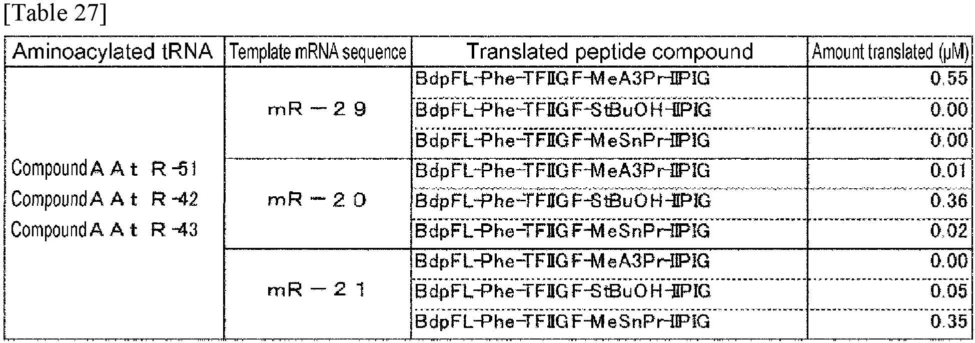

This is a table showing the results of evaluating translation in terms of discrimination of three amino acids in a single codon box (evaluated codons: AGC, AGA, and AGG).

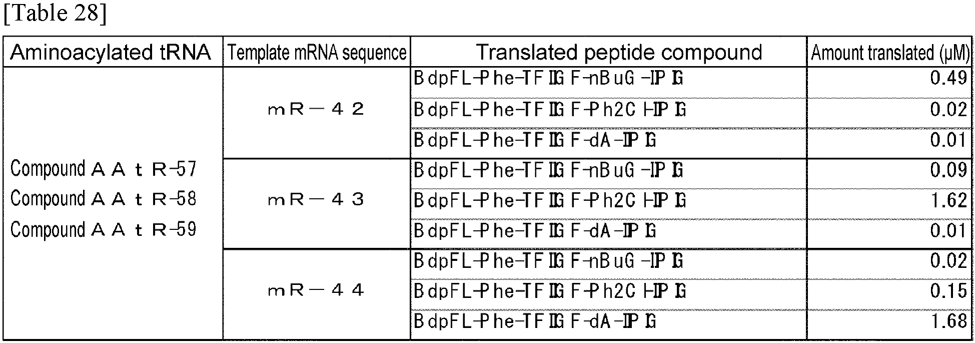

This is a table showing the results of evaluating translation in terms of discrimination of three amino acids in a single codon box (evaluated codons: CGU, CGA, and CGG).

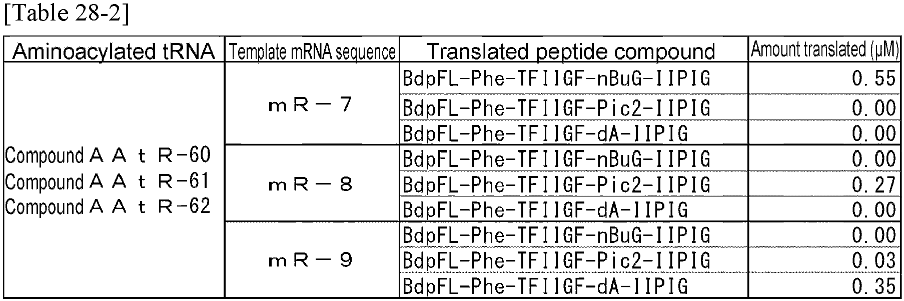

This is a table showing the results of evaluating translation in terms of discrimination of three amino acids in a single codon box (evaluated codons: CAU, CAA, and CAG).

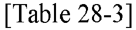

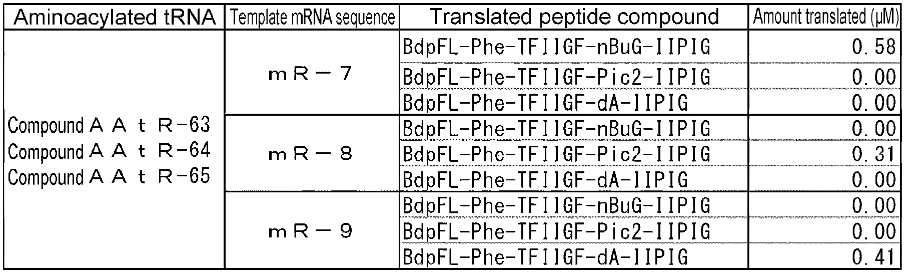

This is a table showing the results of evaluating translation in terms of discrimination of three amino acids in a single codon box (evaluated codons: CAU, CAA, and CAG).

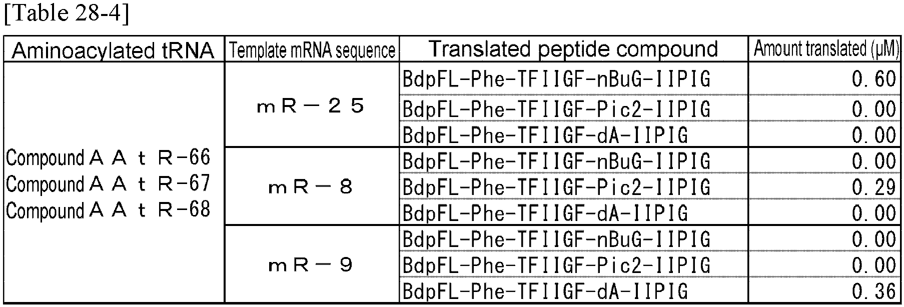

This is a table showing the results of evaluating translation in terms of discrimination of three amino acids in a single codon box (evaluated codons: CAC, CAA, and CAG).

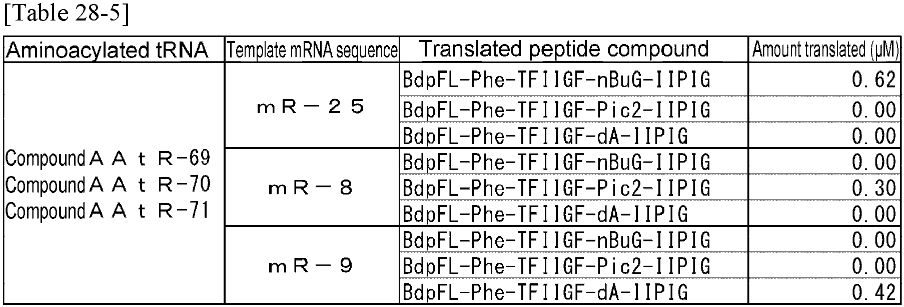

This is a table showing the results of evaluating translation in terms of discrimination of three amino acids in a single codon box (evaluated codons: CAC, CAA, and CAG).

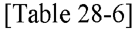

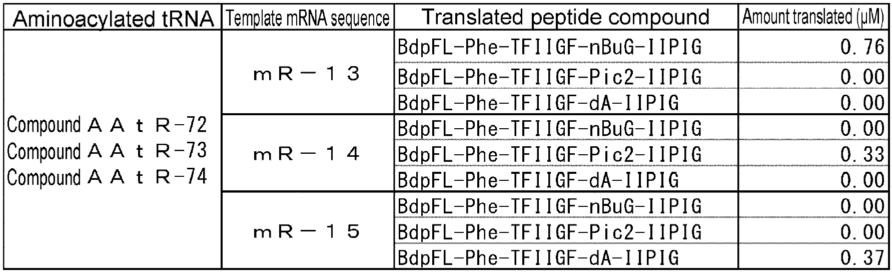

This is a table showing the results of evaluating translation in terms of discrimination of three amino acids in a single codon box (evaluated codons: GAU, GAA, and GAG).

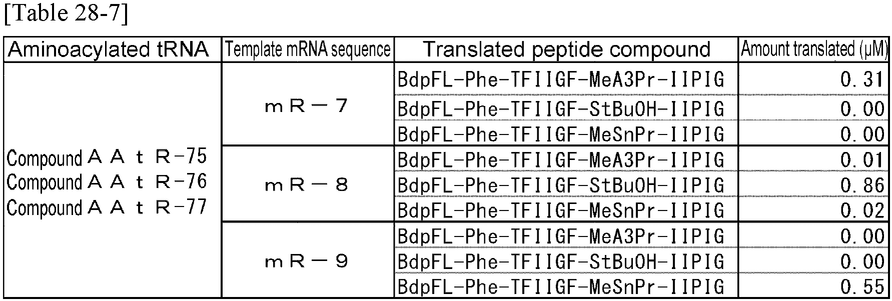

This is a table showing the results of evaluating translation in terms of discrimination of three amino acids in a single codon box (evaluated codons: CAU, CAA, and CAG).

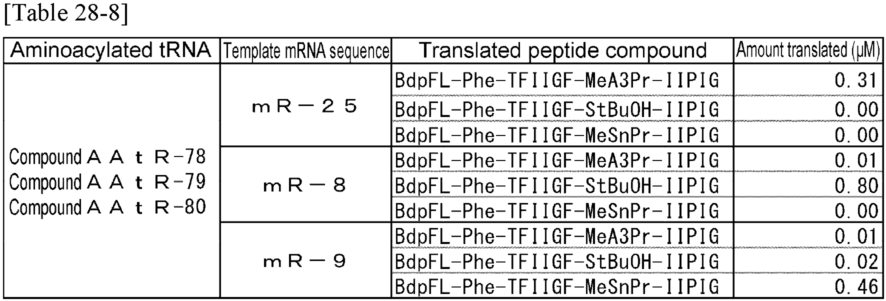

This is a table showing the results of evaluating translation in terms of discrimination of three amino acids in a single codon box (evaluated codons: CAC, CAA, and CAG).

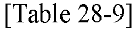

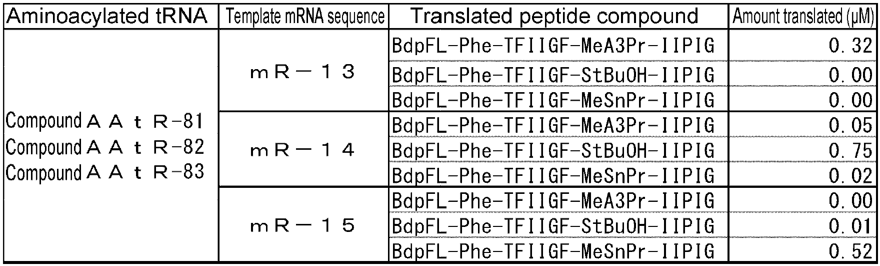

This is a table showing the results of evaluating translation in terms of discrimination of three amino acids in a single codon box (evaluated codons: GAU, GAA, and GAG).

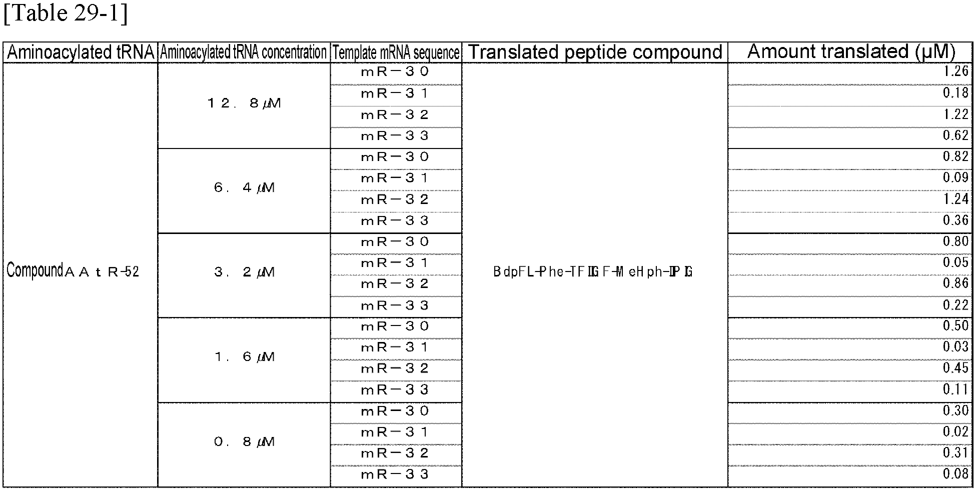

This is a table showing the results of examining, by translation experiments, changes in the amount translated depending on the concentration of aminoacylated tRNA concerning the product of interest and the product of cross-reading in the same codon box, for a single codon (evaluated codons: UCU, UCC, UCA, UCG).

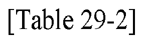

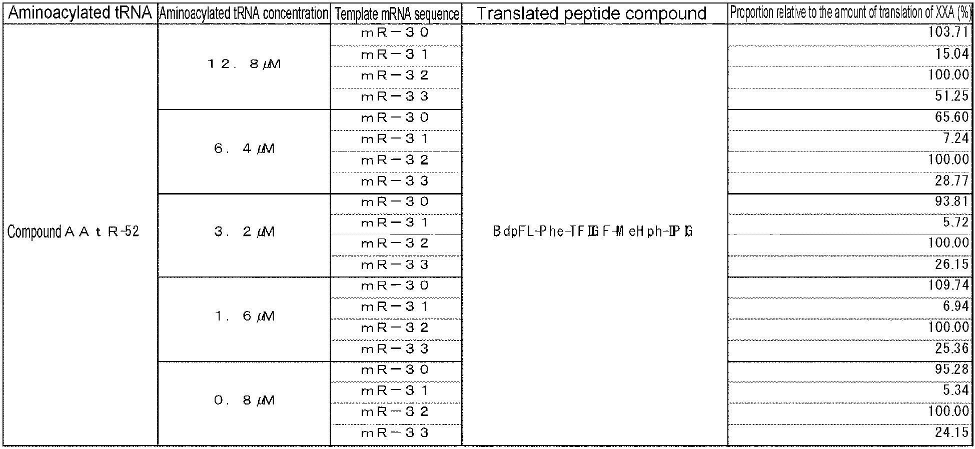

This is a table showing the results of examining, by translation experiments, changes in the amount translated depending on the concentration of aminoacylated tRNA concerning the product of interest and the product of cross-reading in the same codon box, for a single codon, and then calculating proportions relative to the amount of the product of interest (evaluated codons: UCU, UCC, UCA, and UCG).

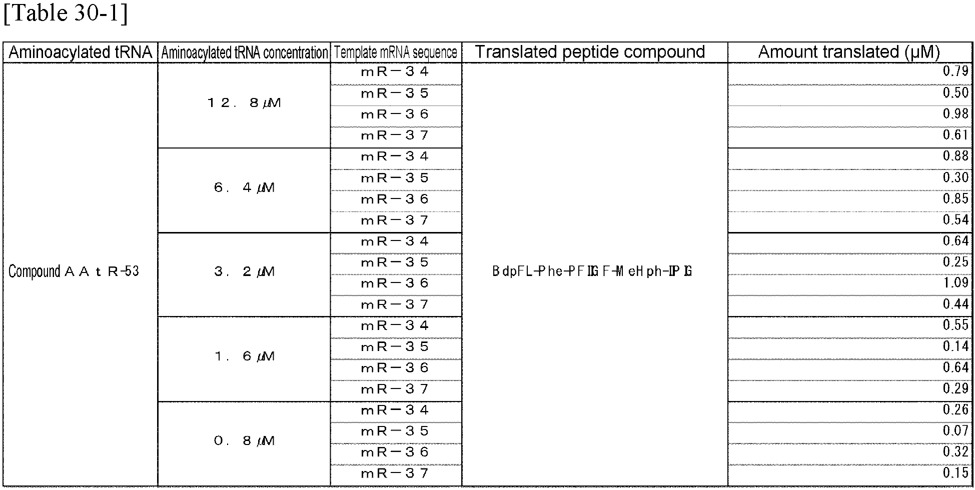

This is a table showing the results of examining, by translation experiments, changes in the amount translated depending on the concentration of aminoacylated tRNA concerning the product of interest and the product of cross-reading in the same codon box, for a single codon (evaluated codons: ACU, ACC, ACA, and ACG).

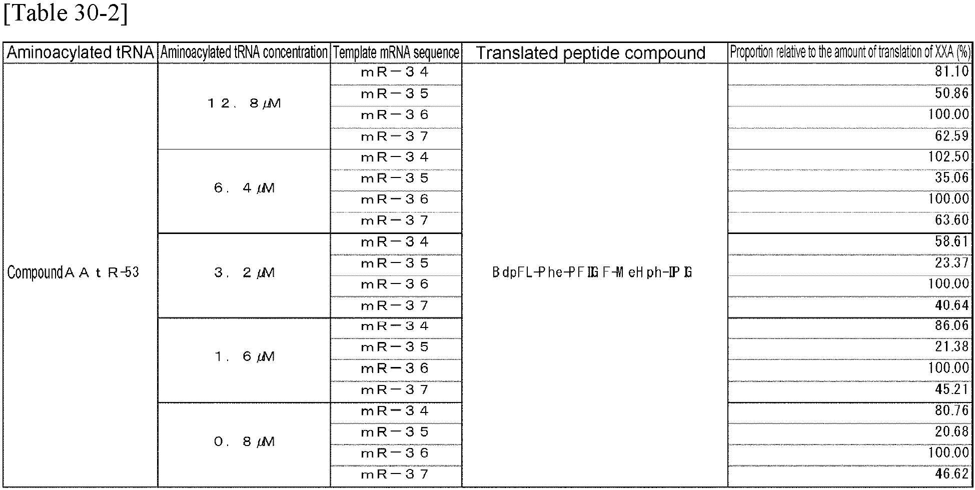

This is a table showing the results of examining, by translation experiments, changes in the amount translated depending on the concentration of aminoacylated tRNA concerning the product of interest and the product of cross-reading in the same codon box, for a single codon, and then calculating proportions relative to the amount of the product of interest (evaluated codons: ACU, ACC, ACA, and ACG).

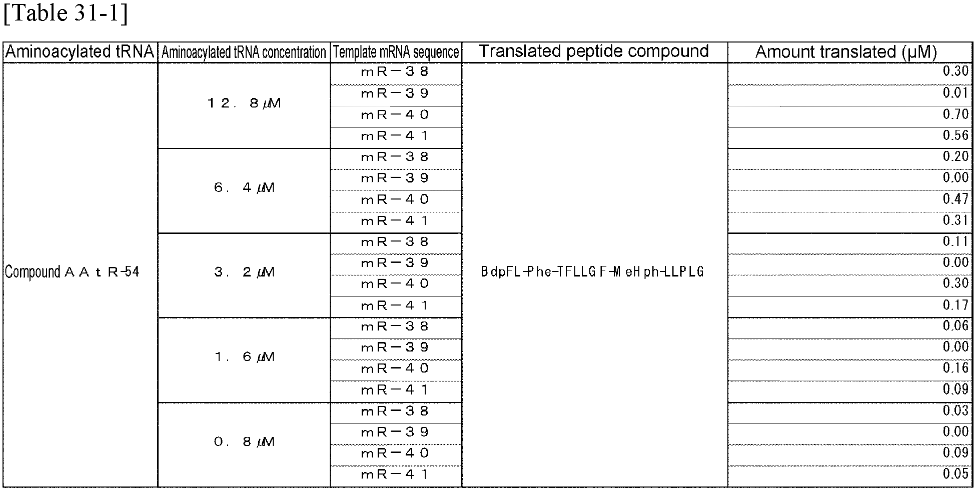

This is a table showing the results of examining, by translation experiments, changes in the amount translated depending on the concentration of aminoacylated tRNA concerning the product of interest and the product of cross-reading in the same codon box, for a single codon (evaluated codons: AUU, AUC, AUA, and AUG).

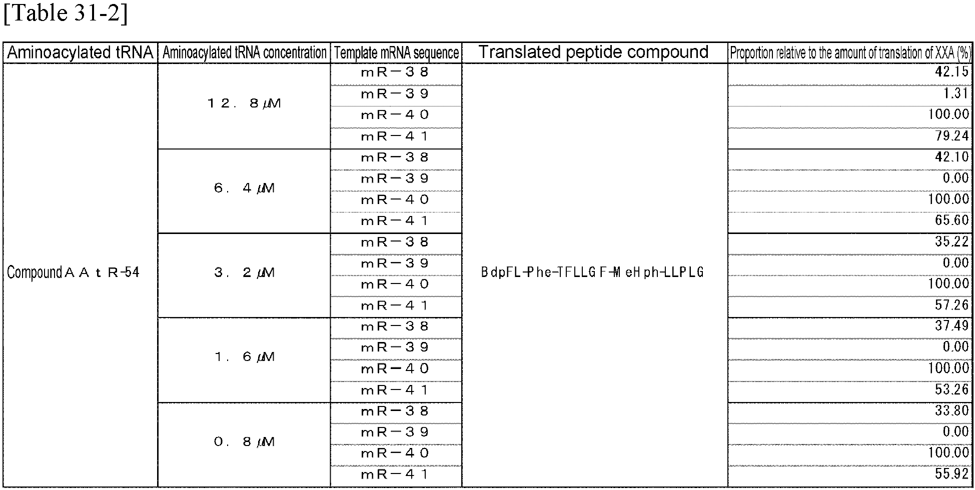

This is a table showing the results of examining, by translation experiments, changes in the amount translated depending on the concentration of aminoacylated tRNA concerning the product of interest and the product of cross-reading in the same codon box, for a single codon, and then calculating proportions relative to the amount of the product of interest (evaluated codons: AUU, AUC, AUA, and AUG).

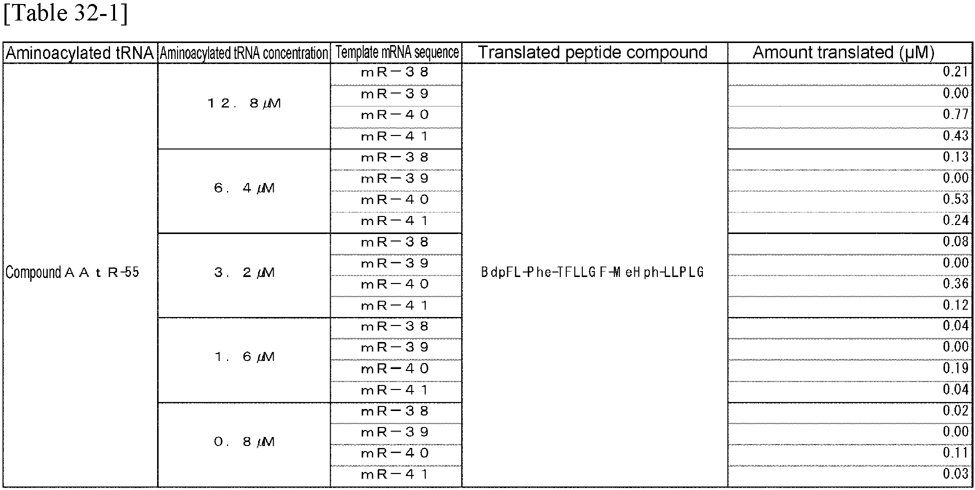

This is a table showing the results of examining, by translation experiments, changes in the amount translated depending on the concentration of aminoacylated tRNA concerning the product of interest and the product of cross-reading in the same codon box, for a single codon (evaluated codons: AUU, AUC, AUA, and AUG).

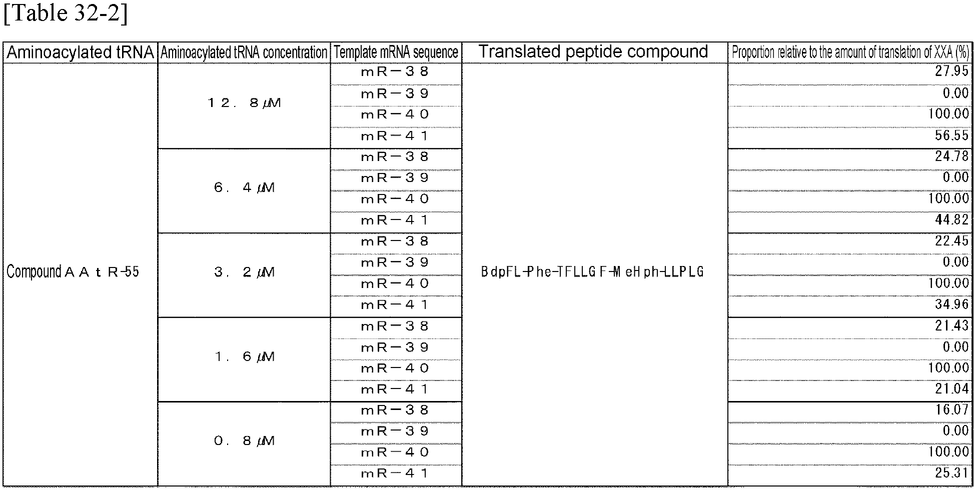

This is a table showing the results of examining, by translation experiments, changes in the amount translated depending on the concentration of aminoacylated tRNA concerning the product of interest and the product of cross-reading in the same codon box, for a single codon, and then calculating proportions relative to the amount of the product of interest (evaluated codons: AUU, AUC, AUA, and AUG).

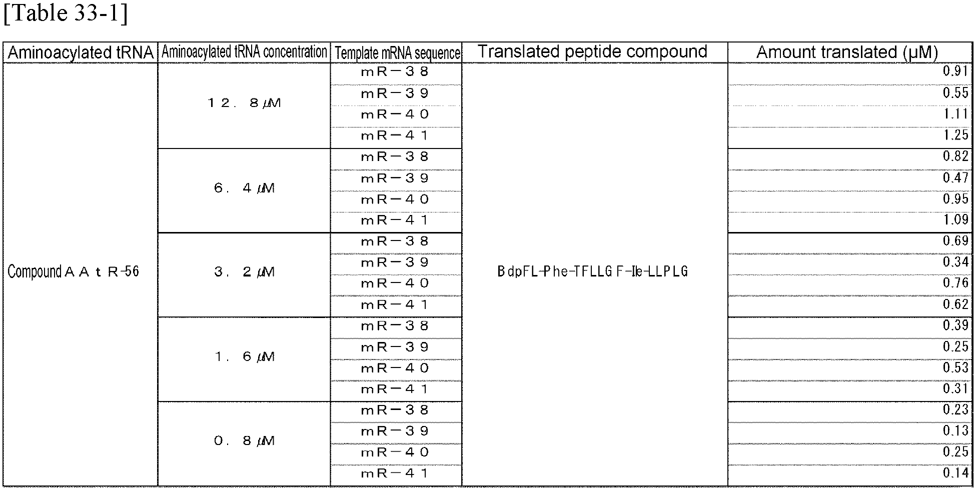

This is a table showing the results of examining, by translation experiments, changes in the amount translated depending on the concentration of aminoacylated tRNA concerning the product of interest and the product of cross-reading in the same codon box, for a single codon (evaluated codons: AUU, AUC, AUA, and AUG).

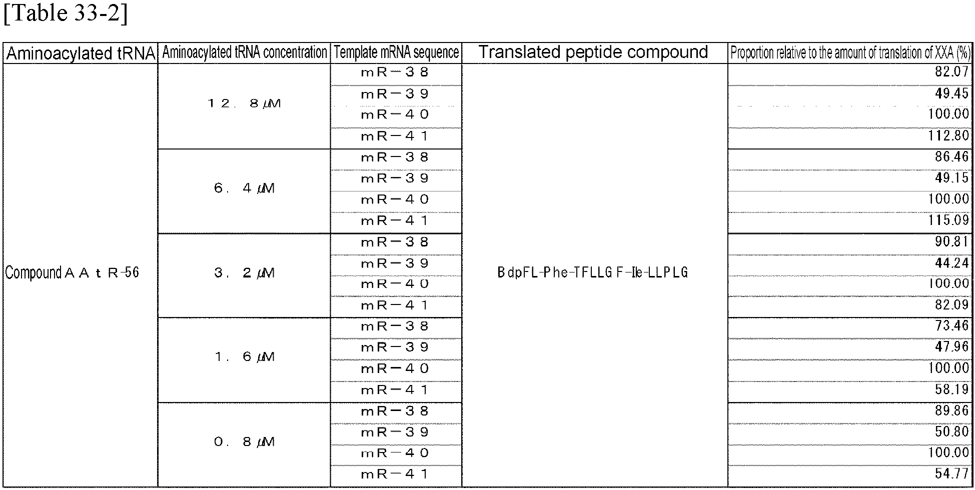

This is a table showing the results of examining, by translation experiments, changes in the amount translated depending on the concentration of aminoacylated tRNA concerning the product of interest and the product of cross-reading in the same codon box, for a single codon, and then calculating proportions relative to the amount of the product of interest (evaluated codons: AUU, AUC, AUA, and AUG).

Although the above invention has been described in detail using examples and illustrations for the purpose of facilitating clear understanding, the descriptions and illustrations herein should not be construed as limiting the scope of the present invention. The disclosures of all patent literature and scientific literature cited herein are hereby expressly incorporated by reference in their entirety.

### [Industrial Applicability]

In one embodiment, the tRNAs of the present disclosure are useful in that they can discriminate the NNA codon and the NNG codon, discriminate the NNU codon, the NNA codon, and the NNG codon, and discriminate the NNC codon, the NNA codon, and the NNG codon, which are not discriminated in the natural genetic code table. In one embodiment, the translation systems of the present disclosure are useful in that they can translate more kinds of amino acids (codon expansion) than translation systems that use the natural genetic code table.

## Claims

1. A translation system, comprising a tRNA having an anticodon complementary to a codon
represented by M₁M₂A and a tRNA having an anticodon complementary to a codon represented by M₁M₂G;
wherein M₁ and M₂ represent nucleosides for the first and second letters of the codon respectively, and each of M₁ and M₂ is independently selected from any of adenosine (A), guanosine (G), cytidine (C), and uridine (U); and
wherein each of the two tRNAs is attached to an amino acid or an amino acid analog that is different from each other.

2. The translation system of claim 1, wherein at least two amino acids or amino acid analogs can be translated from a codon box constituted by M₁M₂U, M₁M₂C, M₁M₂A, and M₁M₂G.

3. A translation system, comprising
(i) a tRNA having an anticodon complementary to a codon represented by M₁M₂U, a tRNA having an anticodon complementary to a codon represented by M₁M₂A, and a tRNA having an anticodon complementary to a codon represented by M₁M₂G, or
(ii) a tRNA having an anticodon complementary to a codon represented by M₁M₂C, a tRNA having an anticodon complementary to a codon represented by M₁M₂A, and an anticodon complementary to a codon represented by M₁M₂G;
wherein M₁ and M₂ represent nucleosides for the first and second letters of the codon respectively, and each of M₁ and M₂ is independently selected from any of adenosine (A), guanosine (G), cytidine (C), and uridine (U); and
wherein each of the three tRNAs in the above-mentioned (i) and (ii) is attached to an amino acid or an amino acid analog that is different from each other.

4. The translation system of claim 1 or 3, wherein at least three amino acids or amino acid analogs can be translated from a codon box constituted by M₁M₂U, M₁M₂C, M₁M₂A, and M₁M₂G.

5. The translation system of any one of claims 1 to 4, wherein M₁ and M₂ are any one of the following:
(i) Mi is uridine (U) and M₂ is uridine (U);
(ii) Mi is uridine (U) and M₂ is adenosine (A);
(iii) Mi is uridine (U) and M₂ is guanosine (G);
(iv) Mi is cytidine (C) and M₂ is adenosine (A);
(v) Mi is cytidine (C) and M₂ is guanosine (G);
(vi) Mi is adenosine (A) and M₂ is uridine (U);
(vii) Mi is adenosine (A) and M₂ is cytidine (C);
(viii) Mi is adenosine (A) and M₂ is adenosine (A);
(ix) Mi is adenosine (A) and M₂ is guanosine (G); or
(x) Mi is guanosine (G) and M₂ is adenosine (A).

6. The translation system of claim 5, wherein M₁ and M₂ are any one of the following:
(i) Mi is uridine (U) and M₂ is uridine (U);
(ii) Mi is uridine (U) and M₂ is guanosine (G);
(iii) Mi is cytidine (C) and M₂ is adenosine (A);
(iv) Mi is cytidine (C) and M₂ is guanosine (G);
(v) Mi is adenosine (A) and M₂ is adenosine (A);
(vi) Mi is adenosine (A) and M₂ is guanosine (G); or
(vii) Mi is guanosine (G) and M₂ is adenosine (A).

7. The translation system of any one of claims 1 to 6, wherein the concentration of the tRNA included in the translation system per codon is any one of (i) 0.8 µM to 1000 µM, (ii) 1.6 µM to 500 µM, (iii) 3.2 µM to 250 µM, (iv) 6.4 µM to 150 µM, or (v) 10 µM to 100 µM.

8. The translation system of any one of claims 1 to 7, wherein the amino acid is a natural amino acid or an unnatural amino acid.

9. The translation system of any one of claims 1 to 8, wherein the tRNA is an initiator tRNA or an elongator tRNA.

10. The translation system of any one of claims 1 to 9, wherein the tRNA is derived from a prokaryote or a eukaryote.

11. The translation system of any one of claims 1 to 10, wherein the anticodon comprises one or more types of nucleosides selected from adenosine (A), guanosine (G), cytidine (C), and uridine (U).

12. The translation system of any one of claims 1 to 11, wherein more than 20 types of amino acids or amino acid analogs can be translated from one genetic code table.

13. The translation system of any one of claims 1 to 12, which is a cell-free translation system.

14. The translation system of claim 13, which is a reconstituted cell-free translation system.

15. The translation system of claim 13 or 14, comprising an *Escherichia* coli-derived ribosome.

16. The translation system of any one of claims 1 to 15, wherein the tRNA has been attached to the amino acid or the amino acid analog outside the translation system.

17. The translation system of any one of claims 1 to 16, wherein among the tRNAs, the tRNA having an anticodon complementary to a codon represented by M₁M₂A and the tRNA having an anticodon complementary to a codon represented by M₁M₂G have been attached to the amino acid or the amino acid analog outside the translation system.

18. The translation system of any one of claims 1 to 17, wherein the tRNA is obtained by the pCpA method, the pdCpA method, a method using an artificial RNA catalyst (flexizyme), or a method using an aminoacyl-tRNA synthetase (ARS).

19. A method for producing the translation system of any one of claims 1 to 18, comprising attaching the amino acid or the amino acid analog to a tRNA outside the translation system.

20. The method of claim 19, wherein attaching the amino acid or the amino acid analog to a tRNA outside the translation system is carried out by the pCpA method, the pdCpA method, a method using an artificial RNA catalyst (flexizyme), or a method using an aminoacyl-tRNA synthetase (ARS).

21. A method for producing a peptide, comprising translating a nucleic acid using the translation system of any one of claims 1 to 18 or a translation system obtained by the method of claim 19 or 20.

22. The method of claim 21, wherein the peptide has a cyclic portion.

23. A peptide produced by the method of claim 21 or 22.

24. A method for producing a peptide library, comprising translating a nucleic acid library using the translation system of any one of claims 1 to 18 or a translation system obtained by the method of claim 19 or 20.

25. A peptide library produced by the method of claim 24.

26. A method for identifying a peptide having binding activity to a target molecule, comprising contacting the target molecule with the peptide library of claim 25.
